# EUROPEAN PATENT APPLICATION

(11) **EP 4 257 599 A2**
(43) Date of publication of application: **11.10.2023**
(21) Application number: 23169890.3
(22) Date of filing: 12.01.2017
(51) Int. Cl.: C07K 16/00

(54) **RODENTS HAVING AN ENGINEERED HEAVY CHAIN DIVERSITY REGION**

(30) Priority: 13.01.2016 US 201662278127 P
(62) Divisional of application: 17704340.3
(71) Applicant: Regeneron Pharmaceuticals, Inc., Tarrytown, NY 10591-6707 (US)
(72) Inventor: MACDONALD, Lynn, Tarrytown, NY 10591 (US); MCWHIRTER, John, Tarrytown, NY 10591 (US); MURPHY, Andrew, J., Tarrytown, NY 10591 (US)
(74) Representative: J A Kemp LLP

(57) **Abstract**

Non-human animals and methods and compositions for making and using them are provided, wherein said non-human animals have a genome comprising an engineered or recombinant diversity cluster within an immunoglobulin heavy chain variable region, which engineered or recombinant diversity cluster comprises an insertion of one or more coding sequences of a non-immunoglobulin polypeptide of interest. Non-human animals described herein express antibodies characterized by complementary determining regions (CDRs), in particular, CDR3s having diversity that directs binding to particular antigens. Methods for producing antibodies from non-human animals are also provided, which antibodies contain human variable regions and mouse constant regions.

## Description

### BACKGROUND

Antibody-based therapeutics offer significant promise in the treatment of several diseases. A variety of formats, including monoclonal, murine, chimeric, humanized, human, full-length, Fab, pegylated, radiolabeled, drug-conjugated, multi-specific, etc. are being developed (see e.g., Reichert, J.M., 2012, mAbs 4:3, 413-415; Nixon, A.E. et al., 2014, mAbs 6:1, 73-85; incorporated herein by reference). Of the more than 40 therapeutic antibody agents that have received marketing approval in the United States or Europe, all have been generated with technologies that rely on assembly of traditional antibody genes from human and/or non-human (e.g., mouse) sources by *in vitro* (e.g., phage display) or *in vivo* (e.g., genetically engineered animals) systems. Still, development of particularly effective antibody agents that bind intractable targets remains a challenge.

### SUMMARY

Disclosed herein is the recognition that it is desirable to engineer non-human animals to permit improved *in vivo* systems for identifying and developing new antibody-based therapeutics and, in some embodiments, antibody agents (e.g., monoclonal antibodies and/or fragments thereof), which can be used for the treatment of a variety of diseases characterized by intractable disease targets. Further, disclosed herein is the recognition that non-human animals having an engineered heavy chain diversity (D_{H}) cluster/region within an immunoglobulin heavy chain variable region (e.g., a heterologous immunoglobulin heavy chain variable region), in particular, an engineered D_{H} cluster (or D_{H} region) containing nucleotide coding sequences not naturally present within an immunoglobulin heavy chain variable region, and/or otherwise expressing, containing, or producing antibodies containing complementary determining regions (CDRs) that are characterized by diversity that directs binding to particular antigens are desirable, for example, for use in identifying and developing antibody-based therapeutics, which may target e.g., membrane-spanning or cytoplasmic polypeptides. In some embodiments, non-human animals disclosed herein are *in vivo* systems for development of antibodies and/or antibody-based therapeutics for administration to humans.

In some embodiments, a non-human animal is provided, whose genome, e.g., germline genome, comprises an immunoglobulin heavy chain variable region that includes an engineered D_{H} region, wherein the engineered D_{H} region includes one or more nucleotide sequences that each encode a non-immunoglobulin polypeptide of interest, or portion thereof.

In another aspect, non-human animals whose genome, e.g., germline genome, are modified to comprise an immunoglobulin heavy chain variable region that includes an engineered D_{H} region, wherein the engineered D_{H} region includes one or more nucleotide sequences that each encode a non-immunoglobulin polypeptide of interest, or portion thereof, may be further modified to express a single rearranged light chain, e.g., a common light chain (ULC).

A single rearranged light chain variable gene sequence operably linked to a light chain constant region, also referred to as common or universal light chain (ULC), may be encoded by a light chain locus comprising a single rearranged V_{L}:J_{L} gene sequence. In some embodiments, the light chain locus comprises a single rearranged V_{L}:J_{L} gene sequence in which the V_{L} sequence is a Vκ gene sequence. In some aspects, the Vκ sequence is selected from Vκ1-39 or Vκ3-20. In some aspects, the J_{L} sequence is a Jκ gene sequence, e.g., a Jκ1 sequence, a Jκ2 sequence, a Jκ3 sequence, a Jκ4 sequence, or a Jκ5 sequence, etc. In some embodiments, the light chain locus comprises a single rearranged Vκ:Jκ sequence selected from the group consisting of Vκ1-39Jκ5 and Vκ3-20Jκ1. In one embodiment, the light chain locus comprises a single rearranged Vκ:Jκ sequence of Vκ1-39Jκ5. In another embodiment, the light chain locus comprises a single rearranged Vκ:Jκ sequence of Vκ3-20Jκ1. In some embodiments, the single rearranged variable gene sequence is operably linked to a non-human light chain constant region gene, e.g., endogenous non-human light constant region gene. In another embodiment, the single rearranged variable gene sequence is operably linked to a human light chain constant region gene. In some aspects, the single rearranged variable gene sequence is a human V:J sequence inserted to the endogenous immunoglobulin light chain locus such that the resulting non-human animal does not comprise functional unrearranged V and/or J gene segments in one or more light chain loci.

In some embodiments, an isolated non-human cell or tissue is provided, whose genome comprises an immunoglobulin heavy chain variable region that includes an engineered D_{H} region, wherein the engineered D_{H} region includes one or more nucleotide sequences that each encode a non-immunoglobulin polypeptide of interest, or portion thereof, and optionally, a common or universal light chain. In some embodiments, a cell is from a lymphoid or myeloid lineage. In some embodiments, a cell is a lymphocyte. In some embodiments, a cell is selected from a B cell, dendritic cell, macrophage, monocyte, and a T cell. In some embodiments, a tissue is selected from adipose, bladder, brain, breast, bone marrow, eye, heart, intestine, kidney, liver, lung, lymph node, muscle, pancreas, plasma, serum, skin, spleen, stomach, thymus, testis, ovum, and a combination thereof.

In some embodiments, an immortalized cell made from an isolated non-human cell as described herein is provided.

In some embodiments, a non-human embryonic stem (ES) cell is provided, whose genome comprises an immunoglobulin heavy chain variable region that includes an engineered D_{H} region, wherein the engineered D_{H} region includes one or more nucleotide sequences that each encode a non-immunoglobulin polypeptide of interest, or portion thereof, and optionally, a common light chain. In some embodiments, a non-human embryonic stem cell is a rodent embryonic stem cell. In some certain embodiments, a rodent embryonic stem cell is a mouse embryonic stem cell and is from a 129 strain, C57BL strain, or a mixture thereof. In some certain embodiments, a rodent embryonic stem cell is a mouse embryonic stem cell and is a mixture of 129 and C57BL strains.

In some embodiments, a non-human germ cell is provided, whose genome comprises an immunoglobulin heavy chain variable region that includes an engineered D_{H} region, wherein the engineered D_{H} region includes one or more nucleotide sequences that each encode a non-immunoglobulin polypeptide of interest, or portion thereof, and optionally, a common light chain. In some embodiments, a non-human germ cell is a rodent germ cell. In some certain embodiments, a rodent germ cell is a mouse germ cell and is from a 129 strain, C57BL strain, or a mixture thereof. In some certain embodiments, a rodent germ cell is a mouse germ cell and is a mixture of 129 and C57BL strains.

In some embodiments, use of a non-human embryonic stem cell or germ cell as described herein to make a non-human animal is provided. In some certain embodiments, a non-human embryonic stem cell or germ cell is a mouse embryonic stem cell or germ cell and is used to make a mouse comprising an immunoglobulin heavy chain variable region that includes an engineered D_{H} region, and optionally a common light chain, as described herein. In some certain embodiments, a non-human embryonic stem cell or germ cell is a rat embryonic stem cell or germ cell and is used to make a rat comprising an immunoglobulin heavy chain variable region that includes an engineered D_{H} region, and optionally a common light chain as described herein.

In some embodiments, a non-human embryo comprising, made from, obtained from, or generated from a non-human embryonic stem cell as described herein is provided. In some certain embodiments, a non-human embryo is a rodent embryo; in some embodiments, a mouse embryo; in some embodiments, a rat embryo.

In some embodiments, use of a non-human embryo described herein to make a non-human animal is provided. In some certain embodiments, a non-human embryo is a mouse embryo and is used to make a mouse comprising an immunoglobulin heavy chain variable region that includes an engineered D_{H} region, and optionally a common light chain locus, as described herein. In some certain embodiments, a non-human embryo is a rat embryo and is used to make a rat comprising an immunoglobulin heavy chain variable region that includes an engineered D_{H} region and optionally a common light chain locus, as described herein.

In some embodiments, a kit is provided, comprising an isolated non-human cell or tissue as described herein, an immortalized cell as described herein, a non-human embryonic stem cell as described herein, a non-human embryo as described herein, or a non-human animal as described herein.

In some embodiments, a kit as described herein is provided, for use in the manufacture and/or development of a drug (e.g., an antibody or antigen-binding fragment thereof) for therapy or diagnosis.

In some embodiments, a kit as described herein is provided, for use in the manufacture and/or development of a drug (e.g., an antibody or antigen-binding fragment thereof) for the treatment, prevention or amelioration of a disease, disorder or condition.

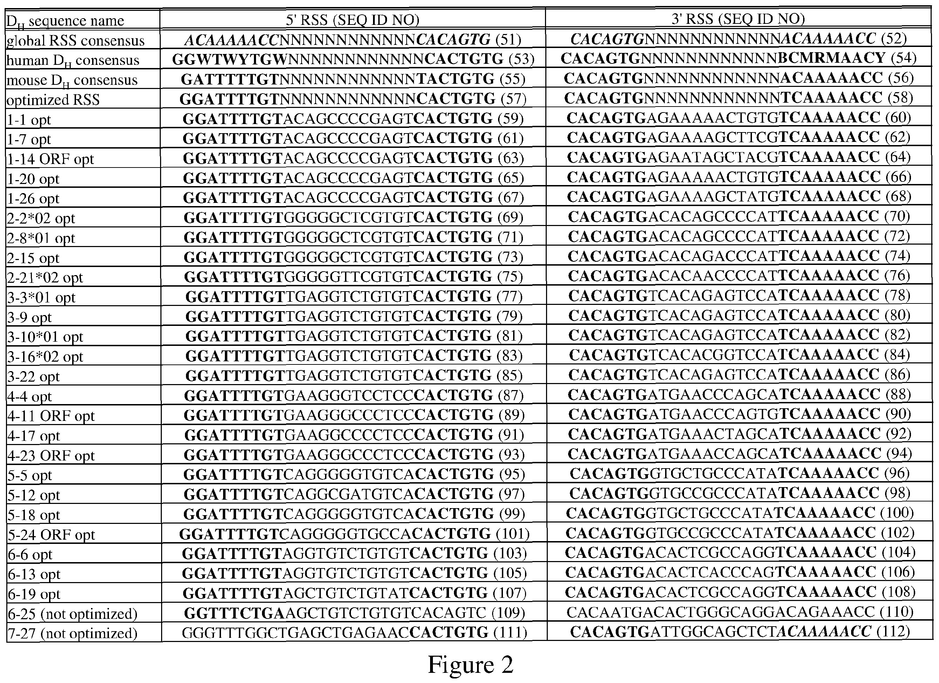

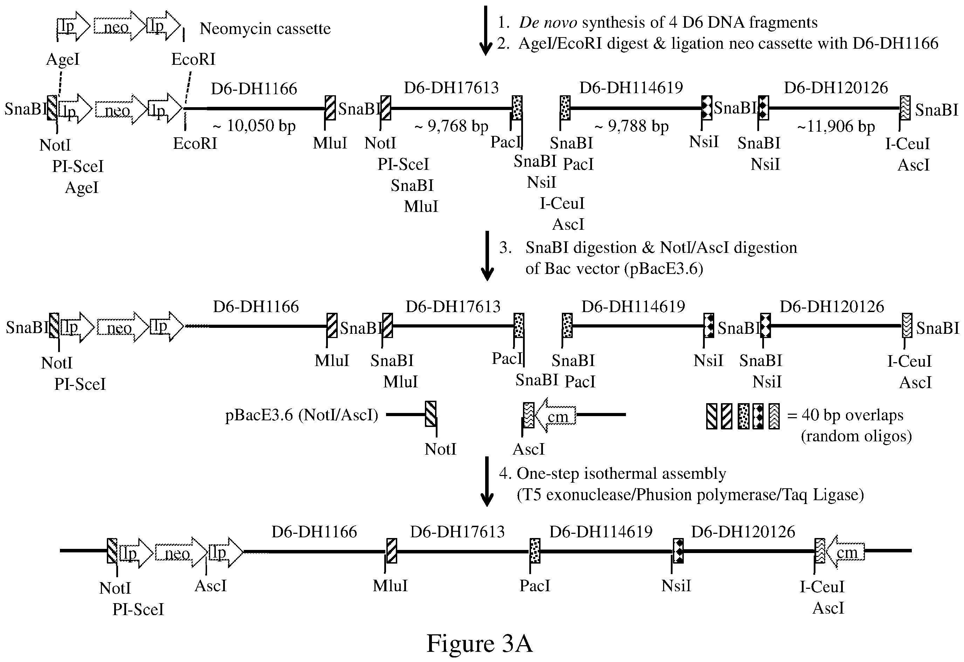

In some embodiments, a transgene, nucleic acid construct, DNA construct, or targeting vector as described herein is provided. In some certain embodiments, a transgene, nucleic acid construct, DNA construct, or targeting vector comprises an engineered D_{H} region as described herein. In some certain embodiments, a transgene, nucleic acid construct, DNA construct, or targeting vector comprises a DNA fragment that includes one or more nucleotide coding sequences described herein. In some certain embodiments, a transgene, nucleic acid construct, DNA construct, or targeting vector comprises an engineered D_{H} region that comprises one or more nucleotide coding sequences selected from Table 3 or Table 4, which one or more nucleotide coding sequences are each flanked by a recombination signal sequence selected from Figure 2. In some certain embodiments, a transgene, nucleic acid construct, DNA construct, or targeting vector further comprises one or more selection markers. In some certain embodiments, a transgene, nucleic acid construct, DNA construct, or targeting vector further comprises one or more site-specific recombination sites (e.g., lox, Frt, or combinations thereof). In some certain embodiments, a transgene, nucleic acid construct, DNA construct, or targeting vector is depicted in any one of Figures 3A, 3B, 4A, 4B, 7A, 7B, 8A and 8B.

In some embodiments, use of a transgene, nucleic acid construct, DNA construct, or targeting vector as described herein to make a non-human embryonic stem cell, non-human cell, non-human embryo and/or non-human animal is provided.

In some embodiments, a non-immunoglobulin polypeptide of interest is a chemokine receptor. In some embodiments, a chemokine receptor is selected from the group consisting of a CC-chemokine receptor (or β-chemokine receptor), CXC-chemokine receptor, CX3C-chemokine receptor and a XC-chemokine receptor. In some embodiments, a chemokine receptor is an atypical chemokine receptor (ACKR). In some embodiments, an ACKR is selected from the group consisting of ACKR1, ACKR2, ACKR3 and ACKR4. In some certain embodiments, an ACKR is ACKR2 or D6 chemokine decoy receptor.

In some embodiments, a non-immunoglobulin polypeptide of interest is a toxin. In some embodiments, a toxin is a toxin that is found in the venom of a tarantula, spider, scorpion or sea anemone.

In some embodiments, a non-immunoglobulin polypeptide of interest is a conotoxin or a tarantula toxin. In some embodiments, a conotoxin is selected from the group consisting of α-conotoxin, δ-conotoxin, κ-conotoxin, µ-conotoxin, ω-conotoxin and combinations thereof. In some certain embodiments, a conotoxin is µ-conotoxin. In some certain embodiments, a tarantula toxin is ProTxI, ProTxII, Huwentoxin-IV (HWTX-IV), or combinations thereof.

In some embodiments, an engineered D_{H} region includes 5, 10, 15, 20, 25 or more nucleotide sequences that each encode an extracellular portion of a D6 chemokine decoy receptor, or that each encode a portion of a conotoxin (e.g., µ-conotoxin), or that each encode a portion of a tarantula toxin (e.g., ProTxI, ProTxII, etc.), or combinations thereof.

In some embodiments, an engineered D_{H} region includes 25 nucleotide sequences that each encode an extracellular portion of a D6 chemokine decoy receptor or includes 26 nucleotide sequences that each encode a portion of a conotoxin (e.g., µ-conotoxin) and/or a tarantula toxin (e.g., ProTxI, ProTxII, etc.).

In some embodiments, an extracellular portion of a D6 chemokine decoy receptor is selected from the group consisting of an N-terminal region, an extracellular loop, and combinations thereof.

In some embodiments, a portion of a conotoxin as described herein includes a sequence that comprises one or more disulfide bonds. In some embodiments, a portion of a conotoxin as described herein includes a sequence that lacks one or more disulfide bonds as compared to a conotoxin sequence that appears in nature (e.g., a reference or parental conotoxin sequence). In some embodiments, a portion of a conotoxin as described herein includes a sequence that exhibits a number and/or pattern of disulfide bonds that is the same or different as compared to a conotoxin sequence that appears in nature (e.g., a reference or parental conotoxin sequence).

In some embodiments, a portion of a tarantula toxin as described herein includes a sequence that comprises a cysteine knot motif that appears in a tarantula toxin sequence found in nature. In some embodiments, a portion of a tarantula toxin as described herein includes a sequence that is or comprises a cysteine knot peptide(s). In some embodiments, a portion of a tarantula toxin as described herein includes a sequence that lacks one or more disulfide bonds as compared to a tarantula toxin sequence that appears in nature (e.g., a reference or parental tarantula toxin sequence). In some embodiments, a portion of a tarantula toxin as described herein includes a sequence exhibits a number and/or pattern of disulfide bonds that is the same or different as compared to a tarantula toxin sequence that appears in nature (e.g., a reference or parental tarantula toxin sequence).

In some embodiments, an engineered D_{H} region includes 25 or 26 nucleotide sequences that is at least 50%, 60%, 70%, 80%, 90%, 95%, or at least 98% identical to a nucleotide sequence that appears in Table 3 or Table 4 and/or encodes an amino acid sequence that appears in Table 3 or Table 4. In some embodiments, an engineered D_{H} region includes 25 or 26 nucleotide sequences that each encode an amino acid sequence that is substantially identical or identical to an amino acid sequence that appears in Table 3 or Table 4, or that has the same function as an amino acid sequence that appears in Table 3 or 4.

In some embodiments, one or more nucleotide sequences comprise one or more nucleotide substitutions that increase somatic hypermutation of the one or more nucleotide sequences.

In some embodiments, an engineered D_{H} region further includes a first and a second recombination signal sequence flanking each of the one or more nucleotide sequences. In some certain embodiments, a first recombination signal sequence comprises a sequence that is at least 50%, 60%, 70%, 80%, 90%, 95%, or at least 98% identical to a first recombination signal sequence that appears in Figure 2. In some certain embodiments, a first recombination signal sequence comprises a sequence that is substantially identical or identical to a first recombination signal sequence that appears in Figure 2. In some certain embodiments, a second recombination signal sequence comprises a sequence that is at least 50%, 60%, 70%, 80%, 90%, 95%, or at least 98% identical to a second recombination signal sequence that appears in Figure 2. In some certain embodiments, a second recombination signal sequence comprises a sequence that is substantially identical or identical to a second recombination signal sequence that appears in Figure 2. In some embodiments, first and second recombination signal sequences are selected from Figure 2.

In some embodiments, the genome of a provided non-human animal lacks one or more wild-type endogenous D_{H} gene segments. In some certain embodiments, the genome of a provided non-human animal lacks all or substantially all wild-type endogenous D_{H} gene segments. In some embodiments, the genome of a provided non-human animal lacks one or more wild-type endogenous recombination signal sequences.

In some embodiments, an engineered D_{H} region comprises one or more wild-type human D_{H} gene segments. In some certain embodiments, an engineered D_{H} region comprises a human D_{H}6-25 gene segment. In some certain embodiments, an engineered D_{H} region lacks a human D_{H}6-25 gene segment.

In some embodiments, an immunoglobulin heavy chain variable region is operably linked to an immunoglobulin heavy chain constant region.

In some embodiments, an immunoglobulin heavy chain constant region is an endogenous immunoglobulin heavy chain constant region.

In some embodiments, an immunoglobulin heavy chain variable region is an unrearranged human immunoglobulin heavy chain variable region, e.g., comprising at least one human (h) unrearranged V_{H} gene segment and/or at least one human (h) unrearranged J_{H} gene segment flanking an engineered (e) D_{H} region. In some embodiments, an immunoglobulin heavy chain variable region comprises a plurality of human (h) unrearranged V_{H} gene segment and/or a plurality of one human (h) unrearranged J_{H} gene segment flanking an engineered (e) D_{H} region. In some embodiments, the unrearranged human immunoglobulin heavy chain variable region is operably linked to an immunoglobulin heavy chain constant region, e.g., a non-human immunoglobulin heavy chain constant region, e.g., at an endogenous non-human heavy chain locus.

In some embodiments, a human immunoglobulin heavy chain variable region comprises a rearranged human immunoglobulin heavy chain variable region, wherein the rearranged human immunoglobulin heavy chain comprises at least one human (h) unrearranged V_{H} gene segment and/or at least one human (h) unrearranged J_{H} gene segment recombined with an engineered (e) D_{H} region to form a rearranged (h)V_{H}/(e)D_{H}/(h)J_{H} gene sequence, that may be operably linked to the heavy chain constant region. In some embodiments, such recombination occurs in a B cell during B cell development.

Accordingly, a non-human animal described herein may comprise
(i) a germ cell comprising an unrearranged human heavy chain variable region comprising
   (a) at least one or a plurality of human (h) unrearranged V_{H} gene segment,
   (b) at least one or a plurality of human (h) unrearranged J_{H} gene segment, and
   (c) an engineered (e) D_{H} region flanked by (a) and (b), wherein (a), (b), and (c) recombine to form a rearranged human heavy chain variable region hV_{H}/eD_{H}/hJ_{H} sequence; and
(ii) a somatic cell, e.g., a B cell, comprising the rearranged human hV_{H}/eD_{H}/hJ_{H} gene sequence, wherein the rearranged hV_{H}/eD_{H}/hJ_{H} gene sequence comprises a CDR3 encoding sequence comprising one or more nucleotide sequences that encode a non-immunoglobulin polypeptide of interest, or portion thereof, or somatically hypermutated variant thereof. In some embodiments, the human unrearranged or rearranged human heavy chain variable region is operably linked to a heavy chain constant region, which may be a non-human heavy chain constant region, e.g., at a non-human endogenous heavy chain locus. In some embodiments, an immunoglobulin heavy chain variable region is a human immunoglobulin heavy chain variable region. In some embodiments, the B cell further expresses the rearranged hV_{H}/eD_{H}/hJ_{H} gene sequence operably linked to a heavy chain constant region as an immunoglobulin heavy chain-like polypeptide comprising a CDR3 comprising a non-immunoglobulin polypeptide of interest, portion thereof, or somatically hypermutated variant thereof.

In some embodiments, a non-human animal as described herein further comprises a humanized light chain locus. In some embodiments, a non-human animal as disclosed herein comprises
(i) in a germ cell:
   (a) an immunoglobulin heavy chain locus comprising an unrearranged immunoglobulin heavy chain variable region and an immunoglobulin heavy chain constant region, wherein the unrearranged immunoglobulin heavy chain variable region comprises at least one unrearranged V_{H} gene segment (which may be a human unrearranged V_{H}gene segment, e.g., an hV_{H}) an engineered D_{H} region (which may include a human D_{H} gene segment and/or engineered D_{H} gene segments, e.g., hD_{H}), and at least one unrearranged, optionally human, J_{H} gene segments, wherein the V_{H} gene segment(s), engineered D_{H} region, and J_{H} gene segment(s) are operably linked such that they can recombine, e.g., in a B cell during B cell development, to form a rearranged immunoglobulin heavy chain hV_{H}/eD_{H}/hJ_{H} variable region gene sequence in operable linkage with an immunoglobulin heavy chain constant region, and
   (b) an immunoglobulin light chain locus comprising human V_{L} and/or J_{L} gene segments, and which may encode a (human) common light chain; and
(ii) in a somatic cell, e.g., a B cell,
   (a) the rearranged immunoglobulin heavy chain hV_{H}/eD_{H}/hJ_{H} variable region gene sequence in operable linkage with an immunoglobulin heavy chain constant region, wherein the engineered D_{H} region comprises a sequence encoding a non-immunoglobulin polypeptide of interest or portion thereof, and wherein rearranged hV_{H}/eD_{H}/hJ_{H} gene sequence comprises a CDR3 encoding sequence comprising one or more nucleotide sequences that encode a non-immunoglobulin polypeptide of interest, or portion thereof, or somatically hypermutated variant thereof, and
   (b) the humanized and/or common immunoglobulin light chain locus or a somatically hypermutated variant thereof. In some embodiments, the human unrearranged or rearranged human heavy chain variable region is operably linked to a heavy chain constant region, which may be a non-human heavy chain constant region, e.g., at a non-human endogenous heavy chain locus. In some embodiments, an immunoglobulin heavy chain variable region is a human immunoglobulin heavy chain variable region. In some embodiments, the B cell further expresses the rearranged hV_{H}/eD_{H}/hJ_{H} gene sequence operably linked to a heavy chain constant region as an immunoglobulin heavy chain-like polypeptide comprising a CDR3 comprising a non-immunoglobulin polypeptide of interest, portion thereof, or somatically hypermutated variant thereof and the human(ized) and/or common light chain as a tetrameric immunoglobulin-like antigen binding protein, wherein the tetramer comprises a dimer of the immunoglobulin heavy chain-like polypeptide, each covalently bound to the human(ized) and/or common light chain.

In some embodiments, a method of making a non-human animal whose genome contains an immunoglobulin heavy chain variable region that includes an engineered D_{H} region is provided, the method comprising (a) inserting a DNA fragment into a non-human embryonic stem cell, said DNA fragment comprising one or more nucleotide sequences that each encode a non-immunoglobulin polypeptide of interest, or portion thereof; (b) obtaining the non-human embryonic stem cell generated in (a); and (c) creating a non-human using the embryonic stem cell of (b).

In one embodiment, the method of making a non-human animal as disclosed herein comprises (a) obtaining a first non-human animal whose genome contains an immunoglobulin heavy chain variable region that includes an engineered D_{H} region as disclosed herein, and (b) breeding the first non-human animal of (a) with a second non-human animal, which in one aspect may be a different strain as the first non-human animal, wherein the second non-human animal expresses a universal light chain, and wherein the breeding results in offspring that produce, e.g., comprise, a genetically engineered heavy chain comprising an amino acid sequence of a non-immunoglobulin protein (or portion thereof), e.g., in a CDR3, and a genetically engineered rearranged light chain (single rearranged light chain; ULC).

In some embodiments, a DNA fragment includes 5, 10, 15, 20, 25 or more nucleotide sequences that each encode an extracellular portion of a D6 chemokine decoy receptor or that each encode a portion of a conotoxin (e.g., µ-conotoxin), a tarantula toxin, or combinations thereof. In some certain embodiments, a DNA fragment includes 25 nucleotide sequences that each encode an extracellular portion of a D6 chemokine decoy receptor or includes 26 nucleotide sequences that each encode a portion of a conotoxin (e.g., µ-conotoxin) and/or a tarantula toxin (e.g., ProTxI, ProTxII, etc.). In some certain embodiments, a DNA fragment further comprises first and second recombination signal sequences flanking each of the 25 or 26 nucleotide sequences.

In some embodiments, a method of making a non-human animal whose genome contains an immunoglobulin heavy chain variable region that includes an engineered D_{H} region is provided, the method comprising modifying the genome of a non-human animal so that it comprises an immunoglobulin heavy chain variable region that includes an engineered D_{H} region, which engineered D_{H} region comprises one or more nucleotide sequences that each encode a non-immunoglobulin polypeptide of interest, or portion thereof, thereby making said non-human animal.

In some embodiments, the genome of the non-human animal is modified to include 5, 10, 15, 20, 25 or more nucleotide sequences that each encode an extracellular portion of a D6 chemokine decoy receptor or that each encode a portion of a conotoxin (e.g., µ-conotoxin), a tarantula toxin, or combinations thereof. In some certain embodiments, the genome of the non-human animal is modified to include 25 nucleotide sequences that each encode an extracellular portion of a D6 chemokine decoy receptor or is modified to include 26 nucleotide sequences that each encode a portion of a conotoxin (e.g., µ-conotoxin) and/or a tarantula toxin (e.g., ProTxI, ProTxII, etc.). In some certain embodiments, the genome of the non-human animal is modified to further include first and second recombination signal sequences flanking each of the 25 or 26 nucleotide sequences.

In some embodiments, a method of producing an antibody in a non-human animal is provided, the method comprising the steps of (a) immunizing a non-human animal with an antigen, which non-human animal has a genome comprising an immunoglobulin heavy chain variable region that includes an engineered D_{H} region, wherein the engineered D_{H} region includes one or more nucleotide sequences that each encode a non-immunoglobulin polypeptide of interest, or portion thereof; (b) maintaining the non-human animal under conditions sufficient that the non-human animal produces an immune response to the antigen; and (c) recovering an antibody from the non-human animal, or a non-human animal cell, that binds the antigen. In some certain embodiments, a non-human cell is a B cell. In some certain embodiments, a non-human cell is a hybridoma.

In some embodiments, a non-human animal is provided whose germ cell genome comprises (a) a human immunoglobulin heavy chain variable region that comprises one or more unrearranged human V_{H} gene segments, an engineered D_{H} region, and one or more unrearranged human J_{H} gene segments, which engineered D_{H} region includes (i) one or more nucleotide sequences that each encode an extracellular portion of an atypical chemokine receptor (ACKR); and (ii) first and second recombination signal sequences flanking each of the one or more nucleotide sequences of (i) so that the one or more unrearranged human V_{H} gene segments, an engineered D_{H} region, and one or more unrearranged human J_{H} gene segments recombine, e.g., in a B cell, such that the non-human animal comprises a B cell genome comprising a human immunoglobulin heavy chain variable region that comprises a rearranged hV_{H}/eD_{H}/hJ_{H} gene sequence; wherein the human immunoglobulin heavy chain variable region is operably linked to one or more endogenous immunoglobulin heavy chain constant region genes so that the non-human animal is characterized in that when it is immunized with an antigen, the rearranged hV_{H}/eD_{H}/hJ_{H} gene sequence operably linked to an immunoglobulin heavy chain constant region gene encodes an antibody comprising a human heavy chain variable domains encoded by one of the human V_{H} gene segments (or portion thereof), an engineered D_{H} region (or portion thereof), and one of the human J_{H} gene segments (or portion thereof) operably linked to non-human animal heavy chain constant domains encoded by the one or more endogenous immunoglobulin constant region genes, and wherein the antibody shows specific binding to the antigen. In some embodiments, the germ cell and, e.g., B cell, of the non-human animal further comprises an immunoglobulin light chain locus encoding a common light chain such that the antibody further comprises the common light chain.

In some embodiments, a non-human animal is provided whose germ cell genome comprises a human immunoglobulin heavy chain variable region that comprises one or more unrearranged human (h) V_{H} gene segments, an engineered (e) D_{H} region, and one or more (h) human J_{H} gene segments, which engineered D_{H} region includes (i) one or more nucleotide sequences that each encode a portion of a toxin (e.g., a µ-conotoxin, tarantula toxin, or combinations thereof); and (ii) first and second recombination signal sequences flanking each of the one or more nucleotide sequences of (i) so that the one or more unrearranged human V_{H} gene segments, engineered D_{H} region, and one or more unrearranged human J_{H} gene segments recombine, e.g., in a B cell, such that the non-human animal comprises a B cell genome comprising a human immunoglobulin heavy chain variable region that comprises a rearranged hV_{H}/eD_{H}/hJ_{H} gene sequence; wherein the human immunoglobulin heavy chain variable region is operably linked to one or more endogenous immunoglobulin heavy chain constant region genes so that the non-human animal is characterized in that when it is immunized with an antigen, the rearranged hV_{H}/eD_{H}/hJ_{H} gene sequence operably linked to an immunoglobulin heavy chain constant region gene encodes an antibody comprising a human heavy chain variable domain encoded by one of the human V_{H} gene segments (or portion thereof), the engineered D_{H} region (or portion thereof), and one of the human J_{H} gene segments (or portion thereof) operably linked to one or more heavy chain constant domains encoded by the one or more endogenous immunoglobulin constant region genes, and wherein the antibodies show specific binding to the antigen. In some embodiments, the germ cell and, e.g., B cell, of the non-human animal further comprises an immunoglobulin light chain locus encoding a common light chain such that the antibody further comprises the common light chain.

In some embodiments, a provided non-human animal further comprises an insertion of one or more human V_{L} gene segments and one or more human J_{L} gene segments into an endogenous light chain locus. In some embodiments, human V_{L} and J_{L} segments are Vκ and Jκ gene segments and are inserted into an endogenous κ light chain locus. In some embodiments, human Vκ and Jκ gene segments are operably linked to a rodent Cκ gene (e.g. a mouse or a rat Cκ gene). In some embodiments, human V_{L} and J_{L} segments are Vλ and Jλ gene segments and are inserted into an endogenous λ light chain locus. In some embodiments, human Vλ and Jλ gene segments are operably linked to a rodent Cλ gene (e.g., a mouse or a rat Cλ gene). In some embodiments, a single rearranged human light chain variable region gene sequence is operably linked to an endogenous non-human light chain constant region gene. In some embodiments, a single rearranged human Vκ/Jκ gene sequence is operably linked to an endogenous Cκ gene (e.g., a mouse or rate Cκ gene). In some embodiments, a single rearranged Vλ/Jλ gene sequence is operably linked to an endogenous Cλ gene.

In some embodiments, a provided non-human animal is homozygous, heterozygous or hemizygous for an engineered D_{H} region as described herein. In some embodiments, a provided non-human animal is transgenic for an engineered D_{H} region as described herein.

Disclosed herein are also cells, e.g., B cells, or hybridomas derived therefrom by fusion with a myeloma cell, each comprising a rearranged (h)V_{H}/eD_{H}/(h)J_{H} sequence, which may be operably linked to a human or non-human heavy chain constant region comprising one or more heavy chain constant region genes. Such cell, e.g., B cell, may be isolated from a non-human animal, e.g., rodent (e.g., rat, mouse, etc.) as described herein.

Also described herein are nucleotide sequences comprising a rearranged variable (h)V_{H}/eD_{H}/(h)J_{H} sequence, which may be operably linked to a human or non-human heavy chain constant region comprising one or more heavy chain constant region genes. Such nucleotide sequences may be isolated from a non-human animal, e.g., rodent (e.g., rat, mouse, etc.) or non-human cell as described herein.

In some embodiments, use of a non-human animal as described herein in the manufacture and/or development of a drug or vaccine for use in medicine, such as use as a medicament, is provided.

In some embodiments, use of a non-human animal as described herein in the manufacture of a medicament for the treatment of a disease, disorder or condition is provided.

In some embodiments, use of a non-human animal as described herein in the manufacture and/or development of an antibody that binds a chemokine or voltage-gated sodium (Na_{V}) channel is provided.

In some embodiments, use of a non-human animal as described herein in the manufacture of a medicament for the treatment or detection of a disease characterized by chemokine or voltage-gated sodium (Na_{V}) channel expression or function is provided (e.g., aberrant expression or function).

In some embodiments, a non-human animal as described herein is provided for use in the manufacture and/or development of a drug for therapy or diagnosis.

In some embodiments, a non-human animal as described herein is provided for use in the manufacture of a medicament for the treatment, prevention or amelioration of a disease, disorder or condition.

In some embodiments, a non-human animal as described herein is provided for use in the manufacture and/or development of an antibody that binds a chemokine or voltage-gated sodium (Na_{V}) channel is provided.

In some embodiments, a disease, disorder or condition is an inflammatory disease, disorder or condition. In some embodiments, a disease, disorder or condition is characterized by chemokine expression or function (e.g., aberrant chemokine expression or function).

In some embodiments, a disease, disorder or condition is a pain disease, disorder or condition. In some embodiments, a disease, disorder or condition is characterized by ion channel expression or function (e.g., aberrant Na_{V} channel expression or function).

In many embodiments, a non-human animal provided herein is a rodent; in some embodiments, a mouse; in some embodiments, a rat.

As used in this application, the terms "about" and "approximately" are used as equivalents. Any numerals used in this application with or without about/approximately are meant to cover any normal fluctuations appreciated by one of ordinary skill in the relevant art.

Other features, objects, and advantages of the non-human animals, cells, nucleic acids and compositions disclosed herein are apparent in the detailed description of certain embodiments that follows. It should be understood, however, that the detailed description, while indicating certain embodiments, is given by way of illustration only, not limitation.

### BRIEF DESCRIPTION OF THE DRAWING

The Drawing included herein, which is composed of the following Figures, is for illustration purposes only and not for limitation.

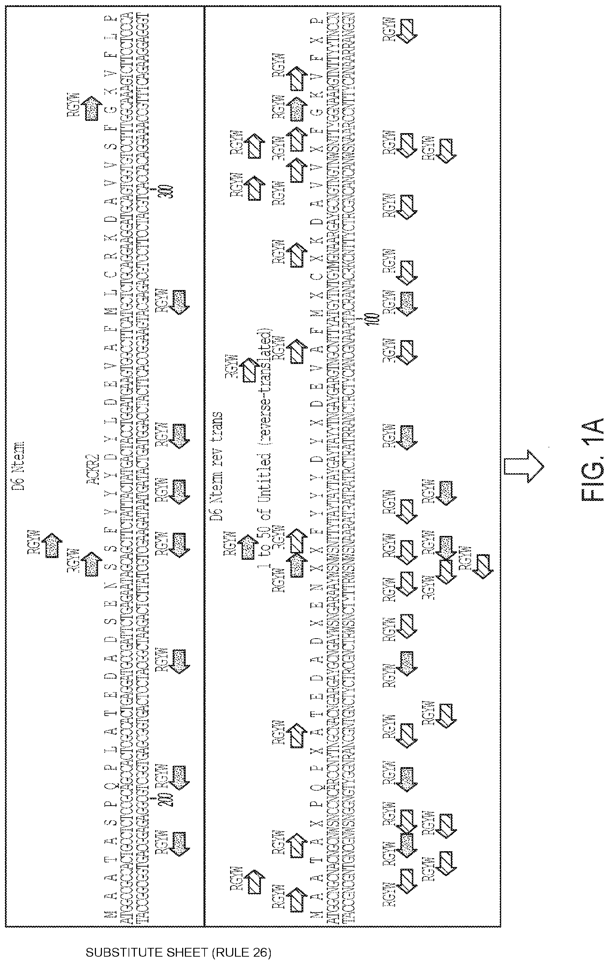

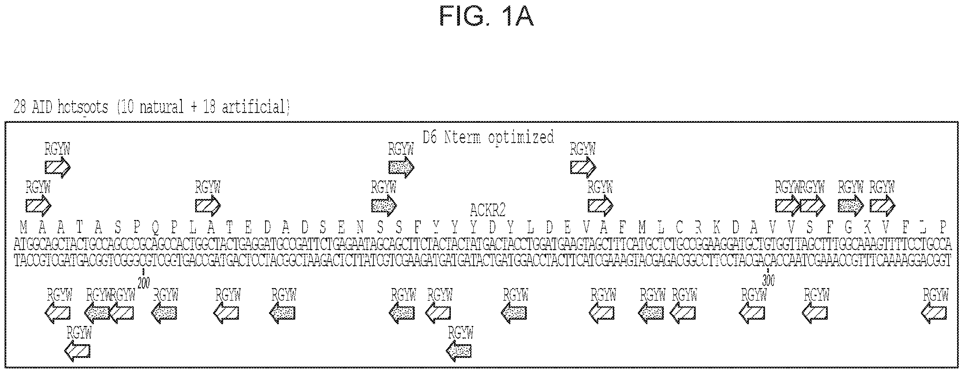

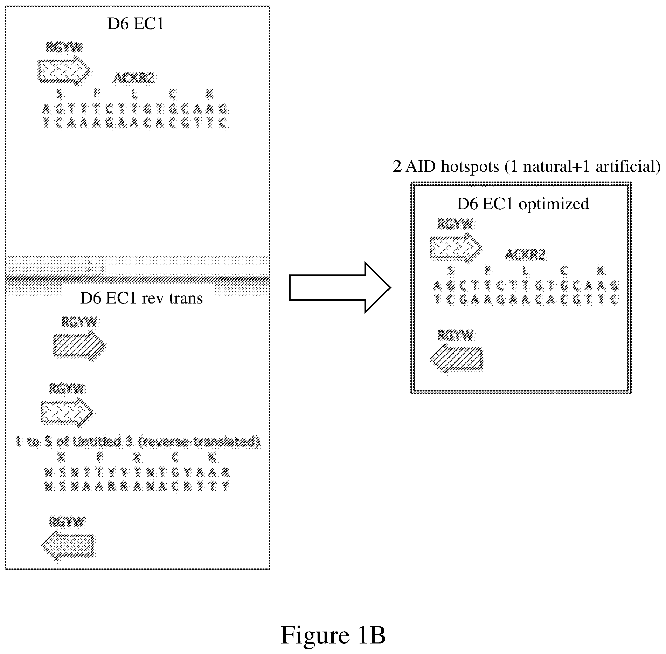

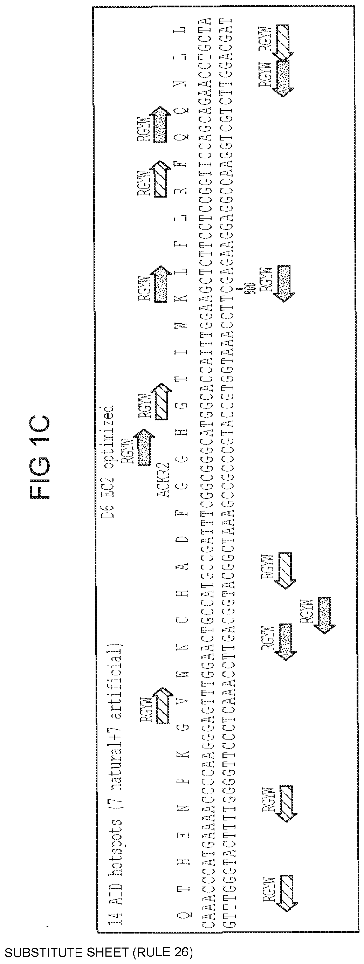

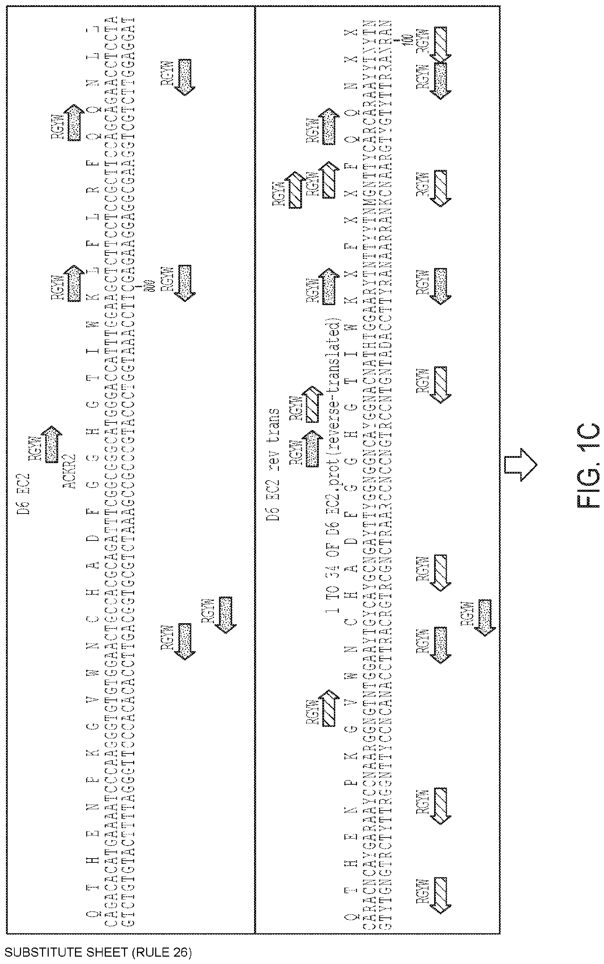

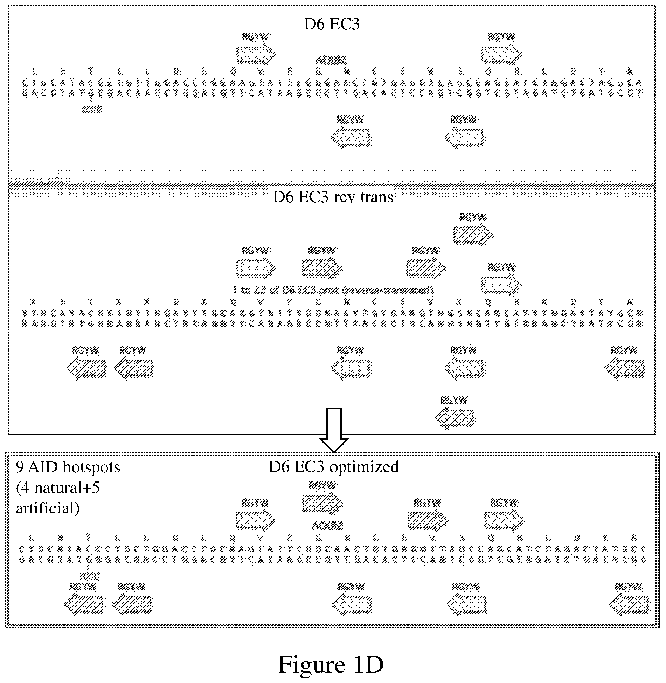

**Figures 1A-1D** shows exemplary optimization of selected D6 chemokine decoy receptor coding sequences to include somatic hypermutation hotspots. **Figure 1A**: optimized Nterm domain of D6 chemokine decoy receptor with locations of natural (broken line fill) and artificial (diagonal line fill) RGYW activation-induced cytidine deaminase (AID) hotspots; **Figure 1B**: optimized EC1 domain of D6 chemokine decoy receptor with locations of natural (broken line fill) and artificial (diagonal line fill) RGYW activation-induced cytidine deaminase (AID) hotspots; **Figure 1C**: optimized EC2 domain of D6 chemokine decoy receptor with locations of natural (broken line fill) and artificial (diagonal line fill) RGYW AID hotspots; **Figure 1D**: optimized EC3 domain of D6 chemokine decoy receptor with locations of natural (broken line fill) and artificial (diagonal line fill) RGYW AID hotspots.
**Figure 2** shows a table of exemplary optimized 5' and 3' recombination signal sequences (RSSs) designed for each nucleotide coding sequence to allow for efficient recombination frequency and equal usage of the nucleotide coding sequences during V(D)J recombination. global RSS consensus 5' RSS (SEQ ID NO:51); global RSS consensus 3' RSS (SEQ ID NO:52); human D_{H} consensus 5' RSS (SEQ ID NO:53); human D_{H} consensus 3' RSS (SEQ ID NO:54); mouse D_{H} consensus 5' RSS (SEQ ID NO:55); mouse D_{H} consensus 3' RSS (SEQ ID NO:56); optimized RSS 5' RSS (SEQ ID NO:57); optimized RSS 3' RSS (SEQ ID NO:58); 1-1 opt 5' RSS (SEQ ID NO:59); 1-1 opt 3' RSS (SEQ ID NO:60); 1-7 opt 5' RSS (SEQ ID NO:61); 1-7 opt 3' RSS (SEQ ID NO:62); 1-14 ORF opt 5' RSS (SEQ ID NO:63); 1-14 ORF opt 3' RSS (SEQ ID NO:64); 1-20 opt 5' RSS (SEQ ID NO:65); 1-20 opt 3' RSS (SEQ ID NO:66); 1-26 opt 5' RSS (SEQ ID NO:67); 1-26 opt 3' RSS (SEQ ID NO:68); 2-2^{∗}02 opt 5' RSS (SEQ ID NO:69); 2-2^{∗}02 opt 3' RSS (SEQ ID NO:70); 2-8^{∗}01 opt 5' RSS (SEQ ID NO:71); 2-8^{∗}01 opt 3' RSS (SEQ ID NO:72); 2-15 opt 5' RSS (SEQ ID NO:73); 2-15 opt 3' RSS (SEQ ID NO:74); 2-21^{∗}02 opt 5' RSS (SEQ ID NO:75); 2-21^{∗}02 opt 3' RSS (SEQ ID NO:76); 3-3^{∗}01 opt 5' RSS (SEQ ID NO:77); 3-3^{∗}01 opt 3' RSS (SEQ ID NO:78); 3-9 opt 5' RSS (SEQ ID NO:79); 3-9 opt 3' RSS (SEQ ID NO:80); 3-10^{∗}01 opt 5' RSS (SEQ ID NO:81); 3-10^{∗}01 opt 3' RSS (SEQ ID NO:82); 3-16^{∗}02 opt 5' RSS (SEQ ID NO:83); 3-16^{∗}02 opt 3' RSS (SEQ ID NO:84); 3-22 opt 5' RSS (SEQ ID NO:85); 3-22 opt 3' RSS (SEQ ID NO:86); 4-4 opt 5' RSS (SEQ ID NO:87); 4-4 opt 3' RSS (SEQ ID NO:88); 4-11 ORF opt 5' RSS (SEQ ID NO:89); 4-11 ORF opt 3' RSS (SEQ ID NO:90); 4-17 opt 5' RSS (SEQ ID NO:91); 4-17 opt 3' RSS (SEQ ID NO:92); 4-23 ORF opt 5' RSS (SEQ ID NO:93); 4-23 ORF opt 3' RSS (SEQ ID NO:94); 5-5 opt 5' RSS (SEQ ID NO:95); 5-5 opt 3' RSS (SEQ ID NO:96); 5-12 opt 5' RSS (SEQ ID NO:97); 5-12 opt 3' RSS (SEQ ID NO:98); 5-18 opt 5' RSS (SEQ ID NO:99); 5-18 opt 3' RSS (SEQ ID NO:100); 5-24 ORF opt 5' RSS (SEQ ID NO:101); 5-24 ORF opt 3' RSS (SEQ ID NO:102); 6-6 opt 5' RSS (SEQ ID NO:103); 6-6 opt 3' RSS (SEQ ID NO:104); 6-13 opt 5' RSS (SEQ ID NO:105); 6-13 opt 3' RSS (SEQ ID NO:106); 6-19 opt 5' RSS (SEQ ID NO:107); 6-19 opt 3' RSS (SEQ ID NO:108); 6-25 (not optimized) 5' RSS (SEQ ID NO:109); 6-25 (not optimized) 3' RSS (SEQ ID NO:110); 7-27 (not optimized) 5' RSS (SEQ ID NO:111); 7-27 (not optimized) 3' RSS (SEQ ID NO:112). Bold and italicized font for global RSS consensus and 7-27 indicates match to global RSS consensus sequence based on rodent and human RSS from immunoglobulin and T cell receptor V, D and J gene segments; bold font for mouse D_{H} consensus indicates match to mouse immunoglobulin D_{H} consensus sequence; bold font for human D_{H} consensus, optimized RSS and all remaining RSS (e.g., 1-1 opt, 1-7 opt, 1-20 opt, etc.) indicates match to the human immunoglobulin D_{H} consensus sequence.

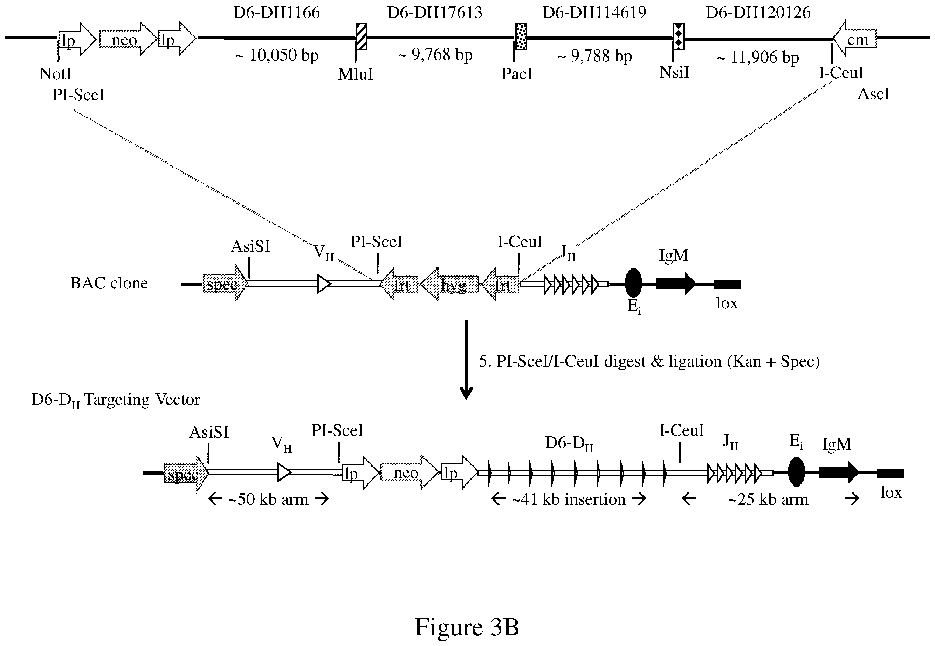

**Figures 3A-3B** shows an illustration, not to scale, of an exemplary strategy for construction of a targeting vector for integration into rodent embryonic stem (ES) cells to create a rodent whose genome comprises an immunoglobulin heavy chain variable region that includes an engineered diversity cluster (i.e., D_{H} region), which diversity cluster includes one or more nucleotide sequences that each encode a portion of a non-immunoglobulin polypeptide (e.g., an extracellular portion of a D6 chemokine decoy receptor). **Figure 3A**: four initial steps highlighting (1) *de novo* synthesis of D6 coding sequences, (2) AgeI/EcoRI digestion and ligation of a selection cassette (e.g., neomycin) and a D6 DNA fragment, (3) SnaBI digestion of D6 DNA fragments and NotI/AscI digestion of a BAC vector (pBacE3.6), and (4) one-step isothermal assembly of digested DNA fragments to create contiguous engineered diversity cluster of D6 chemokine decoy receptor coding sequences in place of traditional D_{H} segments; **Figure 3B**: additional step for creating a targeting vector for integration into the genome of rodent ES cells, (5) PI-SceI/I-CeuI digestion and ligation of 25 synthetic D6 chemokine decoy receptor coding sequences into BAC clone to append 5' and 3' homology arms containing human immunoglobulin V_{H} DNA and J_{H} DNA, respectively. Various restriction enzyme recognition sites are indicated for each of the depicted DNA fragments. lp: *lox*P site; neo: neomycin selection cassette drive by ubiquitin promoter; cm: chloramphenicol selection cassette; frt: Flippase recognition target sequence; hyg: hygromycin selection cassette; Ei: murine heavy chain intronic enhancer; IgM: murine immunoglobulin M constant region gene; lox: *lox*P site sequence of pBACe3.6 vector.

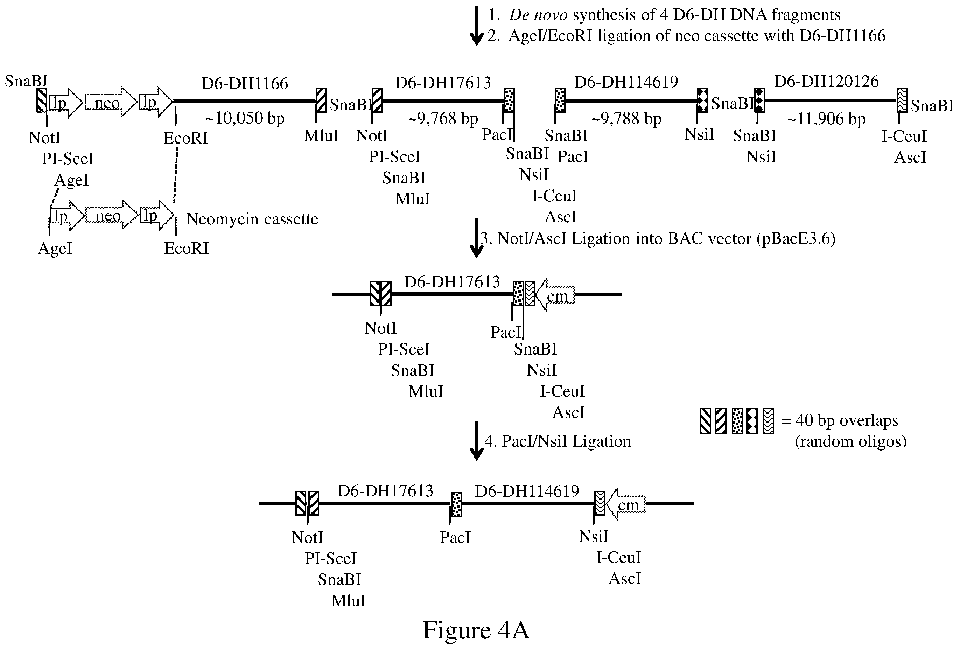

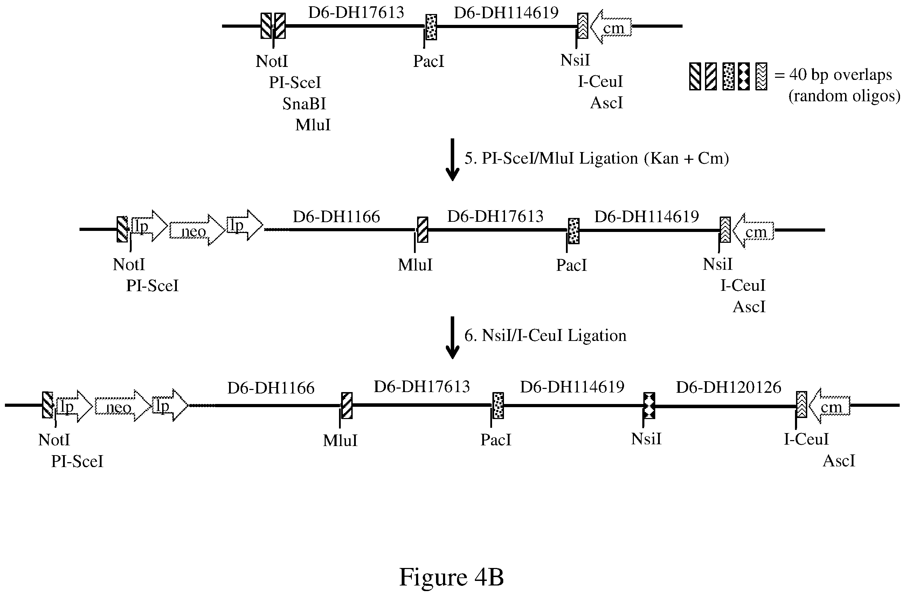

**Figures 4A-4B** shows an illustration, not to scale, of an alternative exemplary strategy to assemble D6 chemokine decoy receptor coding sequences by sequential ligation for construction of a targeting vector for integration into rodent embryonic stem (ES) cells to create a rodent whose genome comprises an immunoglobulin heavy chain variable region that includes an engineered diversity cluster (i.e., an engineered D_{H} region), which diversity cluster includes one or more nucleotide sequences that each encode an extracellular portion of a D6 chemokine decoy receptor. **Figure 4A**: four initial steps highlighting (1) *de novo* synthesis of D6 coding sequences, (2) AgeI/EcoRI digestion and ligation of a selection cassette (e.g., neomycin) and a D6 DNA fragment, (3) NotI/AscI digestion and ligation of a D6 DNA fragment into a BAC vector (pBacE3.6), and (4) PacI/Nsi digestion and ligation of D6 fragments into a BAC vector backbone; **Figure 4B**: two additional steps for creating a targeting vector for integration into the genome of rodent ES cells, (5) PI-SceI/I-CeuI digestion and ligation of an additional D6 DNA fragment into the BAC vector backbone, and (6) NsiI/I-CeuI digestion and ligation of final D6 DNA fragment to create 25 synthetic D6 chemokine decoy receptor coding sequences into a BAC vector backbone. Various restriction enzyme recognition sites are indicated for each of the depicted DNA fragments; lp: *lox*P site sequence; neo: neomycin selection cassette driven by ubiquitin promoter; cm: chloramphenicol selection cassette.

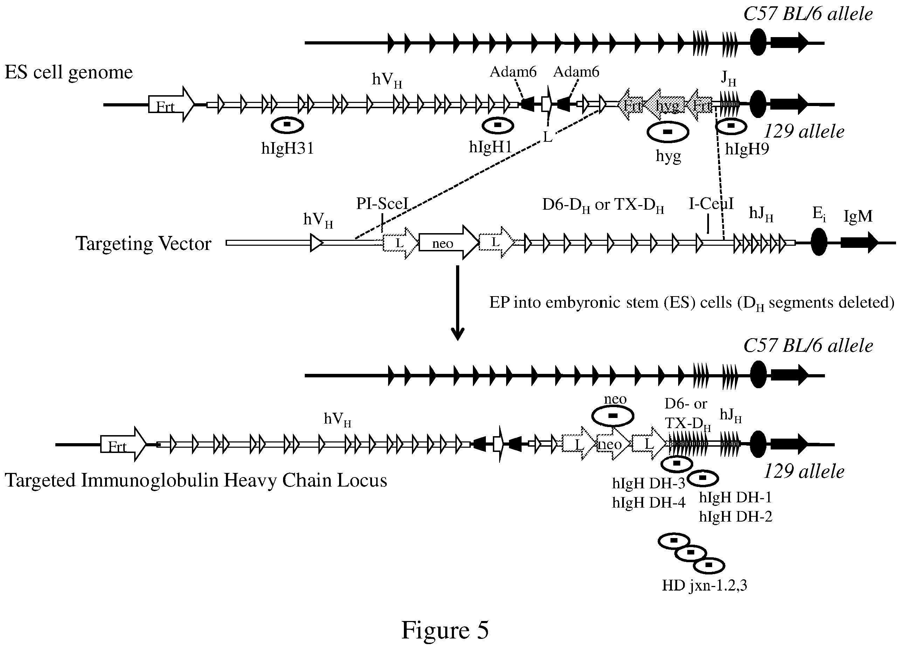

**Figure 5** shows an exemplary screening strategy using genetic material of drug-resistant colonies after electroporation screened by TAQMAN^{™} and karyotyping. Names and approximate locations, not to scale (line encompassed by oval), of various primer/probe sets (see Table 7) are indicated below various alleles shown (not to scale). hyg: hygromycin selection cassette driven by ubiquitin promoter; neo: neomycin selection cassette driven by ubiquitin promoter; L: *lox*P site sequence; Frt: Flippase recognition target sequence.

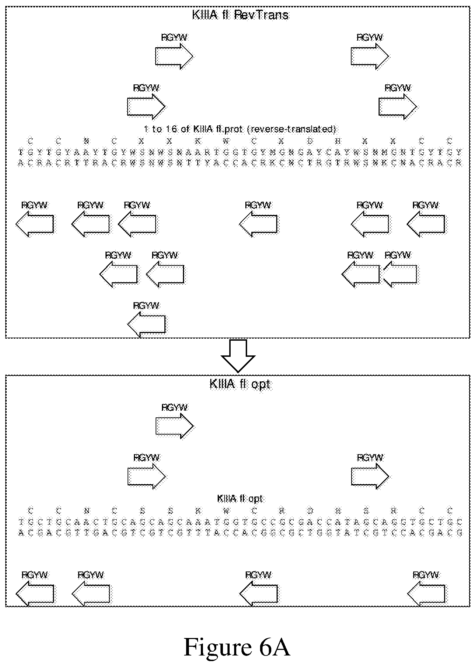

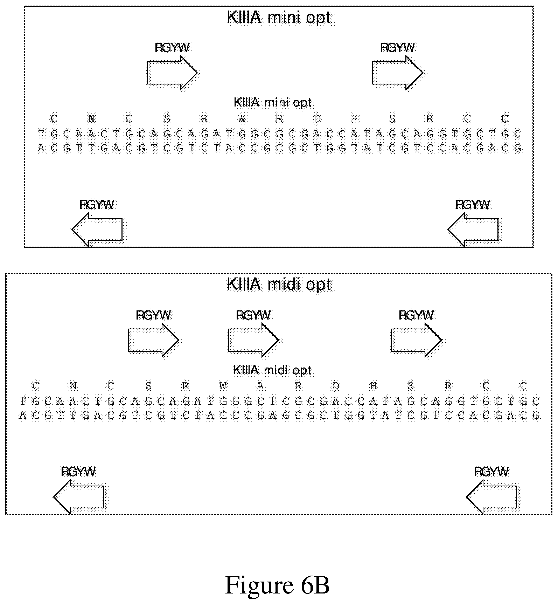

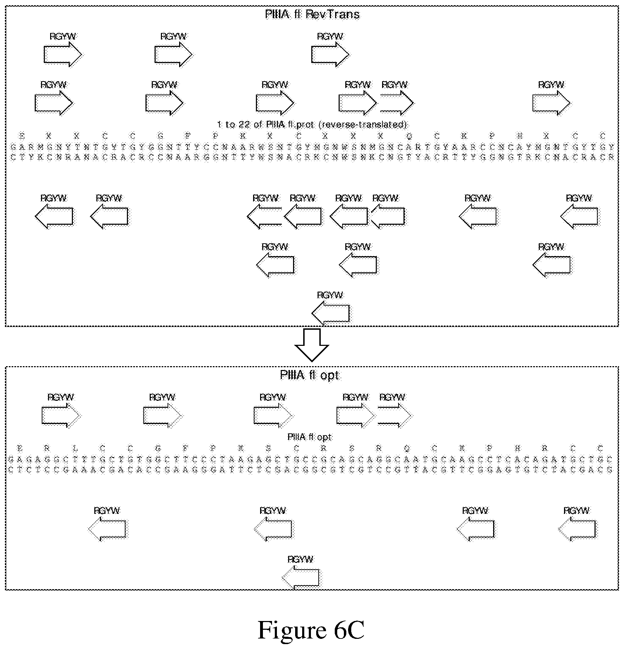

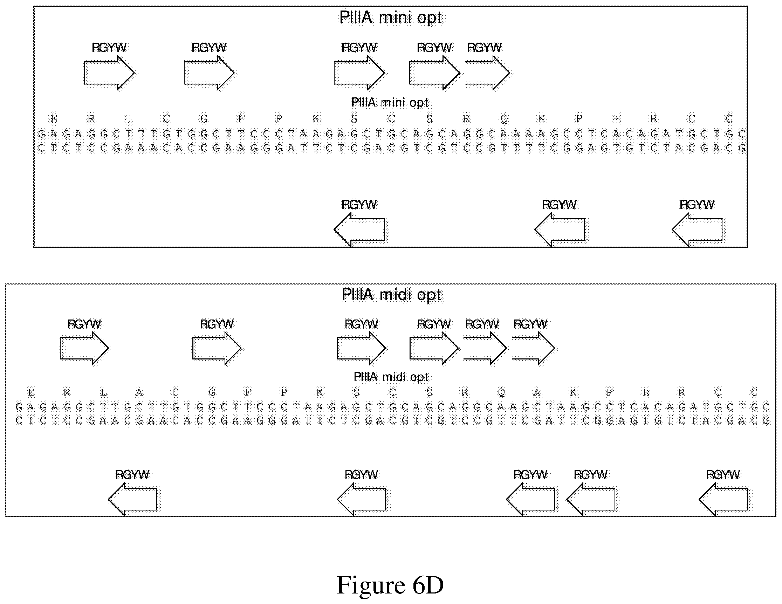

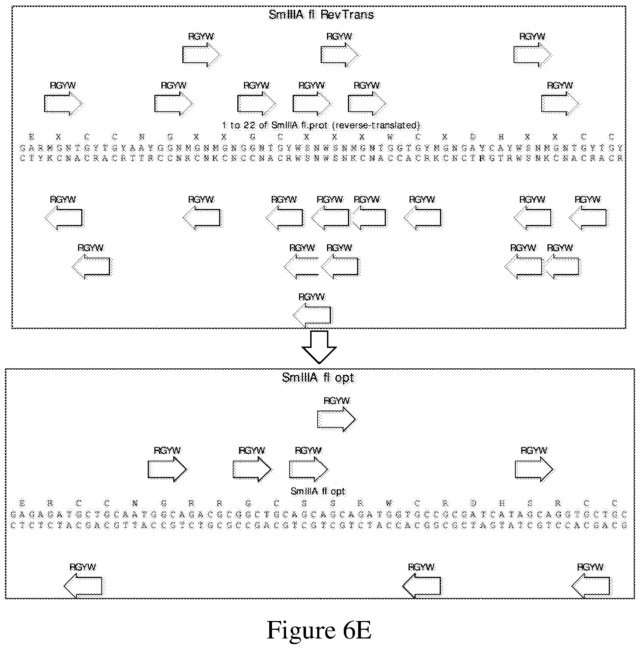

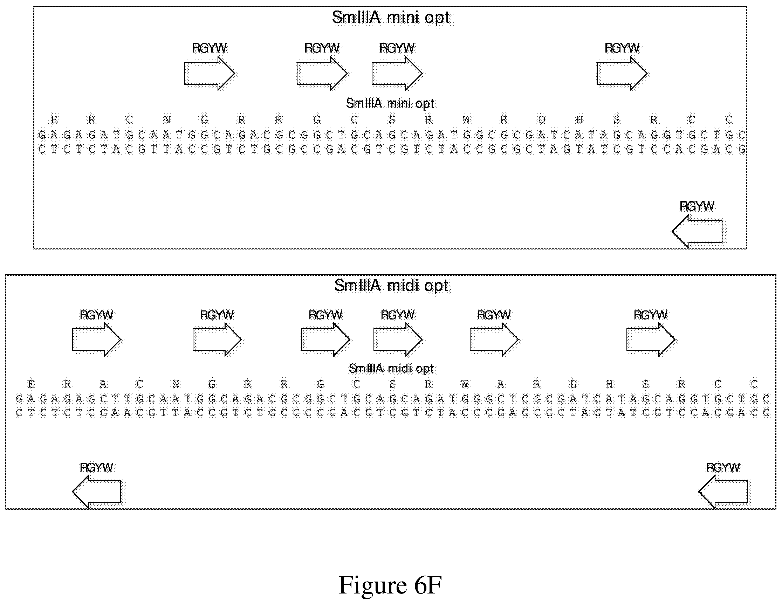

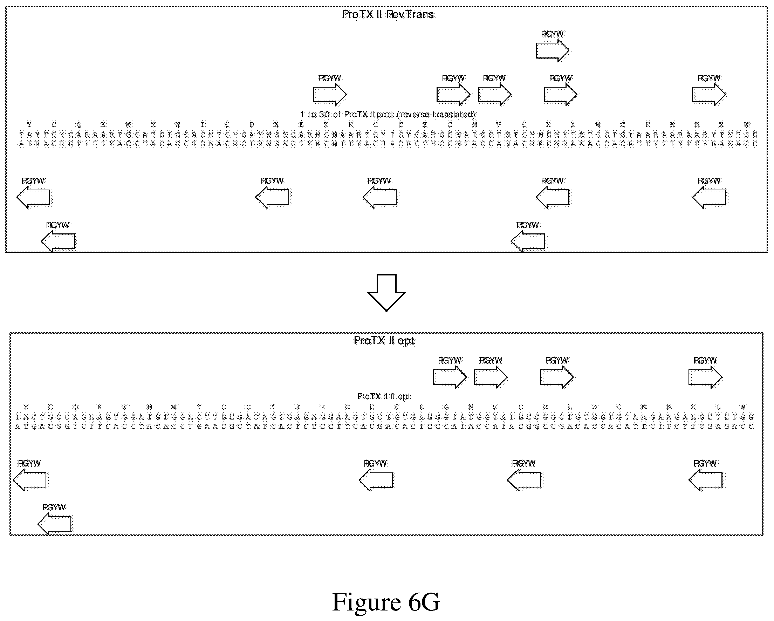

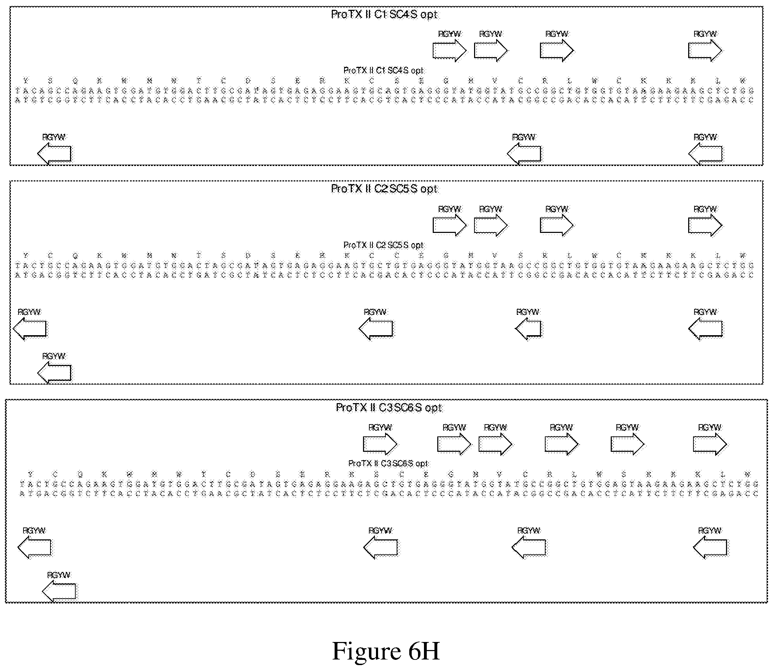

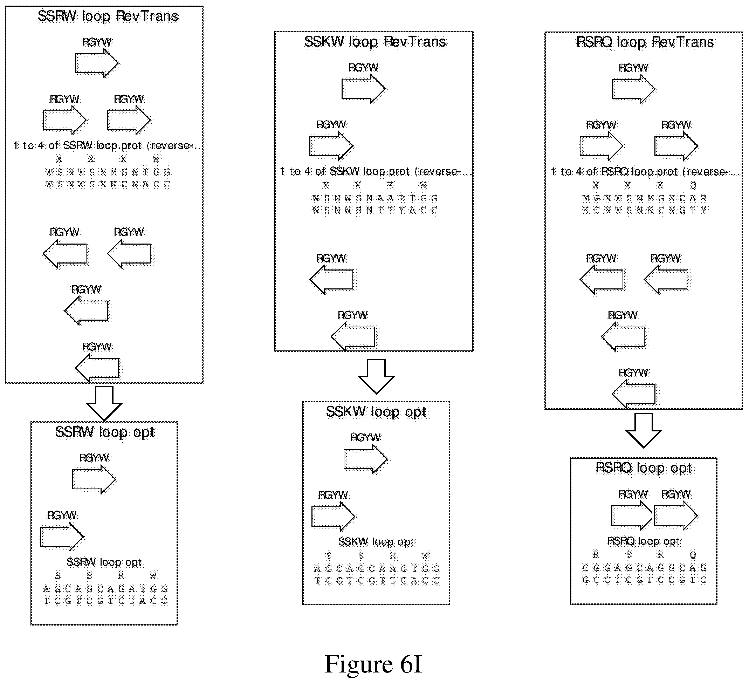

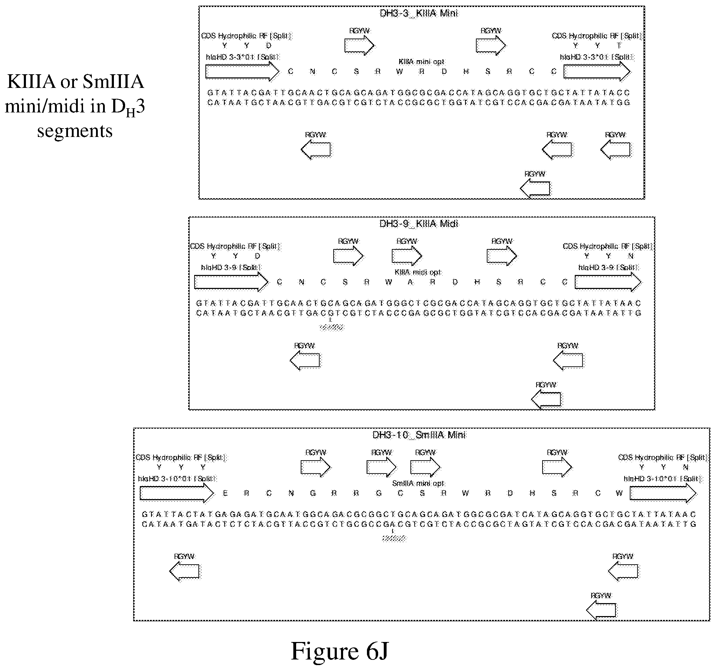

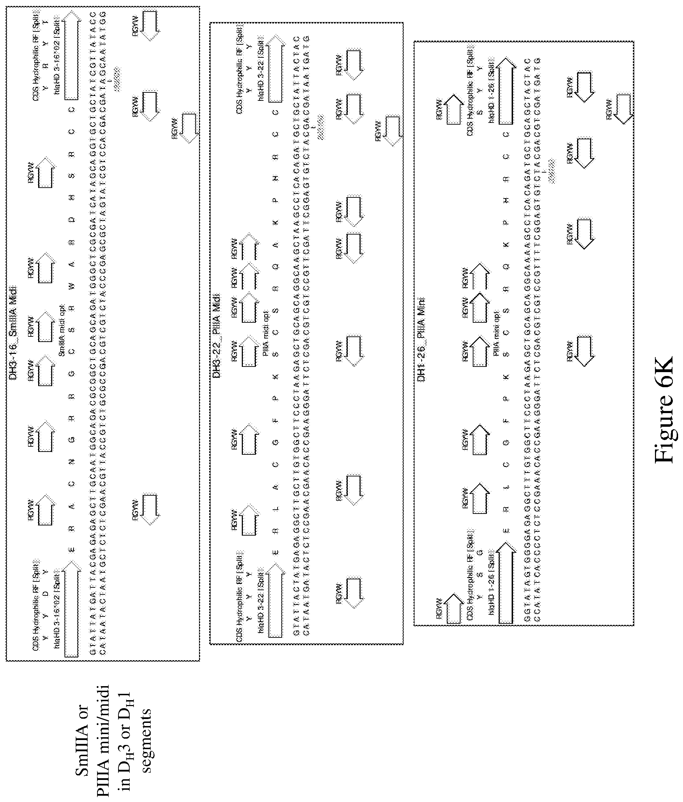

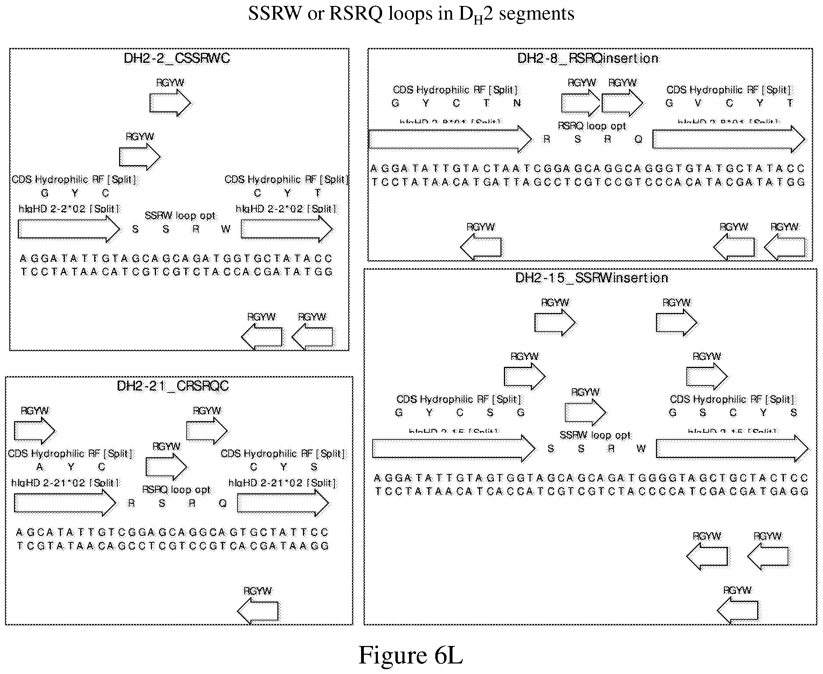

**Figures 6A-6L** shows exemplary optimization of selected µ-conotoxin and tarantula toxin coding sequences to include somatic hypermutation hotspots. **Figure 6A**: optimized KIIIA fl of µ-conotoxin with locations of artificial (diagonal line fill) RGYW activation-induced cytidine deaminase (AID) hotspots; **Figure 6B**: optimized KIIIA mini (top) and KIIIA midi (bottom) of µ-conotoxin with locations of artificial (diagonal line fill) RGYW activation-induced cytidine deaminase (AID) hotspots; **Figure 6C**: optimized PIIIA fl of µ-conotoxin with locations of artificial (diagonal line fill) RGYW AID hotspots; **Figure 6D**: optimized PIIIA mini (top) and PIIIA midi (bottom) of µ-conotoxin with locations of artificial (diagonal line fill) RGYW AID hotspots; **Figure 6E**: optimized SMIIIA fl of µ-conotoxin with locations of artificial (diagonal line fill) RGYW AID hotspots; **Figure 6F**: optimized SmIIIA mini (top) and SmIIIA midi (bottom) of µ-conotoxin with locations of artificial (diagonal line fill) RGYW AID hotspots; **Figure 6G**: optimized ProTxII tarantula toxin with locations of artificial (diagonal line fill) RGYW AID hotspots; **Figure 6H**: optimized tarantula toxin ProTxII C1SC4S (top), ProTxII C2SC5S (middle) and ProTxII C3SC6S (bottom) with locations of artificial (diagonal line fill) RGYW AID hotspots; **Figure 6I**: optimized SmIIIA SSRW loop (left), SmIIIA SSKW loop (middle) and PIIIA RSRQ loop (right) of µ-conotoxin with locations of artificial (diagonal line fill) RGYW AID hotspots; **Figure 6J**: optimized KIIIA or SmIIIA mini/midi of µ-conotoxin in D_{H}3 segment locations with locations of artificial (diagonal line fill) RGYW AID hotspots; **Figure 6K**: optimized SmIIIA or PIIIA mini/midi of µ-conotoxin in D_{H}3 or D_{H}1 segment locations with locations of artificial (diagonal line fill) RGYW AID hotspots; **Figure 6L**: optimized SSRW or RSRQ loops of µ-conotoxin in D_{H}2 segment locations with locations of artificial (diagonal line fill) RGYW AID hotspots.

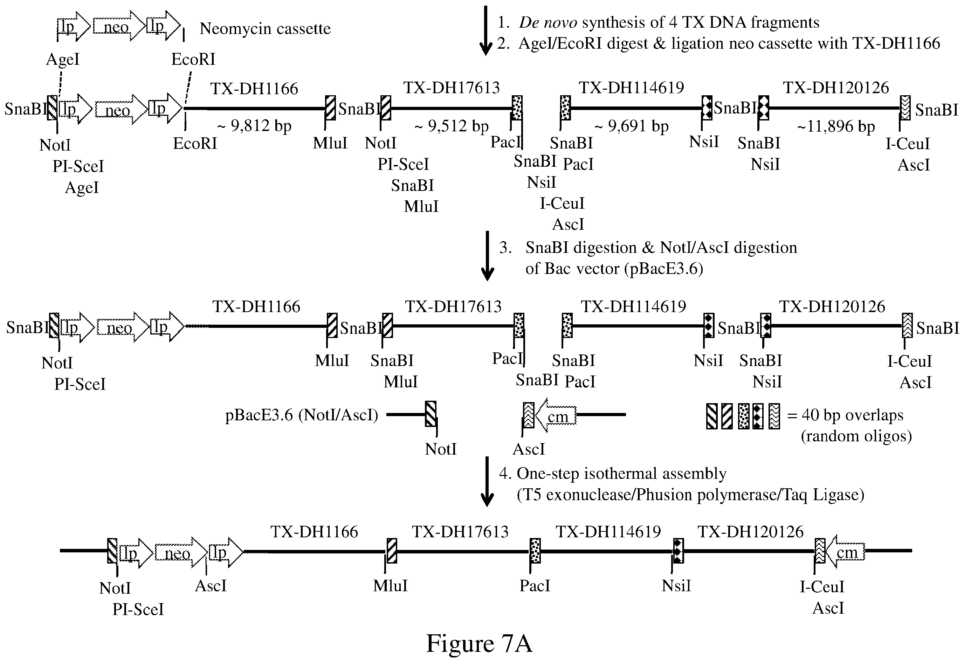

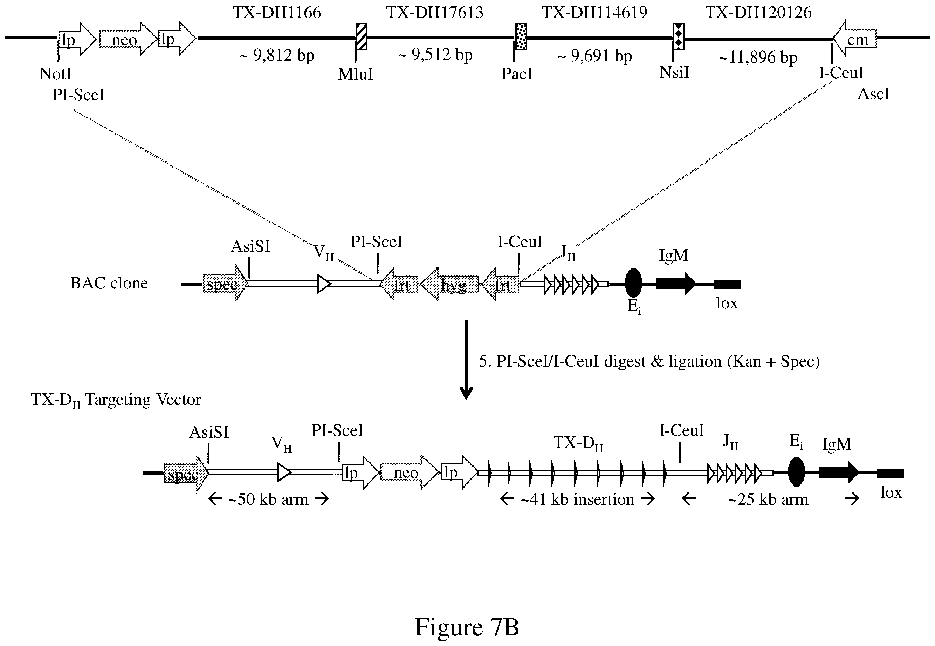

**Figures 7A-7B** shows an illustration, not to scale, of an exemplary strategy for construction of a targeting vector for integration into rodent embryonic stem (ES) cells to create a rodent whose genome comprises an immunoglobulin heavy chain variable region that includes an engineered diversity cluster (i.e., an engineered D_{H} region), which diversity cluster includes one or more nucleotide sequences that each encode a portion of a non-immunoglobulin polypeptide (e.g., a portion of µ-conotoxin and/or tarantula toxin). **Figure 7A**: four initial steps highlighting (1) *de novo* synthesis of toxin coding sequences, (2) AgeI/EcoRI digestion and ligation of a selection cassette (e.g., neomycin) and a toxin DNA fragment (TX-DH1166), (3) SnaBI digestion of toxin DNA fragments and NotI/AscI digestion of a BAC vector (pBacE3.6), and (4) one-step isothermal assembly of digested DNA fragments to create an engineered diversity cluster comprising contiguous toxin coding sequences in place of one or more, and optionally, all functional D_{H} gene segments; **Figure 7B**: additional step for creating a targeting vector for integration into the genome of rodent ES cells, (5) PI-SceI/I-CeuI digestion and ligation of 26 synthetic toxin coding sequences into BAC clone to append 5' and 3' homology arms containing human immunoglobulin V_{H} DNA and J_{H} DNA, respectively. Various restriction enzyme recognition sites are indicated for each of the depicted DNA fragments; lp: *lox*P site; neo: neomycin selection cassette drive by ubiquitin promoter; cm: chloramphenicol selection cassette; frt: Flippase recognition target sequence; hyg: hygromycin selection cassette; Ei: murine heavy chain intronic enhancer; IgM: murine immunoglobulin M constant region gene; lox: *lox*P of pBACe3.6 vector.

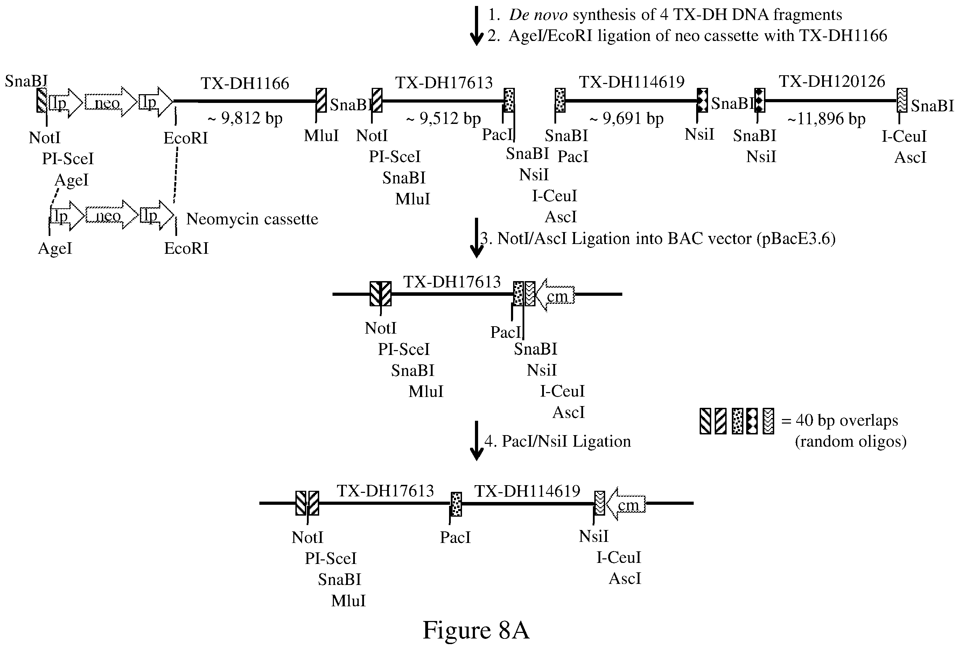

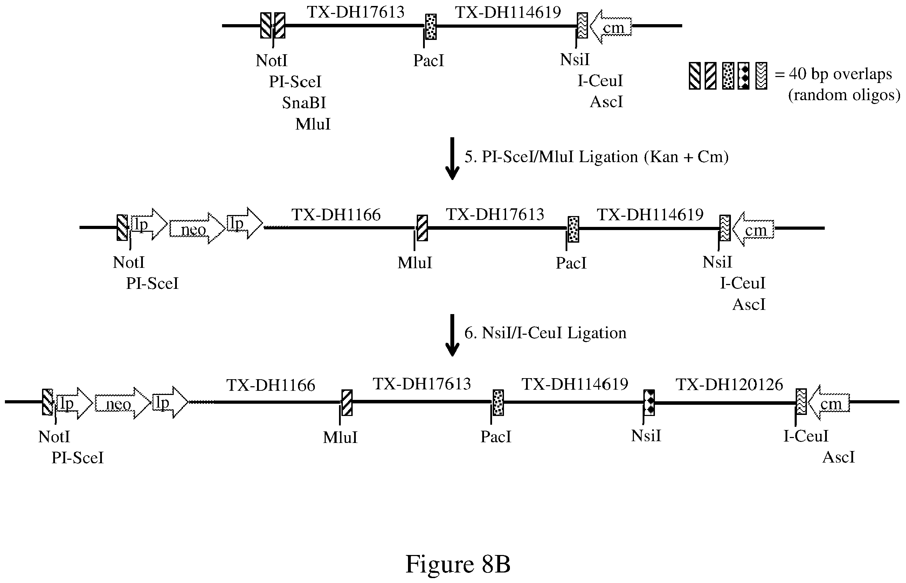

**Figures 8A-8B** shows an illustration, not to scale, of an alternative exemplary strategy to assemble toxin (e.g., µ-conotoxin and tarantula toxin) coding sequences by sequential ligation for construction of a targeting vector for integration into rodent embryonic stem (ES) cells to create a rodent whose genome comprises an immunoglobulin heavy chain variable region that includes an engineered diversity cluster (i.e., D_{H} region), which diversity cluster includes one or more nucleotide sequences that each encode a portion of a toxin peptide (e.g., µ-conotoxin and tarantula toxin ProTxII). **Figure 8A**: four initial steps highlighting (1) *de novo* synthesis of toxin coding sequences, (2) AgeI/EcoRI digestion and ligation of a selection cassette (e.g., neomycin) and a toxin DNA fragment (TX-DH1166), (3) NotI/AscI digestion and ligation of a toxin DNA fragment into a BAC vector (pBacE3.6), and (4) PacI/Nsi digestion and ligation of toxin DNA fragments into a BAC vector backbone; **Figure 8B**: two additional steps for creating a targeting vector for integration into the genome of rodent ES cells, (5) PI-SceI/I-CeuI digestion and ligation of an additional toxin DNA fragment into the BAC vector backbone, and (6) NsiI/I-CeuI digestion and ligation of final toxin DNA fragment to create 26 synthetic toxin coding sequences into a BAC vector backbone. Various restriction enzyme recognition sites are indicated for each of the depicted DNA fragments; lp: *lox*P site; neo: neomycin selection cassette drive by ubiquitin promoter; cm: chloramphenicol selection cassette.

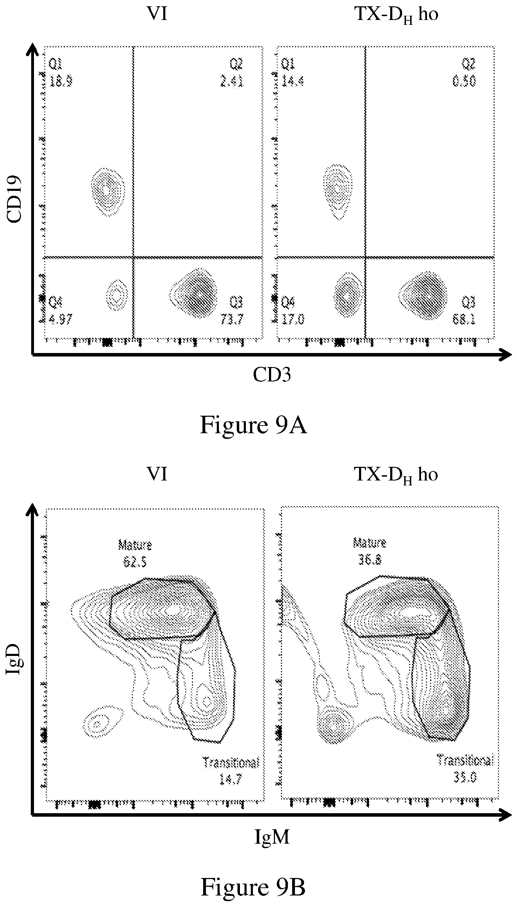

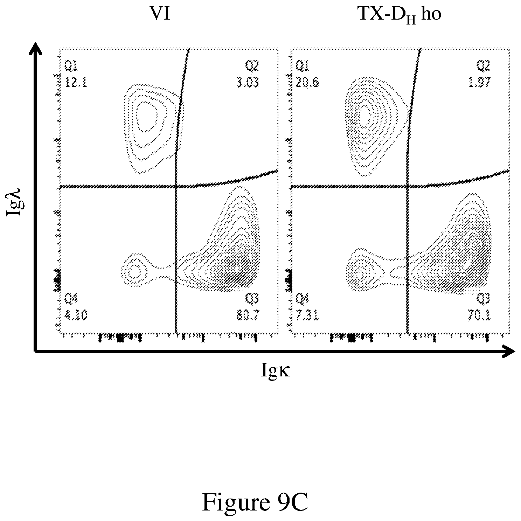

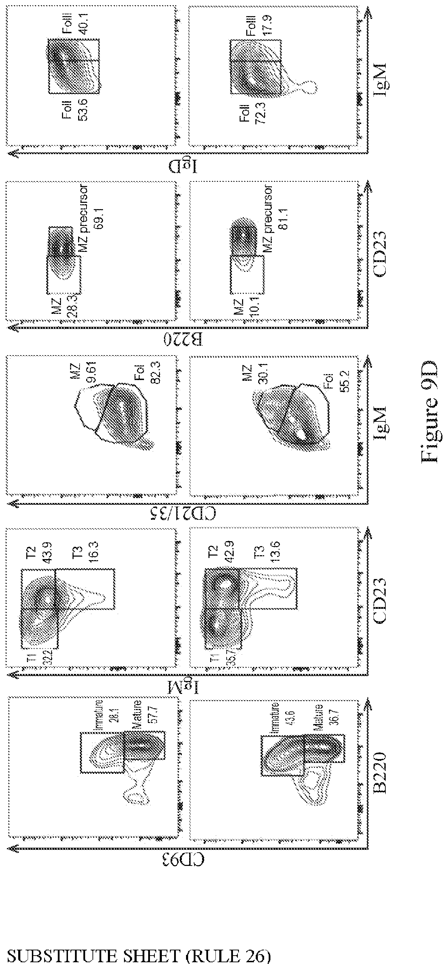

**Figures 9A-9D** show representative contour plots of lymphocytes in spleen harvested from VELOCIMMUNE^{®} (VI) and mice homozygous for an engineered D_{H} region containing toxin coding sequences (6579ho/1293ho, "TX-D_{H} ho"; a rodent strain having a genome comprising a homozygous immunoglobulin heavy chain locus containing a plurality of human V_{H}, engineered D_{H} segments including toxin coding sequences in the place of traditional D_{H} segments, and J_{H} segments operably linked to a rodent immunoglobulin heavy chain constant region including rodent heavy chain enhancers and regulatory regions, and containing an inserted nucleotide sequence encoding one or more murine Adam6 genes [e.g., U.S. Patent Nos. 8,642,835 and 8,697,940]; and a homozygous immunoglobulin κ light chain locus containing human Vκ and Jκ gene segments operably linked to a rodent Cκ region gene including rodent κ light chain enhancers), and stained for cell surface expression of various cell markers. **Figure 9A**: representative contour plot of lymphocytes from spleen gated on singlets illustrating expression of CD19 (y-axis) and CD3 (x-axis). **Figure 9B**: representative contour plot of lymphocytes from spleen singlets gated on CD19⁺ illustrating expression of immunoglobulin D (IgD, y-axis) and immunoglobulin M (IgM, x-axis); mature (CD19⁺ IgD⁺ IgM^{int}) and transitional (CD19⁺ IgD^{int} IgM) B cells are indicated on each dot plot. **Figure 9C**: representative contour plot of lymphocytes from spleen singlets gated on CD19⁺ illustrating expression of Igλ (y-axis) or Igx (x-axis) light chain. **Figure 9D**: shows representative contour plot of B cell maturation illustrating lymphocytes from spleen singlets gated on CD19⁺ and showing expression of [from left to right] CD93 (y-axis) and B220 (x-axis), IgM (y-axis) and CD23 (x-axis); CD21/35 (y-axis) and IgM (x-axis), B220 (y-axis) and CD23 (x-axis), and IgD (y-axis) and IgM (x-axis). Top row: VELOCIMMUNE^{®} mice; Bottom row: TX-D_{H} ho (6579ho/1293ho) mice. Specific B cell populations are indicated on each dot plot: Immature (CD19⁺ CD93⁺ B220⁺), mature (CD19⁺ CD93⁻ B220⁺), T1 (CD19⁺ CD93⁺ B220⁺ IgM⁺ CD23⁻), T2 (CD19⁺ CD93⁺ B220⁺ IgM⁺ CD23⁺), T3 (CD19⁺ CD93⁺ B220⁺ IgM^{int} CD23⁺), MZ (CD19⁺ CD93⁻ B220⁺ CD21/35⁺ IgM⁺ CD23⁻), MZ precursor (CD19⁺ CD93⁻ B220⁺ CD21/35⁺IgM⁺CD23⁺), Fol I (CD19⁺ CD93⁻ B220⁺ CD21/35^{int} IgM^{int} IgD⁺), and Fol II (CD19⁺ CD93⁻ B220⁺ CD21/35^{int} IgM⁺IgD⁺).

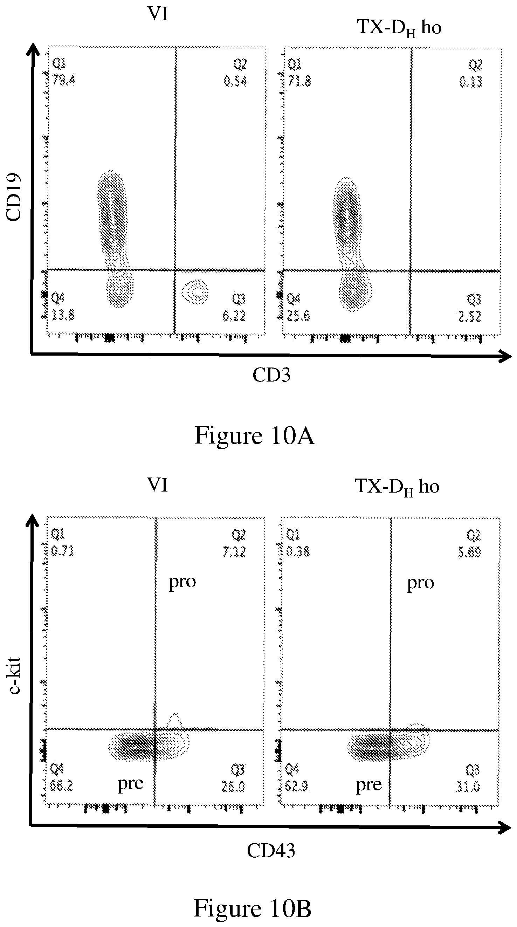

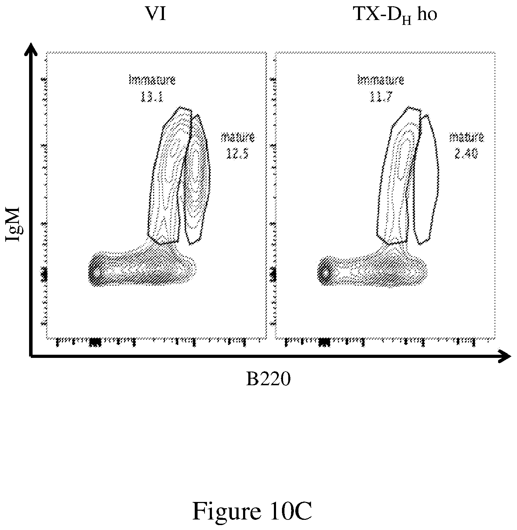

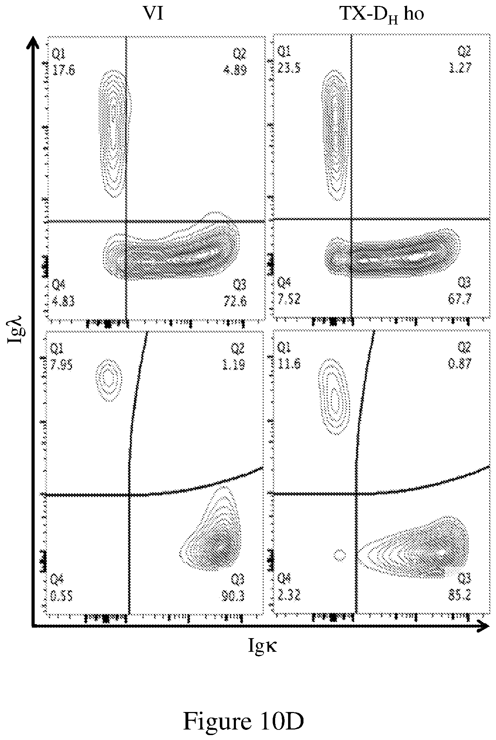

**Figures 10A-10D** show representative contour plots of lymphocytes in bone marrow harvested from VELOCIMMUNE^{®} (VI) and mice homozygous for an engineered D_{H} region containing toxin coding sequences (6579ho/1293ho, "TX-D_{H} ho"; a rodent strain having a genome comprising a homozygous immunoglobulin heavy chain locus containing a plurality of human V_{H}, engineered D_{H} segments including toxin coding sequences in the place of traditional D_{H} segments, and J_{H} segments operably linked to a rodent immunoglobulin heavy chain constant region including rodent heavy chain enhancers and regulatory regions, and containing an inserted nucleotide sequence encoding one or more murine Adam6 genes [e.g., U.S. Patent Nos. 8,642,835 and 8,697,940]; and a homozygous immunoglobulin κ light chain locus containing human Vκ and Jκ gene segments operably linked to a rodent Cκ region gene including rodent κ light chain enhancers), and stained for cell surface expression of various cell markers. **Figure 10A**: representative contour plot of lymphocytes from bone marrow gated on singlets illustrating expression of CD19 (y-axis) and CD3 (x-axis). **Figure 10B**: representative contour plot of lymphocytes from bone marrow gated on CD19⁺IgM^{-/low}IgD⁻ illustrating expression of c-kit (y-axis) and CD43 (x-axis); pre- (c-kit⁻ CD43⁻) and pro-B (c-kit⁺ CD43⁺) cells are indicated on each dot plot. **Figure 10C****:** representative contour plot of lymphocytes from bone marrow gated on singlets illustrating expression of IgM (y-axis) and B220 (x-axis); immature (IgM^{int to +} B220^{int}) and mature (IgM^{int to +} B220⁺) B cells are indicated on each dot plot. **Figure 10D**: representative contour plot of lymphocytes from bone marrow gated on CD19⁺IgM^{int to +} B220^{int} (top row) and CD19⁺IgM^{int/+}B220⁺ (bottom row) illustrating expression of Igλ (y-axis) or Igκ (x-axis) light chain.

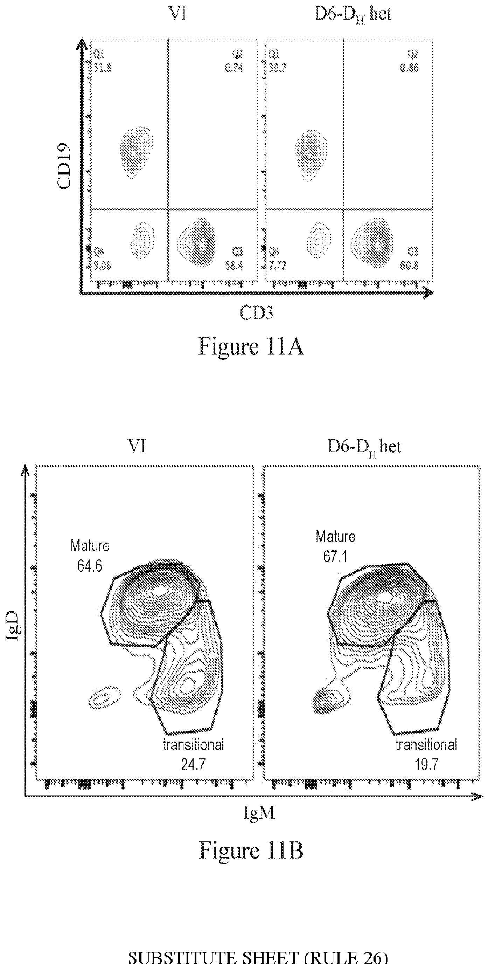

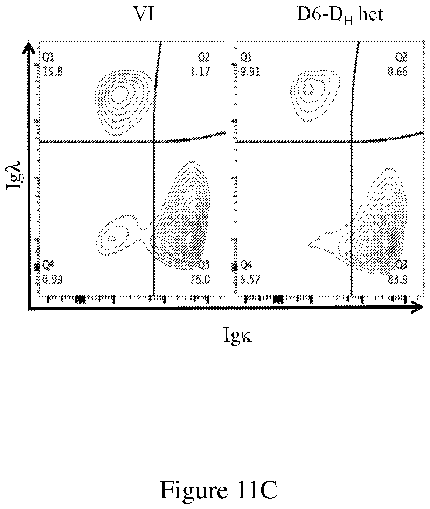

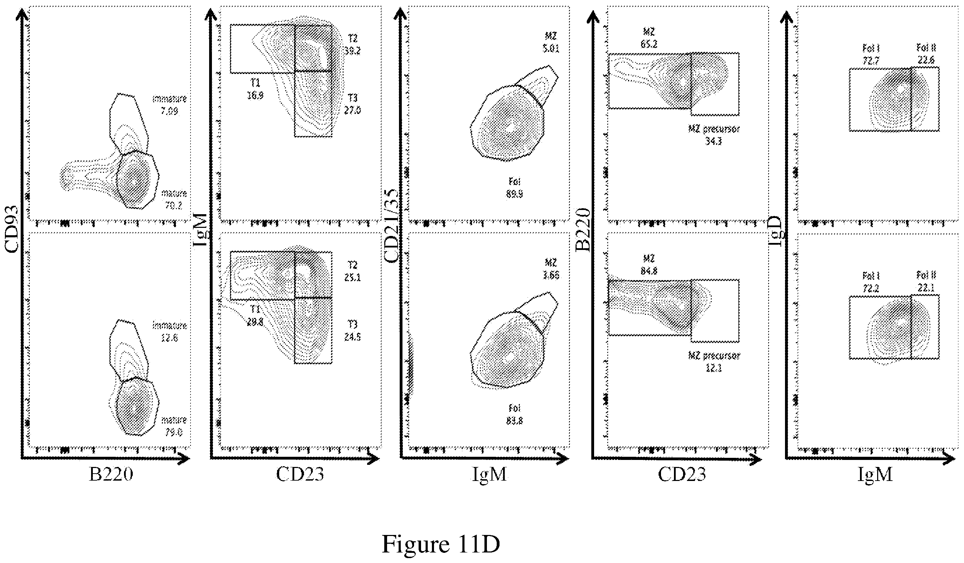

**Figures 11A-11D** show representative contour plots of lymphocytes in spleen harvested from VELOCIMMUNE^{®} (VI) and mice heterozygous for an engineered D_{H} region containing D6 coding sequences (6590het, "D6-D_{H} het"; a rodent strain having a genome comprising a heterozygous immunoglobulin heavy chain locus containing a plurality of human V_{H}, engineered D_{H} segments including D6 chemokine decoy receptor coding sequences in the place of traditional D_{H} segments, and J_{H} segments operably linked to a rodent immunoglobulin heavy chain constant region including rodent heavy chain enhancers and regulatory regions, and containing an inserted nucleotide sequence encoding one or more murine Adam6 genes [e.g., U.S. Patent Nos. 8,642,835 and 8,697,940]; and stained for cell surface expression of various cell markers. **Figure 11A**: representative contour plot of lymphocytes from spleen gated on singlets illustrating expression of CD19 (y-axis) and CD3 (x-axis). **Figure 11B**: representative contour plot of lymphocytes from spleen gated on CD19⁺ singlets illustrating expression of IgD (y-axis) and IgM (x-axis); mature (CD19⁺ IgD⁺ IgM^{int}) and transitional (CD19⁺ IgD^{int} IgM⁺) B cells are indicated on each dot plot. **Figure 11C**: representative contour plot of lymphocytes from spleen gated on CD19⁺ singlets illustrating expression of Igλ (y-axis) or Igκ (x-axis) light chain. **Figure 11D**: shows representative contour plot of B cell maturation illustrating lymphocytes from spleen gated on CD19⁺ singlets and showing expression of [from left to right] CD93 (y-axis) and B220 (x-axis), IgM (y-axis) and CD23 (x-axis); CD21/35 (y-axis) and IgM (x-axis), B220 (y-axis) and CD23 (x-axis), and IgD (y-axis) and IgM (x-axis). Top row: VELOCIMMUNE^{®} mice; Bottom row: D6-D_{H} het (6590het) mice. Specific B cell populations are indicated on each dot plot: Immature (CD19⁺ CD93⁺ B220⁺), mature (CD19⁺ CD93⁻ B220⁺), T1 (CD19⁺ CD93⁺ B220⁺ IgM⁺ CD23⁻), T2 (CD19⁺ CD93⁺ B220⁺ IgM⁺ CD23⁺), T3 (CD19⁺ CD93⁺ B220⁺ IgM^{int} CD23⁺), MZ (CD19⁺ CD93⁻ B220⁺ CD21/35⁺ IgM⁺CD23⁻), MZ precursor (CD19⁺ CD93⁻ B220⁺ CD21/35⁺IgM⁺CD23⁺), Fol I (CD19⁺ CD93⁻ B220⁺ CD21/35^{int} IgM^{int}IgD⁺), and Fol II (CD19⁺ CD93⁻ B220⁺ CD21/35^{int} IgM⁺IgD⁺).

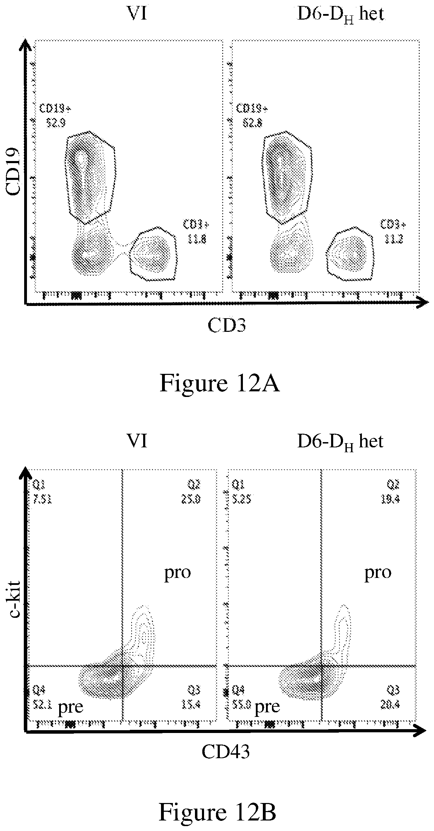

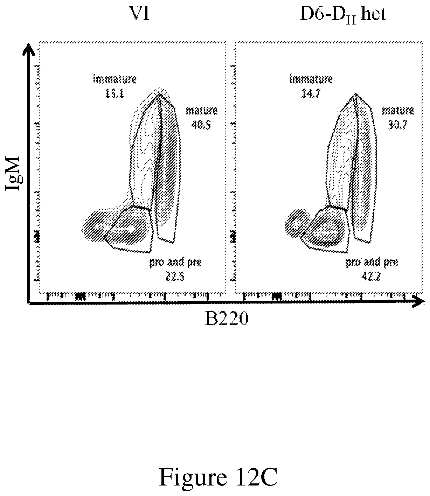

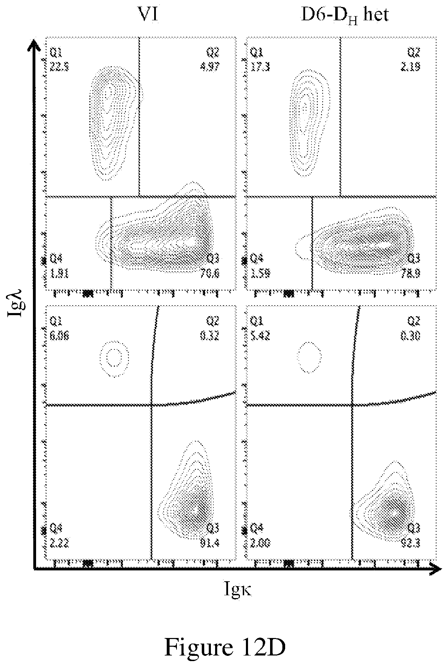

**Figures 12A-12D** show representative contour plots of lymphocytes in bone marrow harvested from VELOCIMMUNE^{®} (VI) and mice heterozygous for an engineered D_{H} region containing D6 coding sequences (6590het, "D6-D_{H} het"; a rodent strain having a genome comprising a heterozygous immunoglobulin heavy chain locus containing a plurality of human V_{H}, engineered D_{H} segments including D6 chemokine decoy receptor coding sequences in the place of traditional D_{H} segments, and J_{H} segments operably linked to a rodent immunoglobulin heavy chain constant region including rodent heavy chain enhancers and regulatory regions, and containing an inserted nucleotide sequence encoding one or more murine Adam6 genes [e.g., U.S. Patent Nos. 8,642,835 and 8,697,940]; and stained for cell surface expression of various cell markers. **Figure 12A**: representative contour plot of lymphocytes from bone marrow gated on singlets illustrating expression of CD19 (y-axis) and CD3 (x-axis). **Figure 12B**: representative contour plot of lymphocytes from bone marrow gated on CD19⁺ IgM^{- to low} IgD⁻ illustrating expression of c-kit (y-axis) and CD43 (x-axis); pre- B (c-kif CD43⁻) and pro-B (c-kit⁺ CD43⁺) cells are indicated on each dot plot. **Figure 12C**: representative contour plot of lymphocytes from bone marrow gated on CD19⁺ illustrating expression of IgM (y-axis) and B220 (x-axis); immature (IgM^{int to +} B220^{int}) and mature (IgM^{int to +} B220⁺), pre- and pro-B cells (IgM^{- to low} B220^{int}) are indicated on each dot plot. **Figure 12D**: representative contour plot of lymphocytes from bone marrow gated on CD19⁺IgM^{int to +}B220^{int} (top row) and CD19⁺IgM ^{int to +}B220⁺ (bottom row) illustrating expression of Igλ (y-axis) or Igκ (x-axis) light chain.

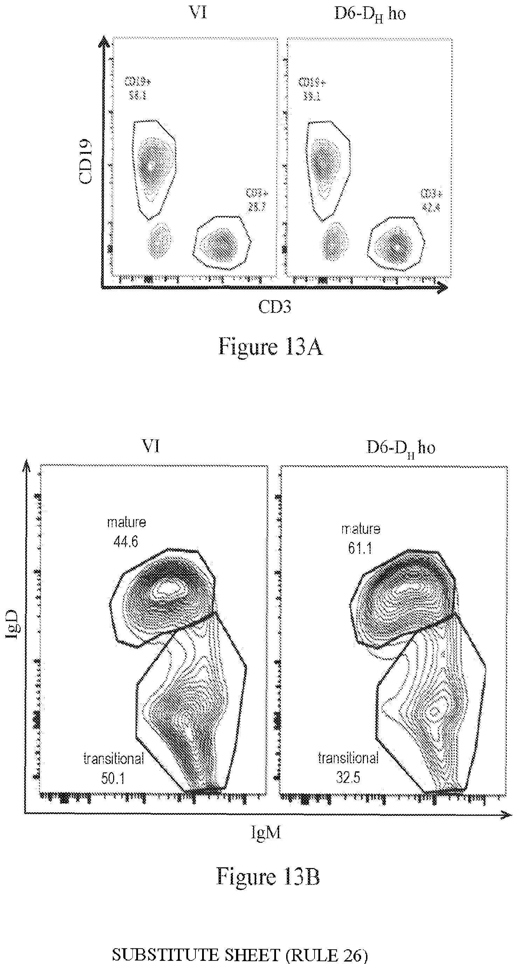

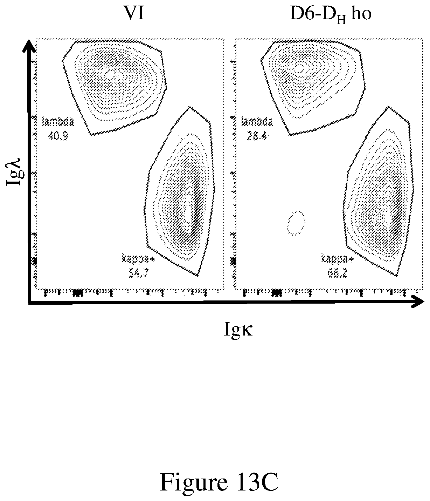

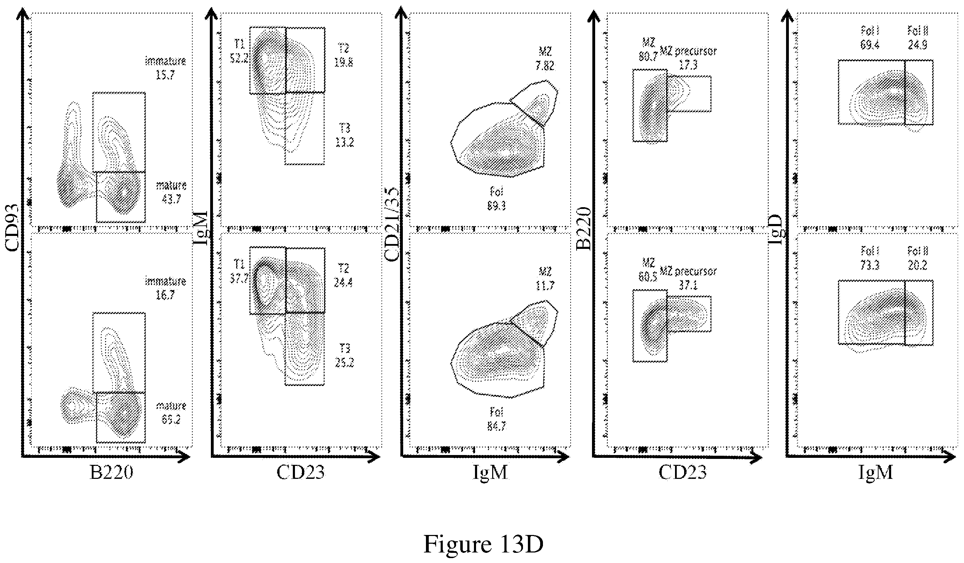

**Figures 13A-13D** show representative contour plots of lymphocytes in spleen harvested from VELOCIMMUNE^{®} (VI) and mice homozygous for an engineered D_{H} region containing D6 coding sequences (6590ho/1293ho, "D6-D_{H} ho"; a rodent strain having a genome comprising a homozygous immunoglobulin heavy chain locus containing a plurality of human V_{H}, engineered D_{H} segments including D6 chemokine decoy receptor coding sequences in the place of traditional D_{H} segments, and J_{H} segments operably linked to a rodent immunoglobulin heavy chain constant region including rodent heavy chain enhancers and regulatory regions, and containing an inserted nucleotide sequence encoding one or more murine Adam6 genes [e.g., U.S. Patent Nos. 8,642,835 and 8,697,940]; and a homozygous immunoglobulin κ light chain locus containing human Vκ and Jκ gene segments operably linked to a rodent Cκ region gene including rodent κ light chain enhancers), and stained for cell surface expression of various cell markers. **Figure 13A**: representative contour plot of lymphocytes from spleen gated on singlets illustrating expression of CD19 (y-axis) and CD3 (x-axis). **Figure 13B**: representative contour plot of lymphocytes from spleen gated on CD19⁺ illustrating expression of IgD (y-axis) and IgM (x-axis); mature (CD19⁺ IgD⁺ IgM^{int}) and transitional (CD19⁺ IgD^{int} IgM⁺) B cells are indicated on each dot plot. **Figure 13C**: representative contour plot of lymphocytes from spleen gated on CD19⁺ illustrating expression of Igλ (y-axis) or Igx (x-axis) light chain. **Figure 13D**: shows representative contour plot of B cell maturation illustrating lymphocytes from spleen gated on CD19⁺ singlets and showing expression of [from left to right] CD93 (y-axis) and B220 (x-axis), IgM (y-axis) and CD23 (x-axis); CD21/35 (y-axis) and IgM (x-axis), B220 (y-axis) and CD23 (x-axis), and IgD (y-axis) and IgM (x-axis). Top row: VELOCIMMUNE^{®} mice; Bottom row: D6-D_{H} ho (6590ho) mice. Specific B cell populations are indicated on each dot plot: Immature (CD19⁺ CD93⁺ B220⁺), mature (CD19⁺ CD93⁻ B220⁺), T1 (CD19⁺ CD93⁺ B220⁺ IgM⁺ CD23⁻), T2 (CD19⁺ CD93⁺ B220⁺ IgM⁺ CD23⁺), T3 (CD19⁺ CD93⁺ B220⁺ IgM^{int} CD23⁺), MZ (CD19⁺ CD93⁻ B220⁺ CD21/35⁺ IgM⁺ CD23⁻), MZ precursor (CD19⁺ CD93⁻ B220⁺ CD21/35⁺ IgM⁺ CD23⁺), Fol I (CD19⁺ CD93⁻ B220⁺ CD21/35^{int} IgM^{int} IgD⁺), and Fol II (CD19⁺ CD93⁻ B220⁺ CD21/35^{int} IgM⁺IgD⁺).

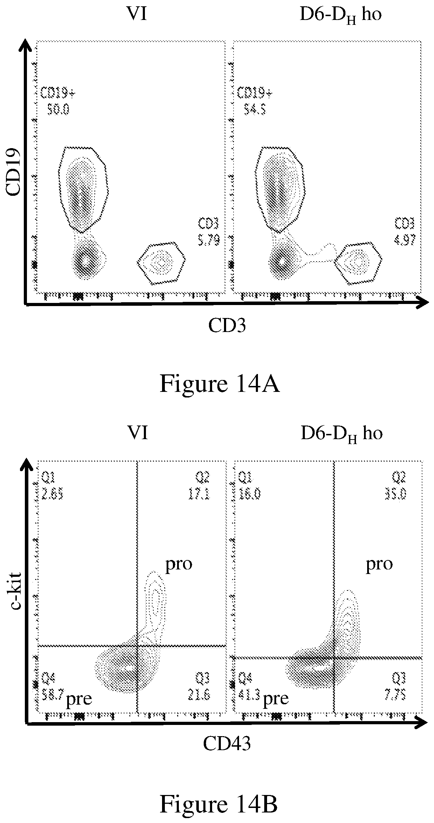

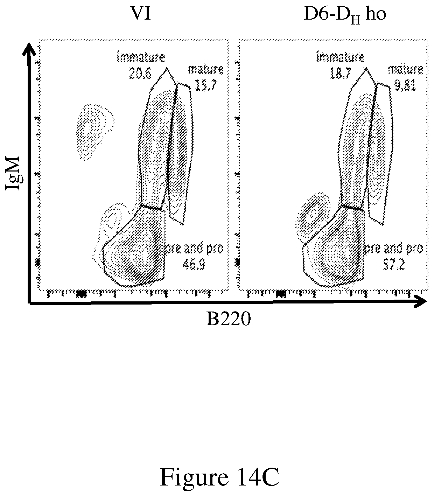

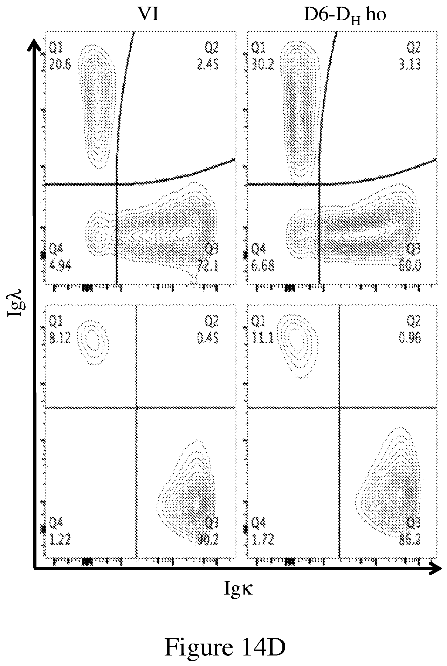

**Figures 14A-14D** show representative contour plots of lymphocytes in bone marrow harvested from VELOCIMMUNE^{®} (VI) and mice homozygous for an engineered D_{H} region containing D6 coding sequences (6590ho/1293ho, "D6-D_{H} ho"; a rodent strain having a genome comprising a heterozygous immunoglobulin heavy chain locus containing a plurality of human V_{H}, engineered D_{H} segments including D6 chemokine decoy receptor coding sequences in the place of traditional D_{H} segments, and J_{H} segments operably linked to a rodent immunoglobulin heavy chain constant region including rodent heavy chain enhancers and regulatory regions, and containing an inserted nucleotide sequence encoding one or more murine Adam6 genes [e.g., U.S. Patent Nos. 8,642,835 and 8,697,940]; and a homozygous immunoglobulin κ light chain locus containing human Vκ and Jκ gene segments operably linked to a rodent Cκ region gene including rodent κ light chain enhancers), and stained for cell surface expression of various cell markers. **Figure 14A**: representative contour plot of lymphocytes from bone marrow gated on singlets illustrating expression of CD19 (y-axis) and CD3 (x-axis). **Figure 14B**: representative contour plot of lymphocytes from bone marrow gated on CD19⁺IgM^{- to low}IgD⁻ illustrating expression of c-kit (y-axis) and CD43 (x-axis); pre- B (c-kif CD43⁻) and pro-B (c-kit⁺ CD43⁺) cells are indicated on each dot plot. **Figure 14C**: representative contour plot of lymphocytes from bone marrow gated on CD19⁺ illustrating expression of IgM (y-axis) and B220 (x-axis); immature (IgM^{int to +}B220^{int}) and mature (IgM^{int to +} B220⁺), pre- and pro-B cells (IgM^{- to low} B220^{int}) are indicated on each dot plot. **Figure 14D**: representative contour plot of lymphocytes from bone marrow gated on CD19⁺IgM^{int to +}B220^{int} (top row) and CD19⁺IgM^{int to +}B220⁺ (bottom row) illustrating expression of Igλ (y-axis) or Igκ (x-axis) light chain.

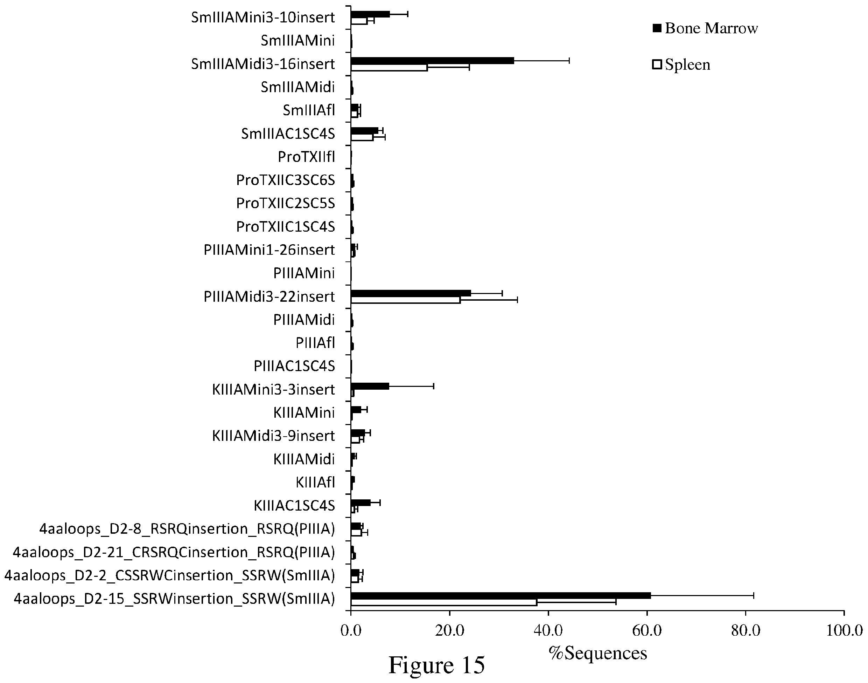

**Figure 15** shows representative usage frequency of toxin coding sequences in an engineered D_{H} region in amplified RNA from spleen and bone marrow (combined all V_{H}-families, not reflective of quantitative V_{H} usage) of three 6579ho/1293ho mice ("TX-D_{H} ho", *supra*). The y-axis indicates the name of each toxin coding sequence within the engineered D_{H} region. The x-axis indicates the frequency (percentage of sequences) of each toxin coding sequence among analyzed sequence reads.

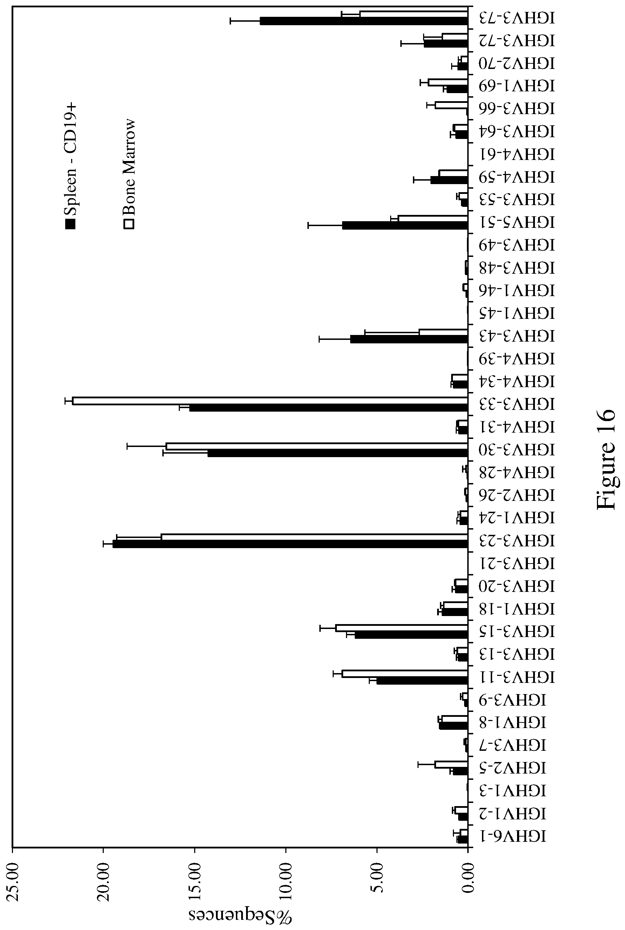

**Figure 16** shows representative percent usage of human V_{H} gene segments in amplified RNA from spleen and bone marrow (combined all V_{H}-families, not reflective of quantitative V_{H} usage) of three 6579ho/1293ho mice ("TX-D_{H} ho", *supra*). The x-axis indicates the name of each human V_{H} gene segment within the humanized heavy chain variable region.

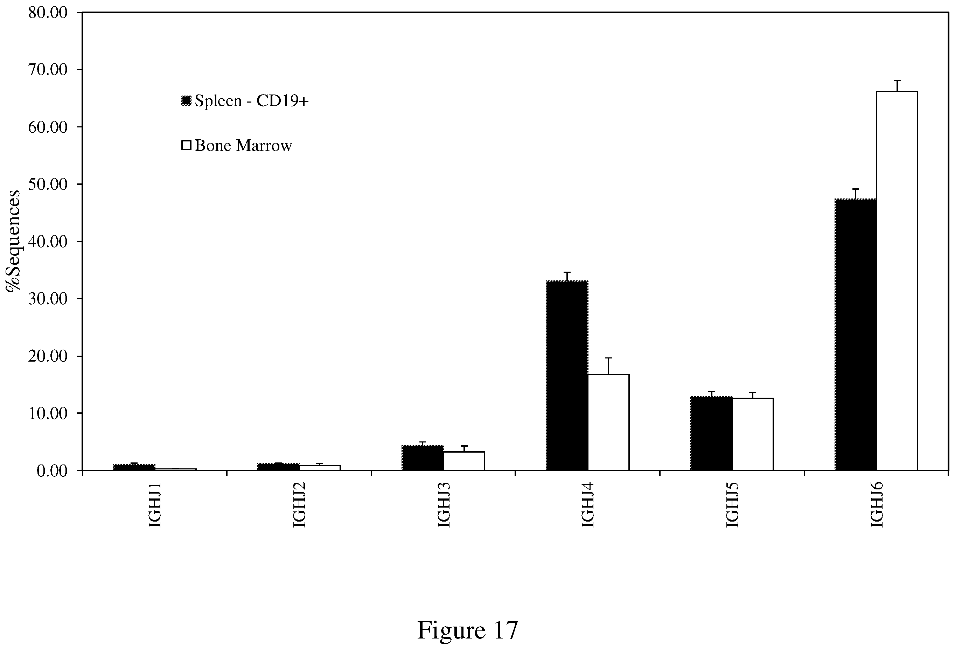

**Figure 17** shows representative percent usage of human J_{H} gene segments in amplified RNA from spleen and bone marrow (combined all V_{H}-families, not reflective of quantitative J_{H} usage) of three 6579ho/1293ho mice ("TX-D_{H} ho", *supra*). The x-axis indicates the name of each human J_{H} gene segment within the humanized heavy chain variable region.

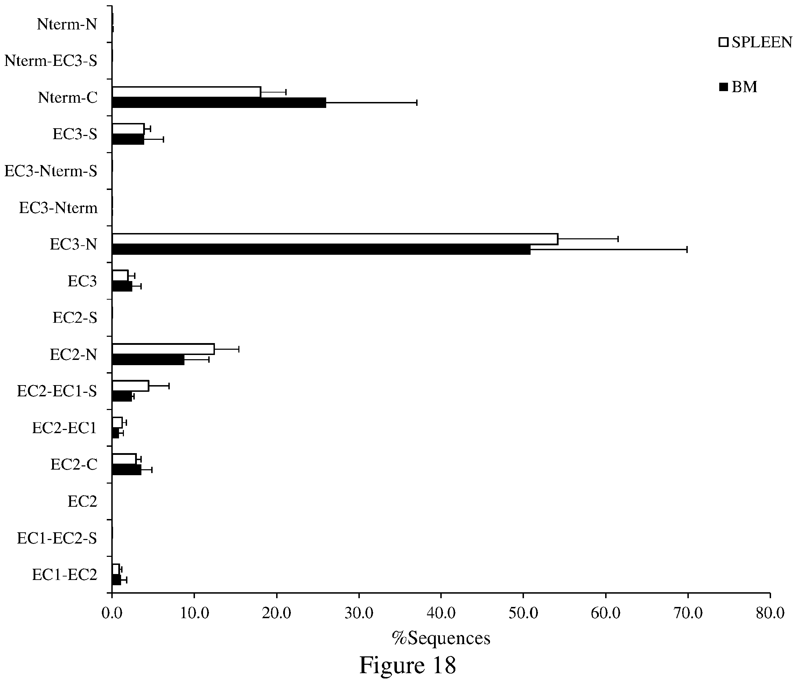

**Figure 18** shows representative usage frequency of selected D6 coding sequences in an engineered D_{H} region in amplified RNA from spleen and bone marrow (combined all V_{H}-families, not reflective of quantitative V_{H} usage) of three 6590hetmice ("D6-D_{H} het", *supra*). The y-axis indicates the name of selected D6 coding sequences within the engineered D_{H} region. The x-axis indicates the frequency (percentage of sequences) of D6 coding sequences among analyzed sequence reads. BM: bone marrow.

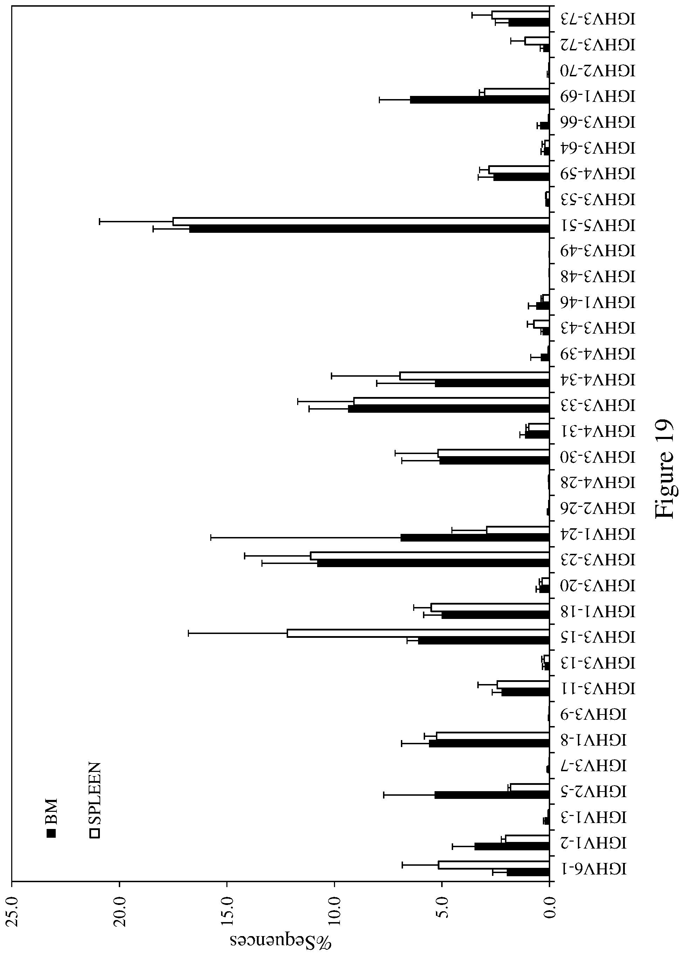

**Figure 19** shows representative percent usage of human V_{H} gene segments in amplified RNA from spleen and bone marrow (combined all V_{H}-families, not reflective of quantitative V_{H} usage) of three 6590het mice ("D6-D_{H} het", *supra*). The x-axis indicates the name of each human V_{H} gene segment within the humanized heavy chain variable region. BM: bone marrow.

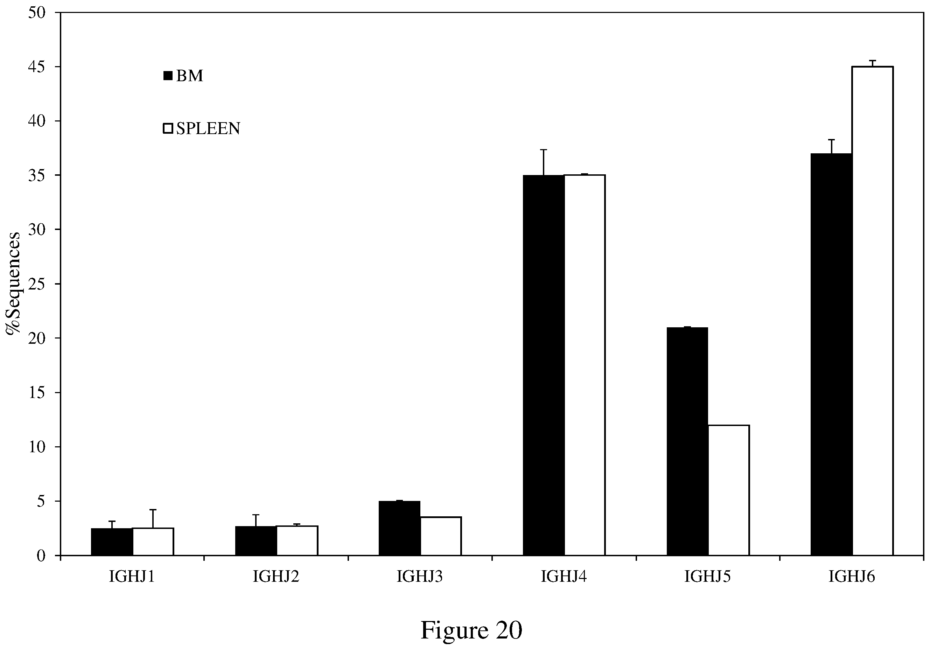

**Figure 20** shows representative percent usage of human J_{H} gene segments in amplified RNA from spleen and bone marrow (combined all V_{H}-families, not reflective of quantitative J_{H} usage) of three 6590het mice ("D6-D_{H} het", *supra*). The x-axis indicates the name of each human J_{H} gene segment within the humanized heavy chain variable region. BM: bone marrow.

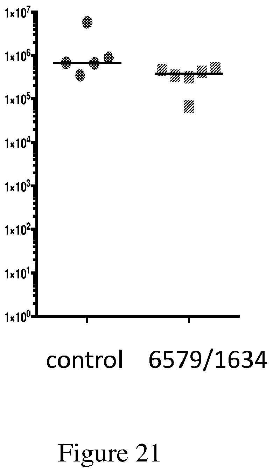

**Figure 21** shows the titer above background (y-axis) from control and 6579HO/1634 animals (x-axis) after immunization with engineered soluble form of a cell surface protein.

### DEFINITIONS

Those skilled in the art, reading the present disclosure, will be aware of various modifications that may be equivalent to such described embodiments, or otherwise within the scope of the instant disclosure. In general, terminology used herein is in accordance with its understood meaning in the art, unless clearly indicated otherwise. Explicit definitions of certain terms are provided herein and below; meanings of these and other terms in particular instances throughout this specification will be clear to those skilled in the art from context. Additional definitions for the following terms and other terms are set forth throughout the specification. References cited within this specification, or relevant portions thereof, are incorporated herein by reference.

***Administration***: refers to the administration of a composition to a subject or system (e.g., to a cell, organ, tissue, organism, or relevant component or set of components thereof). Those of ordinary skill will appreciate that route of administration may vary depending, for example, on the subject or system to which the composition is being administered, the nature of the composition, the purpose of the administration, etc. For example, in certain embodiments, administration to an animal subject (e.g., to a human or a rodent) may be bronchial (including by bronchial instillation), buccal, enteral, interdermal, intra-arterial, intradermal, intragastric, intramedullary, intramuscular, intranasal, intraperitoneal, intrathecal, intravenous, intraventricular, mucosal, nasal, oral, rectal, subcutaneous, sublingual, topical, tracheal (including by intratracheal instillation), transdermal, vaginal and/or vitreal. In some embodiments, administration may involve intermittent dosing. In some embodiments, administration may involve continuous dosing (e.g., perfusion) for at least a selected period of time.

The term "antibody" includes typical immunoglobulin molecules comprising four polypeptide chains, two heavy (H) chains (each of which may comprise an amino acid sequence encoded by an engineered D_{H} cluster) and two light (L) chains (each of which may be a common light chain) inter-connected by disulfide bonds. The term also includes an immunoglobulin that is reactive to an antigen or fragment thereof. Suitable antibodies include, but are not limited to, human antibodies, primatized antibodies, chimeric antibodies, monoclonal antibodies, monospecific antibodies, polyclonal antibodies, poly specific antibodies, nonspecific antibodies, bispecific antibodies, multispecific antibodies, humanized antibodies, synthetic antibodies, recombinant antibodies, hybrid antibodies, mutated antibodies, grafted conjugated antibodies (i.e., antibodies conjugated or fused to other proteins, radiolabels, cytotoxins), and in vitro-generated antibodies. A skilled artisan will readily recognize common antibody isotypes, e.g., antibodies having a heavy chain constant region selected from the group consisting of IgG, IgA, IgM, IgD, and IgE, and any subclass thereof (e.g., IgG1, IgG2, IgG3, and IgG4).

***Approximately***: As applied to one or more values of interest, refers to a value that is similar to a stated reference value. In certain embodiments, the term "*approximately*" or "*about*" refers to a range of values that fall within 25%, 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of the stated reference value unless otherwise stated or otherwise evident from the context (except where such number would exceed 100% of a possible value).

***Biologically active*:** refers to a characteristic of any agent that has activity in a biological system, *in vitro* or *in vivo* (e.g., in an organism). For instance, an agent that, when present in an organism, has a biological effect within that organism is considered to be biologically active. In particular embodiments, where a protein or polypeptide is biologically active, a portion of that protein or polypeptide that shares at least one biological activity of the protein or polypeptide is typically referred to as a "*biologically active*" portion.

***Comparable*:** refers to two or more agents, entities, situations, sets of conditions, etc. that may not be identical to one another but that are sufficiently similar to permit comparison there between so that conclusions may reasonably be drawn based on differences or similarities observed. Those of ordinary skill in the art will understand, in context, what degree of identity is required in any given circumstance for two or more such agents, entities, situations, sets of conditions, etc. to be considered comparable.

The phrase "complementarity determining region," or the term "CDR," includes an amino acid sequence encoded by a nucleic acid sequence of an organism's immunoglobulin genes that normally (i.e., in a wild-type animal) appears between two framework regions in a variable region of a light or a heavy chain of an immunoglobulin molecule (e.g., an antibody or a T cell receptor). A CDR can be encoded by, for example, a germline sequence or a rearranged or unrearranged sequence, and, for example, by a naive or a mature B cell or a T cell. A CDR can be somatically mutated (e.g., vary from a sequence encoded in an animal's germline), humanized, and/or modified with amino acid substitutions, additions, or deletions. In some circumstances (e.g., for a CDR3), CDRs can be encoded by two or more sequences (e.g., germline sequences) that are not contiguous (e.g., in an unrearranged nucleic acid sequence) but are contiguous in a B cell nucleic acid sequence, e.g., as the result of splicing or connecting the sequences (e.g., V-D-J recombination to form a heavy chain CDR3).

***Conservative***: in reference to a conservative amino acid substitution, refers to substitution of an amino acid residue by another amino acid residue having a side chain R group with similar chemical properties (e.g., charge or hydrophobicity). In general, a conservative amino acid substitution will not substantially change the functional properties of interest of a protein, for example, the ability of a receptor to bind to a ligand. Examples of groups of amino acids that have side chains with similar chemical properties include: aliphatic side chains such as glycine, alanine, valine, leucine, and isoleucine; aliphatic-hydroxyl side chains such as serine and threonine; amide-containing side chains such as asparagine and glutamine; aromatic side chains such as phenylalanine, tyrosine, and tryptophan; basic side chains such as lysine, arginine, and histidine; acidic side chains such as aspartic acid and glutamic acid; and sulfur-containing side chains such as cysteine and methionine. Conservative amino acids substitution groups include, for example, valine/leucine/isoleucine, phenylalanine/tyrosine, lysine/arginine, alanine/valine, glutamate/aspartate, and asparagine/glutamine. In some embodiments, a conservative amino acid substitution can be a substitution of any native residue in a protein with alanine, as used in, for example, alanine scanning mutagenesis. In some embodiments, a conservative substitution is made that has a positive value in the PAM250 log-likelihood matrix disclosed in Gonnet, G. H. et al., 1992, Science 256:1443-1445, hereby incorporated by reference. In some embodiments, a substitution is a moderately conservative substitution wherein the substitution has a nonnegative value in the PAM250 log-likelihood matrix.

***Control***: refers to the art-understood meaning of a "*control*" being a standard against which results are compared. Typically, controls are used to augment integrity in experiments by isolating variables in order to make a conclusion about such variables. In some embodiments, a control is a reaction or assay that is performed simultaneously with a test reaction or assay to provide a comparator. A "*control*" may refer to a "*control animal*." A "*control animal*" may have a modification as described herein, a modification that is different as described herein, or no modification (i.e., a wild-type animal). In one experiment, a "*test*" (i.e., a variable being tested) is applied. In a second experiment, the "*control*," the variable being tested is not applied. In some embodiments, a control is a historical control (i.e., of a test or assay performed previously, or an amount or result that is previously known). In some embodiments, a control is or comprises a printed or otherwise saved record. A control may be a positive control or a negative control.

***Disruption***: refers to the result of a homologous recombination event with a DNA molecule (e.g., with an endogenous homologous sequence such as a gene or gene locus). In some embodiments, a disruption may achieve or represent an insertion, deletion, substitution, replacement, missense mutation, or a frame-shift of a DNA sequence(s), or any combination thereof. Insertions may include the insertion of entire genes, fragments of genes, e.g., exons, which may be of an origin other than the endogenous sequence (e.g., a heterologous sequence), or coding sequences derived or isolated from a particular gene of interest. In some embodiments, a disruption may increase expression and/or activity of a gene or gene product (e.g., of a protein encoded by a gene). In some embodiments, a disruption may decrease expression and/or activity of a gene or gene product. In some embodiments, a disruption may alter sequence of a gene or an encoded gene product (e.g., an encoded protein). In some embodiments, a disruption may truncate or fragment a gene or an encoded gene product (e.g., an encoded protein). In some embodiments, a disruption may extend a gene or an encoded gene product. In some such embodiments, a disruption may achieve assembly of a fusion protein. In some embodiments, a disruption may affect level, but not activity, of a gene or gene product. In some embodiments, a disruption may affect activity, but not level, of a gene or gene product. In some embodiments, a disruption may have no significant effect on level of a gene or gene product. In some embodiments, a disruption may have no significant effect on activity of a gene or gene product. In some embodiments, a disruption may have no significant effect on either level or activity of a gene or gene product.

***Determining*, *measuring*, *evaluating*, *assessing*, *assaying*** and ***analyzing***: Are used interchangeably to refer to any form of measurement, and include determining if an element is present or not. These terms include both quantitative and/or qualitative determinations. Assaying may be relative or absolute. "*Assaying for the presence of*" can be determining the amount of something present and/or determining whether or not it is present or absent.

***Endogenous locus* or *endogenous gene***: refers to a genetic locus found in a parent or reference organism prior to introduction of an alteration, disruption, deletion, insertion, modification, substitution or replacement as described herein. In some embodiments, the endogenous locus comprises a sequence, in whole or in part, found in nature. In some embodiments, the endogenous locus is a wild-type locus. In some embodiments, a reference organism is a wild-type organism. In some embodiments, a reference organism is an engineered organism. In some embodiments, a reference organism is a laboratory-bred organism (whether wild-type or engineered).

***Endogenous promoter***: refers to a promoter that is naturally associated, e.g., in a wild-type organism, with an endogenous gene.

***Engineered:*** refers, in general, to the aspect of having been manipulated by the hand of man. For example, in some embodiments, a polynucleotide may be considered to be "*engineered*" when two or more sequences that are not linked together in that order in nature are manipulated by the hand of man to be directly linked to one another in the engineered polynucleotide. In some particular such embodiments, an engineered polynucleotide may comprise a regulatory sequence that is found in nature in operative association with a first coding sequence but not in operative association with a second coding sequence, is linked by the hand of man so that it is operatively associated with the second coding sequence. Alternatively, or additionally, in some embodiments, first and second nucleic acid sequences that each encodes polypeptide elements or domains that in nature are not linked to one another may be linked to one another in a single engineered polynucleotide. Comparably, in some embodiments, a cell or organism may be considered to be "*engineered*" if it has been manipulated so that its genetic information is altered (e.g., new genetic material not previously present has been introduced, or previously present genetic material has been altered or removed). As is common practice and is understood by those in the art, progeny of an engineered polynucleotide or cell are typically still referred to as "*engineered*" even though the actual manipulation was performed on a prior entity. Furthermore, as will be appreciated by those skilled in the art, a variety of methodologies are available through which "*engineering*" as described herein may be achieved. For example, in some embodiments, "*engineering*" may involve selection or design (e.g., of nucleic acid sequences, polypeptide sequences, cells, tissues, and/or organisms) through use of computer systems programmed to perform analysis or comparison, or otherwise to analyze, recommend, and/or select sequences, alterations, etc.). Alternatively, or additionally, in some embodiments, "*engineering*" may involve use of *in vitro* chemical synthesis methodologies and/or recombinant nucleic acid technologies such as, for example, for example, nucleic acid amplification (e.g., via the polymerase chain reaction) hybridization, mutation, transformation, transfection, etc., and/or any of a variety of controlled mating methodologies. As will be appreciated by those skilled in the art, a variety of established such techniques (e.g., for recombinant DNA, oligonucleotide synthesis, and tissue culture and transformation (e.g., electroporation, lipofection, etc.) are well known in the art and described in various general and more specific references that are cited and/or discussed throughout the present specification. See e.g., Sambrook et al., Molecular Cloning: A Laboratory Manual (2d ed., Cold Spring Harbor Laboratory Press, Cold Spring Harbor, N.Y., 1989).

***Gene***: refers to a DNA sequence in a chromosome that codes for a product (e.g., an RNA product and/or a polypeptide product). In some embodiments, a gene includes coding sequence (i.e., sequence that encodes a particular product). In some embodiments, a gene includes non-coding sequence. In some particular embodiments, a gene may include both coding (e.g., exonic) and non-coding (e.g., intronic) sequence. In some embodiments, a gene may include one or more regulatory sequences (*e.g*., promoters, enhancers, *etc*.) and/or intron sequences that, for example, may control or impact one or more aspects of gene expression (e.g., cell-type-specific expression, inducible expression, etc.). For the purpose of clarity we note that, as used in the present application, the term "*gene*" generally refers to a portion of a nucleic acid that encodes a polypeptide; the term may optionally encompass regulatory sequences, as will be clear from context to those of ordinary skill in the art. This definition is not intended to exclude application of the term "*gene*" to non-protein-coding expression units but rather to clarify that, in most cases, the term as used in this document refers to a polypeptide-coding nucleic acid.

The phrase "***gene segment***," or "***segment***" includes reference to a V (light or heavy) or D or J (light or heavy) immunoglobulin gene segment, which includes unrearranged sequences at immunoglobulin loci (in *e.g.*, humans and mice) that can participate in a rearrangement (mediated by, *e.g.,* endogenous recombinases) to form a rearranged V/J (light) or V/D/J (heavy) sequence. Unless indicated otherwise, the V, D, and J segments comprise recombination signal sequences (RSS) that allow for V/J recombination or V/D/J recombination according to the 12/23 rule. Unless indicated otherwise, the segments further comprise sequences with which they are associated in nature or functional equivalents thereof (e.g., for V segments, promoter(s) and leader(s)).

The term "***germline***" in reference to an immunoglobulin nucleic acid sequence includes a nucleic acid sequence that can be passed to progeny, e.g., the germline genome that may be found in a germ cell.

The phrase "***heavy chain***," or "***immunoglobulin heavy chain***" includes an immunoglobulin heavy chain sequence, including immunoglobulin heavy chain constant region sequence, from any organism. Heavy chain variable domains include three heavy chain complementarity determining regions (CDRs) and four FR regions, unless otherwise specified. Fragments of heavy chains include CDRs, CDRs and FRs, and combinations thereof. A typical heavy chain has, following the variable domain (from N-terminal to C-terminal), a C_{H}1 domain, a hinge, a C_{H}2 domain, a C_{H}3 domain, and a C_{H}4 domain (in the context of IgM or IgE). A functional fragment of a heavy chain includes a fragment that is capable of specifically recognizing an epitope (e.g., recognizing the epitope with a KD in the micromolar, nanomolar, or picomolar range), that is capable of expressing and secreting from a cell, and that comprises at least one CDR. A heavy chain variable domain is encoded by a variable region gene sequence, which generally comprises V_{H}, D_{H}, and J_{H} segments derived from a repertoire of V_{H}, D_{H}, and J_{H} segments present in the germline. Sequences, locations and nomenclature for V, D, and J heavy chain segments for various organisms can be viewed at the website of the International Immunogenetics Information System (IMGT) found at www.imgt.org.

The phrase "***light chain***" includes an immunoglobulin light chain sequence from any organism, and unless otherwise specified includes human kappa and lambda light chains and a VpreB, as well as surrogate light chains. Light chain variable domains typically include three light chain complementarity determining regions (CDRs) and four framework (FR) regions, unless otherwise specified. Generally, a full-length light chain includes, from amino terminus to carboxyl terminus, a variable domain that includes FR1-CDR1-FR2-CDR2-FR3-CDR3-FR4, and a light chain constant region. A light chain variable domain is encoded by a light chain variable region gene sequence, which generally comprises V_{L} and J_{L} gene segments, derived from a repertoire of V_{L} and J_{L} gene segments present in the germline. Sequences, locations and nomenclature for V and J light chain segments for various organisms can be viewed at the website of the International Immunogenetics Information System (IMGT) found at www.imgt.org. Light chains include those, e.g., that do not selectively bind either a first or a second epitope selectively bound by the epitope-binding protein in which they appear. Light chains also include those that bind and recognize, or assist the heavy chain with binding and recognizing, one or more epitopes selectively bound by the epitope-binding protein in which they appear. The phrase light chain includes a "***common light chain***," also referred to as a "***universal light chain***" (ULC).

Common or universal light chains (ULCs) include those derived from an immunoglobulin light chain locus comprising a single rearranged immunoglobulin light chain variable region encoding sequence operably linked with a light chain constant region, wherein expression of the immunoglobulin light chain locus produces only a light chain derived from the single rearranged immunoglobulin light chain variable region operably linked to the light chain constant region regardless of the inclusion of other nucleic acid sequences, e.g., other light chain gene segments, in the immunoglobulin light chain locus. Universal light chains include human Vκ1-39Jκ gene (e.g., Vκ1-39Jκ5 gene) or a human Vκ3-20Jκ gene (e.g., Vκ3-20Jκ1 gene), and include somatically mutated (e.g., affinity matured) versions of the same.

***Heterologous***: refers to an agent or entity from a different source. For example, when used in reference to a polypeptide, gene, or gene product present in a particular cell or organism, the term clarifies that the relevant polypeptide or fragment thereof, gene or fragment thereof, or gene product or fragment thereof: 1) was engineered by the hand of man; 2) was introduced into the cell or organism (or a precursor thereof) through the hand of man (e.g., via genetic engineering); and/or 3) is not naturally produced by or present in the relevant cell or organism (e.g., the relevant cell type or organism type). As used herein, the term "*heterologous*" also includes a polypeptide or fragment thereof, gene or fragment thereof, or gene product or fragment thereof that is normally present in a particular native cell or organism, but has been modified, for example, by mutation or placement under the control of non-naturally associated and, in some embodiments, non-endogenous regulatory elements (e.g., a promoter).

***Host cell***: refers to a cell into which a heterologous (e.g., exogenous) nucleic acid or protein has been introduced. Persons of skill upon reading this disclosure will understand that such terms refer not only to the particular subject cell, but also is used to refer to the progeny of such a cell. Because certain modifications may occur in succeeding generations due to either mutation or environmental influences, such progeny may not, in fact, be identical to the parent cell, but are still included within the scope of the term "*host cell*" as used herein. In some embodiments, a host cell is or comprises a prokaryotic or eukaryotic cell. In general, a host cell is any cell that is suitable for receiving and/or producing a heterologous nucleic acid or protein, regardless of the Kingdom of life to which the cell is designated. Exemplary cells include those of prokaryotes and eukaryotes (single-cell or multiple-cell), bacterial cells (e.g., strains of *Escherichia coli*, *Bacillus* spp., *Streptomyces* spp., etc.), mycobacteria cells, fungal cells, yeast cells (e.g., *Saccharomyces cerevisiae, Schizosaccharomyces pombe*, *Pichia pastoris*, *Pichia methanolica*, etc.), plant cells, insect cells (e.g., SF-9, SF-21, baculovirus-infected insect cells, *Trichoplusia ni*, etc.), non-human animal cells, human cells, or cell fusions such as, for example, hybridomas or quadromas. In some embodiments, the cell is a human, monkey, ape, hamster, rat, or mouse cell. In some embodiments, the cell is eukaryotic and is selected from the following cells: CHO (e.g., CHO K1, DXB-11 CHO, Veggie-CHO), COS (e.g., COS-7), retinal cell, Vero, CV1, kidney (e.g., HEK293, 293 EBNA, MSR 293, MDCK, HaK, BHK), HeLa, HepG2, WI38, MRC 5, Colo205, HB 8065, HL-60, (e.g., BHK21), Jurkat, Daudi, A431 (epidermal), CV-1, U937, 3T3, L cell, C127 cell, SP2/0, NS-0, MMT 060562, Sertoli cell, BRL 3A cell, HT1080 cell, myeloma cell, tumor cell, and a cell line derived from an aforementioned cell. In some embodiments, the cell comprises one or more viral genes, e.g., a retinal cell that expresses a viral gene (e.g., a PER.C6^{®} cell). In some embodiments, a host cell is or comprises an isolated cell. In some embodiments, a host cell is part of a tissue. In some embodiments, a host cell is part of an organism.

***Identity***: used in connection with a comparison of sequences, refers to identity as determined by a number of different algorithms known in the art that can be used to measure nucleotide and/or amino acid sequence identity. In some embodiments, identities as described herein are determined using a ClustalW v. 1.83 (slow) alignment employing an open gap penalty of 10.0, an extend gap penalty of 0.1, and using a Gonnet similarity matrix (MACVECTOR^{™} 10.0.2, Mac Vector Inc., 2008).

***In vitro***: refers to events that occur in an artificial environment, e.g., in a test tube or reaction vessel, in cell culture, etc., rather than within a multi-cellular organism.

***In vivo***: refers to events that occur within a multi-cellular organism, such as a human and a non-human animal. In the context of cell-based systems, the term may be used to refer to events that occur within a living cell (as opposed to, for example, *in vitro* systems).

***Isolated***: refers to a substance and/or entity that has been (1) separated from at least some of the components with which it was associated when initially produced (whether in nature and/or in an experimental setting), and/or (2) designed, produced, prepared, and/or manufactured by the hand of man. Isolated substances and/or entities may be separated from about 10%, about 20%, about 30%, about 40%, about 50%, about 60%, about 70%, about 80%, about 90%, about 91%, about 92%, about 93%, about 94%, about 95%, about 96%, about 97%, about 98%, about 99%, or more than about 99% of the other components with which they were initially associated. In some embodiments, isolated agents are about 80%, about 85%, about 90%, about 91%, about 92%, about 93%, about 94%, about 95%, about 96%, about 97%, about 98%, about 99%, or more than about 99% pure. As used herein, a substance is "*pure*" if it is substantially free of other components. In some embodiments, as will be understood by those skilled in the art, a substance may still be considered *"isolated"* or even "*pure*", after having been combined with certain other components such as, for example, one or more carriers or excipients (e.g., buffer, solvent, water, etc.); in such embodiments, percent isolation or purity of the substance is calculated without including such carriers or excipients. To give but one example, in some embodiments, a biological polymer such as a polypeptide or polynucleotide that occurs in nature is considered to be *"isolated"* when: a) by virtue of its origin or source of derivation is not associated with some or all of the components that accompany it in its native state in nature; b) it is substantially free of other polypeptides or nucleic acids of the same species from the species that produces it in nature; or c) is expressed by or is otherwise in association with components from a cell or other expression system that is not of the species that produces it in nature. Thus, for instance, in some embodiments, a polypeptide that is chemically synthesized or is synthesized in a cellular system different from that which produces it in nature is considered to be an *"isolated"* polypeptide. Alternatively or additionally, in some embodiments, a polypeptide that has been subjected to one or more purification techniques may be considered to be an *"isolated"* polypeptide to the extent that it has been separated from other components: a) with which it is associated in nature; and/or b) with which it was associated when initially produced.

***Non-human animal***: refers to any vertebrate organism that is not a human. In some embodiments, a non-human animal is a cyclostome, a bony fish, a cartilaginous fish (e.g., a shark or a ray), an amphibian, a reptile, a mammal, and a bird. In some embodiments, a non-human mammal is a primate, a goat, a sheep, a pig, a dog, a cow, or a rodent. In some embodiments, a non-human animal is a rodent such as a rat or a mouse.

***Nucleic acid***: in its broadest sense, refers to any compound and/or substance that is or can be incorporated into an oligonucleotide chain. In some embodiments, a *"nucleic acid"* is a compound and/or substance that is or can be incorporated into an oligonucleotide chain via a phosphodiester linkage. As will be clear from context, in some embodiments, *"nucleic acid"* refers to individual nucleic acid residues (e.g., nucleotides and/or nucleosides); in some embodiments, *"nucleic acid"* refers to an oligonucleotide chain comprising individual nucleic acid residues. In some embodiments, a *"nucleic acid"* is or comprises RNA; in some embodiments, a *"nucleic acid"* is or comprises DNA. In some embodiments, a *"nucleic acid"* is, comprises, or consists of one or more natural nucleic acid residues. In some embodiments, a *"nucleic acid"* is, comprises, or consists of one or more nucleic acid analogs. In some embodiments, a nucleic acid analog differs from a "*nucleic acid*" in that it does not utilize a phosphodiester backbone. For example, in some embodiments, a "*nucleic acid*" is, comprises, or consists of one or more "*peptide nucleic acids*", which are known in the art and have peptide bonds instead of phosphodiester bonds in the backbone. Alternatively, or additionally, in some embodiments, a *"nucleic acid"* has one or more phosphorothioate and/or 5'-N-phosphoramidite linkages rather than phosphodiester bonds. In some embodiments, a "*nucleic acid*" is, comprises, or consists of one or more natural nucleosides (e.g., adenosine, thymidine, guanosine, cytidine, uridine, deoxyadenosine, deoxythymidine, deoxyguanosine, and deoxycytidine). In some embodiments, a "*nucleic acid*" is, comprises, or consists of one or more nucleoside analogs (e.g., 2-aminoadenosine, 2-thiothymidine, inosine, pyrrolo-pyrimidine, 3-methyl adenosine, 5-methylcytidine, C-5 propynyl-cytidine, C-5 propynyl-uridine, 2-aminoadenosine, C5-bromouridine, C5-fluorouridine, C5-iodouridine, C5-propynyl-uridine, C5-propynyl-cytidine, C5-methylcytidine, 2-aminoadenosine, 7-deazaadenosine, 7-deazaguanosine, 8-oxoadenosine, 8-oxoguanosine, O(6)-methylguanine, 2-thiocytidine, methylated bases, intercalated bases, and combinations thereof). In some embodiments, a "*nucleic acid*" comprises one or more modified sugars (e.g., 2'-fluororibose, ribose, 2'-deoxyribose, arabinose, and hexose) as compared with those in natural nucleic acids. In some embodiments, a "*nucleic acid*" has a nucleotide sequence that encodes a functional gene product such as an RNA or protein. In some embodiments, a "*nucleic acid*" has a nucleotide sequence that encodes polypeptide fragment (e.g., a peptide). In some embodiments, a "*nucleic acid*" includes one or more introns. In some embodiments, a "*nucleic acid*" includes one or more exons. In some embodiments, a "*nucleic acid*" includes one or more coding sequences. In some embodiments, a "*nucleic acid*" is prepared by one or more of isolation from a natural source, enzymatic synthesis by polymerization based on a complementary template (*in vivo* or *in vitro*), reproduction in a recombinant cell or system, and chemical synthesis. In some embodiments, a "*nucleic acid*" is at least 3, 4, 5, 6, 7, 8, 9, 10, 15, 20, 25, 30, 35, 40, 45, 50, 55, 60, 65, 70, 75, 80, 85, 90, 95, 100, 110, 120, 130, 140, 150, 160, 170, 180, 190, 20, 225, 250, 275, 300, 325, 350, 375, 400, 425, 450, 475, 500, 600, 700, 800, 900, 1000, 1500, 2000, 2500, 3000, 3500, 4000, 4500, 5000 or more residues long. In some embodiments, a *"nucleic acid"* is single stranded; in some embodiments, a "*nucleic acid*" is double stranded. In some embodiments, a "*nucleic acid*" has a nucleotide sequence comprising at least one element that encodes, or is the complement of a sequence that encodes, a polypeptide or fragment thereof. In some embodiments, a "*nucleic acid*" has enzymatic activity.

***Operably linked***: refers to a juxtaposition wherein the components described are in a relationship permitting them to function in their intended manner. A control sequence "*operably linked*" to a coding sequence is ligated in such a way that expression of the coding sequence is achieved under conditions compatible with the control sequences. "*Operably linked*" sequences include both expression control sequences that are contiguous with the gene of interest and expression control sequences that act in trans or at a distance to control the gene of interest. The term "*expression control sequence*", as used herein, refers to polynucleotide sequences, which are necessary to affect the expression and processing of coding sequences to which they are ligated. "*Expression control sequences*" include: appropriate transcription initiation, termination, promoter and enhancer sequences; efficient RNA processing signals such as splicing and polyadenylation signals; sequences that stabilize cytoplasmic mRNA; sequences that enhance translation efficiency (i.e., Kozak consensus sequence); sequences that enhance protein stability; and when desired, sequences that enhance protein secretion. The nature of such control sequences differs depending upon the host organism. For example, in prokaryotes, such control sequences generally include promoter, ribosomal binding site and transcription termination sequence, while in eukaryotes typically, such control sequences include promoters and transcription termination sequence. The term "*control sequences*" is intended to include components whose presence is essential for expression and processing, and can also include additional components whose presence is advantageous, for example, leader sequences and fusion partner sequences.

***Physiological conditions***: includes its art-understood meaning referencing conditions under which cells or organisms live and/or reproduce. In some embodiments, the term refers to conditions of the external or internal milieu that may occur in nature for an organism or cell system. In some embodiments, physiological conditions are those conditions present within the body of a human or non-human animal, especially those conditions present at and/or within a surgical site. Physiological conditions typically include, e.g., a temperature range of 20-40°C, atmospheric pressure of 1, pH of 6-8, glucose concentration of 1-20 mM, oxygen concentration at atmospheric levels, and gravity as it is encountered on earth. In some embodiments, conditions in a laboratory are manipulated and/or maintained at physiological conditions. In some embodiments, physiological conditions are encountered in an organism (e.g., non-human animal).

***Polypeptide***: refers to any polymeric chain of amino acids. In some embodiments, a polypeptide has an amino acid sequence that occurs in nature. In some embodiments, a polypeptide has an amino acid sequence that does not occur in nature. In some embodiments, a polypeptide has an amino acid sequence that contains portions that occur in nature separately from one another (i.e., from two or more different organisms, for example, human and non-human portions). In some embodiments, a polypeptide has an amino acid sequence that is engineered in that it is designed and/or produced through action of the hand of man. In some embodiments, a polypeptide may comprise or consist of a plurality of fragments, each of which is found in the same parent polypeptide in a different spatial arrangement relative to one another than is found in the polypeptide of interest (e.g., fragments that are directly linked in the parent may be spatially separated in the polypeptide of interest or vice versa, and/or fragments may be present in a different order in the polypeptide of interest than in the parent), so that the polypeptide of interest is a derivative of its parent polypeptide.

***Recombinant***: refers to polypeptides that are designed, engineered, prepared, expressed, created or isolated by recombinant means, such as polypeptides expressed using a recombinant expression vector transfected into a host cell, polypeptides isolated from a recombinant, combinatorial human polypeptide library (Hoogenboom H. R., 1997 TIB Tech. 15:62-70; Hoogenboom H., and Chames P., 2000, Immunology Today 21:371-378; Azzazy H., and Highsmith W. E., 2002, Clin. Biochem. 35:425-445; Gavilondo J. V., and Larrick J. W., 2002, BioTechniques 29:128-145), antibodies isolated from an animal (e.g., a mouse) that is transgenic for human immunoglobulin genes (see e.g., Taylor, L. D., et al., 1992, Nucl. Acids Res. 20:6287-6295; Little M. et al., 2000, Immunology Today 21:364-370; Kellermann S. A. and Green L. L., 2002, Current Opinion in Biotechnology 13:593-597; Murphy, A.J., et al., 2014, Proc. Natl. Acad. Sci. U. S. A. 111(14):5153-5158) or polypeptides prepared, expressed, created or isolated by any other means that involves splicing selected sequence elements to one another. In some embodiments, one or more of such selected sequence elements is found in nature. In some embodiments, one or more of such selected sequence elements is designed *in silico.* In some embodiments, one or more such selected sequence elements result from mutagenesis (e.g., *in vivo* or *in vitro*) of a known sequence element, e.g., from a natural or synthetic source. For example, in some embodiments, a recombinant polypeptide comprises sequences found in the genome (or polypeptide) of a source organism of interest (e.g., human, mouse, etc.). In some embodiments, a recombinant polypeptide comprises sequences that occur in nature separately from one another (i.e., from two or more different organisms, for example, human and non-human portions) in two different organisms (e.g., a human and a non-human organism). In some embodiments, a recombinant polypeptide has an amino acid sequence that resulted from mutagenesis (e.g., *in vitro* or *in vivo*, for example in a non-human animal), so that the amino acid sequences of the recombinant polypeptides are sequences that, while originating from and related to polypeptide sequences, may not naturally exist within the genome of a non-human animal *in vivo*.

***Reference***: is intended to describe a standard or control agent, animal, cohort, individual, population, sample, sequence or value against which an agent, animal, cohort, individual, population, sample, sequence or value of interest is compared. In some embodiments, a reference agent, animal, cohort, individual, population, sample, sequence or value is tested and/or determined substantially simultaneously with the testing or determination of the agent, animal, cohort, individual, population, sample, sequence or value of interest. In some embodiments, a reference agent, animal, cohort, individual, population, sample, sequence or value is a historical reference, optionally embodied in a tangible medium. In some embodiments, a reference may refer to a control. As used herein, a "*reference*" may refer to a "*reference animal*". A "*reference animal*" may have a modification as described herein, a modification that is different as described herein or no modification (i.e., a wild-type animal). Typically, as would be understood by those skilled in the art, a reference agent, animal, cohort, individual, population, sample, sequence or value is determined or characterized under conditions comparable to those utilized to determine or characterize the agent, animal (e.g., a mammal), cohort, individual, population, sample, sequence or value of interest.

Immunoglobulins participate in a cellular mechanism, termed somatic hypermutation, which produces affinity-matured antibody variants characterized by high affinity to their target. Although somatic hypermutation largely occurs within the CDRs of antibody variable regions, mutations are preferentially targeted to certain sequence motifs that are referred to as hot spots, e.g., RGYW activation-induced cytidine deaminase (AID) hotspots (see, e.g., Li, Z. et al., 2004, Genes Dev. 18:1-11; Teng, G. and F.N. Papavasiliou, 2007, Annu. Rev. Genet. 41:107-20; hereby incorporated by reference). The non-immunoglobulin peptides of interest, or portion thereof, disclosed herein useful for the generation of an engineered D_{H} region may comprise one or more natural and/or artificial hotspots. The phrase "**somatically mutated**" includes reference to a nucleic acid sequence from a B cell that has undergone class-switching, wherein the nucleic acid sequence of an immunoglobulin variable region (e.g., nucleotide sequence encoding a heavy chain variable domain or including a heavy chain CDR or FR sequence) in the class-switched B cell is not identical to the nucleic acid sequence in the B cell prior to class-switching, such as, for example, a difference in a CDR or framework nucleic acid sequence between a B cell that has not undergone class-switching and a B cell that has undergone class-switching. "Somatically mutated" includes reference to nucleic acid sequences from affinity-matured B cells that are not identical to corresponding immunoglobulin variable region sequences in B cells that are not affinity-matured (i.e., sequences in the genome of germline cells). The phrase "somatically mutated" also includes reference to an immunoglobulin variable region nucleic acid sequence from a B cell after exposure of the B cell to an epitope of interest, wherein the nucleic acid sequence differs from the corresponding nucleic acid sequence prior to exposure of the B cell to the epitope of interest. The phrase "somatically mutated" refers to sequences from binding proteins that have been generated in an animal, e.g., a mouse having human immunoglobulin variable region nucleic acid sequences, in response to an immunogen challenge, and that result from the selection processes inherently operative in such an animal.

***Substantially***: refers to the qualitative condition of exhibiting total or near-total extent or degree of a characteristic or property of interest. One of ordinary skill in the biological arts will understand that biological and chemical phenomena rarely, if ever, go to completion and/or proceed to completeness or achieve or avoid an absolute result. The term "*substantially*" is therefore used herein to capture the potential lack of completeness inherent in many biological and chemical phenomena.

***Substantial homology***: refers to a comparison between amino acid or nucleic acid sequences. As will be appreciated by those of ordinary skill in the art, two sequences are generally considered to be "*substantially homologous*" if they contain homologous residues in corresponding positions. Homologous residues may be identical residues. Alternatively, homologous residues may be non-identical residues with appropriately similar structural and/or functional characteristics. For example, as is well known by those of ordinary skill in the art, certain amino acids are typically classified as "*hydrophobic*" or "*hydrophilic*" amino acids, and/or as having "*polar*" or "*non-polar*" side chains. Substitution of one amino acid for another of the same type may often be considered a "*homologous*" substitution. Typical amino acid categorizations are summarized below.

| | | | | | |
|---|---|---|---|---|---|
| Alanine | Ala | A | Nonpolar | Neutral | 1.8 |
| Arginine | Arg | R | Polar | Positive | -4.5 |
| Asparagine | Asn | N | Polar | Neutral | -3.5 |
| Aspartic acid | Asp | D | Polar | Negative | -3.5 |
| Cysteine | Cys | C | Nonpolar | Neutral | 2.5 |
| Glutamic acid | Glu | E | Polar | Negative | -3.5 |
| Glutamine | Gln | Q | Polar | Neutral | -3.5 |
| Glycine | Gly | G | Nonpolar | Neutral | -0.4 |
| Histidine | His | H | Polar | Positive | -3.2 |
| Isoleucine | Ile | I | Nonpolar | Neutral | 4.5 |
| Leucine | Leu | L | Nonpolar | Neutral | 3.8 |
| Lysine | Lys | K | Polar | Positive | -3.9 |
| Methionine | Met | M | Nonpolar | Neutral | 1.9 |
| Phenylalanin e | Phe | F | Nonpolar | Neutral | 2.8 |
| Proline | Pro | P | Nonpolar | Neutral | -1.6 |
| Serine | Ser | S | Polar | Neutral | -0.8 |
| Threonine | Thr | T | Polar | Neutral | -0.7 |
| Tryptophan | Trp | W | Nonpolar | Neutral | -0.9 |
| Tyrosine | Tyr | Y | Polar | Neutral | -1.3 |
| Valine | Val | V | Nonpolar | Neutral | 4.2 |

| Ambiguous Amino Acids | 3-Letter | 1-Letter |
|---|---|---|
| Asparagine or aspartic acid | Asx | B |
| Glutamine or glutamic acid | Glx | Z |
| Leucine or Isoleucine | Xle | J |
| Unspecified or unknown amino acid | Xaa | X |

As is well known in this art, amino acid or nucleic acid sequences may be compared using any of a variety of algorithms, including those available in commercial computer programs such as BLASTN for nucleotide sequences and BLASTP, gapped BLAST, and PSI-BLAST for amino acid sequences. Exemplary such programs are described in Altschul, S. F. et al., 1990, J. Mol. Biol., 215(3): 403-410; Altschul, S. F. et al., 1997, Methods in Enzymology; Altschul, S. F. et al., 1997, Nucleic Acids Res., 25:3389-3402; Baxevanis, A.D., and B. F. F. Ouellette (eds.) Bioinformatics: A Practical Guide to the Analysis of Genes and Proteins, Wiley, 1998; and Misener et al. (eds.) Bioinformatics Methods and Protocols (Methods in Molecular Biology, Vol. 132), Humana Press, 1998. In addition to identifying homologous sequences, the programs mentioned above typically provide an indication of the degree of homology. In some embodiments, two sequences are considered to be substantially homologous if at least 50%, 55%, 60%, 65%, 70%, 75%, 80%, 85%, 90%, 91%, 92%, 93%, 94%, 95%, 96%, 97%, 98%, 99% or more of their corresponding residues are homologous over a relevant stretch of residues. In some embodiments, the relevant stretch is a complete sequence. In some embodiments, the relevant stretch is at least 9, 10, 11, 12, 13, 14, 15, 16, 17 or more residues. In some embodiments, the relevant stretch includes contiguous residues along a complete sequence. In some embodiments, the relevant stretch includes discontinuous residues along a complete sequence, for example, noncontiguous residues brought together by the folded conformation of a polypeptide or a portion thereof. In some embodiments, the relevant stretch is at least 10, 15, 20, 25, 30, 35, 40, 45, 50, or more residues.

***Substantial identity***: refers to a comparison between amino acid or nucleic acid sequences. As will be appreciated by those of ordinary skill in the art, two sequences are generally considered to be "*substantially identical*" if they contain identical residues in corresponding positions. As is well known in this art, amino acid or nucleic acid sequences may be compared using any of a variety of algorithms, including those available in commercial computer programs such as BLASTN for nucleotide sequences and BLASTP, gapped BLAST, and PSI-BLAST for amino acid sequences. Exemplary such programs are described in Altschul, S. F. et al., 1990, J. Mol. Biol., 215(3): 403-410; Altschul, S. F. et al., 1997, Methods in Enzymology; Altschul, S. F. et al., 1997, Nucleic Acids Res., 25:3389-3402; Baxevanis, A.D., and B. F. F. Ouellette (eds.) Bioinformatics: A Practical Guide to the Analysis of Genes and Proteins, Wiley, 1998; and Misener et al. (eds.) Bioinformatics Methods and Protocols (Methods in Molecular Biology, Vol. 132), Humana Press, 1998. In addition to identifying identical sequences, the programs mentioned above typically provide an indication of the degree of identity. In some embodiments, two sequences are considered to be substantially identical if at least 50%, 55%, 60%, 65%, 70%, 75%, 80%, 85%, 90%, 91%, 92%, 93%, 94%, 95%, 96%, 97%, 98%, 99% or more of their corresponding residues are identical over a relevant stretch of residues. In some embodiments, the relevant stretch is a complete sequence. In some embodiments, the relevant stretch is at least 10, 15, 20, 25, 30, 35, 40, 45, 50, or more residues.

***Transformation***: refers to any process by which exogenous DNA is introduced into a host cell. Transformation may occur under natural or artificial conditions using various methods well known in the art. Transformation may rely on any known method for the insertion of foreign nucleic acid sequences into a prokaryotic or eukaryotic host cell. In some embodiments, a particular transformation methodology is selected based on the host cell being transformed and may include, but is not limited to, viral infection, electroporation, mating, lipofection. In some embodiments, a "*transformed*" cell is stably transformed in that the inserted DNA is capable of replication either as an autonomously replicating plasmid or as part of the host chromosome. In some embodiments, a transformed cell transiently expresses introduced nucleic acid for limited periods of time.

***Targeting vector*** or ***targeting construct***: refers to a polynucleotide molecule that comprises a targeting region. A targeting region comprises a sequence that is identical or substantially identical to a sequence in a target cell, tissue or animal and provides for integration of the targeting construct into a position within the genome of the cell, tissue or animal via homologous recombination. Targeting regions that target using site-specific recombinase recognition sites (e.g., *lox*P or Frt sites) are also included. In some embodiments, a targeting construct as described herein further comprises a nucleic acid sequence or gene of particular interest, a selectable marker, control and or regulatory sequences, and other nucleic acid sequences that allow for recombination mediated through exogenous addition of proteins that aid in or facilitate recombination involving such sequences. In some embodiments, a targeting construct further comprises a gene of interest in whole or in part, wherein the gene of interest is a heterologous gene that encodes a polypeptide, in whole or in part, that has a similar function as a protein encoded by an endogenous sequence. In some embodiments, a targeting construct further comprises a humanized gene of interest, in whole or in part, wherein the humanized gene of interest encodes a polypeptide, in whole or in part, that has a similar function as a polypeptide encoded by an endogenous sequence. In some embodiments, a targeting construct (or targeting vector) may comprise a nucleic acid sequence manipulated by the hand of man. For example, in some embodiments, a targeting construct (or targeting vector) may be constructed to contain an engineered or recombinant polynucleotide that contains two or more sequences that are not linked together in that order in nature yet manipulated by the hand of man to be directly linked to one another in the engineered or recombinant polynucleotide.

***Transgene*** or ***transgene construct***: refers to a nucleic acid sequence (encoding e.g., a polypeptide of interest, in whole or in part) that has been introduced into a cell by the hand of man such as by the methods described herein. A transgene could be partly or entirely heterologous, i.e., foreign, to the transgenic animal or cell into which it is introduced. A transgene can include one or more transcriptional regulatory sequences and any other nucleic acid, such as introns or promoters, which may be necessary for expression of a selected nucleic acid sequence.

***Transgenic animal*, *transgenic non-human animal*** or ***Tg*⁺**: may be used interchangeably and refer to any non-naturally occurring non-human animal in which one or more of the cells of the non-human animal contain heterologous nucleic acid and/or gene encoding a polypeptide of interest, in whole or in part. In some embodiments, a heterologous nucleic acid and/or gene is introduced into the cell, directly or indirectly by introduction into a precursor cell, by way of deliberate genetic manipulation, such as by microinjection or by infection with a recombinant virus. The term genetic manipulation does not include classic breeding techniques, but rather is directed to introduction of recombinant DNA molecule(s). This molecule may be integrated within a chromosome, or it may be extrachromosomally replicating DNA. The term "*Tg*⁺" includes animals that are heterozygous or homozygous for a heterologous nucleic acid and/or gene, and/or animals that have single or multi-copies of a heterologous nucleic acid and/or gene.

***Variant***: refers to an entity that shows significant structural identity with a reference entity, but differs structurally from the reference entity in the presence or level of one or more chemical moieties as compared with the reference entity. In many embodiments, a "*variant*" also differs functionally from its reference entity. In general, whether a particular entity is properly considered to be a "*variant*" of a reference entity is based on its degree of structural identity with the reference entity. As will be appreciated by those skilled in the art, any biological or chemical reference entity has certain characteristic structural elements. A "*variant*", by definition, is a distinct chemical entity that shares one or more such characteristic structural elements. To give but a few examples, a small molecule may have a characteristic core structural element (e.g., a macrocycle core) and/or one or more characteristic pendent moieties so that a variant of the small molecule is one that shares the core structural element and the characteristic pendent moieties but differs in other pendent moieties and/or in types of bonds present (single vs. double, E vs. Z, etc.) within the core, a polypeptide may have a characteristic sequence element comprised of a plurality of amino acids having designated positions relative to one another in linear or three-dimensional space and/or contributing to a particular biological function, a nucleic acid may have a characteristic sequence element comprised of a plurality of nucleotide residues having designated positions relative to on another in linear or three-dimensional space. For example, a "*variant polypeptide*" may differ from a reference polypeptide as a result of one or more differences in amino acid sequence and/or one or more differences in chemical moieties (e.g., carbohydrates, lipids, etc.) covalently attached to the polypeptide backbone. In some embodiments, a "*variant polypeptide*" shows an overall sequence identity with a reference polypeptide that is at least 85%, 86%, 87%, 88%, 89%, 90%, 91%, 92%, 93%, 94%, 95%, 96%, 97%, or 99%. Alternatively or additionally, in some embodiments, a "*variant polypeptide*" does not share at least one characteristic sequence element with a reference polypeptide. In some embodiments, the reference polypeptide has one or more biological activities. In some embodiments, a "*variant polypeptide*" shares one or more of the biological activities of the reference polypeptide. In some embodiments, a "*variant polypeptide*" lacks one or more of the biological activities of the reference polypeptide. In some embodiments, a "*variant polypeptide*" shows a reduced level of one or more biological activities as compared with the reference polypeptide. In many embodiments, a polypeptide of interest is considered to be a "*variant*" of a parent or reference polypeptide if the polypeptide of interest has an amino acid sequence that is identical to that of the parent but for a small number of sequence alterations at particular positions. Typically, fewer than 20%, 15%, 10%, 9%, 8%, 7%, 6%, 5%, 4%, 3%, or 2% of the residues in the variant are substituted as compared with the parent. In some embodiments, a "*variant*" has 10, 9, 8, 7, 6, 5, 4, 3, 2, or 1 substituted residue(s) as compared with a parent. Often, a "*variant*" has a very small number (e.g., fewer than 5, 4, 3, 2, or 1) number of substituted functional residues (i.e., residues that participate in a particular biological activity). Furthermore, a "*variant*" typically has not more than 5, 4, 3, 2, or 1 additions or deletions, and often has no additions or deletions, as compared with the parent. Moreover, any additions or deletions are typically fewer than about 25, about 20, about 19, about 18, about 17, about 16, about 15, about 14, about 13, about 10, about 9, about 8, about 7, about 6, and commonly are fewer than about 5, about 4, about 3, or about 2 residues. In some embodiments, the parent or reference polypeptide is one found in nature. As will be understood by those of ordinary skill in the art, a plurality of variants of a particular polypeptide of interest may commonly be found in nature, particularly when the polypeptide of interest is an infectious agent polypeptide.

***Vector***: refers to a nucleic acid molecule capable of transporting another nucleic acid to which it is associated. In some embodiment, vectors are capable of extra-chromosomal replication and/or expression of nucleic acids to which they are linked in a host cell such as a eukaryotic and/or prokaryotic cell. Vectors capable of directing the expression of operably linked genes are referred to herein as "*expression vectors*."

***Wild-type***: includes its art-understood meaning that refers to an entity having a structure and/or activity as found in nature in a "*normal*" (as contrasted with mutant, diseased, altered, etc.) state or context. Those of ordinary skill in the art will appreciate that wild-type genes and polypeptides often exist in multiple different forms (e.g., alleles).

### DETAILED DESCRIPTION OF CERTAIN EMBODIMENTS

Disclosed herein are, among other things, transgenic non-human animals having heterologous genetic material encoding one or more portions (functional fragments, binding portions, etc.) of a polypeptide of interest, which heterologous genetic material is inserted into the diversity cluster (i.e., D_{H} region) of an immunoglobulin heavy chain variable region so that the heterologous genetic material is operably linked with heavy chain variable (V_{H}) and joining (J_{H}) segments. It is contemplated that such non-human animals demonstrate a capacity to generate antibodies to intractable disease targets. It is also contemplated that such non-human animals demonstrate an antibody population characterized by heavy chain variable regions having an increase in CDR3 diversity as compared to an antibody population having immunoglobulin heavy chain variable CDR3 diversity generated from traditional immunoglobulin D_{H} gene segments (or immunoglobulin D_{H} gene segments that appear in nature). Therefore, the non-human animals described herein may be useful for the development of antibody-based therapeutics that bind particular antigens, in particular, antigens associated with low and/or poor immunogenicity. In particular, disclosed herein is the introduction of exemplary nucleotide coding sequences that each encode a portion of a polypeptide of interest (e.g., an extracellular portion of an atypical chemokine receptor, a portion of a conotoxin, or a portion of a tarantula toxin) into the D_{H} region of an immunoglobulin heavy chain variable region resulting in expression of antibodies having heavy chain variable regions and, in particular, CDR3 regions, generated from V(D)J recombination involving an inserted nucleotide coding sequence. In some embodiments, inserted nucleotide coding sequences of a polypeptide of interest (e.g., an atypical chemokine receptor (ACKR), conotoxin, tarantula toxin, or combinations thereof) replace all or substantially all traditional D_{H} segments (i.e., wild-type D_{H} segments) within an immunoglobulin heavy chain diversity cluster (i.e., D_{H} region) as described herein. In some embodiments, inserted nucleotide coding sequences of a polypeptide of interest (e.g., an atypical chemokine receptor (ACKR), conotoxin, tarantula toxin, or combinations thereof) partially replace one or more traditional D_{H} segments within an immunoglobulin heavy chain diversity cluster (i.e., D_{H} region) as described herein. In some embodiments, nucleotide coding sequences of a polypeptide of interest (e.g., an atypical chemokine receptor (ACKR), conotoxin, tarantula toxin, or combinations thereof) are inserted into one or more traditional D_{H} segments within an immunoglobulin heavy chain diversity cluster (i.e., D_{H} region) as described herein, so that said nucleotide coding sequences are flanked by sequences that are normally or naturally found in or associated with the one or more traditional D_{H} segments. In some embodiments, one or more traditional D_{H} segments remain intact within an immunoglobulin heavy chain diversity cluster as described herein. In some embodiments, one or more traditional D_{H} segments are deleted, removed or otherwise rendered non-functional from an immunoglobulin heavy chain diversity cluster as described herein. In some certain embodiments, an immunoglobulin heavy chain diversity cluster as described herein lacks all or substantially all traditional D_{H} segments. In some certain embodiments, an immunoglobulin heavy chain diversity cluster as described herein comprises synthetic D_{H} segments made or generated using nucleotide coding sequences described herein. Such transgenic non-human animals provide an *in vivo* system for identifying and developing antibodies and/or antibody-based therapeutics that bind disease targets beyond the targeting capabilities of established drug discovery technologies. Further, such transgenic non-human animals provide a useful animal model system for the development of antibodies and/or antibody-based therapeutics centered on or designed for disrupting protein-protein interactions that are central to various diseases and/or disease pathologies that affect humans.

In some embodiments, non-human animals described herein comprise an immunoglobulin heavy chain variable region containing an engineered diversity cluster (i.e., an engineered D_{H} region) characterized by the presence of one or more nucleotide coding sequences corresponding to a portion(s) of a polypeptide of interest such as, for example, an extracellular domain of an ACKR (e.g., a D6 chemokine decoy receptor), a toxin (e.g., an ion channel blocker such as, for example, a conotoxin, spider toxin, tarantula toxin, sea anemone toxin, or scorpion toxin), a G-protein-coupled receptor, long heavy chain CDRs of selected antibodies (e.g., neutralizing antibodies that bind viruses including, for example, HIV, HCV, HPV, influenza, etc.), glucagon-like peptide-1 receptor agonists (e.g., exenatide, liraglutide, lixisenatide, albiglutide, dulaglutide, taspoglutide, etc.), heavy chain diversity (D_{H}) gene segments from a non-human species (e.g., bird, chicken, cow, rabbit, swine, etc.), etc. In such embodiments, antibodies containing CDR3s generated from recombination involving such nucleotide coding sequences can be characterized as having increased diversity to direct binding to particular antigens (e.g., membrane-spanning polypeptides). In some embodiments, antibodies produced by non-human animals described herein have an immunoglobulin heavy chain variable region sequence that contains a CDR3 region corresponding to a peptide encoded by the one or more nucleotide coding sequences. In some embodiments, non-human animals described herein comprise heavy chain variable (V_{H}) and joining (J_{H}) gene segments operably linked with the one or more nucleotide coding sequences so that V(D)J recombination occurs between said V_{H}, J_{H} and one or more nucleotide coding sequences to create a heavy chain variable region that binds an antigen of interest. In some embodiments, non-human animals described herein comprise a plurality of V_{H} and J_{H} gene segments operably linked to 5, 10, 15, 20, 25 or more (e.g., a plurality) nucleotide coding sequences at an immunoglobulin heavy chain variable region in the genome of the non-human animal. In many embodiments, V_{H} and J_{H} segments are human V_{H} and human J_{H} gene segments. In some embodiments, non-human animals described herein further comprise a human or humanized immunoglobulin light chain locus (e.g., κ and/or λ) such that the non-human animals produce antibodies comprising human variable regions (i.e., heavy and light) and non-human constant regions. In some certain embodiments, said human or humanized immunoglobulin light chain locus comprises human V_{L} and J_{L} gene segments operably linked to a rodent light chain constant region (e.g., a rodent Cκ or Cλ). In some embodiments, non-human animals described herein further comprise an immunoglobulin light chain locus as described in U.S. Patent Application Publication Nos. 2011-0195454 A1, 2012-0021409 A1, 2012-0192300 A1, 2013-0045492 A1, 2013-0185821 A1, 2013-0198880 A1, 2013-0302836 A1, 2015-0059009 A1; International Patent Application Publication Nos. WO 2011/097603, WO 2012/148873, WO 2013/134263, WO 2013/184761, WO 2014/160179, WO 2014/160202; all of which are hereby incorporated by reference).

Various aspects of the compositions and methods are described in detail in the following sections. The use of sections is not meant to limit any embodiment. Each section can apply to any embodiment specifically described. In this application, the use of "or" means "and/or" unless stated otherwise.

### V(D)J Recombination

A series of recombination events, involving several genetic components, serves to assemble immunoglobulins from ordered arrangement of gene segments (e.g., V, D and J). This assembly of gene segments is known to be imprecise and, therefore, immunoglobulin diversity is achieved both by combination of different gene segments and formation of unique junctions through imprecise joining. Further diversity is generated through a process known as somatic hypermutation in which the variable region sequence of immunoglobulins is altered to increase affinity and specificity for antigen. The immunoglobulin is a Y-shaped polypeptide composed of two identical heavy and two identical light chains, each of which have two structural components: one variable domain and one constant domain. It is the variable domains of heavy and light chains that are formed by the assembly of gene segments, while constant domains are fused to variable domains through RNA splicing. Although the mechanism of assembling (or joining) gene segments is similar for heavy and light chains, only one joining event is required for light chains (i.e., V to J) while two are required for heavy chains (i.e., D to J and V to DJ).

The assembly of gene segments for heavy and light chain variable regions is guided by conserved noncoding DNA sequences that flank each gene segment, termed recombination signal sequences (RSSs), which ensure DNA rearrangements at precise locations relative to V, D and J coding sequences (see, e.g., Ramsden, D.A. et al., 1994, Nuc. Acids Res. 22(10):1785-96). Each RSS consists of a conserved block of seven nucleotides (heptamer) that is contiguous with a coding sequence (e.g., a V segment) followed by a conserved spacer (either 12 or 23bp) and a second conserved block of nine nucleotides (nonamer). Although considerable sequence divergence among individuals is tolerated, the length of these sequences typically does not vary. Recombination between immunoglobulin gene segments follows a rule commonly referred to as the 12/23 rule, in which gene segments flanked by an RSS with a 12bp spacer are typically joined to a gene segment flanked by a 23bp spacer (see, e.g., Hiom, K. and M. Gellert, 1998, Mol. Cell. 1(7): 1011-9). The sequence of an RSS has been reported to influence the efficiency and/or the frequency of recombination with a particular gene segment (see, e.g., Ramsden, D.A and G.E. Wu, 1991, Proc. Natl. Acad. Sci. U.S.A. 88:10721-5; Boubnov, N.V. et al., 1995, Nuc. Acids Res. 23:1060-7; Ezekiel, U.R. et al., 1995, Immunity 2:381-9; Sadofsky, M. et al., 1995, Genes Dev. 9:2193-9; Cuomo, C.A. et al., 1996, Mol. Cell Biol. 16:5683-90; Ramsden, D.A. et al., 1996, EMBO J 15:3197-3206). Indeed, many reports point to a highly biased and variable usage of gene segments, in particular, D_{H} segments, among individuals.

In some embodiments, non-human animals described herein comprise one or more RSSs flanking coding sequences that is or are optimized for recombination to a V and/or a J gene segment. In various embodiments, coding sequences are inserted into an immunoglobulin heavy chain locus in the place of (or within) traditional D_{H} gene segments (or D_{H} gene segments that appear in nature). Optimization of RSSs may be achieved using standard techniques known in the art such as, for example, site-directed mutagenesis of known RSS sequences or *in silico* generation of synthetic RSS sequences followed by *de novo* synthesis. To give but one example, an RSS that is associated with low or poor recombination efficiency and/or frequency may be optimized by comparison to an RSS that is associated with a high or optimal recombination efficiency and/or frequency. Recombination efficiency and/or frequency may be determined, in some embodiments, by usage frequencies of gene segments in a population of antibody sequences (e.g., from an individual or group of individuals; see e.g., Arnaout, R. et al., 2011, PLoS One 6(8):e22365; Glanville, J. et al., 2011, Proc. Natl. Acad. Sci. U.S.A. 108(50):20066-71). Thus, non-human animals described herein may, in some embodiments, comprise one or more optimized RSSs flanking coding sequences (or gene segments) so that recombination of coding sequences (or gene segments) occurs at equal or about equal frequencies. Exemplary optimized RSSs are set forth in Figure 2.

Assembly of gene segments to form heavy and light chain variable regions results in the formation of antigen-binding regions (or sites) of immunoglobulins. Such antigen-binding regions are characterized, in part, by the presence of hypervariable regions, which are commonly referred to as complementary determining regions (CDRs). There are three CDRs for both heavy and light chains (i.e., for a total of six CDRs) with both CDR1 and CDR2 being entirely encoded by the V gene segment. CDR3, however, is encoded by the sequence resulting from the joining of the V and J segments for light chains, and the V, D and J segments for heavy chains. Thus, the additional gene segment employed during recombination to form a heavy chain variable region coding sequence significantly increases the diversity of the antigen-binding sites of heavy chains.

### Chemokines and chemokine receptors

Immune and inflammatory responses are complex biological processes involving several types of immune cells and molecular components. Migration of leukocytes has been reported as an important factor in the initiation, maintenance and resolution of immune and inflammatory responses, some of which is achieved through the action of chemokines and their receptors. Indeed, several chemokines and corresponding receptors have been reported. Chemokine receptors have a structure characterized by a seven-transmembrane domain that couples to a G-protein for signal transduction in the intracellular compartment and are divided into multiple different families corresponding to the subsets of chemokines they bind: CC-chemokine receptors (β-chemokine receptors), CXC-chemokine receptors, CX3C-chemokine receptors and XC-chemokine receptors. In addition to these traditional chemokine receptors, other chemokine receptors (termed atypical chemokine receptors or ACKRs) that share a similar structure yet lacking the ability to initiate signaling in response to ligand binding have been reported (see, e.g., Bonecchi, R. et al., 2010, Curr. Top. Microbiol. Immunol. 341:15-36; Nibbs, R.J.B. and G.J. Graham, 2013, Nature Rev. 13:815-29).

Among ACKRs, ACKR2 (also known as CCBP2 and D6 chemokine decoy receptor) is a seven-transmembrane protein receptor similar to other G protein-coupled receptors and encoded by the *CCBP2* gene (Nibbs, R.J.B. et al., 1997, J. Biol. Chem. 272(51):32078-83). In contrast to other chemokine receptors, ACKR2 contains a DKYLEIV motif in the second intracellular loop in place of the canonical DRYLAIV motif and is incapable of signaling through Gαᵢ proteins. ACKR2 is expressed on lymphatic endothelial cells of skin, gut and lung, as well as on B cells and dendritic cells. ACKR is a promiscuous receptor for many pro-inflammatory β-chemokines (CC chemokines), but does not bind constitutive CCL chemokines. ACKR2 has been suggested to be a scavenger receptor that internalizes CC chemokines and targets them for lysosomal degradation thereby limiting inflammatory responses via clearance of CC chemokines (Jamieson, T. et al., 2005, Nature Immunol. 6(4):403-11; Bonecchi, R. et al., *supra*; Hansell, C.A.H. et al., 2011, Immunol. Cell Biol. 89(2):197-206; Nibbs, R.J.B. and G.J. Graham, *supra*).

### Peptide Toxins

Toxins are naturally occurring substances found in plants and animals that can be poisonous to humans. For example, cone snails produce neurotoxic peptides, called conotoxins, in their venom that modulate the activity of various receptors in humans. In particular, several conotoxins have been shown to modulate ion channels (e.g., Na_{V} channels). Typically, conotoxins are peptides 10 to 30 amino acids in length that include one or more disulfide bonds, and are characterized based on the target upon which they act: α-conotoxins (acetylcholine receptors), δ-conotoxins (voltage-gated sodium channels), κ-conotoxins (potassium channels), µ-conotoxins (voltage-gated sodium channels) and ω-conotoxins (voltage-gated calcium channels). Conotoxins are known to be highly polymorphic among species of snails and, as a result, the genes that encode them are not conserved (see, for example, Terlau, H. and B.M. Olivera, 2004, Physiol. Rev. 84(1):41-68; Biggs, J.S. et al., 2010, Mol. Phylogenet. Evol. 56(1):1-12; Olivera, B.M. et al., 2012, Ann. N.Y. Acad. Sci. 1267(1):61-70; Wong, E.S. and K. Belov, 2012, Gene 496(1):1-7).

Among conotoxins, µ-conotoxins have been reported to have two types of cysteine patterns and act on voltage-gated sodium channels in muscle tissue (Cruz, L.J. et al., 1985, J. Biol. Chem. 260(16):9280-8; Zeikus, R.D. et al., 1985, J. Biol. Chem. 260(16):9280-8; McIntosh, J.M. and R.M. Jones, 2001, Toxicon. 39(10):1447-51; Nielsen, K.J. et al., 2002, J. Biol. Chem. 277(30):27247-55; Floresca, C.Z., 2003, Toxicol. Appl. Pharmacol. 190(2):95-101; Priest, B.T. et al., 2007, Toxicon. 49(2):194-201; Schmalhofer, W.A. et al., 2008, Mol. Pharmacol. 74(5):1476-84; Ekberg, J. et al., 2008, Int. J. Biochem. Cell Biol. 40(11):2363-8). Indeed, µ-conotoxins have been the subject of investigation for their potential pharmacological use (see, e.g., Olivera, B.M. and R.W. Teichert, 2007, Mol. Interv. 7(5):251-60; Stevens, M. et al., 2012, J. Biol. Chem. 287(37):31382-92). Although, several studies have been conducted to elucidate the mechanism of action of various µ-conotoxins, much remains unknown.

Other examples of toxins include the venom of sea anemones, scorpions, spiders and tarantulas, which have been reported to act on ion channels by inhibiting activation and blocking neuronal transmission. Indeed, several scorpion toxins have been identified and their structures solved (see, e.g., Rochat, H. and J. Gregoire, 1983, Toxicon. 21(1):153-62; Zhou, X.H. et al., 1989, Biochem. J. 257(2):509-17; Granier, C. et al., 1990, FEBS Lett. 261(2):423-6). Further, neurotoxin-based libraries have been developed for potassium channels using toxins from scorpion venom (see, e.g., Takacs, Z. et al., 2009, Proc. Natl. Acad. Sci. U.S.A. 106(52):22211-6). To give yet another example, peptides from tarantula venom have been reported to specifically act on voltage-gated sodium channels such as Na_{V}1.5 and Na_{V}1.7 (see, e.g., Priest, B.T. et al., 2007, Toxicon. 49(2):194-201; Xiao, Y. et al., 2010, Mol. Pharmacol. 78(6):1124-34). Described herein is the finding that a particularly useful set of nucleotide coding sequences for construction an engineered D_{H} region is or comprises nucleotide coding sequences from tarantula toxin and µ-conotoxin sequences (see, e.g., Table 4). Disclosed herein is the use of toxin coding sequences that act on voltage-gated sodium channels (e.g., Na_{V}1.7) for construction of an engineered D_{H} region. The methods described herein can be employed to utilize any set of coding sequences derived from any desired toxin peptide(s), or combination of toxin peptides (or toxin peptide sequence fragments) from multiple (i.e., two, three, four, five, etc.) toxin peptides as desired.

### Provided in vivo systems

Described herein is recognition that particular antigens are associated with low and/or poor immunogenicity and, therefore, are poor targets for antibody-based therapeutics. Indeed, many disease targets (e.g., membrane-spanning proteins) have been characterized as intractable or undruggable. Thus, disclosed herein is the creation of an *in vivo* system for the development of antibodies and antibody-based therapeutics that overcome deficiencies associated with established drug discovery technologies. The present disclosure specifically demonstrates the construction of a transgenic rodent whose genome comprises an immunoglobulin heavy chain variable region that includes an engineered D_{H} region, which engineered D_{H} region includes one or more heterologous nucleotide coding sequences that each encode a portion (e.g., an extracellular portion, binding portion, functional fragment, etc.) of a heterologous polypeptide or peptide of interest. The methods described herein can be adapted to employ a set of heterologous nucleotide coding sequences that each encode a portion of any polypeptide or peptide of interest (e.g., a membrane-spanning protein, a toxin, a G-protein-coupled receptor, etc.) for creating an engineered D_{H} region. The engineered D_{H} region, once integrated into an immunoglobulin heavy chain variable region (i.e., placed in operable linkage with V and J gene segments and/or one or more constant regions), provides for recombination of gene segments (i.e., V and J) with the one or more heterologous nucleotide coding sequences to generate antibodies characterized by heavy chains having added diversity (i.e., CDR3 diversity) to direct binding to particular antigens.

Described herein is the recognition that particularly useful heterologous polypeptides of interest from which to design a set of nucleotide coding sequences (or combinations of nucleotide coding sequences) for constructing an engineered D_{H} region as described herein include chemokine receptors, conotoxins, tarantula toxins and/or combinations thereof.

In some embodiments, chemokine receptors include CC-chemokine receptors, CXC-chemokine receptors, CX3C-chemokine receptors, XC-chemokine receptors and combinations thereof.

In some embodiments, CC-chemokine receptors (also known as β-chemokine receptors) include CCR1, CCR2, CCR3, CCR4, CCR5, CCR6, CCR7, CCR8, CCR9, CCR10 and CCR11.

In some embodiments, CXC-chemokine receptors include CXCR1, CXCR2, CXCR3, CXCR4, CXCR5, CXCR6 and CXCR7.

In some embodiments, CX3C-chemokine receptors include CX3CR1.

In some embodiments, XC-chemokine receptors include XCR1.

In some embodiments, conotoxins include α-conotoxins, δ-conotoxins, κ-conotoxins, µ-conotoxins, ω-conotoxins and combinations thereof.

In some embodiments, conotoxins are or comprise µ-conotoxins.

In some embodiments, tarantula toxins include ProTxI, ProTxII, Huwentoxin-IV (HWTX-IV), and combinations thereof.

Without wishing to be bound by any particular theory, we note that data provided herein demonstrate that, in some embodiments, rodents whose genome comprises an immunoglobulin heavy chain variable locus that includes an engineered D_{H} region characterized by the inclusion of one or more heterologous nucleotide coding sequences derived from an extracellular portion of a heterologous atypical chemokine receptor (e.g., ACKR2, also known as D6 chemokine decoy receptor) effectively generate an immunoglobulin heavy chain variable region locus that produces antibodies characterized by CDR3s having added diversity to bind ligands of a heterologous atypical chemokine receptor (e.g., a heterologous D6 chemokine decoy receptor). We also note that data provided herein demonstrate that, in some embodiments, rodents whose genome comprises an immunoglobulin heavy chain variable locus that includes an engineered D_{H} region characterized by the inclusion of one or more heterologous nucleotide coding sequences derived from a portion of one or more toxins (e.g., µ-conotoxin and/or ProTxII) effectively generate an immunoglobulin heavy chain variable region locus that produces antibodies characterized by CDR3s having added diversity to bind a heterologous voltage-gated sodium channel (e.g., a heterologous Naᵥ channel).

In particular, the present disclosure specifically demonstrates, among other things, exemplary nucleotide coding sequences from a human atypical chemokine receptor (ACKR) such as, for example, ACKR2 (i.e., D6 chemokine decoy receptor) that are particularly useful for integration into a D_{H} region of an immunoglobulin heavy chain locus for the generation of antibodies characterized by CDR3s having diversity resulting from recombination of the nucleotide coding sequences with V_{H} and J_{H} segments, and that block several inflammatory cytokines (e.g., CCL2, CCL3, CCL4, CCL5, CCL7, CCL8, CCL11, CCL12, CCL13, CCL14, CCL17, CCL22 and CCL3L1). The present disclosure also demonstrates exemplary nucleotide coding sequences from a conotoxin (e.g., µ-conotoxin) and a tarantula toxin (e.g., ProTxII) that are particularly useful for integration into a D_{H} region of an immunoglobulin heavy chain locus for the generation of antibodies characterized by CDR3s having diversity resulting from recombination of the nucleotide coding sequences with V_{H} and J_{H} segments, and that block and/or inhibit the activation and/or function of a voltage-gated sodium channel(s) (e.g., Na_{V}1.7). Thus, the present disclosure, in at least some embodiments, embraces the development of an *in vivo* system for generating antibodies and/or antibody-based therapeutics to intractable disease targets.

Exemplary human ACKRs (along with their associated ligands) are set forth in Table 1 (see, e.g., Nibbs, R.J.B. and G.J. Graham, 2013, Nature Reviews 13:815-29). Exemplary toxins (along with their associated targets) are set forth in Table 2 (see, e.g., Terlau, H. and B.M. Olivera, 2004, Physiol. Rev. 84(1):41-68).

**TABLE 1**

| ACKR | Ligands |
|---|---|
| ACKR1 (also known as DARC) | CCL2, CCL5, CCL7, CCL11, CCL13, CCL14, CCL17, CXCL1, CXCL2, CXCL3, CXCL5, CXCL6, CXCL8, CXCL11 |
| ACKR2 (also known as D6) | CCL2, CCL3, CCL4, CCL5, CCL7, CCL8, CCL11, CCL12, CCL13, CCL14, CCL17, CCL22, CCL3L1 |
| ACKR3 (also known as CXCR7) | CXCL11, CXCL12 |
| ACKR4 (also known as CCRL1, CCX-CKR or CCR11) | CCL19, CCL21, CCL25 |

**TABLE 2**

| Toxin | Target |
|---|---|
| α-conotoxins | Acetylcholine receptors |
| δ-conotoxins | Voltage-gated sodium channels |
| κ-conotoxins | Potassium channels |
| µ-conotoxins | Voltage-gated sodium channels |
| ω-conotoxins | N-type voltage-dependent calcium channels |
| ProTxI | Selective Ca_{V}3.1 channel blocker; inhibits Na_{V}1 subtypes and K_{V}2.1 channels |
| ProTxII | Selective Na_{V}1.7 inhibitor |
| Huwentoxin-IV | Selective Na_{V}1.7 inhibitor |

### ACKR2 (D6 chemokine decoy receptor) coding sequences

Exemplary nucleotide coding sequences (DNA and amino acid (AA)) of a human ACKR2 (D6 chemokine decoy receptor) for construction of an engineered D_{H} region as described herein are set forth in Table 3. The set of human ACKR2 nucleotide coding sequences set forth in Table 3 are characterized by four extracellular domains, four extracellular domains with Cys to Ser substitutions to remove disulfide bonds, four Cys crossovers (Nterm-EC3, EC3-Nterm, EC1-EC2, EC2-EC1), four Cys crossovers with Cys to Ser substitutions to remove disulfide bonds, two loop fusions (with Cys retained; Nterm+EC3, EC1+EC2), and seven partial domains.

**TABLE 3**

| |
|---|
| **Nterm DNA** |
| ATGGCAGCTACTGCCAGCCCGCAGCCACTGGCTACTGAGGATGCCGATTCTGAGAATAGCAGCT TCTACTACTATGACTACCTGGATGAAGTAGCTTTCATGCTCTGCCGGAAGGATGCTGTGGTTAG CTTTGGCAAAGTTTTCCTGCCA (SEQ ID NO:1) |
| **Nterm** AA |
| MAATASPQPLATEDADSENSSFYYYDYLDEVAFMLCRKDAVVSFGKVFLP (SEQ ID NO:2) |
| **EC1 DNA** |
| AGCTTCTTGTGCAAG (SEQ ID NO:3) |
| **EC1 AA** |
| SFLCK (SEQ ID NO:4) |
| **EC2 DNA** |
| CAAACCCATGAAAACCCCAAGGGAGTTTGGAACTGCCATGCCGATTTCGGCGGGCATGGCACC ATTTGGAAGCTCTTCCTCCGGTTCCAGCAGAACCTGCTA (SEQ ID NO:5) |
| **EC2 AA** |
| QTHENPKGVWNCHADFGGHGTIWKLFLRFQQNLL (SEQ ID NO:6) |
| **EC3 DNA** |
| CTGCATACCCTGCTGGACCTGCAAGTATTCGGCAACTGTGAGGTTAGCCAGCATCTAGACTATG CC (SEQ ID NO:7) |
| **EC3 AA** |
| LHTLLDLQVFGNCEVSQHLDYA (SEQ ID NO:8) |
| **Nterm-S DNA** |
| ATGGCAGCTACTGCCAGCCCGCAGCCACTGGCTACTGAGGATGCCGATTCTGAGAATAGCAGCT TCTACTACTATGACTACCTGGATGAAGTAGCTTTCATGCTCAGCCGGAAGGATGCTGTGGTTAG CTTTGGCAAAGTTTTCCTGCCA (SEQ ID NO:9) |
| **Nterm-S AA** |
| MAATASPQPLATEDADSENSSFYYYDYLDEVAFMLSRKDAVVSFGKVFLP (SEQ ID NO: 10) |
| **EC1-S DNA** |
| AGCTTCTTGAGCAAG (SEQ ID NO:11) |
| **EC1-S AA** |
| SFLSK (SEQ ID NO:12) |
| **EC2-S DNA** |
| CAAACCCATGAAAACCCCAAGGGAGTTTGGAACAGCCATGCCGATTTCGGCGGGCATGGCACC ATTTGGAAGCTCTTCCTCCGGTTCCAGCAGAACCTGCTA (SEQ ID NO:13) |
| **EC2-S AA** |
| QTHENPKGVWNSHADFGGHGTIWKLFLRFQQNLL (SEQ ID NO:14) |
| **EC3-S DNA** |
| CTGCATACCCTGCTGGACCTGCAAGTATTCGGCAACAGTGAGGTTAGCCAGCATCTAGACTATG CC (SEQ ID NO:15) |
| **EC3-S AA** |
| LHTLLDLQVFGNSEVSQHLDYA (SEQ ID NO:16) |
| **Nterm-EC3 DNA** |
| ATGGCAGCTACTGCCAGCCCGCAGCCACTGGCTACTGAGGATGCCGATTCTGAGAATAGCAGCT TCTACTACTATGACTACCTGGATGAAGTAGCTTTCATGCTCTGCGAGGTTAGCCAGCATCTAGA CTATGCC (SEQ ID NO:17) |
| **Nterm-EC3 AA** |
| MAATASPQPLATEDADSENSSFYYYDYLDEVAFMLCEVSQHLDYA (SEQ ID NO:18) |
| **EC3-Nterm DNA** |
| CTGCATACCCTGCTGGACCTGCAAGTATTCGGCAACTGTCGGAAGGATGCTGTGGTTAGCTTTG GCAAAGTTTTCCTGCCA (SEQ ID NO:19) |
| **EC3-Nterm AA** |
| LHTLLDLQVFGNCRKDAVVSFGKVFLP (SEQ ID NO:20) |
| **EC1-EC2 DNA** |
| AGCTTCTTGTGCCATGCCGATTTCGGCGGGCATGGCACCATTTGGAAGCTCTTCCTCCGGTTCCA GCAGAACCTGCTA (SEQ ID NO:21) |
| **EC1-EC2 AA** |
| SFLCHADFGGHGTIWKLFLRFQQNLL (SEQ ID NO:22) |
| **EC2-EC1 DNA** |
| CAAACCCATGAAAACCCCAAGGGAGTTTGGAACTGCAAG (SEQ ID NO:23) |
| **EC2 EC1 AA** |
| QTHENPKGVWNCK (SEQ ID NO:24) |
| **Nterm-EC3-S DNA** |
| ATGGCAGCTACTGCCAGCCCGCAGCCACTGGCTACTGAGGATGCCGATTCTGAGAATAGCAGCT TCTACTACTATGACTACCTGGATGAAGTAGCTTTCATGCTCAGCGAGGTTAGCCAGCATCTAGA CTATGCC (SEQ ID NO:25) |
| **Nterm-EC3-S AA** |
| MAATASPQPLATEDADSENSSFYYYDYLDEVAFMLSEVSQHLDYA (SEQ ID NO:26) |
| **EC3-Nterm-S DNA** |
| CTGCATACCCTGCTGGACCTGCAAGTATTCGGCAACAGTCGGAAGGATGCTGTGGTTAGCTTTG GCAAAGTTTTCCTGCCA (SEQ ID NO:27) |
| **EC3-Nterm-S AA** |
| LHTLLDLQVFGNSRKDAVVSFGKVFLP (SEQ ID NO:28) |
| **EC1-EC2-S DNA** |
| AGCTTCTTGAGCCATGCCGATTTCGGCGGGCATGGCACCATTTGGAAGCTCTTCCTCCGGTTCCA GCAGAACCTGCTA (SEQ ID NO:29) |
| **EC1-EC2-S AA** |
| SFLSHADFGGHGTIWKLFLRFQQNLL (SEQ ID NO:30) |
| **EC2-EC1-S DNA** |
| CAAACCCATGAAAACCCCAAGGGAGTTTGGAACAGCAAG (SEQ ID NO:31) |
| **EC2-EC1-S** AA |
| QTHENPKGVWNSK (SEQ ID NO:32) |
| **Nterm+EC3 DNA** |
| ATGGCAGCTACTGCCAGCCCGCAGCCACTGGCTACTGAGGATGCCGATTCTGAGAATAGCAGCT TCTACTACTATGACTACCTGGATGAAGTAGCTTTCATGCTCTGCCGGAAGGATGCTGTGGTTAG CTTTGGCAAAGTTTTCCTGCCACTGCATACCCTGCTGGACCTGCAAGTATTCGGCAACTGTGAG GTTAGCCAGCATCTAGACTATGCC (SEQ ID NO:33) |
| **Nterm+EC3 AA** |
| MAATASPQPLATEDADSENSSFYYYDYLDEVAFMLCRKDAVVSFGKVFLPLHTLLDLQVFGNCEVS QHLDYA (SEQ ID NO:34) |
| **EC1+EC2 DNA** |
| AGCTTCTTGTGCAAGCAAACCCATGAAAACCCCAAGGGAGTTTGGAACTGCCATGCCGATTTCG GCGGGCATGGCACCATTTGGAAGCTCTTCCTCCGGTTCCAGCAGAACCTGCTA (SEQ ID NO:35) |
| **EC1+EC2 AA** |
| SFLCKQTHENPKGVWNCHADFGGHGTIWKLFLRFQQNLL (SEQ ID NO:36) |
| **Nterm-N DNA** |
| ATGGCAGCTACTGCCAGCCCGCAGCCACTGGCTACTGAGGATGCCGATTCTGAGAATAGCAGCT TCTACTACTATGACTACCTGGATGAAGTAGCTTTCATGCTC (SEQ ID NO:37) |
| **Nterm-N AA** |
| MAATASPQPLATEDADSENSSFYYYDYLDEVAFML (SEQ ID NO:38) |
| **Nterm-C DNA** |
| CGGAAGGATGCTGTGGTTAGCTTTGGCAAAGTTTTCCTGCCA (SEQ ID NO:39) |
| **Nterm-C AA** |
| RKDAVVSFGKVFLP (SEQ ID NO:40) |
| **EC1-N DNA** |
| AGCTTCTTG (SEQ ID NO:41) |
| **EC1-N AA** |
| SFL (SEQ ID NO:42) |
| **EC2-N DNA** |
| CAAACCCATGAAAACCCCAAGGGAGTTTGGAAC (SEQ ID NO:43) |
| **EC2-N AA** |
| QTHENPKGVWN (SEQ ID NO:44) |
| **EC2-C DNA** |
| CATGCCGATTTCGGCGGGCATGGCACCATTTGGAAGCTCTTCCTCCGGTTCCAGCAGAACCTGC TA (SEQ ID NO:45) |
| **EC2-C AA** |
| HADFGGHGTIWKLFLRFQQNLL (SEQ ID NO:46) |
| **EC3-N DNA** |
| CTGCATACCCTGCTGGACCTGCAAGTATTCGGCAAC (SEQ ID NO:47) |
| **EC3-N AA** |
| LHTLLDLQVFGN (SEQ ID NO:48) |
| **EC3-C DNA** |
| GAGGTTAGCCAGCATCTAGACTATGCC (SEQ ID NO:49) |
| **EC3-C AA** |
| EVSQHLDYA (SEQ ID NO:50) |

Exemplary DNA fragments containing human ACKR2 (D6 chemokine decoy receptor) nucleotide coding sequences for construction of an engineered D_{H} region are provided below.

D6-DH1166 (SEQ ID NO:131) includes D6 coding sequences inserted in positions corresponding to D_{H}1-1 to D_{H}6-6:

TACGTAGCCGTTTCGATCCTCCCGAATTGACTAGTGGGTAGGCCTGGCGGCCGCTGCCAT
TTCATTACCTCTTTCTCCGCACCCGACATAGATACCGGTGGATTCGAATTCTCCCCGTTGA
AGCTGACCTGCCCAGAGGGGCCTGGGCCCACCCCACACACCGGGGCGGAATGTGTACAG
GCCCCGGTCTCTGTGGGTGTTCCGCTAACTGGGGCTCCCAGTGCTCACCCCACAACTAAA
GCGAGCCCCAGCCTCCAGAGCCCCCGAAGGAGATGCCGCCCACAAGCCCAGCCCCCATC
CAGGAGGCCCCAGAGCTCAGGGCGCCGGGGCGGATTTTGTACAGCCCCGAGTCACTGTG
CGGAAGGATGCTGTGGTTAGCTTTGGCAAAGTTTTCCTGCCACCACAGTGAGAAAAACTG
TGTCAAAAACCGTCTCCTGGCCCCTGCTGGAGGCCGCGCCAGAGAGGGGAGCAGCCGCC
CCGAACCTAGGTCCTGCTCAGCTCACACGACCCCCAGCACCCAGAGCACAACGGAGTCC
CCATTGAATGGTGAGGACGGGGACCAGGGCTCCAGGGGGTCATGGAAGGGGCTGGACCC
CATCCTACTGCTATGGTCCCAGTGCTCCTGGCCAGAACTGACCCTACCACCGACAAGAGT
CCCTCAGGGAAACGGGGGTCACTGGCACCTCCCAGCATCAACCCCAGGCAGCACAGGCA
TAAACCCCACATCCAGAGCCGACTCCAGGAGCAGAGACACCCCAGTACCCTGGGGGACA
CCGACCCTGATGACTCCCCACTGGAATCCACCCCAGAGTCCACCAGGACCAAAGACCCC
GCCCCTGTCTCTGTCCCTCACTCAGGACCTGCTGCGGGGCGGGCCATGAGACCAGACTCG
GGCTTAGGGAACACCACTGTGGCCCCAACCTCGACCAGGCCACAGGCCCTTCCTTCCTGC
CCTGCGGCAGCACAGACTTTGGGGTCTGTGCAGAGAGGAATCACAGAGGCCCCAGGCTG
AGGTGGTGGGGGTGGAAGACCCCCAGGAGGTGGCCCACTTCCCTTCCTCCCAGCTGGAA
CCCACCATGACCTTCTTAAGATAGGGGTGTCATCCGAGGCAGGTCCTCCATGGAGCTCCC
TTCAGGCTCCTCCCCGGTCCTCACTAGGCCTCAGTCCCGGCTGCGGGAATGCAGCCACCA
CAGGCACACCAGGCAGCCCAGACCCAGCCAGCCTGCAGTGCCCAAGCCCACATTCTGGA
GCAGAGCAGGCTGTGTCTGGGAGAGTCTGGGCTCCCCACCGCCCCCCCGCACACCCCAC
CCACCCCTGTCCAGGCCCTATGCAGGAGGGTCAGAGCCCCCCATGGGGTATGGACTTAG
GGTCTCACTCACGTGGCTCCCCTCCTGGGTGAAGGGGTCTCATGCCCAGATCCCCACAGC
AGAGCTGGTCAAAGGTGGAGGCAGTGGCCCCAGGGCCACCCTGACCTGGACCCTCAGGC
TCCTCTAGCCCTGGCTGCCCTGCTGTCCCTGGGAGGCCTGGACTCCACCAGACCACAGGT

CCAGGGCACCGCCCATAGGTGCTGCCCACACTCAGTTCACAGGAAGAAGATAAGCTCCA
GACCCCCAAGACTGGGACCTGCCTTCCTGCCACCGCTTGTAGCTCCAGACCTCCGTGCCT
CCCCCGACCACTTACACACGGGCCAGGGAGCTGTTCCACAAAGATCAACCCCAAACCGG
GACCGCCTGGCACTCGGGCCGCTGCCACTTCCCTCTCCATTTGTTCCCAGCACCTCTGTGC
TCCCTCCCTCCTCCCTCCTTCAGGGGAACAGCCTGTGCAGCCCCTCCCTGCACCCCACACC
CTGGGGAGGCCCAACCCTGCCTCCAGCCCTTTCTCCCCCGCTGCTCTTCCTGCCCATCCAG
ACAACCCTGGGGTCCCATCCCTGCAGCCTACACCCTGGTCTCCACCCAGACCCCTGTCTC
TCCCTCCAGACACCCCTCCCAGGCCAACCCTGCACATGCAGGCCCTCCCCTTTTCTGCTGC
CAGAGCCTCAGTTTCTACCCTCTGTGCCTACCCCCTGCCTCCTCCTGCCCACAACTCGAGC
TCTTCCTCTCCTGGGGCCCCTGAGCCATGGCACTGACCGTGCACTCCCACCCCCACACTG
CCCATGCCCTCACCTTCCTCCTGGACACTCTGACCCCGCTCCCCTCTTGGACCCAGCCCTG
GTATTTCCAGGACAAAGGCTCACCCAAGTCTTCCCCATGCAGGCCCTTGCCCTCACTGCC
CGGTTACACGGCAGCCTCCTGTGCACAGAAGCAGGGAGCTCAGCCCTTCCACAGGCAGA
AGGCACTGAAAGAAATCGGCCTCCAGCACCCTGATGCACGTCCGCCTGTGTCTCTCACTG
CCCGCACCTGCAGGGAGGCTCGGCACTCCCTGTAAAGACGAGGGATCCAGGCAGCAACA
TCATGGGAGAATGCAGGGCTCCCAGACAGCCCAGCCCTCTCGCAGGCCTCTCCTGGGAA
GAGACCTGCAGCCACCACTGAACAGCCACGGAGCCCGCTGGATAGTAACTGAGTCAGTG
ACCGACCTGGAGGGCAGGGGAGCAGTGAACCGGAGCCCAGACCATAGGGACAGAGACC
AGCCGCTGACATCCCGAGCCCCTCACTGGCGGCCCCAGAACACCGCGTGGAAACAGAAC
AGACCCACATTCCCACCTGGAACAGGGCAGACACTGCTGAGCCCCCAGCACCAGCCCTG
AGAAACACCAGGCAACGGCATCAGAGGGGGCTCCTGAGAAAGAAAGGAGGGGAGGTCT
CCTTCACCAGCAAGTACTTCCCTTGACCAAAAACAGGGTCCACGCAACTCCCCCAGGACA
AAGGAGGAGCCCCCTGTACAGCACTGGGCTCAGAGTCCTCTCCCACACACCCTGAGTTTC
AGACAAAAACCCCCTGGAAATCATAGTATCAGCAGGAGAACTAGCCAGAGACAGCAAG
AGGGGACTCAGTGACTCCCGCGGGGACAGGAGGATTTTGTGGGGGCTCGTGTCACTGTG
CTGCATACCCTGCTGGACCTGCAAGTATTCGGCAACTGTGAGGTTAGCCAGCATCTAGAC
TATGCCCACAGTGACACAGCCCCATTCAAAAACCCCTGCTGTAAACGCTTCCACTTCTGG
AGCTGAGGGGCTGGGGGGAGCGTCTGGGAAGTAGGGCCTAGGGGTGGCCATCAATGCCC
AAAACGCACCAGACTCCCCCCCAGACATCACCCCACTGGCCAGTGAGCAGAGTAAACAG
AAAATGAGAAGCAGCTGGGAAGCTTGCACAGGCCCCAAGGAAAGAGCTTTGGCGGGTGT
GCAAGAGGGGATGCGGGCAGAGCCTGAGCAGGGCCTTTTGCTGTTTCTGCTTTCCTGTGC
AGATAGTTCCATAAACTGGTGTTCAAGATCGATGGCTGGGAGTGAGCCCAGGAGGACAG
TGTGGGAAGGGCACAGGGAAGGAGAAGCAGCCGCTATCCTACACTGTCATCTTTCAAGA
GTTTGCCCTGTGCCCACAATGCTGCATCATGGGATGCTTAACAGCTGATGTAGACACAGC
TAAAGAGAGAATCAGTGAAATGGATTTGCAGCACAGATCTGAATAAATTCTCCAGAATG
TGGAGCCACACAGAAGCAAGCACAAGGAAAGTGCCTGATGCAAGGGCAAAGTACAGTG
TGTACCTTCAGGCTGGGCACAGACACTCTGAAAAGCCTTGGCAGGAACTCCCTGCAACA
AAGCAGAGCCCTGCAGGCAATGCCAGCTCCAGAGCCCTCCCTGAGAGCCTCATGGGCAA
AGATGTGCACAACAGGTGTTTCTCATAGCCCCAAACTGAGAATGAAGCAAACAGCCATC
TGAAGGAAAACAGGCAAATAAACGATGGCAGGTTCATGAAATGCAAACCCAGACAGCC
AGAAGGACAACAGTGAGGGTTACAGGTGACTCTGTGGTTGAGTTCATGACAATGCTGAG
TAATTGGAGTAACAAAGGAAAGTCCAAAAAATACTTTCAATGTGATTTCTTCTAAATAAA
ATTTACAGCCGGCAAAATGAACTATCTTCTTAAGGGATAAACTTTCCACTAGGAAAACTA
TAAGGAAAATCAAGAAAAGGATGATCACATAAACACAGTGGTCGTTACTTCTACTGGGG
AAGGAAGAGGGTATGAACTGAGACACACAGGGTTGGCAAGTCTCCTAACAAGAACAGA
ACAAATACATTACAGTACCTTGAAAACAGCAGTTAAAATTCTAAATTGCAAGAAGAGGA
AAATGCACACAGCTGTGTTTAGAAAATTCTCAGTCCAGCACTGTTCATAATAGCAAAGAC
ATTAACCCAGGTTGGATAAATAAACGATGACACAGGCAATTGCACAATGATACAGACAT

ACATTCAGTATATGAGACATTGATGATGTATCCCCAAAGAAATGACTTTAAAGAGAAAA
GGCCTGATATGTGGTGGCACTCACCTCCCTGGGCATCCCCGGACAGGCTGCAGGCACACT
GTGTGGCAGGGCAGGCTGGTACCTGCTGGCAGCTCCTGGGGCCTGATGTGGAGCAGGCA
CAGAGCCGTATCCCCCCGAGGACATATACCCCCAAGGACGGCACAGTTGGTACATTCCG
GAGACAAGCAACTCAGCCACACTCCCAGGCCAGAGCCCGAGAGGGACGCCCATGCACA
GGGAGGCAGAGCCCAGCTCCTCCACAGCCAGCAGCACCCGTGCAGGGGCCGCCATCTGG
CAGGCACAGAGCATGGGCTGGGAGGAGGGGCAGGGACACCAGGCAGGGTTGGCACCAA
CTGAAAATTACAGAAGTCTCATACATCTACCTCAGCCTTGCCTGACCTGGGCCTCACCTG
ACCTGGACCTCACCTGGCCTGGACCTCACCTGGCCTAGACCTCACCTCTGGGCTTCACCT
GAGCTCGGCCTCACCTGACTTGGACCTTGCCTGTCCTGAGCTCACATGATCTGGGCCTCA
CCTGACCTGGGTTTCACCTGACCTGGGCTTCACCTGACCTGGGCCTCATCTGACCTGGGC
CTCACTGGCCTGGACCTCACCTGGCCTGGGCTTCACCTGGCCTCAGGCCTCATCTGCACC
TGCTCCAGGTCTTGCTGGAACCTCAGTAGCACTGAGGCTGCAGGGGCTCATCCAGGGTTG
CAGAATGACTCTAGAACCTCCCACATCTCAGCTTTCTGGGTGGAGGCACCTGGTGGCCCA
GGGAATATAAAAAGCCTGAATGATGCCTGCGTGATTTGGGGGCAATTTATAAACCCAAA
AGGACATGGCCATGCAGCGGGTAGGGACAATACAGACAGATATCAGCCTGAAATGGAG
CCTCAGGGCACAGGTGGGCACGGACACTGTCCACCTAAGCCAGGGGCAGACCCGAGTGT
CCCCGCAGTAGACCTGAGAGCGCTGGGCCCACAGCCTCCCCTCGGTGCCCTGCTACCTCC
TCAGGTCAGCCCTGGACATCCCGGGTTTCCCCAGGCCTGGCGGTAGGATTTTGTTGAGGT
CTGTGTCACTGTGCATGGCAGCTACTGCCAGCCCGCAGCCACTGGCTACTGAGGATGCCG
ATTCTGAGAATAGCAGCTTCTACTACTATGACTACCTGGATGAAGTAGCTTTCATGCTCT
GCCGGAAGGATGCTGTGGTTAGCTTTGGCAAAGTTTTCCTGCCACCACAGTGTCACAGAG
TCCATCAAAAACCCATCCCTGGGAACCTTCTGCCACAGCCCTCCCTGTGGGGCACCGCCG
CGTGCCATGTTAGGATTTTGACTGAGGACACAGCACCATGGGTATGGTGGCTACCGCAGC
AGTGCAGCCCGTGACCCAAACACACAGGGCAGCAGGCACAACAGACAAGCCCACAAGT
GACCACCCTGAGCTCCTGCCTGCCAGCCCTGGAGACCATGAAACAGATGGCCAGGATTA
TCCCATAGGTCAGCCAGACCTCAGTCCAACAGGTCTGCATCGCTGCTGCCCTCCAATACC
AGTCCGGATGGGGACAGGGCTGGCCCACATTACCATTTGCTGCCATCCGGCCAACAGTCC
CAGAAGCCCCTCCCTCAAGGCTGGGCCACATGTGTGGACCCTGAGAGCCCCCCATGTCTG
AGTAGGGGCACCAGGAAGGTGGGGCTGGCCCTGTGCACTGTCCCTGCCCCTGTGGTCCCT
GGCCTGCCTGGCCCTGACACCTGGGCCTCTCCTGGGTCATTTCCAAGACAGAAGACATTC
CCAGGACAGCTGGAGCTGGGAGTCCATCATCCTGCCTGGCCGTCCTGAGTCCTGCGCCTT
TCCAAACCTCACCCGGGAAGCCAACAGAGGAATCACCTCCCACAGGCAGAGACAAAGAC
CTTCCAGAAATCTCTGTCTCTCTCCCCAGTGGGCACCCTCTTCCAGGGCAGTCCTCAGTGA
TATCACAGTGGGAACCCACATCTGGATCGGGACTGCCCCCAGAACACAAGATGGCCCAC
AGGGACAGCCCCACAGCCCAGCCCTTCCCAGACCCCTAAAAGGCGTCCCACCCCCTGCA
TCTGCCCCAGGGCTCAAACTCCAGGAGGACTGACTCCTGCACACCCTCCTGCCAGACATC
ACCTCAGCCCCTCCTGGAAGGGACAGGAGCGCGCAAGGGTGAGTCAGACCCTCCTGCCC
TCGATGGCAGGCGGAGAAGATTCAGAAAGGTCTGAGATCCCCAGGACGCAGCACCACTG
TCAATGGGGGCCCCAGACGCCTGGACCAGGGCCTGCGTGGGAAAGGCCTCTGGGCACAC
TCAGGGGGATTTTGTGAAGGGTCCTCCCACTGTGCATGGCAGCTACTGCCAGCCCGCAGC
CACTGGCTACTGAGGATGCCGATTCTGAGAATAGCAGCTTCTACTACTATGACTACCTGG
ATGAAGTAGCTTTCATGCTCTGCCGGAAGGATGCTGTGGTTAGCTTTGGCAAAGTTTTCC
TGCCACTGCATACCCTGCTGGACCTGCAAGTATTCGGCAACTGTGAGGTTAGCCAGCATC
TAGACTATGCCCACAGTGATGAACCCAGCATCAAAAACCGACCGGACTCCCAAGGTTTA
TGCACACTTCTCCGCTCAGAGCTCTCCAGGATCAGAAGAGCCGGGCCCAAGGGTTTCTGC
CCAGACCCTCGGCCTCTAGGGACATCTTGGCCATGACAGCCCATGGGCTGGTGCCCCACA
CATCGTCTGCCTTCAAACAAGGGCTTCAGAGGGCTCTGAGGTGACCTCACTGATGACCAC

AGGTGCCCTGGCCCCTTCCCCACCAGCTGCACCAGACCCCGTCATGACAGATGCCCCGAT
TCCAACAGCCAATTCCTGGGGCCAGGAATCGCTGTAGACACCAGCCTCCTTCCAACACCT
CCTGCCAATTGCCTGGATTCCCATCCCGGTTGGAATCAAGAGGACAGCATCCCCCAGGCT
CCCAACAGGCAGGACTCCCACACCCTCCTCTGAGAGGCCGCTGTGTTCCGTAGGGCCAG
GCTGCAGACAGTCCCCCTCACCTGCCACTAGACAAATGCCTGCTGTAGATGTCCCCACCT
GGAAAATACCACTCATGGAGCCCCCAGCCCCAGGTACAGCTGTAGAGAGAGTCTCTGAG
GCCCCTAAGAAGTAGCCATGCCCAGTTCTGCCGGGACCCTCGGCCAGGCTGACAGGAGT
GGACGCTGGAGCTGGGCCCATACTGGGCCACATAGGAGCTCACCAGTGAGGGCAGGAGA
GCACATGCCGGGGAGCACCCAGCCTCCTGCTGACCAGAGGCCCGTCCCAGAGCCCAGGA
GGCTGCAGAGGCCTCTCCAGGGGGACACTGTGCATGTCTGGTCCCTGAGCAGCCCCCCAC
GTCCCCAGTCCTGGGGGCCCCTGGCACAGCTGTCTGGACCCTCTCTATTCCCTGGGAAGC
TCCTCCTGACAGCCCCGCCTCCAGTTCCAGGTGTGGATTTTGTCAGGGGGTGTCACACTG
TGCAGCTTCTTGTGCAAGCACAGTGGTGCTGCCCATATCAAAAACCAGGCCAAGTAGAC
AGGCCCCTGCTGTGCAGCCCCAGGCCTCCAGCTCACCTGCTTCTCCTGGGGCTCTCAAGG
CTGCTGTTTTCTGCACTCTCCCCTCTGTGGGGAGGGTTCCCTCAGTGGGAGATCTGTTCTC
AACATCCCACGGCCTCATTCCTGCAAGGAAGGCCAATGGATGGGCAACCTCACATGCCG
CGGCTAAGATAGGGTGGGCAGCCTGGCGGGGACAGGACATCCTGCTGGGGTATCTGTCA
CTGTGCCTAGTGGGGCACTGGCTCCCAAACAACGCAGTCCTTGCCAAAATCCCCACGGCC
TCCCCCGCTAGGGGCTGGCCTGATCTCCTGCAGTCCTAGGAGGCTGCTGACCTCCAGAAT
GGCTCCGTCCCCAGTTCCAGGGCGAGAGCAGATCCCAGGCCGGCTGCAGACTGGGAGGC
CACCCCCTCCTTCCCAGGGTTCACTGCAGGTGACCAGGGCAGGAAATGGCCTGAACACA
GGGATAACCGGGCCATCCCCCAACAGAGTCCACCCCCTCCTGCTCTGTACCCCGCACCCC
CCAGGCCAGCCCATGACATCCGACAACCCCACACCAGAGTCACTGCCCGGTGCTGCCCT
AGGGAGGACCCCTCAGCCCCCACCCTGTCTAGAGGACTGGGGAGGACAGGACACGCCCT
CTCCTTATGGTTCCCCCACCTGGCTCTGGCTGGGACCCTTGGGGTGTGGACAGAAAGGAC
GCTTGCCTGATTGGCCCCCAGGAGCCCAGAACTTCTCTCCAGGGACCCCAGCCCGAGCAC
CCCCTTACCCAGGACCCAGCCCTGCCCCTCCTCCCCTCTGCTCTCCTCTCATCACCCCATG
GGAATCCAGAATCCCCAGGAAGCCATCAGGAAGGGCTGAGGGAGGAAGTGGGGCCACT
GCACCACCAGGCAGGAGGCTCTGTCTTTGTGAACCCAGGGAGGTGCCAGCCTCCTAGAG
GGTATGGTCCACCCTGCCTATGGCTCCCACAGTGGCAGGCTGCAGGGAAGGACCAGGGA
CGGTGTGGGGGAGGGCTCAGGGCCCCGCGGGTGCTCCATCTTGGATGAGCCTATCTCTCT
CACCCACGGACTCGCCCACCTCCTCTTCACCCTGGCCACACGTCGTCCACACCATCCTAA
GTCCCACCTACACCAGAGCCGGCACAGCCAGTGCAGACAGAGGCTGGGGTGCAGGGGG
GCCGACTGGGCAGCTTCGGGGAGGGAGGAATGGAGGAAGGGGAGTTCAGTGAAGAGGC
CCCCCTCCCCTGGGTCCAGGATCCTCCTCTGGGACCCCCGGATCCCATCCCCTCCAGGCT
CTGGGAGGAGAAGCAGGATGGGAGAATCTGTGCGGGACCCTCTCACAGTGGAATACCTC
CACAGCGGCTCAGGCCAGATACAAAAGCCCCTCAGTGAGCCCTCCACTGCAGTGCTGGG
CCTGGGGGCAGCCGCTCCCACACAGGATGAACCCAGCACCCCGAGGATGTCCTGCCAGG
GGGAGCTCAGAGCCATGAAGGAGCAGGATATGGGACCCCCGATACAGGCACAGACCTC
AGCTCCATTCAGGACTGCCACGTCCTGCCCTGGGAGGAACCCCTTTCTCTAGTCCCTGCA
GGCCAGGAGGCAGCTGACTCCTGACTTGGACGCCTATTCCAGACACCAGACAGAGGGGC
AGGCCCCCCAGAACCAGGGATGAGGACGCCCCGTCAAGGCCAGAAAAGACCAAGTTGC
GCTGAGCCCAGCAAGGGAAGGTCCCCAAACAAACCAGGAGGATTTTGTAGGTGTCTGTG
TCACTGTGCAAACCCATGAAAACCCCAAGGGAGTTTGGAACTGCCATGCCGATTTCGGC
GGGCATGGCACCATTTGGAAGCTCTTCCTCCGGTTCCAGCAGAACCTGCTACCACAGTGA
CACTCGCCAGGTCAAAAACCCCATCCCAAGTCAGCGGAATGCAGAGAGAGCAGGGAGG
ACATGTTTAGGATCTGAGGCCGCACCTGACACCCAGGCCAGCAGACGTCTCCTGTCCACG
GCACCCTGCCATGTCCTGCATTTCTGGAAGAACAAGGGCAGGCTGAAGGGGGTCCAGGA

CCAGGAGATGGGTCCGCTCTACCCAGAGAAGGAGCCAGGCAGGACACAAGCCCCCACGC
GTGGGCTCGTAGTTTGACGTGCGTGAAGTGTGGGTAAGAAAGTACGTA

D6-D17613 (SEQ ID NO:132) includes D6 coding sequences inserted in positions corresponding to D_{H}1-7 to D_{H}6-13:

GCGGCCGCTGCCATTTCATTACCTCTTTCTCCGCACCCGACATAGATTACGTAACGCGTG
GGCTCGTAGTTTGACGTGCGTGAAGTGTGGGTAAGAAAGTCCCCATTGAGGCTGACCTGC
CCAGAGGGTCCTGGGCCCACCCAACACACCGGGGCGGAATGTGTGCAGGCCTCGGTCTC
TGTGGGTGTTCCGCTAGCTGGGGCTCACAGTGCTCACCCCACACCTAAAACGAGCCACAG
CCTCCGGAGCCCCTGAAGGAGACCCCGCCCACAAGCCCAGCCCCCACCCAGGAGGCCCC
AGAGCACAGGGCGCCCCGTCGGATTTTGTACAGCCCCGAGTCACTGTGCAGCTTCTTGCA
CAGTGAGAAAAGCTTCGTCAAAAACCGTCTCCTGGCCACAGTCGGAGGCCCCGCCAGAG
AGGGGAGCAGCCACCCCAAACCCATGTTCTGCCGGCTCCCATGACCCCGTGCACCTGGA
GCCCCACGGTGTCCCCACTGGATGGGAGGACAAGGGCCGGGGGCTCCGGCGGGTCGGGG
CAGGGGCTTGATGGCTTCCTTCTGCCGTGGCCCCATTGCCCCTGGCTGGAGTTGACCCTTC
TGACAAGTGTCCTCAGAGAGTCAGGGATCAGTGGCACCTCCCAACATCAACCCCACGCA
GCCCAGGCACAAACCCCACATCCAGGGCCAACTCCAGGAACAGAGACACCCCAATACCC
TGGGGGACCCCGACCCTGATGACTCCCGTCCCATCTCTGTCCCTCACTTGGGGCCTGCTG
CGGGGCGAGCACTTGGGAGCAAACTCAGGCTTAGGGGACACCACTGTGGGCCTGACCTC
GAGCAGGCCACAGACCCTTCCCTCCTGCCCTGGTGCAGCACAGACTTTGGGGTCTGGGCA
GGGAGGAACTTCTGGCAGGTCACCAAGCACAGAGCCCCCAGGCTGAGGTGGCCCCAGGG
GGAACCCCAGCAGGTGGCCCACTACCCTTCCTCCCAGCTGGACCCCATGTCTTCCCCAAG
ATAGGGGTGCCATCCAAGGCAGGTCCTCCATGGAGCCCCCTTCAGGCTCCTCTCCAGACC
CCACTGGGCCTCAGTCCCCACTCTAGGAATGCAGCCACCACGGGCACACCAGGCAGCCC
AGGCCCAGCCACCCTGCAGTGCCCAAGCCCACACCCTGGAGGAGAGCAGGGTGCGTCTG
GGAGGGGCTGGGCTCCCCACCCCCACCCCCACCTGCACACCCCACCCACCCTTGCCCGGG
CCCCCTGCAGGAGGGTCAGAGCCCCCATGGGATATGGACTTAGGGTCTCACTCACGCAC
CTCCCCTCCTGGGAGAAGGGGTCTCATGCCCAGATCCCCCCAGCAGCGCTGGTCACAGGT
AGAGGCAGTGGCCCCAGGGCCACCCTGACCTGGCCCCTCAGGCTCCTCTAGCCCTGGCTG
CCCTGCTGTCCCTGGGAGGCCTGGGCTCCACCAGACCACAGGTCTAGGGCACCGCCCAC
ACTGGGGCCGCCCACACACAGCTCACAGGAAGAAGATAAGCTCCAGACCCCCAGGCCCG
GGACCTGCCTTGCTGCTACGACTTCCTGCCCCAGACCTCGTTGCCCTCCCCCGTCCACTTA
CACACAGGCCAGGAAGCTGTTCCCACACAGACCAACCCCAGACGGGGACCACCTGGCAC
TCAGGTCACTGCCATTTCCTTCTCCATTCACTTCCAATGCCTCTGTGCTTCCTCCCTCCTCC
TTCCTTCGGGGGAGCACCCTGTGCAGCTCCTCCCTGCAGTCCACACCCTGGGGAGACCCG
ACCCTGCAGCCCACACCCTGGGGAGACCTGACCCTCCTCCAGCCCTTTCTCCCCCGCTGC
TCTTGCCACCCACCAAGACAGCCCTGGGGTCCTGTCCCTACAGCCCCCACCCAGTTCTCT
ACCTAGACCCGTCTTCCTCCCTCTAAACACCTCTCCCAGGCCAACCCTACACCTGCAGGC
CCTCCCCTCCACTGCCAAAGACCCTCAGTTTCTCCTGCCTGTGCCCACCCCCGTGCTCCTC
CTGCCCACAGCTCGAGCTCTTCCTCTCCTAGGGCCCCTGAGGGATGGCATTGACCGTGCC
CTCGCACCCACACACTGCCCATGCCCTCACATTCCTCCTGGCCACTCCAGCCCCACTCCCC
TCTCAGGCCTGGCTCTGGTATTTCTGGGACAAAGCCTTACCCAAGTCTTTCCCATGCAGG
CCTGGGCCCTTACCCTCACTGCCCGGTTACAGGGCAGCCTCCTGTGCACAGAAGCAGGGA
GCTCAGCCCTTCCACAGGCAGAAGGCACTGAAAGAAATCGGCCTCCAGCGCCTTGACAC
ACGTCTGCCTGTGTCTCTCACTGCCCGCACCTGCAGGGAGGCTCGGCACTCCCTCTAAAG
ACGAGGGATCCAGGCAGCAGCATCACAGGAGAATGCAGGGCTACCAGACATCCCAGTCC
TCTCACAGGCCTCTCCTGGGAAGAGACCTGAAGACGCCCAGTCAACGGAGTCTAACACC

AAACCTCCCTGGAGGCCGATGGGTAGTAACGGAGTCATTGCCAGACCTGGAGGCAGGGG
AGCAGTGAGCCCGAGCCCACACCATAGGGCCAGAGGACAGCCACTGACATCCCAAGCCA
CTCACTGGTGGTCCCACAACACCCCATGGAAAGAGGACAGACCCACAGTCCCACCTGGA
CCAGGGCAGAGACTGCTGAGACCCAGCACCAGAACCAACCAAGAAACACCAGGCAACA
GCATCAGAGGGGGCTCTGGCAGAACAGAGGAGGGGAGGTCTCCTTCACCAGCAGGCGCT
TCCCTTGACCGAAGACAGGATCCATGCAACTCCCCCAGGACAAAGGAGGAGCCCCTTGT
TCAGCACTGGGCTCAGAGTCCTCTCCAAGACACCCAGAGTTTCAGACAAAAACCCCCTG
GAATGCACAGTCTCAGCAGGAGAGCCAGCCAGAGCCAGCAAGATGGGGCTCAGTGACA
CCCGCAGGGACAGGAGGATTTTGTGGGGGCTCGTGTCACTGTGCTGCATACCCTGCTGGA
CCTGCAAGTATTCGGCAACAGTGAGGTTAGCCAGCATCTAGACTATGCCCACAGTGACA
CAGCCCCATTCAAAAACCCCTACTGCAAACGCATTCCACTTCTGGGGCTGAGGGGCTGGG
GGAGCGTCTGGGAAATAGGGCTCAGGGGTGTCCATCAATGCCCAAAACGCACCAGACTC
CCCTCCATACATCACACCCACCAGCCAGCGAGCAGAGTAAACAGAAAATGAGAAGCAAG
CTGGGGAAGCTTGCACAGGCCCCAAGGAAAGAGCTTTGGCGGGTGTGTAAGAGGGGATG
CGGGCAGAGCCTGAGCAGGGCCTTTTGCTGTTTCTGCTTTCCTGTGCAGAGAGTTCCATA
AACTGGTGTTCGAGATCAATGGCTGGGAGTGAGCCCAGGAGGACAGCGTGGGAAGAGC
ACAGGGAAGGAGGAGCAGCCGCTATCCTACACTGTCATCTTTCGAAAGTTTGCCTTGTGC
CCACACTGCTGCATCATGGGATGCTTAACAGCTGATGTAGACACAGCTAAAGAGAGAAT
CAGTGAGATGGATTTGCAGCACAGATCTGAATAAATTCTCCAGAATGTGGAGCAGCACA
GAAGCAAGCACACAGAAAGTGCCTGATGCAAGGACAAAGTTCAGTGGGCACCTTCAGGC
ATTGCTGCTGGGCACAGACACTCTGAAAAGCCCTGGCAGGAACTCCCTGTGACAAAGCA
GAACCCTCAGGCAATGCCAGCCCCAGAGCCCTCCCTGAGAGCCTCATGGGCAAAGATGT
GCACAACAGGTGTTTCTCATAGCCCCAAACTGAGAGCAAAGCAAACGTCCATCTGAAGG
AGAACAGGCAAATAAACGATGGCAGGTTCATGAAATGCAAACCCAGACAGCCACAAGC
ACAAAAGTACAGGGTTATAAGCGACTCTGGTTGAGTTCATGACAATGCTGAGTAATTGG
AGTAACAAAGTAAACTCCAAAAAATACTTTCAATGTGATTTCTTCTAAATAAAATTTACA
CCCTGCAAAATGAACTGTCTTCTTAAGGGATACATTTCCCAGTTAGAAAACCATAAAGAA
AACCAAGAAAAGGATGATCACATAAACACAGTGGTGGTTACTTCTGCTGGGGAAGGAAG
AGGGTATGAACTGAGATACACAGGGTGGGCAAGTCTCCTAACAAGAACAGAACGAATAC
ATTACAGTACCTTGAAAACAGCAGTTAAACTTCTAAATTGCAAGAAGAGGAAAATGCAC
ACAGTTGTGTTTAGAAAATTCTCAGTCCAGCACTGTTCATAATAGCAAAGACATTAACCC
AGGTCGGATAAATAAGCGATGACACAGGCAATTGCACAATGATACAGACATATATTTAG
TATATGAGACATCGATGATGTATCCCCAAATAAACGACTTTAAAGAGATAAAGGGCTGA
TGTGTGGTGGCATTCACCTCCCTGGGATCCCCGGACAGGTTGCAGGCTCACTGTGCAGCA
GGGCAGGCGGGTACCTGCTGGCAGTTCCTGGGGCCTGATGTGGAGCAAGCGCAGGGCCA
TATATCCCGGAGGACGGCACAGTCAGTGAATTCCAGAGAGAAGCAACTCAGCCACACTC
CCCAGGCAGAGCCCGAGAGGGACGCCCACGCACAGGGAGGCAGAGCCCAGCACCTCCG
CAGCCAGCACCACCTGCGCACGGGCCACCACCTTGCAGGCACAGAGTGGGTGCTGAGAG
GAGGGGCAGGGACACCAGGCAGGGTGAGCACCCAGAGAAAACTGCAGACGCCTCACAC
ATCCACCTCAGCCTCCCCTGACCTGGACCTCACTGGCCTGGGCCTCACTTAACCTGGGCT
TCACCTGACCTTGGCCTCACCTGACTTGGACCTCGCCTGTCCCAAGCTTTACCTGACCTGG
GCCTCAACTCACCTGAACGTCTCCTGACCTGGGTTTAACCTGTCCTGGAACTCACCTGGC
CTTGGCTTCCCCTGACCTGGACCTCATCTGGCCTGGGCTTCACCTGGCCTGGGCCTCACCT
GACCTGGACCTCATCTGGCCTGGACCTCACCTGGCCTGGACTTCACCTGGCCTGGGCTTC
ACCTGACCTGGACCTCACCTGGCCTCGGGCCTCACCTGCACCTGCTCCAGGTCTTGCTGG
AGCCTGAGTAGCACTGAGGGTGCAGAAGCTCATCCAGGGTTGGGGAATGACTCTAGAAG
TCTCCCACATCTGACCTTTCTGGGTGGAGGCAGCTGGTGGCCCTGGGAATATAAAAATCT
CCAGAATGATGACTCTGTGATTTGTGGGCAACTTATGAACCCGAAAGGACATGGCCATG

GGGTGGGTAGGGACATAGGGACAGATGCCAGCCTGAGGTGGAGCCTCAGGACACAGGT
GGGCACGGACACTATCCACATAAGCGAGGGATAGACCCGAGTGTCCCCACAGCAGACCT
GAGAGCGCTGGGCCCACAGCCTCCCCTCAGAGCCCTGCTGCCTCCTCCGGTCAGCCCTGG
ACATCCCAGGTTTCCCCAGGCCTGGCGGTAGGATTTTGTTGAGGTCTGTGTCACTGTGCA
TGGCAGCTACTGCCAGCCCGCAGCCACTGGCTACTGAGGATGCCGATTCTGAGAATAGC
AGCTTCTACTACTATGACTACCTGGATGAAGTAGCTTTCATGCTCCACAGTGTCACAGAG
TCCATCAAAAACCCATGCCTGGAAGCTTCCCGCCACAGCCCTCCCCATGGGGCCCTGCTG
CCTCCTCAGGTCAGCCCCGGACATCCCGGGTTTCCCCAGGCTGGGCGGTAGGATTTTGTT
GAGGTCTGTGTCACTGTGCCAAACCCATGAAAACCCCAAGGGAGTTTGGAACAGCCATG
CCGATTTCGGCGGGCATGGCACCATTTGGAAGCTCTTCCTCCGGTTCCAGCAGAACCTGC
TACCCACAGTGTCACAGAGTCCATCAAAAACCCATCCCTGGGAGCCTCCCGCCACAGCCC
TCCCTGCAGGGGACCGGTACGTGCCATGTTAGGATTTTGATCGAGGAGACAGCACCATG
GGTATGGTGGCTACCACAGCAGTGCAGCCTGTGACCCAAACCCGCAGGGCAGCAGGCAC
GATGGACAGGCCCGTGACTGACCACGCTGGGCTCCAGCCTGCCAGCCCTGGAGATCATG
AAACAGATGGCCAAGGTCACCCTACAGGTCATCCAGATCTGGCTCCGAGGGGTCTGCAT
CGCTGCTGCCCTCCCAACGCCAGTCCAAATGGGACAGGGACGGCCTCACAGCACCATCT
GCTGCCATCAGGCCAGCGATCCCAGAAGCCCCTCCCTCAAGGCTGGGCACATGTGTGGA
CACTGAGAGCCCTCATATCTGAGTAGGGGCACCAGGAGGGAGGGGCTGGCCCTGTGCAC
TGTCCCTGCCCCTGTGGTCCCTGGCCTGCCTGGCCCTGACACCTGAGCCTCTCCTGGGTCA
TTTCCAAGACAGAAGACATTCCTGGGGACAGCCGGAGCTGGGCGTCGCTCATCCTGCCC
GGCCGTCCTGAGTCCTGCTCATTTCCAGACCTCACCGGGGAAGCCAACAGAGGACTCGCC
TCCCACATTCAGAGACAAAGAACCTTCCAGAAATCCCTGCCTCTCTCCCCAGTGGACACC
CTCTTCCAGGACAGTCCTCAGTGGCATCACAGCGGCCTGAGATCCCCAGGACGCAGCAC
CGCTGTCAATAGGGGCCCCAAATGCCTGGACCAGGGCCTGCGTGGGAAAGGCCTCTGGC
CACACTCGGGGATTTTGTGAAGGGCCCTCCCACTGTGCCAGCTTCTTGAGCCATGCCGAT
TTCGGCGGGCATGGCACCATTTGGAAGCTCTTCCTCCGGTTCCAGCAGAACCTGCTACCA
CAGTGATGAACCCAGTGTCAAAAACCGGCTGGAAACCCAGGGGCTGTGTGCACGCCTCA
GCTTGGAGCTCTCCAGGAGCACAAGAGCCGGGCCCAAGGATTTGTGCCCAGACCCTCAG
CCTCTAGGGACACCTGGGTCATCTCAGCCTGGGCTGGTGCCCTGCACACCATCTTCCTCC
AAATAGGGGCTTCAGAGGGCTCTGAGGTGACCTCACTCATGACCACAGGTGACCTGGCC
CTTCCCTGCCAGCTATACCAGACCCTGTCTTGACAGATGCCCCGATTCCAACAGCCAATT
CCTGGGACCCTGAATAGCTGTAGACACCAGCCTCATTCCAGTACCTCCTGCCAATTGCCT
GGATTCCCATCCTGGCTGGAATCAAGAAGGCAGCATCCGCCAGGCTCCCAACAGGCAGG
ACTCCCGCACACCCTCCTCTGAGAGGCCGCTGTGTTCCGCAGGGCCAGGCCCTGGACAGT
TCCCCTCACCTGCCACTAGAGAAACACCTGCCATTGTCGTCCCCACCTGGAAAAGACCAC
TCGTGGAGCCCCCAGCCCCAGGTACAGCTGTAGAGACAGTCCTCGAGGCCCCTAAGAAG
GAGCCATGCCCAGTTCTGCCGGGACCCTCGGCCAGGCCGACAGGAGTGGACGCTGGAGC
TGGGCCCACACTGGGCCACATAGGAGCTCACCAGTGAGGGCAGGAGAGCACATGCCGGG
GAGCACCCAGCCTCCTGCTGACCAGAGGCCCGTCCCAGAGCCCAGGAGGCTGCAGAGGC
CTCTCCAGGGAGACACTGTGCATGTCTGGTACCTAAGCAGCCCCCCACGTCCCCAGTCCT
GGGGGCCCCTGGCTCAGCTGTCTGGGCCCTCCCTGCTCCCTGGGAAGCTCCTCCTGACAG
CCCCGCCTCCAGTTCCAGGTGTGGATTTTGTCAGGCGATGTCACACTGTGCAGCTTCTTG
AGCAAGCACAGTGGTGCCGCCCATATCAAAAACCAGGCCAAGTAGACAGGCCCCTGCTG
CGCAGCCCCAGGCATCCACTTCACCTGCTTCTCCTGGGGCTCTCAAGGCTGCTGTCTGTCC
TCTGGCCCTCTGTGGGGAGGGTTCCCTCAGTGGGAGGTCTGTGCTCCAGGGCAGGGATGA
TTGAGATAGAAATCAAAGGCTGGCAGGGAAAGGCAGCTTCCCGCCCTGAGAGGTGCAGG
CAGCACCACGGAGCCACGGAGTCACAGAGCCACGGAGCCCCCATTGTGGGCATTTGAGA
GTGCTGTGCCCCCGGCAGGCCCAGCCCTGATGGGGAAGCCTGTCCCATCCCACAGCCCG

GGTCCCACGGGCAGCGGGCACAGAAGCTGCCAGGTTGTCCTCTATGATCCTCATCCCTCC
AGCAGCATCCCCTCCACAGTGGGGAAACTGAGGCTTGGAGCACCACCCGGCCCCCTGGA
AATGAGGCTGTGAGCCCAGACAGTGGGCCCAGAGCACTGTGAGTACCCCGGCAGTACCT
GGCTGCAGGGATCAGCCAGAGATGCCAAACCCTGAGTGACCAGCCTACAGGAGGATCCG
GCCCCACCCAGGCCACTCGATTAATGCTCAACCCCCTGCCCTGGAGACCTCTTCCAGTAC
CACCAGCAGCTCAGCTTCTCAGGGCCTCATCCCTGCAAGGAAGGTCAAGGGCTGGGCCT
GCCAGAAACACAGCACCCTCCCTAGCCCTGGCTAAGACAGGGTGGGCAGACGGCTGTGG
ACGGGACATATTGCTGGGGCATTTCTCACTGTCACTTCTGGGTGGTAGCTCTGACAAAAA
CGCAGACCCTGCCAAAATCCCCACTGCCTCCCGCTAGGGGCTGGCCTGGAATCCTGCTGT
CCTAGGAGGCTGCTGACCTCCAGGATGGCTCCGTCCCCAGTTCCAGGGCGAGAGCAGAT
CCCAGGCAGGCTGTAGGCTGGGAGGCCACCCCTGCCCTTGCCGGGGTTGAATGCAGGTG
CCCAAGGCAGGAAATGGCATGAGCACAGGGATGACCGGGACATGCCCCACCAGAGTGC
GCCCCTTCCTGCTCTGCACCCTGCACCCCCCAGGCCAGCCCACGACGTCCAACAACTGGG
CCTGGGTGGCAGCCCCACCCAGACAGGACAGACCCAGCACCCTGAGGAGGTCCTGCCAG
GGGGAGCTAAGAGCCATGAAGGAGCAAGATATGGGGCCCCCGATACAGGCACAGATGT
CAGCTCCATCCAGGACCACCCAGCCCACACCCTGAGAGGAACGTCTGTCTCCAGCCTCTG
CAGGTCGGGAGGCAGCTGACCCCTGACTTGGACCCCTATTCCAGACACCAGACAGAGGC
GCAGGCCCCCCAGAACCAGGGTTGAGGGACGCCCCGTCAAAGCCAGACAAAACCAAGG
GGTGTTGAGCCCAGCAAGGGAAGGCCCCCAAACAGACCAGGAGGATTTTGTAGGTGTCT
GTGTCACTGTGCATGGCAGCTACTGCCAGCCCGCAGCCACTGGCTACTGAGGATGCCGAT
TCTGAGAATAGCAGCTTCTACTACTATGACTACCTGGATGAAGTAGCTTTCATGCTCAGC
CGGAAGGATGCTGTGGTTAGCTTTGGCAAAGTTTTCCTGCCACCCACAGTGACACTCACC
CAGTCAAAAACCCCATTCCAAGTCAGCGGAAGCAGAGAGAGCAGGGAGGACACGTTTA
GGATCTGAGACTGCACCTGACACCCAGGCCAGCAGACGTCTCCCCTCCAGGGCACCCCA
CCCTGTCCTGCATTTCTGCAAGATCAGGGGCGGCCTGAGGGGGGGTCTAGGGTGAGGAG
ATGGGTCCCCTGTACACCAAGGAGGAGTTAGGCAGGTCCCGAGCACTCTTAATTAAACG
ACGCCTCGAATGGAACTACTACAACGAATGGTTGCTCTACGTAATGCATTCGCTACCTTA
GGACCGTTATAGTTAGGCGCGCC

D6-DH114619 (SEQ ID NO:133) includes D6 coding sequence inserted in positions corresponding to D_{H}1-14 to D_{H}6-19:

TACGTATTAATTAAACGACGCCTCGAATGGAACTACTACAACGAATGGTTGCTCTCCCCA
TTGAGGCTGACCTGCCCAGAGAGTCCTGGGCCCACCCCACACACCGGGGCGGAATGTGT
GCAGGCCTCGGTCTCTGTGGGTGTTCCGCTAGCTGGGGCTCACAGTGCTCACCCCACACC
TAAAATGAGCCACAGCCTCCGGAGCCCCCGCAGGAGACCCCGCCCACAAGCCCAGCCCC
CACCCAGGAGGCCCCAGAGCTCAGGGCGCCCCGTCGGATTTTGTACAGCCCCGAGTCAC
TGTGCAAACCCATGAAAACCCCAAGGGAGTTTGGAACCACAGTGAGAATAGCTACGTCA
AAAACCGTCCAGTGGCCACTGCCGGAGGCCCCGCCAGAGAGGGCAGCAGCCACTCTGAT
CCCATGTCCTGCCGGCTCCCATGACCCCCAGCACGCGGAGCCCCACAGTGTCCCCACTGG
ATGGGAGGACAAGAGCTGGGGATTCCGGCGGGTCGGGGCAGGGGCTTGATCGCATCCTT
CTGCCGTGGCTCCAGTGCCCCTGGCTGGAGTTGACCCTTCTGACAAGTGTCCTCAGAGAG
ACAGGCATCACCGGCGCCTCCCAACATCAACCCCAGGCAGCACAGGCACAAACCCCACA
TCCAGAGCCAACTCCAGGAGCAGAGACACCCCAATACCCTGGGGGACCCCGACCCTGAT
GACTTCCCACTGGAATTCGCCGTAGAGTCCACCAGGACCAAAGACCCTGCCTCTGCCTCT
GTCCCTCACTCAGGACCTGCTGCCGGGCGAGGCCTTGGGAGCAGACTTGGGCTTAGGGG
ACACCAGTGTGACCCCGACCTTGACCAGGACGCAGACCTTTCCTTCCTTTCCTGGGGCAG
CACAGACTTTGGGGTCTGGGCCAGGAGGAACTTCTGGCAGGTCGCCAAGCACAGAGGCC

ACAGGCTGAGGTGGCCCTGGAAAGACCTCCAGGAGGTGGCCACTCCCCTTCCTCCCAGCT
GGACCCCATGTCCTCCCCAAGATAAGGGTGCCATCCAAGGCAGGTGCTCCTTGGAGCCCC
ATTCAGACTCCTCCCTGGACCCCACTGGGCCTCAGTCCCAGCTCTGGGGATGAAGCCACC
ACAAGCACACCAGGCAGCCCAGGCCCAGCCACCCTGCAGTGCCCAAGCACACACTCTGG
AGCAGAGCAGGGTGCCTCTGGGAGGGGCTGAGCTCCCCACCCCACCCCCACCTGCACAC
CCCACCCACCCCTGCCCAGCGGCTCTGCAGGAGGGTCAGAGCCCCACATGGGGTATGGA
CTTAGGGTCTCACTCACGTGGCTCCCATCATGAGTGAAGGGGCCTCAAGCCCAGGTTCCC
ACAGCAGCGCCTGTCGCAAGTGGAGGCAGAGGCCCGAGGGCCACCCTGACCTGGTCCCT
GAGGTTCCTGCAGCCCAGGCTGCCCTGCTGTCCCTGGGAGGCCTGGGCTCCACCAGACCA
CAGGTCCAGGGCACCGGGTGCAGGAGCCACCCACACACAGCTCACAGGAAGAAGATAA
GCTCCAGACCCCCAGGGCCAGAACCTGCCTTCCTGCTACTGCTTCCTGCCCCAGACCTGG
GCGCCCTCCCCCGTCCACTTACACACAGGCCAGGAAGCTGTTCCCACACAGAACAACCCC
AAACCAGGACCGCCTGGCACTCAGGTGGCTGCCATTTCCTTCTCCATTTGCTCCCAGCGC
CTCTGTCCTCCCTGGTTCCTCCTTCGGGGGAACAGCCTGTGCAGCCAGTCCCTGCAGCCC
ACACCCTGGGGAGACCCAACCCTGCCTGGGGCCCTTCCAACCCTGCTGCTCTTACTGCCC
ACCCAGAAAACTCTGGGGTCCTGTCCCTGCAGTCCCTACCCTGGTCTCCACCCAGACCCC
TGTGTATCACTCCAGACACCCCTCCCAGGCAAACCCTGCACCTGCAGGCCCTGTCCTCTT
CTGTCGCTAGAGCCTCAGTTTCTCCCCCCTGTGCCCACACCCTACCTCCTCCTGCCCACAA
CTCTAACTCTTCTTCTCCTGGAGCCCCTGAGCCATGGCATTGACCCTGCCCTCCCACCACC
CACAGCCCATGCCCTCACCTTCCTCCTGGCCACTCCGACCCCGCCCCCTCTCAGGCCAAG
CCCTGGTATTTCCAGGACAAAGGCTCACCCAAGTCTTTCCCAGGCAGGCCTGGGCTCTTG
CCCTCACTTCCCGGTTACACGGGAGCCTCCTGTGCACAGAAGCAGGGAGCTCAGCCCTTC
CACAGGCAGAAGGCACTGAAAGAAATCGGCCTCCAGCACCTTGACACACGTCCGCCCGT
GTCTCTCACTGCCCGCACCTGCAGGGAGGCTCCGCACTCCCTCTAAAGACAAGGGATCCA
GGCAGCAGCATCACGGGAGAATGCAGGGCTCCCAGACATCCCAGTCCTCTCACAGGCCT
CTCCTGGGAAGAGACCTGCAGCCACCACCAAACAGCCACAGAGGCTGCTGGATAGTAAC
TGAGTCAATGACCGACCTGGAGGGCAGGGGAGCAGTGAGCCGGAGCCCATACCATAGG
GACAGAGACCAGCCGCTGACATCCCGAGCTCCTCAATGGTGGCCCCATAACACACCTAG
GAAACATAACACACCCACAGCCCCACCTGGAACAGGGCAGAGACTGCTGAGCCCCCAGC
ACCAGCCCCAAGAAACACCAGGCAACAGTATCAGAGGGGGCTCCCGAGAAAGAGAGGA
GGGGAGATCTCCTTCACCATCAAATGCTTCCCTTGACCAAAAACAGGGTCCACGCAACTC
CCCCAGGACAAAGGAGGAGCCCCCTATACAGCACTGGGCTCAGAGTCCTCTCTGAGACA
CCCTGAGTTTCAGACAACAACCCGCTGGAATGCACAGTCTCAGCAGGAGAACAGACCAA
AGCCAGCAAAAGGGACCTCGGTGACACCAGTAGGGACAGGAGGATTTTGTGGGGGCTCG
TGTCACTGTGCAAACCCATGAAAACCCCAAGGGAGTTTGGAACTGCAAGCACAGTGACA
CAGACCCATTCAAAAACCCCTACTGCAAACACACCCACTCCTGGGGCTGAGGGGCTGGG
GGAGCGTCTGGGAAGTAGGGTCCAGGGGTGTCTATCAATGTCCAAAATGCACCAGACTC
CCCGCCAAACACCACCCCACCAGCCAGCGAGCAGGGTAAACAGAAAATGAGAGGCTCTG
GGAAGCTTGCACAGGCCCCAAGGAAAGAGCTTTGGCGGGTGTGCAAGAGGGGATGCAG
GCAGAGCCTGAGCAGGGCCTTTTGCTGTTTCTGCTTTCCTGTGCAGAGAGTTCCATAAAC
TGGTGTTCAAGATCAGTGGCTGGGAATGAGCCCAGGAGGGCAGTCTGTGGGAAGAGCAC
AGGGAAGGAGGAGCAGCCGCTATCCTACACTGTCATCTTTCAAAAGTTTGCCTTGTGACC
ACACTATTGCATCATGGGATGCTTAAGAGCTGATGTAGACACAGCTAAAGAGAGAATCA
GTGAGATGAATTTGCAGCATAGATCTGAATAAACTCTCCAGAATGTGGAGCAGTACAGA
AGCAAACACACAGAAAGTGCCTGATGCAAGGACAAAGTTCAGTGGGCACCTTCAGGCAT
TGCTGCTGGGCACAGACACTCTGAAAAGCCTTGGCAGGATCTCCCTGCGACAAAGCAGA
ACCCTCAGGCAATGCCAGCCCCAGAGCCCTCCCTGAGAGCGTCATGGGGAAAGATGTGC
AGAACAGCTGATTATCATAGACTCAAACTGAGAACAGAGCAAACGTCCATCTGAAGAAC

AGTCAAATAAGCAATGGTAGGTTCATGCAATGCAAACCCAGACAGCCAGGGGACAACAG
TAGAGGGCTACAGGCGGCTTTGCGGTTGAGTTCATGACAATGCTGAGTAATTGGAGTAA
CAGAGGAAAGCCCAAAAAATACTTTTAATGTGATTTCTTCTAAATAAAATTTACACCAGG
CAAAATGAACTGTCTTCTTAAGGGATAAACTTTCCCCTGGAAAAACTACAAGGAAAATT
AAGAAAACGATGATCACATAAACACAGTTGTGGTTACTTCTACTGGGGAAGGAAGAGGG
TATGAGCTGAGACACACAGAGTCGGCAAGTCTCCAAGCAAGCACAGAACGAATACATTA
CAGTACCTTGAATACAGCAGTTAAACTTCTAAATCGCAAGAACAGGAAAATGCACACAG
CTGTGTTTAGAAAATTCTCAGTCCAGCACTATTCATAATAGCAAAGACATTAACCCAGGT
TGGATAAATAAATGATGACACAGGCAATTGCACAATGATACAGACATACATTTAGTACA
TGAGACATCGATGATGTATCCCCAAAGAAATGACTTTAAAGAGAAAAGGCCTGATGTGT
GGTGGCACTCACCTCCCTGGGATCCCCGGACAGGTTGCAGGCACACTGTGTGGCAGGGC
AGGCTGGTACATGCTGGCAGCTCCTGGGGCCTGATGTGGAGCAAGCGCAGGGCTGTATA
CCCCCAAGGATGGCACAGTCAGTGAATTCCAGAGAGAAGCAGCTCAGCCACACTGCCCA
GGCAGAGCCCGAGAGGGACGCCCACGTACAGGGAGGCAGAGCCCAGCTCCTCCACAGC
CACCACCACCTGTGCACGGGCCACCACCTTGCAGGCACAGAGTGGGTGCTGAGAGGAGG
GGCAGGGACACCAGGCAGGGTGAGCACCCAGAGAAAACTGCAGAAGCCTCACACATCC
ACCTCAGCCTCCCCTGACCTGGACCTCACCTGGTCTGGACCTCACCTGGCCTGGGCCTCA
CCTGACCTGGACCTCACCTGGCCTGGGCTTCACCTGACCTGGACCTCACCTGGCCTCCGG
CCTCACCTGCACCTGCTCCAGGTCTTGCTGGAACCTGAGTAGCACTGAGGCTGCAGAAGC
TCATCCAGGGTTGGGGAATGACTCTGGAACTCTCCCACATCTGACCTTTCTGGGTGGAGG
CATCTGGTGGCCCTGGGAATATAAAAAGCCCCAGAATGGTGCCTGCGTGATTTGGGGGC
AATTTATGAACCCGAAAGGACATGGCCATGGGGTGGGTAGGGACATAGGGACAGATGCC
AGCCTGAGGTGGAGCCTCAGGACACAGTTGGACGCGGACACTATCCACATAAGCGAGGG
ACAGACCCGAGTGTTCCTGCAGTAGACCTGAGAGCGCTGGGCCCACAGCCTCCCCTCGGT
GCCCTGCTGCCTCCTCAGGTCAGCCCTGGACATCCCGGGTTTCCCCAGGCCAGATGGTAG
GATTTTGTTGAGGTCTGTGTCACTGTGCATGGCAGCTACTGCCAGCCCGCAGCCACTGGC
TACTGAGGATGCCGATTCTGAGAATAGCAGCTTCTACTACTATGACTACCTGGATGAAGT
AGCTTTCATGCTCTGCGAGGTTAGCCAGCATCTAGACTATGCCCACAGTGTCACACGGTC
CATCAAAAACCCATGCCACAGCCCTCCCCGCAGGGGACCGCCGCGTGCCATGTTACGATT
TTGATCGAGGACACAGCGCCATGGGTATGGTGGCTACCACAGCAGTGCAGCCCATGACC
CAAACACACAGGGCAGCAGGCACAATGGACAGGCCTGTGAGTGACCATGCTGGGCTCCA
GCCCGCCAGCCCCGGAGACCATGAAACAGATGGCCAAGGTCACCCCACAGTTCAGCCAG
ACATGGCTCCGTGGGGTCTGCATCGCTGCTGCCCTCTAACACCAGCCCAGATGGGGACAA
GGCCAACCCCACATTACCATCTCCTGCTGTCCACCCAGTGGTCCCAGAAGCCCCTCCCTC
ATGGCTGAGCCACATGTGTGAACCCTGAGAGCACCCCATGTCAGAGTAGGGGCAGCAGA
AGGGCGGGGCTGGCCCTGTGCACTGTCCCTGCACCCATGGTCCCTCGCCTGCCTGGCCCT
GACACCTGAGCCTCTTCTGAGTCATTTCTAAGATAGAAGACATTCCCGGGGACAGCCGGA
GCTGGGCGTCGCTCATCCCGCCCGGCCGTCCTGAGTCCTGCTTGTTTCCAGACCTCACCA
GGGAAGCCAACAGAGGACTCACCTCACACAGTCAGAGACAAAGAACCTTCCAGAAATCC
CTGTCTCACTCCCCAGTGGGCACCTTCTTCCAGGACATTCCTCGGTCGCATCACAGCAGG
CACCCACATCTGGATCAGGACGGCCCCCAGAACACAAGATGGCCCATGGGGACAGCCCC
ACAACCCAGGCCTTCCCAGACCCCTAAAAGGCGTCCCACCCCCTGCACCTGCCCCAGGGC
TAAAAATCCAGGAGGCTTGACTCCCGCATACCCTCCAGCCAGACATCACCTCAGCCCCCT
CCTGGAGGGGACAGGAGCCCGGGAGGGTGAGTCAGACCCACCTGCCCTCGATGGCAGGC
GGGGAAGATTCAGAAAGGCCTGAGATCCCCAGGACGCAGCACCACTGTCAATGGGGGCC
CCAGACGCCTGGACCAGGGCCTGCGTGGGAAAGGCCGCTGGGCACACTCAGGGGGATTT
TGTGAAGGCCCCTCCCACTGTGCAGCTTCTTGTGCAAGCAAACCCATGAAAACCCCAAGG
GAGTTTGGAACTGCCATGCCGATTTCGGCGGGCATGGCACCATTTGGAAGCTCTTCCTCC

GGTTCCAGCAGAACCTGCTACCACAGTGATGAAACTAGCATCAAAAACCGGCCGGACAC
CCAGGGACCATGCACACTTCTCAGCTTGGAGCTCTCCAGGACCAGAAGAGTCAGGTCTG
AGGGTTTGTAGCCAGACCCTCGGCCTCTAGGGACACCCTGGCCATCACAGCGGATGGGC
TGGTGCCCCACATGCCATCTGCTCCAAACAGGGGCTTCAGAGGGCTCTGAGGTGACTTCA
CTCATGACCACAGGTGCCCTGGCCCCTTCCCCGCCAGCTACACCGAACCCTGTCCCAACA
GCTGCCCCAGTTCCAACAGCCAATTCCTGGGGCCCAGAATTGCTGTAGACACCAGCCTCG
TTCCAGCACCTCCTGCCAATTGCCTGGATTCACATCCTGGCTGGAATCAAGAGGGCAGCA
TCCGCCAGGCTCCCAACAGGCAGGACTCCCGCACACCCTCCTCTGAGAGGCCGCTGTGTT
CCGCAGGGCCAGGCCCTGGACAGTTCCCCTCACCTGCCACTAGAGAAACACCTGCCATTG
TCGTCCCCACCTGGAAAAGACCACTCGTGGAGCCCCCAGCCCCAGGTACAGCTGTAGAG
AGACTCCCCGAGGGATCTAAGAAGGAGCCATGCGCAGTTCTGCCGGGACCCTCGGCCAG
GCCGACAGGAGTGGACACTGGAGCTGGGCCCACACTGGGCCACATAGGAGCTCACCAGT
GAGGGCAGGAGAGCACATGCCGGGGAGCACCCAGCCTCCTGCTGACCAGAGGCCCGTCC
CAGAGCCCAGGAGGCTGCAGAGGCCTCTCCAGGGGGACACTGTGCATGTCTGGTCCCTG
AGCAGCCCCCCACGTCCCCAGTCCTGGGGGCCCCTGGCACAGCTGTCTGGACCCTCCCTG
TTCCCTGGGAAGCTCCTCCTGACAGCCCCGCCTCCAGTTCCAGGTGTGGATTTTGTCAGG
GGGTGTCACACTGTGCTGCATACCCTGCTGGACCTGCAAGTATTCGGCAACTGTCGGAAG
GATGCTGTGGTTAGCTTTGGCAAAGTTTTCCTGCCACCACAGTGGTGCTGCCCATATCAA
AAACCAGGCCAAGTAGACAGGCCCCTGCTGTGCAGCCCCAGGCCTCCACTTCACCTGCTT
CTCCTGGGGCTCTCAAGGTCACTGTTGTCTGTACTCTGCCCTCTGTGGGGAGGGTTCCCTC
AGTGGGAGGTCTGTTCTCAACATCCCAGGGCCTCATGTCTGCACGGAAGGCCAATGGAT
GGGCAACCTCACATGCCGCGGCTAAGATAGGGTGGGCAGCCTGGCGGGGGACAGTACAT
ACTGCTGGGGTGTCTGTCACTGTGCCTAGTGGGGCACTGGCTCCCAAACAACGCAGTCCT
CGCCAAAATCCCCACAGCCTCCCCTGCTAGGGGCTGGCCTGATCTCCTGCAGTCCTAGGA
GGCTGCTGACCTCCAGAATGTCTCCGTCCCCAGTTCCAGGGCGAGAGCAGATCCCAGGCC
GGCTGCAGACTGGGAGGCCACCCCCTCCTTCCCAGGGTTCACTGGAGGTGACCAAGGTA
GGAAATGGCCTTAACACAGGGATGACTGCGCCATCCCCCAACAGAGTCAGCCCCCTCCT
GCTCTGTACCCCGCACCCCCCAGGCCAGTCCACGAAAACCAGGGCCCCACATCAGAGTC
ACTGCCTGGCCCGGCCCTGGGGCGGACCCCTCAGCCCCCACCCTGTCTAGAGGACTTGGG
GGGACAGGACACAGGCCCTCTCCTTATGGTTCCCCCACCTGCCTCCGGCCGGGACCCTTG
GGGTGTGGACAGAAAGGACACCTGCCTAATTGGCCCCCAGGAACCCAGAACTTCTCTCC
AGGGACCCCAGCCCGAGCACCCCCTTACCCAGGACCCAGCCCTGCCCCTCCTCCCCTCTG
CTCTCCTCTCATCACCCCATGGGAATCCGGTATCCCCAGGAAGCCATCAGGAAGGGCTGA
AGGAGGAAGCGGGGCCGTGCACCACCGGGCAGGAGGCTCCGTCTTCGTGAACCCAGGGA
AGTGCCAGCCTCCTAGAGGGTATGGTCCACCCTGCCTGGGGCTCCCACCGTGGCAGGCTG
CGGGGAAGGACCAGGGACGGTGTGGGGGAGGGCTCAGGGCCCTGCGGGTGCTCCTCCAT
CTTCGGTGAGCCTCCCCCTTCACCCACCGTCCCGCCCACCTCCTCTCCACCCTGGCTGCAC
GTCTTCCACACCATCCTGAGTCCTACCTACACCAGAGCCAGCAAAGCCAGTGCAGACAA
AGGCTGGGGTGCAGGGGGGCTGCCAGGGCAGCTTCGGGGAGGGAAGGATGGAGGGAGG
GGAGGTCAGTGAAGAGGCCCCCTTCCCCTGGGTCCAGGATCCTCCTCTGGGACCCCCGGA
TCCCATCCCCTCCTGGCTCTGGGAGGAGAAGCAGGATGGGAGAATCTGTGCGGGACCCT
CTCACAGTGGAATATCCCCACAGCGGCTCAGGCCAGACCCAAAAGCCCCTCAGTGAGCC
CTCCACTGCAGTCCTGGGCCTGGGTAGCAGCCCCTCCCACAGAGGACAGACCCAGCACC
CCGAAGAAGTCCTGCCAGGGGGAGCTCAGAGCCATGAAAGAGCAGGATATGGGGTCCCC
GATACAGGCACAGACCTCAGCTCCATCCAGGCCCACCGGGACCCACCATGGGAGGAACA
CCTGTCTCCGGGTTGTGAGGTAGCTGGCCTCTGTCTCGGACCCCACTCCAGACACCAGAC
AGAGGGGCAGGCCCCCCAAAACCAGGGTTGAGGGATGATCCGTCAAGGCAGACAAGAC
CAAGGGGCACTGACCCCAGCAAGGGAAGGCTCCCAAACAGACGAGGAGGATTTTGTAGC

TGTCTGTATCACTGTGCAGCTTCTTGTGCCATGCCGATTTCGGCGGGCATGGCACCATTTG
GAAGCTCTTCCTCCGGTTCCAGCAGAACCTGCTACCACAGTGACACTCGCCAGGTCAAAA
ACCCCGTCCCAAGTCAGCGGAAGCAGAGAGAGCAGGGAGGACACGTTTAGGATCTGAG
GCCGCACCTGACACCCAGGGCAGCAGACGTCTCCCCTCCAGGGCACCCTCCACCGTCCTG
CGTTTCTTCAAGAATAGGGGCGGCCTGAGGGGGTCCAGGGCCAGGCGATAGGTCCCCTC
TACCCCAAGGAGGAGCCAGGCAGGACCCGAGCACCGATGCATCTAACGCAGTCATGTAA
TGCTGGGTGACAGTCAGTTCGCCTACGTA

D6-DH120126 (SEQ ID NO:134) includes D6 coding sequence inserted in positions corresponding to D_{H}1-20 to D_{H}1-26:

TACGTAATGCATCTAACGCAGTCATGTAATGCTGGGTGACAGTCAGTTCGCCTCCCCATT
GAGGCTGACCTGCCCAGACGGGCCTGGGCCCACCCCACACACCGGGGCGGAATGTGTGC
AGGCCCCAGTCTCTGTGGGTGTTCCGCTAGCTGGGGCCCCCAGTGCTCACCCCACACCTA
AAGCGAGCCCCAGCCTCCAGAGCCCCCTAAGCATTCCCCGCCCAGCAGCCCAGCCCCTG
CCCCCACCCAGGAGGCCCCAGAGCTCAGGGCGCCTGGTCGGATTTTGTACAGCCCCGAG
TCACTGTGCATGCCGATTTCGGCGGGCATGGCACCATTTGGAAGCTCTTCCTCCGGTTCC
AGCAGAACCTGCTACCACAGTGAGAAAAACTGTGTCAAAAACCGACTCCTGGCAGCAGT
CGGAGGCCCCGCCAGAGAGGGGAGCAGCCGGCCTGAACCCATGTCCTGCCGGTTCCCAT
GACCCCCAGCACCCAGAGCCCCACGGTGTCCCCGTTGGATAATGAGGACAAGGGCTGGG
GGCTCCGGTGGTTTGCGGCAGGGACTTGATCACATCCTTCTGCTGTGGCCCCATTGCCTCT
GGCTGGAGTTGACCCTTCTGACAAGTGTCCTCAGAAAGACAGGGATCACCGGCACCTCC
CAATATCAACCCCAGGCAGCACAGACACAAACCCCACATCCAGAGCCAACTCCAGGAGC
AGAGACACCCCAACACTCTGGGGGACCCCAACCGTGATAACTCCCCACTGGAATCCGCC
CCAGAGTCTACCAGGACCAAAGGCCCTGCCCTGTCTCTGTCCCTCACTCAGGGCCTCCTG
CAGGGCGAGCGCTTGGGAGCAGACTCGGTCTTAGGGGACACCACTGTGGGCCCCAACTT
TGATGAGGCCACTGACCCTTCCTTCCTTTCCTGGGGCAGCACAGACTTTGGGGTCTGGGC
AGGGAAGAACTACTGGCTGGTGGCCAATCACAGAGCCCCCAGGCCGAGGTGGCCCCAAG
AAGGCCCTCAGGAGGTGGCCACTCCACTTCCTCCCAGCTGGACCCCAGGTCCTCCCCAAG
ATAGGGGTGCCATCCAAGGCAGGTCCTCCATGGAGCCCCCTTCAGACTCCTCCCGGGACC
CCACTGGACCTCAGTCCCTGCTCTGGGAATGCAGCCACCACAAGCACACCAGGAAGCCC
AGGCCCAGCCACCCTGCAGTGGGCAAGCCCACACTCTGGAGCAGAGCAGGGTGCGTCTG
GGAGGGGCTAACCTCCCCACCCCCCACCCCCCATCTGCACACAGCCACCTACCACTGCCC
AGACCCTCTGCAGGAGGGCCAAGCCACCATGGGGTATGGACTTAGGGTCTCACTCACGT
GCCTCCCCTCCTGGGAGAAGGGGCCTCATGCCCAGATCCCTGCAGCACTAGACACAGCT
GGAGGCAGTGGCCCCAGGGCCACCCTGACCTGGCATCTAAGGCTGCTCCAGCCCAGACA
GCACTGCCGTTCCTGGGAAGCCTGGGCTCCACCAGACCACAGGTCCAGGGCACAGCCCA
CAGGAGCCACCCACACACAGCTCACAGGAAGAAGATAAGCTCCAGACCCCAGGGCGGG
ACCTGCCTTCCTGCCACCACTTACACACAGGCCAGGGAGCTGTTCCCACACAGATCAACC
CCAAACCGGGACTGCCTGGCACTAGGGTCACTGCCATTTCCCTCTCCATTCCCTCCCAGT
GCCTCTGTGCTCCCTCCTTCTGGGGAACACCCTGTGCAGCCCCTCCCTGCAGCCCACACG
CTGGGGAGACCCCACCCTGCCTCGGGCCTTTTCTACCTGCTGCACTTGCCGCCCACCCAA
ACAACCCTGGGTACGTGACCCTGCAGTCCTCACCCTGATCTGCAACCAGACCCCTGTCCC
TCCCTCTAAACACCCCTCCCAGGCCAACTCTGCACCTGCAGGCCCTCCGCTCTTCTGCCAC
AAGAGCCTCAGGTTTTCCTACCTGTGCCCACCCCCTAACCCCTCCTGCCCACAACTTGAG
TTCTTCCTCTCCTGGAGCCCTTGAGCCATGGCACTGACCCTACACTCCCACCCACACACTG
CCCATGCCATCACCTTCCTCCTGGACACTCTGACCCCGCTCCCCTCCCTCTCAGACCCGGC
CCTGGTATTTCCAGGACAAAGGCTCACCCAAGTCTTCCCCATGCAGGCCCTTGCCCTCAC

TGCCTGGTTACACGGGAGCCTCCTGTGCGCAGAAGCAGGGAGCTCAGCTCTTCCACAGG
CAGAAGGCACTGAAAGAAATCAGCCTCCAGTGCCTTGACACACGTCCGCCTGTGTCTCTC
ACTGCCTGCACCTGCAGGGAGGCTCCGCACTCCCTCTAAAGATGAGGGATCCAGGCAGC
AACATCACGGGAGAATGCAGGGCTCCCAGACAGCCCAGCCCTCTCGCAGGCCTCTCCTG
GGAAGAGACCTGCAGCCACCACTGAACAGCCACGGAGGTCGCTGGATAGTAACCGAGTC
AGTGACCGACCTGGAGGGCAGGGGAGCAGTGAACCGGAGCCCATACCATAGGGACAGA
GACCAGCCGCTAACATCCCGAGCCCCTCACTGGCGGCCCCAGAACACCCCGTGGAAAGA
GAACAGACCCACAGTCCCACCTGGAACAGGGCAGACACTGCTGAGCCCCCAGCACCAGC
CCCAAGAAACACTAGGCAACAGCATCAGAGGGGGCTCCTGAGAAAGAGAGGAGGGGAG
GTCTCCTTCACCATCAAATGCTTCCCTTGACCAAAAACAGGGTCCACGCAACTCCCCCAG
GACAAAGGAGGAGCCCCCTGTACAGCACTGGGCTCAGAGTCCTCTCTGAGACAGGCTCA
GTTTCAGACAACAACCCGCTGGAATGCACAGTCTCAGCAGGAGAGCCAGGCCAGAGCCA
GCAAGAGGAGACTCGGTGACACCAGTCTCCTGTAGGGACAGGAGGATTTTGTGGGGGTT
CGTGTCACTGTGCAAACCCATGAAAACCCCAAGGGAGTTTGGAACAGCAAGCACAGTGA
CACAACCCCATTCAAAAACCCCTACTGCAAACGCACCCACTCCTGGGACTGAGGGGCTG
GGGGAGCGTCTGGGAAGTATGGCCTAGGGGTGTCCATCAATGCCCAAAATGCACCAGAC
TCTCCCCAAGACATCACCCCACCAGCCAGTGAGCAGAGTAAACAGAAAATGAGAAGCAG
CTGGGAAGCTTGCACAGGCCCCAAGGAAAGAGCTTTGGCAGGTGTGCAAGAGGGGATGT
GGGCAGAGCCTCAGCAGGGCCTTTTGCTGTTTCTGCTTTCCTGTGCAGAGAGTTCCATAA
ACTGGTATTCAAGATCAATGGCTGGGAGTGAGCCCAGGAGGACAGTGTGGGAAGAGCAC
AGGGAAGGAGGAGCAGCCGCTATCCTACACTGTCATCTTTTGAAAGTTTGCCCTGTGCCC
ACAATGCTGCATCATGGGATGCTTAACAGCTGATGTAGACACAGCTAAAGAGAGAATCA
GTGAAATGGATTTGCAGCACAGATCTGAATAAATCCTCCAGAATGTGGAGCAGCACAGA
AGCAAGCACACAGAAAGTGCCTGATGCCAAGGCAAAGTTCAGTGGGCACCTTCAGGCAT
TGCTGCTGGGCACAGACACTCTGAAAAGCACTGGCAGGAACTGCCTGTGACAAAGCAGA
ACCCTCAGGCAATGCCAGCCCTAGAGCCCTTCCTGAGAACCTCATGGGCAAAGATGTGC
AGAACAGCTGTTTGTCATAGCCCCAAACTATGGGGCTGGACAAAGCAAACGTCCATCTG
AAGGAGAACAGACAAATAAACGATGGCAGGTTCATGAAATGCAAACTAGGACAGCCAG
AGGACAACAGTAGAGAGCTACAGGCGGCTTTGCGGTTGAGTTCATGACAATGCTGAGTA
ATTGGAGTAACAGAGGAAAGCCCAAAAAATACTTTTAATGTGATTTCTTCTAAATAAAAT
TTACACCCGGCAAAATGAACTATCTTCTTAAGGGATAAACTTTCCCCTGGAAAAACTATA
AGGAAAATCAAGAAAACGATGATCACATAAACACAGTGGTGGTTACTTCTACTGGGGAA
GGAAGAGGGTATGAGCTGAGACACACAGAGTCGGCAAGTCTCCTAACAAGAACAGAAC
AAATACATTACAGTACCTTGAAAACAGCAGTTAAACTTCTAAATCGCAAGAAGAGGAAA
ATGCACACACCTGTGTTTAGAAAATTCTCAGTCCAGCACTGTTCATAATAGCAAAGACAT
TAACCCAGGTTGGATAAATAAGCGATGACACAGGCAATTGCACAATGATACAGACATAC
ATTCAGTATATGAGACATCGATGATGTATCCCCAAAGAAATGACTTTAAAGAGAAAAGG
CCTGATGTGTGGTGGCAATCACCTCCCTGGGCATCCCCGGACAGGCTGCAGGCTCACTGT
GTGGCAGGGCAGGCAGGCACCTGCTGGCAGCTCCTGGGGCCTGATGTGGAGCAGGCACA
GAGCTGTATATCCCCAAGGAAGGTACAGTCAGTGCATTCCAGAGAGAAGCAACTCAGCC
ACACTCCCTGGCCAGAACCCAAGATGCACACCCATGCACAGGGAGGCAGAGCCCAGCAC
CTCCGCAGCCACCACCACCTGCGCACGGGCCACCACCTTGCAGGCACAGAGTGGGTGCT
GAGAGGAGGGGCAGGGACACCAGGCAGGGTGAGCACCCAGAGAAAACTGCAGAAGCCT
CACACATCCCTCACCTGGCCTGGGCTTCACCTGACCTGGACCTCACCTGGCCTCGGGCCT
CACCTGCACCTGCTCCAGGTCTTGCTGGAGCCTGAGTAGCACTGAGGCTGTAGGGACTCA
TCCAGGGTTGGGGAATGACTCTGCAACTCTCCCACATCTGACCTTTCTGGGTGGAGGCAC
CTGGTGGCCCAGGGAATATAAAAAGCCCCAGAATGATGCCTGTGTGATTTGGGGGCAAT
TTATGAACCCGAAAGGACATGGCCATGGGGTGGGTAGGGACAGTAGGGACAGATGTCAG

CCTGAGGTGAAGCCTCAGGACACAGGTGGGCATGGACAGTGTCCACCTAAGCGAGGGAC
AGACCCGAGTGTCCCTGCAGTAGACCTGAGAGCGCTGGGCCCACAGCCTCCCCTCGGGG
CCCTGCTGCCTCCTCAGGTCAGCCCTGGACATCCCGGGTTTCCCCAGGCCTGGCGGTAGG
ATTTTGTTGAGGTCTGTGTCACTGTGCATGGCAGCTACTGCCAGCCCGCAGCCACTGGCT
ACTGAGGATGCCGATTCTGAGAATAGCAGCTTCTACTACTATGACTACCTGGATGAAGTA
GCTTTCATGCTCAGCGAGGTTAGCCAGCATCTAGACTATGCCCACAGTGTCACAGAGTCC
ATCAAAAACCCATGCCTGGGAGCCTCCCACCACAGCCCTCCCTGCGGGGGACCGCTGCA
TGCCGTGTTAGGATTTTGATCGAGGACACGGCGCCATGGGTATGGTGGCTACCACAGCA
GTGCAGCCCATGACCCAAACACACGGGGCAGCAGAAACAATGGACAGGCCCACAAGTG
ACCATGATGGGCTCCAGCCCACCAGCCCCAGAGACCATGAAACAGATGGCCAAGGTCAC
CCTACAGGTCATCCAGATCTGGCTCCAAGGGGTCTGCATCGCTGCTGCCCTCCCAACGCC
AAACCAGATGGAGACAGGGCCGGCCCCATAGCACCATCTGCTGCCGTCCACCCAGCAGT
CCCGGAAGCCCCTCCCTGAACGCTGGGCCACGTGTGTGAACCCTGCGAGCCCCCCATGTC
AGAGTAGGGGCAGCAGGAGGGCGGGGCTGGCCCTGTGCACTGTCACTGCCCCTGTGGTC
CCTGGCCTGCCTGGCCCTGACACCTGAGCCTCTCCTGGGTCATTTCCAAGACATTCCCAG
GGACAGCCGGAGCTGGGAGTCGCTCATCCTGCCTGGCTGTCCTGAGTCCTGCTCATTTCC
AGACCTCACCAGGGAAGCCAACAGAGGACTCACCTCACACAGTCAGAGACAACGAACCT
TCCAGAAATCCCTGTTTCTCTCCCCAGTGAGAGAAACCCTCTTCCAGGGTTTCTCTTCTCT
CCCACCCTCTTCCAGGACAGTCCTCAGCAGCATCACAGCGGGAACGCACATCTGGATCA
GGACGGCCCCCAGAACACGCGATGGCCCATGGGGACAGCCCAGCCCTTCCCAGACCCCT
AAAAGGTATCCCCACCTTGCACCTGCCCCAGGGCTCAAACTCCAGGAGGCCTGACTCCTG
CACACCCTCCTGCCAGATATCACCTCAGCCCCCTCCTGGAGGGGACAGGAGCCCGGGAG
GGTGAGTCAGACCCACCTGCCCTCAATGGCAGGCGGGGAAGATTCAGAAAGGCCTGAGA
TCCCCAGGACGCAGCACCACTGTCAATGGGGGCCCCAGACGCCTGGACCAGGGCCTGTG
TGGGAAAGGCCTCTGGCCACACTCAGGGGGATTTTGTGAAGGGCCCTCCCACTGTGGAG
GTTAGCCAGCATCTAGACTATGCCCACAGTGATGAAACCAGCATCAAAAACCGACCGGA
CTCGCAGGGTTTATGCACACTTCTCGGCTCGGAGCTCTCCAGGAGCACAAGAGCCAGGCC
CGAGGGTTTGTGCCCAGACCCTCGGCCTCTAGGGACACCCGGGCCATCTTAGCCGATGGG
CTGATGCCCTGCACACCGTGTGCTGCCAAACAGGGGCTTCAGAGGGCTCTGAGGTGACTT
CACTCATGACCACAGGTGCCCTGGTCCCTTCACTGCCAGCTGCACCAGACCCTGTTCCGA
GAGATGCCCCAGTTCCAAAAGCCAATTCCTGGGGCCGGGAATTACTGTAGACACCAGCC
TCATTCCAGTACCTCCTGCCAATTGCCTGGATTCCCATCCTGGCTGGAATCAAGAGGGCA
GCATCCGCCAGGCTCCCAACAGGCAGGACTCCCACACACCCTCCTCTGAGAGGCCGCTGT
GTTCCGCAGGGCCAGGCCGCAGACAGTTCCCCTCACCTGCCCATGTAGAAACACCTGCCA
TTGTCGTCCCCACCTGGCAAAGACCACTTGTGGAGCCCCCAGCCCCAGGTACAGCTGTAG
AGAGAGTCCTCGAGGCCCCTAAGAAGGAGCCATGCCCAGTTCTGCCGGGACCCTCGGCC
AGGCCGACAGGAGTGGACGCTGGAGCTGGGCCCACACTGGGCCACATAGGAGCTCACCA
GTGAGGGCAGGAGAGCACATGCCGGGGAGCACCCAGCCTCCTGCTGACCAGAGACCCGT
CCCAGAGCCCAGGAGGCTGCAGAGGCCTCTCCAGGGGGACACAGTGCATGTCTGGTCCC
TGAGCAGCCCCCAGGCTCTCTAGCACTGGGGGCCCCTGGCACAGCTGTCTGGACCCTCCC
TGTTCCCTGGGAAGCTCCTCCTGACAGCCCCGCCTCCAGTTCCAGGTGTGGATTTTGTCA
GGGGGTGCCACACTGTGCTGCATACCCTGCTGGACCTGCAAGTATTCGGCAACAGTCGG
AAGGATGCTGTGGTTAGCTTTGGCAAAGTTTTCCTGCCACCACAGTGGTGCCGCCCATAT
CAAAAACCAGGCCAAGTAGACAGACCCCTGCCACGCAGCCCCAGGCCTCCAGCTCACCT
GCTTCTCCTGGGGCTCTCAAGGCTGCTGTCTGCCCTCTGGCCCTCTGTGGGGAGGGTTCCC
TCAGTGGGAGGTCTGTGCTCCAGGGCAGGGATGACTGAGATAGAAATCAAAGGCTGGCA
GGGAAAGGCAGCTTCCCGCCCTGAGAGGTGCAGGCAGCACCACAGAGCCATGGAGTCAC
AGAGCCACGGAGCCCCCAGTGTGGGCGTGTGAGGGTGCTGGGCTCCCGGCAGGCCCAGC

CCTGATGGGGAAGCCTGCCCCGTCCCACAGCCCAGGTCCCCAGGGGCAGCAGGCACAGA
AGCTGCCAAGCTGTGCTCTACGATCCTCATCCCTCCAGCAGCATCCACTCCACAGTGGGG
AAACTGAGCCTTGGAGAACCACCCAGCCCCCTGGAAACAAGGCGGGGAGCCCAGACAGT
GGGCCCAGAGCACTGTGTGTATCCTGGCACTAGGTGCAGGGACCACCCGGAGATCCCCA
TCACTGAGTGGCCAGCCTGCAGAAGGACCCAACCCCAACCAGGCCGCTTGATTAAGCTC
CATCCCCCTGTCCTGGGAACCTCTTCCCAGCGCCACCAACAGCTCGGCTTCCCAGGCCCT
CATCCCTCCAAGGAAGGCCAAAGGCTGGGCCTGCCAGGGGCACAGTACCCTCCCTTGCC
CTGGCTAAGACAGGGTGGGCAGACGGCTGCAGATAGGACATATTGCTGGGGCATCTTGC
TCTGTGACTACTGGGTACTGGCTCTCAACGCAGACCCTACCAAAATCCCCACTGCCTCCC
CTGCTAGGGGCTGGCCTGGTCTCCTCCTGCTGTCCTAGGAGGCTGCTGACCTCCAGGATG
GCTTCTGTCCCCAGTTCTAGGGCCAGAGCAGATCCCAGGCAGGCTGTAGGCTGGGAGGC
CACCCCTGTCCTTGCCGAGGTTCAGTGCAGGCACCCAGGACAGGAAATGGCCTGAACAC
AGGGATGACTGTGCCATGCCCTACCTAAGTCCGCCCCTTTCTACTCTGCAACCCCCACTC
CCCAGGTCAGCCCATGACGACCAACAACCCAACACCAGAGTCACTGCCTGGCCCTGCCC
TGGGGAGGACCCCTCAGCCCCCACCCTGTCTAGAGGAGTTGGGGGGACAGGACACAGGC
TCTCTCCTTATGGTTCCCCCACCTGGCTCCTGCCGGGACCCTTGGGGTGTGGACAGAAAG
GACGCCTGCCTAATTGGCCCCCAGGAACCCAGAACTTCTCTCCAGGGACCCCAGCCCGA
GCACCCCCTTACCCAGGACCCAGCCCTGCCCCTCCTCCCCTCTGCTCTCCTCTCATCACTC
CATGGGAATCCAGAATCCCCAGGAAGCCATCAGGAAGGGCTGAAGGAGGAAGCGGGGC
CGCTGCACCACCGGGCAGGAGGCTCCGTCTTCGTGAACCCAGGGAAGTGCCAGCCTCCT
AGAGGGTATGGTCCACCCTGCCTGGGGCTCCCACCGTGGCAGGCTGCGGGGAAGGACCA
GGGACGGTGTGGGGGAGGGCTCAGGGCCCTGCAGGTGCTCCATCTTGGATGAGCCCATC
CCTCTCACCCACCGACCCGCCCACCTCCTCTCCACCCTGGCCACACGTCGTCCACACCAT
CCTGAGTCCCACCTACACCAGAGCCAGCAGAGCCAGTGCAGACAGAGGCTGGGGTGCAG
GGGGGCCGCCAGGGCAGCTTTGGGGAGGGAGGAATGGAGGAAGGGGAGGTCAGTGAAG
AGGCCCCCCTCCCCTGGGTCTAGGATCCACCTTTGGGACCCCCGGATCCCATCCCCTCCA
GGCTCTGGGAGGAGAAGCAGGATGGGAGATTCTGTGCAGGACCCTCTCACAGTGGAATA
CCTCCACAGCGGCTCAGGCCAGATACAAAAGCCCCTCAGTGAGCCCTCCACTGCAGTGC
AGGGCCTGGGGGCAGCCCCTCCCACAGAGGACAGACCCAGCACCCCGAAGAAGTCCTGC
CAGGGGGAGCTCAGAGCCATGAAGGAGCAAGATATGGGGACCCCAATACTGGCACAGA
CCTCAGCTCCATCCAGGCCCACCAGGACCCACCATGGGTGGAACACCTGTCTCCGGCCCC
TGCTGGCTGTGAGGCAGCTGGCCTCTGTCTCGGACCCCCATTCCAGACACCAGACAGAGG
GACAGGCCCCCCAGAACCAGTGTTGAGGGACACCCCTGTCCAGGGCAGCCAAGTCCAAG
AGGCGCGCTGAGCCCAGCAAGGGAAGGCCCCCAAACAAACCAGGAGGTTTCTGAAGCTG
TCTGTGTCACAGTCGGGCATAGCCACGGCTACCACAATGACACTGGGCAGGACAGAAAC
CCCATCCCAAGTCAGCCGAAGGCAGAGAGAGCAGGCAGGACACATTTAGGATCTGAGGC
CACACCTGACACTCAAGCCAACAGATGTCTCCCCTCCAGGGCGCCCTGCCCTGTTCAGTG
TTCCTGAGAAAACAGGGGCAGCCTGAGGGGATCCAGGGCCAGGAGATGGGTCCCCTCTA
CCCCGAGGAGGAGCCAGGCGGGAATCCCAGCCCCCTCCCCATTGAGGCCATCCTGCCCA
GAGGGGCCCGGACCCACCCCACACACCCAGGCAGAATGTGTGCAGGCCTCAGGCTCTGT
GGGTGCCGCTAGCTGGGGCTGCCAGTCCTCACCCCACACCTAAGGTGAGCCACAGCCGC
CAGAGCCTCCACAGGAGACCCCACCCAGCAGCCCAGCCCCTACCCAGGAGGCCCCAGAG
CTCAGGGCGCCTGGGTGGATTTTGTACAGCCCCGAGTCACTGTGCTGCATACCCTGCTGG
ACCTGCAAGTATTCGGCAACCACAGTGAGAAAAGCTATGTCAAAAACCGTCTCCCGGCC
ACTGCTGGAGGCCCAGCCAGAGAAGGGACCAGCCGCCCGAACATACGACCTTCCCAGAC
CTCATGACCCCCAGCACTTGGAGCTCCACAGTGTCCCCATTGGATGGTGAGGATGGGGGC
CGGGGCCATCTGCACCTCCCAACATCACCCCCAGGCAGCACAGGCACAAACCCCAAATC
CAGAGCCGACACCAGGAACACAGACACCCCAATACCCTGGGGGACCCTGGCCCTGGTGA

CTTCCCACTGGGATCCACCCCCGTGTCCACCTGGATCAAAGACCCCACCGCTGTCTCTGT
CCCTCACTCAGGGCCTGCTGAGGGGCGGGTGCTTTGGAGCAGACTCAGGTTTAGGGGCC
ACCATTGTGGGGCCCAACCTCGACCAGGACACAGATTTTTCTTTCCTGCCCTGGGGCAAC
ACAGACTTTGGGGTCTGTGCAGGGAGGACCTTCTGGAAAGTCACCAAGCACAGAGCCCT
GACTGAGGTGGTCTCAGGAAGACCCCCAGGAGGGGGCTTGTGCCCCTTCCTCTCATGTGG
ACCCCATGCCCCCCAAGATAGGGGCATCATGCAGGGCAGGTCCTCCATGCAGCCACCAC
TAGGCAACTCCCTGGCGCCGGTCCCCACTGCGCCTCCATCCCGGCTCTGGGGATGCAGCC
ACCATGGCCACACCAGGCAGCCCGGGTCCAGCAACCCTGCAGTGCCCAAGCCCTTGGCA
GGATTCCCAGAGGCTGGAGCCCACCCCTCCTCATCCCCCCACACCTGCACACACACACCT
ACCCCCTGCCCAGTCCCCCTCCAGGAGGGTTGGAGCCGCCCATAGGGTGGGGGCTCCAG
GTCTCACTCACTCGCTTCCCTTCCTGGGCAAAGGAGCCTCGTGCCCCGGTCCCCCCTGAC
GGCGCTGGGCACAGGTGTGGGTACTGGGCCCCAGGGCTCCTCCAGCCCCAGCTGCCCTG
CTCTCCCTGGGAGGCCTGGGCACCACCAGACCACCAGTCCAGGGCACAGCCCCAGGGAG
CCGCCCACTGCCAGCTCACAGGAAGAAGATAAGCTTCAGACCCTCAGGGCCGGGAGCTG
CCTTCCTGCCACCCCTTCCTGCCCCAGACCTCCATGCCCTCCCCCAACCACTTACACACAA
GCCAGGGAGCTGTTTCCACACAGTTCAACCCCAAACCAGGACGGCCTGGCACTCGGGTC
ACTGCCATTTCTGTCTGCATTCGCTCCCAGCGCCCCTGTGTTCCCTCCCTCCTCCCTCCTTC
CTTTCTTCCTGCATTGGGTTCATGCCGCAGAGTGCCAGGTGCAGGTCAGCCCTGAGCTTG
GGGTCACCTCCTCACTGAAGGCAGCCTCAGGGTGCCCAGGGGCAGGCAGGGTGGGGGTG
AGGCTTCCAGCTCCAACCGCTTCGCTACCTTAGGACCGTTATAGTTAGGCGCGCCGTCGA
CCAATTCTCATGTTTGACAGCTTATCATCGAATTTCTACGTA

### Toxin coding sequences

Exemplary nucleotide coding sequences (DNA and amino acid (AA)) of toxins (e.g., µ-conotoxin and tarantula toxin ProTxII) for construction of an engineered D_{H} region as described herein are set forth in Table 4.

**TABLE 4**

| |
|---|
| **SmIIIA C1SC4S DNA** |
| GAGAGAAGCTGCAATGGCAGACGCGGCTGCAGCAGCAGATGGAGCCGCGATCATAGCAGGTG CTGC (SEQ ID NO:180) |
| **SmIIIA C1SC4S AA** |
| ERSCNGRRGCSSRWSRDHSRCC (SEQ ID NO:181) |
| **CSSRWC DNA** |
| AGGATATTGTAGCAGCAGATGGTGCTATACC (SEQ ID NO:182) |
| **CSSRWC AA** |
| GYCSSRWCYT (SEQ ID NO:183) |
| **KIIIA mini DNA** |
| GTATTACGATTGCAACTGCAGCAGATGGCGCGACCATAGCAGGTGCTGCTATTATACC (SEQ ID NO: 184) |
| **KIIIA mini AA** |
| YYDCNCSRWRDHSRCCYYT (SEQ ID NO:185) |
| **PIIIA C1SC4S DNA** |
| GAGAGGCTTAGCTGTGGCTTCCCTAAGAGCTGCCGCAGCAGGCAAAGCAAGCCTCACAGATGC |
| TGC (SEQ ID NO:186) |
| **PIIIA C1SC4S AA** |
| ERLSCGFPKSCRSRQSKPHRCC (SEQ ID NO:187) |
| **ProTxII C1SC4S DNA** |
| TACAGCCAGAAGTGGATGTGGACTTGCGATAGTGAGAGGAAGTGCAGTGAGGGTATGGTATGC CGGCTGTGGTGTAAGAAGAAGCTCTGG (SEQ ID NO:188) |
| **ProTxII C1SC4S AA** |
| YSQKWMWTCDSERKCSEGMVCRLWCKKKLW (SEQ ID NO:189) |
| **KIIIA C1SC4S DNA** |
| AGCTGCAACTGCAGCAGCAAATGGAGCCGCGACCATAGCAGGTGCTGC (SEQ ID NO:190) |
| **KIIIA C1SC4S AA** |
| SCNCSSKWSRDHSRCC (SEQ ID NO:191) |
| **SmIIIA mini DNA** |
| GAGAGATGCAATGGCAGACGCGGCTGCAGCAGATGGCGCGATCATAGCAGGTGCTGC (SEQ ID NO: 192) |
| **SmIIIA mini AA** |
| ERCNGRRGCSRWRDHSRCC (SEQ ID NO:193) |
| **RSRQ insertion DNA** |
| AGGATATTGTACTAATCGGAGCAGGCAGGGTGTATGCTATACC (SEQ ID NO:194) |
| **RSRQ insertion AA** |
| GYCTNRSRQGVCYT (SEQ ID NO:195) |
| **KIIIA midi DNA** |
| GTATTACGATTGCAACTGCAGCAGATGGGCTCGCGACCATAGCAGGTGCTGCTATTATAAC (SEQ ID NO:196) |
| **KIIIA midi AA** |
| YYDCNCSRWARDHSRCCYYN (SEQ ID NO:197) |
| **SmIIIA mini DNA** |
| GTATTACTATGAGAGATGCAATGGCAGACGCGGCTGCAGCAGATGGCGCGATCATAGCAGGTG CTGCTATTATAAC (SEQ ID NO:198) |
| **SmIIIA mini AA** |
| YYYERCNGRRGCSRWRDHSRCCYYN (SEQ ID NO:199) |
| **PIIIA mini DNA** |
| GAGAGGCTTTGTGGCTTCCCTAAGAGCTGCAGCAGGCAAAAGCCTCACAGATGCTGC (SEQ ID NO:200) |
| **PIIIA mini AA** |
| ERLCGFPKSCSRQKPHRCC (SEQ ID NO:201) |
| **ProTxII C2SC5S DNA** |
| TACTGCCAGAAGTGGATGTGGACTAGCGATAGTGAGAGGAAGTGCTGTGAGGGTATGGTAAGC CGGCTGTGGTGTAAGAAGAAGCTCTGG (SEQ ID NO:202) |
| **ProTxII C2SC5S AA** |
| YCQKWMWTSDSERKCCEGMVSRLWCKKKLW (SEQ ID NO:203) |
| **KIIIA mini DNA** |
| TGCAACTGCAGCAGATGGCGCGACCATAGCAGGTGCTG (SEQ ID NO:204) |
| **KIIIA mini AA** |
| CNCSRWRDHSRC (SEQ ID NO:205) |
| **SmIIIA fl DNA** |
| GAGAGATGCTGCAATGGCAGACGCGGCTGCAGCAGCAGATGGTGCCGCGATCATAGCAGGTGC |
| TGC (SEQ ID NO:206) |
| **SmIIIA fl AA** |
| ERCCNGRRGCSSRWCRDHSRCC (SEQ ID NO:207) |
| **SSRW insertion DNA** |
| AGGATATTGTAGTGGTAGCAGCAGATGGGGTAGCTGCTACTCC (SEQ ID NO:208) |
| **SSRW insertion AA** |
| GYCSGSSRWGSCYS (SEQ ID NO:209) |
| **SmIIIA midi DNA** |
| GTATTATGATTACGAGAGAGCTTGCAATGGCAGACGCGGCTGCAGCAGATGGGCTCGCGATCA TAGCAGGTGCTGCTATCGTTATACC (SEQ ID NO:210) |
| **SmIIIA midi AA** |
| YYDYERACNGRRGCSRWARDHSRCCYRYT (SEQ ID NO:211) |
| **PIIIA midi DNA** |
| GAGAGGCTTGCTTGTGGCTTCCCTAAGAGCTGCAGCAGGCAAGCTAAGCCTCACAGATGCTGC (SEQ ID NO:212) |
| **PIIIA midi AA** |
| ERLACGFPKSCSRQAKPHRCC (SEQ ID NO:213) |
| **ProTxII C3SC6S DNA** |
| TACTGCCAGAAGTGGATGTGGACTTGCGATAGTGAGAGGAAGAGCTGTGAGGGTATGGTATGC CGGCTGTGGAGTAAGAAGAAGCTCTGG (SEQ ID NO:214) |
| **ProTxII C3SC6S AA** |
| YCQKWMWTCDSERKSCEGMVCRLWSKKKLW (SEQ ID NO:215) |
| **KIIIA midi DNA** |
| TGCAACTGCAGCAGATGGGCTCGCGACCATAGCAGGTGCTG (SEQ ID NO:216) |
| **KIIIA midi AA** |
| CNCSRWARDHSRC (SEQ ID NO:217) |
| **SmIIIA midi DNA** |
| GAGAGAGCTTGCAATGGCAGACGCGGCTGCAGCAGATGGGCTCGCGATCATAGCAGGTGCTGC (SEQ ID NO:218) |
| **SmIIIA midi AA** |
| ERACNGRRGCSRWARDHSRCC (SEQ ID NO:219) |
| **CRSRQC DNA** |
| AGCATATTGTCGGAGCAGGCAGTGCTATTCC (SEQ ID NO:220) |
| **CRSRQC AA** |
| AYCRSRQCYS (SEQ ID NO:221) |
| **PIIIA midi DNA** |
| GTATTACTATGAGAGGCTTGCTTGTGGCTTCCCTAAGAGCTGCAGCAGGCAAGCTAAGCCTCAC AGATGCTGCTATTACTAC (SEQ ID NO:222) |
| **PIIIA midi AA** |
| YYYERLACGFPKSCSRQAKPHRCCYYY (SEQ ID NO:223) |
| **PIIA fl DNA** |
| GAGAGGCTTTGCTGTGGCTTCCCTAAGAGCTGCCGCAGCAGGCAATGCAAGCCTCACAGATGCT GC (SEQ ID NO:224) |
| **PIIA fl AA** |
| ERLCCGFPKSCRSRQCKPHRCC (SEQ ID NO:225) |
| **ProTxII fl DNA** |
| TACTGCCAGAAGTGGATGTGGACTTGCGATAGTGAGAGGAAGTGCTGTGAGGGTATGGTATGC |
| CGGCTGTGGTGTAAGAAGAAGCTCTGG (SEQ ID NO:226) |
| **ProTxII fl AA** |
| YCQKWMWTCDSERKCCEGMVCRLWCKKKLW (SEQ ID NO:227) |
| **KIIIA fl DNA** |
| TGCTGCAACTGCAGCAGCAAATGGTGCCGCGACCATAGCAGGTGCTGC (SEQ ID NO:228) |
| **KIIIA fl AA** |
| CCNCSSKWCRDHSRCC (SEQ ID NO:229) |
| **PIIIA mini DNA** |
| GGTATAGTGGGGAGAGGCTTTGTGGCTTCCCTAAGAGCTGCAGCAGGCAAAAGCCTCACAGAT GCTGCAGCTACTAC (SEQ ID NO:230) |
| **PIIIA mini AA** |
| YSGERLCGFPKSCSRQKPHRCCSYY (SEQ ID NO:231) |

Exemplary DNA fragments containing toxin nucleotide coding sequences for construction of an engineered D_{H} region are provided below.

TX-DH1166 (SEQ ID NO:232) includes toxin coding sequences inserted in positions corresponding to D_{H}1-1 to D_{H}6-6:

TACGTAGCCGTTTCGATCCTCCCGAATTGACTAGTGGGTAGGCCTGGCGGCCGCTGCCAT
TTCATTACCTCTTTCTCCGCACCCGACATAGATACCGGTGGATTCGAATTCTCCCCGTTGA
AGCTGACCTGCCCAGAGGGGCCTGGGCCCACCCCACACACCGGGGCGGAATGTGTACAG
GCCCCGGTCTCTGTGGGTGTTCCGCTAACTGGGGCTCCCAGTGCTCACCCCACAACTAAA
GCGAGCCCCAGCCTCCAGAGCCCCCGAAGGAGATGCCGCCCACAAGCCCAGCCCCCATC
CAGGAGGCCCCAGAGCTCAGGGCGCCGGGGCGGATTTTGTACAGCCCCGAGTCACTGTG
GAGAGAAGCTGCAATGGCAGACGCGGCTGCAGCAGATGGAGCCGCGATCATAGCA
GGTGCTGCCACAGTGAGAAAAACTGTGTCAAAAACCGTCTCCTGGCCCCTGCTGGAGGC
CGCGCCAGAGAGGGGAGCAGCCGCCCCGAACCTAGGTCCTGCTCAGCTCACACGACCCC
CAGCACCCAGAGCACAACGGAGTCCCCATTGAATGGTGAGGACGGGGACCAGGGCTCCA
GGGGGTCATGGAAGGGGCTGGACCCCATCCTACTGCTATGGTCCCAGTGCTCCTGGCCAG
AACTGACCCTACCACCGACAAGAGTCCCTCAGGGAAACGGGGGTCACTGGCACCTCCCA
GCATCAACCCCAGGCAGCACAGGCATAAACCCCACATCCAGAGCCGACTCCAGGAGCAG
AGACACCCCAGTACCCTGGGGGACACCGACCCTGATGACTCCCCACTGGAATCCACCCC
AGAGTCCACCAGGACCAAAGACCCCGCCCCTGTCTCTGTCCCTCACTCAGGACCTGCTGC
GGGGCGGGCCATGAGACCAGACTCGGGCTTAGGGAACACCACTGTGGCCCCAACCTCGA
CCAGGCCACAGGCCCTTCCTTCCTGCCCTGCGGCAGCACAGACTTTGGGGTCTGTGCAGA
GAGGAATCACAGAGGCCCCAGGCTGAGGTGGTGGGGGTGGAAGACCCCCAGGAGGTGG
CCCACTTCCCTTCCTCCCAGCTGGAACCCACCATGACCTTCTTAAGATAGGGGTGTCATC
CGAGGCAGGTCCTCCATGGAGCTCCCTTCAGGCTCCTCCCCGGTCCTCACTAGGCCTCAG
TCCCGGCTGCGGGAATGCAGCCACCACAGGCACACCAGGCAGCCCAGACCCAGCCAGCC
TGCAGTGCCCAAGCCCACATTCTGGAGCAGAGCAGGCTGTGTCTGGGAGAGTCTGGGCT
CCCCACCGCCCCCCCGCACACCCCACCCACCCCTGTCCAGGCCCTATGCAGGAGGGTCAG
AGCCCCCCATGGGGTATGGACTTAGGGTCTCACTCACGTGGCTCCCCTCCTGGGTGAAGG
GGTCTCATGCCCAGATCCCCACAGCAGAGCTGGTCAAAGGTGGAGGCAGTGGCCCCAGG
GCCACCCTGACCTGGACCCTCAGGCTCCTCTAGCCCTGGCTGCCCTGCTGTCCCTGGGAG
GCCTGGACTCCACCAGACCACAGGTCCAGGGCACCGCCCATAGGTGCTGCCCACACTCA

GTTCACAGGAAGAAGATAAGCTCCAGACCCCCAAGACTGGGACCTGCCTTCCTGCCACC
GCTTGTAGCTCCAGACCTCCGTGCCTCCCCCGACCACTTACACACGGGCCAGGGAGCTGT
TCCACAAAGATCAACCCCAAACCGGGACCGCCTGGCACTCGGGCCGCTGCCACTTCCCTC
TCCATTTGTTCCCAGCACCTCTGTGCTCCCTCCCTCCTCCCTCCTTCAGGGGAACAGCCTG
TGCAGCCCCTCCCTGCACCCCACACCCTGGGGAGGCCCAACCCTGCCTCCAGCCCTTTCT
CCCCCGCTGCTCTTCCTGCCCATCCAGACAACCCTGGGGTCCCATCCCTGCAGCCTACAC
CCTGGTCTCCACCCAGACCCCTGTCTCTCCCTCCAGACACCCCTCCCAGGCCAACCCTGC
ACATGCAGGCCCTCCCCTTTTCTGCTGCCAGAGCCTCAGTTTCTACCCTCTGTGCCTACCC
CCTGCCTCCTCCTGCCCACAACTCGAGCTCTTCCTCTCCTGGGGCCCCTGAGCCATGGCAC
TGACCGTGCACTCCCACCCCCACACTGCCCATGCCCTCACCTTCCTCCTGGACACTCTGAC
CCCGCTCCCCTCTTGGACCCAGCCCTGGTATTTCCAGGACAAAGGCTCACCCAAGTCTTC
CCCATGCAGGCCCTTGCCCTCACTGCCCGGTTACACGGCAGCCTCCTGTGCACAGAAGCA
GGGAGCTCAGCCCTTCCACAGGCAGAAGGCACTGAAAGAAATCGGCCTCCAGCACCCTG
ATGCACGTCCGCCTGTGTCTCTCACTGCCCGCACCTGCAGGGAGGCTCGGCACTCCCTGT
AAAGACGAGGGATCCAGGCAGCAACATCATGGGAGAATGCAGGGCTCCCAGACAGCCC
AGCCCTCTCGCAGGCCTCTCCTGGGAAGAGACCTGCAGCCACCACTGAACAGCCACGGA
GCCCGCTGGATAGTAACTGAGTCAGTGACCGACCTGGAGGGCAGGGGAGCAGTGAACCG
GAGCCCAGACCATAGGGACAGAGACCAGCCGCTGACATCCCGAGCCCCTCACTGGCGGC
CCCAGAACACCGCGTGGAAACAGAACAGACCCACATTCCCACCTGGAACAGGGCAGACA
CTGCTGAGCCCCCAGCACCAGCCCTGAGAAACACCAGGCAACGGCATCAGAGGGGGCTC
CTGAGAAAGAAAGGAGGGGAGGTCTCCTTCACCAGCAAGTACTTCCCTTGACCAAAAAC
AGGGTCCACGCAACTCCCCCAGGACAAAGGAGGAGCCCCCTGTACAGCACTGGGCTCAG
AGTCCTCTCCCACACACCCTGAGTTTCAGACAAAAACCCCCTGGAAATCATAGTATCAGC
AGGAGAACTAGCCAGAGACAGCAAGAGGGGACTCAGTGACTCCCGCGGGGACAGGAGG
ATTTTGTGGGGGCTCGTGTCACTGTGAGGATATTGTAGCAGCAGATGGTGCTATACCCAC
AGTGACACAGCCCCATTCAAAAACCCCTGCTGTAAACGCTTCCACTTCTGGAGCTGAGGG
GCTGGGGGGAGCGTCTGGGAAGTAGGGCCTAGGGGTGGCCATCAATGCCCAAAACGCAC
CAGACTCCCCCCCAGACATCACCCCACTGGCCAGTGAGCAGAGTAAACAGAAAATGAGA
AGCAGCTGGGAAGCTTGCACAGGCCCCAAGGAAAGAGCTTTGGCGGGTGTGCAAGAGG
GGATGCGGGCAGAGCCTGAGCAGGGCCTTTTGCTGTTTCTGCTTTCCTGTGCAGATAGTT
CCATAAACTGGTGTTCAAGATCGATGGCTGGGAGTGAGCCCAGGAGGACAGTGTGGGAA
GGGCACAGGGAAGGAGAAGCAGCCGCTATCCTACACTGTCATCTTTCAAGAGTTTGCCCT
GTGCCCACAATGCTGCATCATGGGATGCTTAACAGCTGATGTAGACACAGCTAAAGAGA
GAATCAGTGAAATGGATTTGCAGCACAGATCTGAATAAATTCTCCAGAATGTGGAGCCA
CACAGAAGCAAGCACAAGGAAAGTGCCTGATGCAAGGGCAAAGTACAGTGTGTACCTTC
AGGCTGGGCACAGACACTCTGAAAAGCCTTGGCAGGAACTCCCTGCAACAAAGCAGAGC
CCTGCAGGCAATGCCAGCTCCAGAGCCCTCCCTGAGAGCCTCATGGGCAAAGATGTGCA
CAACAGGTGTTTCTCATAGCCCCAAACTGAGAATGAAGCAAACAGCCATCTGAAGGAAA
ACAGGCAAATAAACGATGGCAGGTTCATGAAATGCAAACCCAGACAGCCAGAAGGACA
ACAGTGAGGGTTACAGGTGACTCTGTGGTTGAGTTCATGACAATGCTGAGTAATTGGAGT
AACAAAGGAAAGTCCAAAAAATACTTTCAATGTGATTTCTTCTAAATAAAATTTACAGCC
GGCAAAATGAACTATCTTCTTAAGGGATAAACTTTCCACTAGGAAAACTATAAGGAAAA
TCAAGAAAAGGATGATCACATAAACACAGTGGTCGTTACTTCTACTGGGGAAGGAAGAG
GGTATGAACTGAGACACACAGGGTTGGCAAGTCTCCTAACAAGAACAGAACAAATACAT
TACAGTACCTTGAAAACAGCAGTTAAAATTCTAAATTGCAAGAAGAGGAAAATGCACAC
AGCTGTGTTTAGAAAATTCTCAGTCCAGCACTGTTCATAATAGCAAAGACATTAACCCAG
GTTGGATAAATAAACGATGACACAGGCAATTGCACAATGATACAGACATACATTCAGTA
TATGAGACATTGATGATGTATCCCCAAAGAAATGACTTTAAAGAGAAAAGGCCTGATAT

GTGGTGGCACTCACCTCCCTGGGCATCCCCGGACAGGCTGCAGGCACACTGTGTGGCAG
GGCAGGCTGGTACCTGCTGGCAGCTCCTGGGGCCTGATGTGGAGCAGGCACAGAGCCGT
ATCCCCCCGAGGACATATACCCCCAAGGACGGCACAGTTGGTACATTCCGGAGACAAGC
AACTCAGCCACACTCCCAGGCCAGAGCCCGAGAGGGACGCCCATGCACAGGGAGGCAG
AGCCCAGCTCCTCCACAGCCAGCAGCACCCGTGCAGGGGCCGCCATCTGGCAGGCACAG
AGCATGGGCTGGGAGGAGGGGCAGGGACACCAGGCAGGGTTGGCACCAACTGAAAATT
ACAGAAGTCTCATACATCTACCTCAGCCTTGCCTGACCTGGGCCTCACCTGACCTGGACC
TCACCTGGCCTGGACCTCACCTGGCCTAGACCTCACCTCTGGGCTTCACCTGAGCTCGGC
CTCACCTGACTTGGACCTTGCCTGTCCTGAGCTCACATGATCTGGGCCTCACCTGACCTG
GGTTTCACCTGACCTGGGCTTCACCTGACCTGGGCCTCATCTGACCTGGGCCTCACTGGC
CTGGACCTCACCTGGCCTGGGCTTCACCTGGCCTCAGGCCTCATCTGCACCTGCTCCAGG
TCTTGCTGGAACCTCAGTAGCACTGAGGCTGCAGGGGCTCATCCAGGGTTGCAGAATGA
CTCTAGAACCTCCCACATCTCAGCTTTCTGGGTGGAGGCACCTGGTGGCCCAGGGAATAT
AAAAAGCCTGAATGATGCCTGCGTGATTTGGGGGCAATTTATAAACCCAAAAGGACATG
GCCATGCAGCGGGTAGGGACAATACAGACAGATATCAGCCTGAAATGGAGCCTCAGGGC
ACAGGTGGGCACGGACACTGTCCACCTAAGCCAGGGGCAGACCCGAGTGTCCCCGCAGT
AGACCTGAGAGCGCTGGGCCCACAGCCTCCCCTCGGTGCCCTGCTACCTCCTCAGGTCAG
CCCTGGACATCCCGGGTTTCCCCAGGCCTGGCGGTAGGATTTTGTTGAGGTCTGTGTCAC
TGTGGTATTACGATTGCAACTGCAGCAGATGGCGCGACCATAGCAGGTGCTGCTATTATA
CCCACAGTGTCACAGAGTCCATCAAAAACCCATCCCTGGGAACCTTCTGCCACAGCCCTC
CCTGTGGGGCACCGCCGCGTGCCATGTTAGGATTTTGACTGAGGACACAGCACCATGGGT
ATGGTGGCTACCGCAGCAGTGCAGCCCGTGACCCAAACACACAGGGCAGCAGGCACAAC
AGACAAGCCCACAAGTGACCACCCTGAGCTCCTGCCTGCCAGCCCTGGAGACCATGAAA
CAGATGGCCAGGATTATCCCATAGGTCAGCCAGACCTCAGTCCAACAGGTCTGCATCGCT
GCTGCCCTCCAATACCAGTCCGGATGGGGACAGGGCTGGCCCACATTACCATTTGCTGCC
ATCCGGCCAACAGTCCCAGAAGCCCCTCCCTCAAGGCTGGGCCACATGTGTGGACCCTG
AGAGCCCCCCATGTCTGAGTAGGGGCACCAGGAAGGTGGGGCTGGCCCTGTGCACTGTC
CCTGCCCCTGTGGTCCCTGGCCTGCCTGGCCCTGACACCTGGGCCTCTCCTGGGTCATTTC
CAAGACAGAAGACATTCCCAGGACAGCTGGAGCTGGGAGTCCATCATCCTGCCTGGCCG
TCCTGAGTCCTGCGCCTTTCCAAACCTCACCCGGGAAGCCAACAGAGGAATCACCTCCCA
CAGGCAGAGACAAAGACCTTCCAGAAATCTCTGTCTCTCTCCCCAGTGGGCACCCTCTTC
CAGGGCAGTCCTCAGTGATATCACAGTGGGAACCCACATCTGGATCGGGACTGCCCCCA
GAACACAAGATGGCCCACAGGGACAGCCCCACAGCCCAGCCCTTCCCAGACCCCTAAAA
GGCGTCCCACCCCCTGCATCTGCCCCAGGGCTCAAACTCCAGGAGGACTGACTCCTGCAC
ACCCTCCTGCCAGACATCACCTCAGCCCCTCCTGGAAGGGACAGGAGCGCGCAAGGGTG
AGTCAGACCCTCCTGCCCTCGATGGCAGGCGGAGAAGATTCAGAAAGGTCTGAGATCCC
CAGGACGCAGCACCACTGTCAATGGGGGCCCCAGACGCCTGGACCAGGGCCTGCGTGGG
AAAGGCCTCTGGGCACACTCAGGGGGATTTTGTGAAGGGTCCTCCCACTGTGGAGAGGC
TTAGCTGTGGCTTCCCTAAGAGCTGCCGCAGCAGGCAAAGCAAGCCTCACAGATGCTGC
CACAGTGATGAACCCAGCATCAAAAACCGACCGGACTCCCAAGGTTTATGCACACTTCTC
CGCTCAGAGCTCTCCAGGATCAGAAGAGCCGGGCCCAAGGGTTTCTGCCCAGACCCTCG
GCCTCTAGGGACATCTTGGCCATGACAGCCCATGGGCTGGTGCCCCACACATCGTCTGCC
TTCAAACAAGGGCTTCAGAGGGCTCTGAGGTGACCTCACTGATGACCACAGGTGCCCTG
GCCCCTTCCCCACCAGCTGCACCAGACCCCGTCATGACAGATGCCCCGATTCCAACAGCC
AATTCCTGGGGCCAGGAATCGCTGTAGACACCAGCCTCCTTCCAACACCTCCTGCCAATT
GCCTGGATTCCCATCCCGGTTGGAATCAAGAGGACAGCATCCCCCAGGCTCCCAACAGG
CAGGACTCCCACACCCTCCTCTGAGAGGCCGCTGTGTTCCGTAGGGCCAGGCTGCAGACA
GTCCCCCTCACCTGCCACTAGACAAATGCCTGCTGTAGATGTCCCCACCTGGAAAATACC

ACTCATGGAGCCCCCAGCCCAGGTACAGCTGTAGAGAGAGTCTCTGAGGCCCCTAAGA
AGTAGCCATGCCCAGTTCTGCCGGGACCCTCGGCCAGGCTGACAGGAGTGGACGCTGGA
GCTGGGCCCATACTGGGCCACATAGGAGCTCACCAGTGAGGGCAGGAGAGCACATGCCG
GGGAGCACCCAGCCTCCTGCTGACCAGAGGCCCGTCCCAGAGCCCAGGAGGCTGCAGAG
GCCTCTCCAGGGGGACACTGTGCATGTCTGGTCCCTGAGCAGCCCCCACGTCCCCAGTC
CTGGGGGCCCTGGCACAGCTGTCTGGACCCTCTCTATTCCCTGGGAAGCTCCTCCTGAC
AGCCCCGCCTCCAGTTCCAGGTGTGGATTTTGTCAGGGGGTGTCACACTGTGTACAGCCA
GAAGTGGATGTGGACTTGCGATAGTGAGAGGAAGTGCAGTGAGGGTATGGTATGCCGGC
TGTGGTGTAAGAAGAAGCTCTGGCACAGTGGTGCTGCCCATATCAAAAACCAGGCCAAG
TAGACAGGCCCCTGCTGTGCAGCCCCAGGCCTCCAGCTCACCTGCTTCTCCTGGGGCTCT
CAAGGCTGCTGTTTCTGCACTCTCCCCTCTGTGGGGAGGGTTCCCTCAGTGGGAGATCT
GTTCTCAACATCCCACGGCCTCATTCCTGCAAGGAAGGCCAATGGATGGGCAACCTCACA
TGCCGCGGCTAAGATAGGGTGGGCAGCCTGGCGGGGACAGGACATCCTGCTGGGGTATC
TGTCACTGTGCCTAGTGGGGCACTGGCTCCCAAACAACGCAGTCCTTGCCAAAATCCCCA
CGGCCTCCCCCGCTAGGGGCTGGCCTGATCTCCTGCAGTCCTAGGAGGCTGCTGACCTCC
AGAATGGCTCCGTCCCCAGTTCCAGGGCGAGAGCAGATCCCAGGCCGGCTGCAGACTGG
GAGGCCACCCCCTCCTTCCCAGGGTTCACTGCAGGTGACCAGGGCAGGAAATGGCCTGA
ACACAGGGATAACCGGGCCATCCCCAACAGAGTCCACCCCCTCCTGCTCTGTACCCCGC
ACCCCCCAGGCCAGCCCATGACATCCGACAACCCCACACCAGAGTCACTGCCCGGTGCT
GCCCTAGGGAGGACCCCTCAGCCCCCACCCTGTCTAGAGGACTGGGGAGGACAGGACAC
GCCCTCTCCTTATGGTTCCCCCACCTGGCTCTGGCTGGGACCCTTGGGGTGTGGACAGAA
AGGACGCTTGCCTGATTGGCCCCCAGGAGCCCAGAACTTCTCTCCAGGGACCCCAGCCCG
AGCACCCCCTTACCCAGGACCCAGCCCTGCCCCTCCTCCCCTCTGCTCTCCTCTCATCACC
CCATGGGAATCCAGAATCCCCAGGAAGCCATCAGGAAGGGCTGAGGGAGGAAGTGGGG
CCACTGCACCACCAGGCAGGAGGCTCTGTCTTTGTGAACCCAGGGAGGTGCCAGCCTCCT
AGAGGGTATGGTCCACCCTGCCTATGGCTCCCACAGTGGCAGGCTGCAGGGAAGGACCA
GGGACGGTGTGGGGGAGGGCTCAGGGCCCCGCGGGTGCTCCATCTTGGATGAGCCTATC
TCTCTCACCCACGGACTCGCCCACCTCCTCTTCACCCTGGCCACACGTCGTCCACACCATC
CTAAGTCCCACCTACACCAGAGCCGGCACAGCCAGTGCAGACAGAGGCTGGGGTGCAGG
GGGGCCGACTGGGCAGCTTCGGGGAGGGAGGAATGGAGGAAGGGGAGTTCAGTGAAGA
GGCCCCCCTCCCCTGGGTCCAGGATCCTCCTCTGGGACCCCCGGATCCCATCCCCTCCAG
GCTCTGGGAGGAGAAGCAGGATGGGAGAATCTGTGCGGGACCCTCTCACAGTGGAATAC
CTCCACAGCGGCTCAGGCCAGATACAAAAGCCCCTCAGTGAGCCCTCCACTGCAGTGCT
GGGCCTGGGGGCAGCCGCTCCCACACAGGATGAACCCAGCACCCCGAGGATGTCCTGCC
AGGGGGAGCTCAGAGCCATGAAGGAGCAGGATATGGGACCCCCGATACAGGCACAGAC
CTCAGCTCCATTCAGGACTGCCACGTCCTGCCCTGGGAGGAACCCCTTTCTCTAGTCCCT
GCAGGCCAGGAGGCAGCTGACTCCTGACTTGGACGCCTATTCCAGACACCAGACAGAGG
GGCAGGCCCCCCAGAACCAGGGATGAGGACGCCCCGTCAAGGCCAGAAAAGACCAAGT
TGCGCTGAGCCCAGCAAGGGAAGGTCCCCAAACAAACCAGGAGGATTTGTAGGTGTCT
GTGTCACTGTGAGCTGCAACTGCAGCAGCAAATGGAGCCGCGACCATAGCAGGTGCTGC
CACAGTGACACTCGCCAGGTCAAAAACCCCATCCCAAGTCAGCGGAATGCAGAGAGAGC
AGGGAGGACATGTTTAGGATCTGAGGCCGCACCTGACACCCAGGCCAGCAGACGTCTCC
TGTCCACGGCACCCTGCCATGTCCTGCATTTCTGGAAGAACAAGGGCAGGCTGAAGGGG
GTCCAGGACCAGGAGATGGGTCCGCTCTACCCAGAGAAGGAGCCAGGCAGGACACAAG
CCCCCACGCGTGGGCTCGTAGTTTGACGTGCGTGAAGTGTGGGTAAGAAAGTACGTA

TX-DH17613 (SEQ ID NO:233) includes toxin coding sequences inserted in positions corresponding to D_{H}1-7 to D_{H}6-13:

GCGGCCGCTGCCATTTCATTACCTCTTTCTCCGCACCCGACATAGATTACGTAACGCGTG
GGCTCGTAGTTTGACGTGCGTGAAGTGTGGGTAAGAAAGTCCCCATTGAGGCTGACCTGC
CCAGAGGGTCCTGGGCCCACCCAACACACCGGGGCGGAATGTGTGCAGGCCTCGGTCTC
TGTGGGTGTTCCGCTAGCTGGGGCTCACAGTGCTCACCCCACACCTAAAACGAGCCACAG
CCTCCGGAGCCCCTGAAGGAGACCCCGCCCACAAGCCCAGCCCCCACCCAGGAGGCCCC
AGAGCACAGGGCGCCCCGTCGGATTTTGTACAGCCCCGAGTCACTGTGGAGAGATGCAA
TGGCAGACGCGGCTGCAGCAGATGGCGCGATCATAGCAGGTGCTGCCACAGTGAGAAAA
GCTTCGTCAAAAACCGTCTCCTGGCCACAGTCGGAGGCCCCGCCAGAGAGGGGAGCAGC
CACCCCAAACCCATGTTCTGCCGGCTCCCATGACCCCGTGCACCTGGAGCCCCACGGTGT
CCCCACTGGATGGGAGGACAAGGGCCGGGGGCTCCGGCGGGTCGGGGCAGGGGCTTGAT
GGCTTCCTTCTGCCGTGGCCCCATTGCCCCTGGCTGGAGTTGACCCTTCTGACAAGTGTCC
TCAGAGAGTCAGGGATCAGTGGCACCTCCCAACATCAACCCCACGCAGCCCAGGCACAA
ACCCCACATCCAGGGCCAACTCCAGGAACAGAGACACCCCAATACCCTGGGGGACCCCG
ACCCTGATGACTCCCGTCCCATCTCTGTCCCTCACTTGGGGCCTGCTGCGGGGCGAGCAC
TTGGGAGCAAACTCAGGCTTAGGGGACACCACTGTGGGCCTGACCTCGAGCAGGCCACA
GACCCTTCCCTCCTGCCCTGGTGCAGCACAGACTTTGGGGTCTGGGCAGGGAGGAACTTC
TGGCAGGTCACCAAGCACAGAGCCCCCAGGCTGAGGTGGCCCCAGGGGGAACCCCAGCA
GGTGGCCCACTACCCTTCCTCCCAGCTGGACCCCATGTCTTCCCCAAGATAGGGGTGCCA
TCCAAGGCAGGTCCTCCATGGAGCCCCCTTCAGGCTCCTCTCCAGACCCCACTGGGCCTC
AGTCCCCACTCTAGGAATGCAGCCACCACGGGCACACCAGGCAGCCCAGGCCCAGCCAC
CCTGCAGTGCCCAAGCCCACACCCTGGAGGAGAGCAGGGTGCGTCTGGGAGGGGCTGGG
CTCCCCACCCCCACCCCCACCTGCACACCCCACCCACCCTTGCCCGGGCCCCCTGCAGGA
GGGTCAGAGCCCCCATGGGATATGGACTTAGGGTCTCACTCACGCACCTCCCCTCCTGGG
AGAAGGGGTCTCATGCCCAGATCCCCCCAGCAGCGCTGGTCACAGGTAGAGGCAGTGGC
CCCAGGGCCACCCTGACCTGGCCCCTCAGGCTCCTCTAGCCCTGGCTGCCCTGCTGTCCC
TGGGAGGCCTGGGCTCCACCAGACCACAGGTCTAGGGCACCGCCCACACTGGGGCCGCC
CACACACAGCTCACAGGAAGAAGATAAGCTCCAGACCCCCAGGCCCGGGACCTGCCTTG
CTGCTACGACTTCCTGCCCCAGACCTCGTTGCCCTCCCCCGTCCACTTACACACAGGCCA
GGAAGCTGTTCCCACACAGACCAACCCCAGACGGGGACCACCTGGCACTCAGGTCACTG
CCATTTCCTTCTCCATTCACTTCCAATGCCTCTGTGCTTCCTCCCTCCTCCTTCCTTCGGGG
GAGCACCCTGTGCAGCTCCTCCCTGCAGTCCACACCCTGGGGAGACCCGACCCTGCAGCC
CACACCCTGGGGAGACCTGACCCTCCTCCAGCCCTTTCTCCCCCGCTGCTCTTGCCACCCA
CCAAGACAGCCCTGGGGTCCTGTCCCTACAGCCCCCACCCAGTTCTCTACCTAGACCCGT
CTTCCTCCCTCTAAACACCTCTCCCAGGCCAACCCTACACCTGCAGGCCCTCCCCTCCACT
GCCAAAGACCCTCAGTTTCTCCTGCCTGTGCCCACCCCCGTGCTCCTCCTGCCCACAGCTC
GAGCTCTTCCTCTCCTAGGGCCCCTGAGGGATGGCATTGACCGTGCCCTCGCACCCACAC
ACTGCCCATGCCCTCACATTCCTCCTGGCCACTCCAGCCCCACTCCCCTCTCAGGCCTGGC
TCTGGTATTTCTGGGACAAAGCCTTACCCAAGTCTTTCCCATGCAGGCCTGGGCCCTTAC
CCTCACTGCCCGGTTACAGGGCAGCCTCCTGTGCACAGAAGCAGGGAGCTCAGCCCTTCC
ACAGGCAGAAGGCACTGAAAGAAATCGGCCTCCAGCGCCTTGACACACGTCTGCCTGTG
TCTCTCACTGCCCGCACCTGCAGGGAGGCTCGGCACTCCCTCTAAAGACGAGGGATCCAG
GCAGCAGCATCACAGGAGAATGCAGGGCTACCAGACATCCCAGTCCTCTCACAGGCCTC
TCCTGGGAAGAGACCTGAAGACGCCCAGTCAACGGAGTCTAACACCAAACCTCCCTGGA
GGCCGATGGGTAGTAACGGAGTCATTGCCAGACCTGGAGGCAGGGGAGCAGTGAGCCCG

AGCCCACACCATAGGGCCAGAGGACAGCCACTGACATCCCAAGCCACTCACTGGTGGTC
CCACAACACCCCATGGAAAGAGGACAGACCCACAGTCCCACCTGGACCAGGGCAGAGA
CTGCTGAGACCCAGCACCAGAACCAACCAAGAAACACCAGGCAACAGCATCAGAGGGG
GCTCTGGCAGAACAGAGGAGGGGAGGTCTCCTTCACCAGCAGGCGCTTCCCTTGACCGA
AGACAGGATCCATGCAACTCCCCCAGGACAAAGGAGGAGCCCCTTGTTCAGCACTGGGC
TCAGAGTCCTCTCCAAGACACCCAGAGTTTCAGACAAAAACCCCCTGGAATGCACAGTCT
CAGCAGGAGAGCCAGCCAGAGCCAGCAAGATGGGGCTCAGTGACACCCGCAGGGACAG
GAGGATTTTGTGGGGGCTCGTGTCACTGTGAGGATATTGTACTAATCGGAGCAGGCAGG
GTGTATGCTATACCCACAGTGACACAGCCCCATTCAAAAACCCCTACTGCAAACGCATTC
CACTTCTGGGGCTGAGGGGCTGGGGGAGCGTCTGGGAAATAGGGCTCAGGGGTGTCCAT
CAATGCCCAAAACGCACCAGACTCCCCTCCATACATCACACCCACCAGCCAGCGAGCAG
AGTAAACAGAAAATGAGAAGCAAGCTGGGGAAGCTTGCACAGGCCCCAAGGAAAGAGC
TTTGGCGGGTGTGTAAGAGGGGATGCGGGCAGAGCCTGAGCAGGGCCTTTTGCTGTTTCT
GCTTTCCTGTGCAGAGAGTTCCATAAACTGGTGTTCGAGATCAATGGCTGGGAGTGAGCC
CAGGAGGACAGCGTGGGAAGAGCACAGGGAAGGAGGAGCAGCCGCTATCCTACACTGT
CATCTTTCGAAAGTTTGCCTTGTGCCCACACTGCTGCATCATGGGATGCTTAACAGCTGA
TGTAGACACAGCTAAAGAGAGAATCAGTGAGATGGATTTGCAGCACAGATCTGAATAAA
TTCTCCAGAATGTGGAGCAGCACAGAAGCAAGCACACAGAAAGTGCCTGATGCAAGGAC
AAAGTTCAGTGGGCACCTTCAGGCATTGCTGCTGGGCACAGACACTCTGAAAAGCCCTG
GCAGGAACTCCCTGTGACAAAGCAGAACCCTCAGGCAATGCCAGCCCCAGAGCCCTCCC
TGAGAGCCTCATGGGCAAAGATGTGCACAACAGGTGTTTCTCATAGCCCCAAACTGAGA
GCAAAGCAAACGTCCATCTGAAGGAGAACAGGCAAATAAACGATGGCAGGTTCATGAA
ATGCAAACCCAGACAGCCACAAGCACAAAAGTACAGGGTTATAAGCGACTCTGGTTGAG
TTCATGACAATGCTGAGTAATTGGAGTAACAAAGTAAACTCCAAAAAATACTTTCAATGT
GATTTCTTCTAAATAAAATTTACACCCTGCAAAATGAACTGTCTTCTTAAGGGATACATTT
CCCAGTTAGAAAACCATAAAGAAAACCAAGAAAAGGATGATCACATAAACACAGTGGT
GGTTACTTCTGCTGGGGAAGGAAGAGGGTATGAACTGAGATACACAGGGTGGGCAAGTC
TCCTAACAAGAACAGAACGAATACATTACAGTACCTTGAAAACAGCAGTTAAACTTCTA
AATTGCAAGAAGAGGAAAATGCACACAGTTGTGTTTAGAAAATTCTCAGTCCAGCACTG
TTCATAATAGCAAAGACATTAACCCAGGTCGGATAAATAAGCGATGACACAGGCAATTG
CACAATGATACAGACATATATTTAGTATATGAGACATCGATGATGTATCCCCAAATAAAC
GACTTTAAAGAGATAAAGGGCTGATGTGTGGTGGCATTCACCTCCCTGGGATCCCCGGAC
AGGTTGCAGGCTCACTGTGCAGCAGGGCAGGCGGGTACCTGCTGGCAGTTCCTGGGGCC
TGATGTGGAGCAAGCGCAGGGCCATATATCCCGGAGGACGGCACAGTCAGTGAATTCCA
GAGAGAAGCAACTCAGCCACACTCCCCAGGCAGAGCCCGAGAGGGACGCCCACGCACA
GGGAGGCAGAGCCCAGCACCTCCGCAGCCAGCACCACCTGCGCACGGGCCACCACCTTG
CAGGCACAGAGTGGGTGCTGAGAGGAGGGGCAGGGACACCAGGCAGGGTGAGCACCCA
GAGAAAACTGCAGACGCCTCACACATCCACCTCAGCCTCCCCTGACCTGGACCTCACTGG
CCTGGGCCTCACTTAACCTGGGCTTCACCTGACCTTGGCCTCACCTGACTTGGACCTCGCC
TGTCCCAAGCTTTACCTGACCTGGGCCTCAACTCACCTGAACGTCTCCTGACCTGGGTTTA
ACCTGTCCTGGAACTCACCTGGCCTTGGCTTCCCCTGACCTGGACCTCATCTGGCCTGGG
CTTCACCTGGCCTGGGCCTCACCTGACCTGGACCTCATCTGGCCTGGACCTCACCTGGCC
TGGACTTCACCTGGCCTGGGCTTCACCTGACCTGGACCTCACCTGGCCTCGGGCCTCACC
TGCACCTGCTCCAGGTCTTGCTGGAGCCTGAGTAGCACTGAGGGTGCAGAAGCTCATCCA
GGGTTGGGGAATGACTCTAGAAGTCTCCCACATCTGACCTTTCTGGGTGGAGGCAGCTGG
TGGCCCTGGGAATATAAAAATCTCCAGAATGATGACTCTGTGATTTGTGGGCAACTTATG
AACCCGAAAGGACATGGCCATGGGGTGGGTAGGGACATAGGGACAGATGCCAGCCTGA
GGTGGAGCCTCAGGACACAGGTGGGCACGGACACTATCCACATAAGCGAGGGATAGACC

CGAGTGTCCCCACAGCAGACCTGAGAGCGCTGGGCCCACAGCCTCCCCTCAGAGCCCTG
CTGCCTCCTCCGGTCAGCCCTGGACATCCCAGGTTTCCCCAGGCCTGGCGGTAGGATTTT
GTTGAGGTCTGTGTCACTGTGGTATTACGATTGCAACTGCAGCAGATGGGCTCGCGACCA
TAGCAGGTGCTGCTATTATAACCACAGTGTCACAGAGTCCATCAAAAACCCATGCCTGGA
AGCTTCCCGCCACAGCCCTCCCCATGGGGCCCTGCTGCCTCCTCAGGTCAGCCCCGGACA
TCCCGGGTTTCCCCAGGCTGGGCGGTAGGATTTTGTTGAGGTCTGTGTCACTGTGGTATT
ACTATGAGAGATGCAATGGCAGACGCGGCTGCAGCAGATGGCGCGATCATAGCAGGTGC
TGCTATTATAACCACAGTGTCACAGAGTCCATCAAAAACCCATCCCTGGGAGCCTCCCGC
CACAGCCCTCCCTGCAGGGGACCGGTACGTGCCATGTTAGGATTTTGATCGAGGAGACA
GCACCATGGGTATGGTGGCTACCACAGCAGTGCAGCCTGTGACCCAAACCCGCAGGGCA
GCAGGCACGATGGACAGGCCCGTGACTGACCACGCTGGGCTCCAGCCTGCCAGCCCTGG
AGATCATGAAACAGATGGCCAAGGTCACCCTACAGGTCATCCAGATCTGGCTCCGAGGG
GTCTGCATCGCTGCTGCCCTCCCAACGCCAGTCCAAATGGGACAGGGACGGCCTCACAG
CACCATCTGCTGCCATCAGGCCAGCGATCCCAGAAGCCCCTCCCTCAAGGCTGGGCACAT
GTGTGGACACTGAGAGCCCTCATATCTGAGTAGGGGCACCAGGAGGGAGGGGCTGGCCC
TGTGCACTGTCCCTGCCCCTGTGGTCCCTGGCCTGCCTGGCCCTGACACCTGAGCCTCTCC
TGGGTCATTTCCAAGACAGAAGACATTCCTGGGGACAGCCGGAGCTGGGCGTCGCTCAT
CCTGCCCGGCCGTCCTGAGTCCTGCTCATTTCCAGACCTCACCGGGGAAGCCAACAGAGG
ACTCGCCTCCCACATTCAGAGACAAAGAACCTTCCAGAAATCCCTGCCTCTCTCCCCAGT
GGACACCCTCTTCCAGGACAGTCCTCAGTGGCATCACAGCGGCCTGAGATCCCCAGGAC
GCAGCACCGCTGTCAATAGGGGCCCCAAATGCCTGGACCAGGGCCTGCGTGGGAAAGGC
CTCTGGCCACACTCGGGGATTTTGTGAAGGGCCCTCCCACTGTGGAGAGGCTTTGTGGCT
TCCCTAAGAGCTGCAGCAGGCAAAAGCCTCACAGATGCTGCCACAGTGATGAACCCAGT
GTCAAAAACCGGCTGGAAACCCAGGGGCTGTGTGCACGCCTCAGCTTGGAGCTCTCCAG
GAGCACAAGAGCCGGGCCCAAGGATTTGTGCCCAGACCCTCAGCCTCTAGGGACACCTG
GGTCATCTCAGCCTGGGCTGGTGCCCTGCACACCATCTTCCTCCAAATAGGGGCTTCAGA
GGGCTCTGAGGTGACCTCACTCATGACCACAGGTGACCTGGCCCTTCCCTGCCAGCTATA
CCAGACCCTGTCTTGACAGATGCCCCGATTCCAACAGCCAATTCCTGGGACCCTGAATAG
CTGTAGACACCAGCCTCATTCCAGTACCTCCTGCCAATTGCCTGGATTCCCATCCTGGCTG
GAATCAAGAAGGCAGCATCCGCCAGGCTCCCAACAGGCAGGACTCCCGCACACCCTCCT
CTGAGAGGCCGCTGTGTTCCGCAGGGCCAGGCCCTGGACAGTTCCCCTCACCTGCCACTA
GAGAAACACCTGCCATTGTCGTCCCCACCTGGAAAAGACCACTCGTGGAGCCCCCAGCC
CCAGGTACAGCTGTAGAGACAGTCCTCGAGGCCCCTAAGAAGGAGCCATGCCCAGTTCT
GCCGGGACCCTCGGCCAGGCCGACAGGAGTGGACGCTGGAGCTGGGCCCACACTGGGCC
ACATAGGAGCTCACCAGTGAGGGCAGGAGAGCACATGCCGGGGAGCACCCAGCCTCCTG
CTGACCAGAGGCCCGTCCCAGAGCCCAGGAGGCTGCAGAGGCCTCTCCAGGGAGACACT
GTGCATGTCTGGTACCTAAGCAGCCCCCCACGTCCCCAGTCCTGGGGGCCCCTGGCTCAG
CTGTCTGGGCCCTCCCTGCTCCCTGGGAAGCTCCTCCTGACAGCCCCGCCTCCAGTTCCA
GGTGTGGATTTTGTCAGGCGATGTCACACTGTGTACTGCCAGAAGTGGATGTGGACTAGC
GATAGTGAGAGGAAGTGCTGTGAGGGTATGGTAAGCCGGCTGTGGTGTAAGAAGAAGCT
CTGGCACAGTGGTGCCGCCCATATCAAAAACCAGGCCAAGTAGACAGGCCCCTGCTGCG
CAGCCCCAGGCATCCACTTCACCTGCTTCTCCTGGGGCTCTCAAGGCTGCTGTCTGTCCTC
TGGCCCTCTGTGGGGAGGGTTCCCTCAGTGGGAGGTCTGTGCTCCAGGGCAGGGATGATT
GAGATAGAAATCAAAGGCTGGCAGGGAAAGGCAGCTTCCCGCCCTGAGAGGTGCAGGC
AGCACCACGGAGCCACGGAGTCACAGAGCCACGGAGCCCCCATTGTGGGCATTTGAGAG
TGCTGTGCCCCCGGCAGGCCCAGCCCTGATGGGGAAGCCTGTCCCATCCCACAGCCCGG
GTCCCACGGGCAGCGGGCACAGAAGCTGCCAGGTTGTCCTCTATGATCCTCATCCCTCCA
GCAGCATCCCCTCCACAGTGGGGAAACTGAGGCTTGGAGCACCACCCGGCCCCCTGGAA

ATGAGGCTGTGAGCCCAGACAGTGGGCCCAGAGCACTGTGAGTACCCCGGCAGTACCTG
GCTGCAGGGATCAGCCAGAGATGCCAAACCCTGAGTGACCAGCCTACAGGAGGATCCGG
CCCCACCCAGGCCACTCGATTAATGCTCAACCCCCTGCCCTGGAGACCTCTTCCAGTACC
ACCAGCAGCTCAGCTTCTCAGGGCCTCATCCCTGCAAGGAAGGTCAAGGGCTGGGCCTG
CCAGAAACACAGCACCCTCCCTAGCCCTGGCTAAGACAGGGTGGGCAGACGGCTGTGGA
CGGGACATATTGCTGGGGCATTTCTCACTGTCACTTCTGGGTGGTAGCTCTGACAAAAAC
GCAGACCCTGCCAAAATCCCCACTGCCTCCCGCTAGGGGCTGGCCTGGAATCCTGCTGTC
CTAGGAGGCTGCTGACCTCCAGGATGGCTCCGTCCCCAGTTCCAGGGCGAGAGCAGATC
CCAGGCAGGCTGTAGGCTGGGAGGCCACCCCTGCCCTTGCCGGGGTTGAATGCAGGTGC
CCAAGGCAGGAAATGGCATGAGCACAGGGATGACCGGGACATGCCCCACCAGAGTGCG
CCCCTTCCTGCTCTGCACCCTGCACCCCCCAGGCCAGCCCACGACGTCCAACAACTGGGC
CTGGGTGGCAGCCCCACCCAGACAGGACAGACCCAGCACCCTGAGGAGGTCCTGCCAGG
GGGAGCTAAGAGCCATGAAGGAGCAAGATATGGGGCCCCCGATACAGGCACAGATGTC
AGCTCCATCCAGGACCACCCAGCCCACACCCTGAGAGGAACGTCTGTCTCCAGCCTCTGC
AGGTCGGGAGGCAGCTGACCCCTGACTTGGACCCCTATTCCAGACACCAGACAGAGGCG
CAGGCCCCCCAGAACCAGGGTTGAGGGACGCCCCGTCAAAGCCAGACAAAACCAAGGG
GTGTTGAGCCCAGCAAGGGAAGGCCCCCAAACAGACCAGGAGGATTTTGTAGGTGTCTG
TGTCACTGTGTGCAACTGCAGCAGATGGCGCGACCATAGCAGGTGCTGCCACAGTGACA
CTCACCCAGTCAAAAACCCCATTCCAAGTCAGCGGAAGCAGAGAGAGCAGGGAGGACA
CGTTTAGGATCTGAGACTGCACCTGACACCCAGGCCAGCAGACGTCTCCCCTCCAGGGCA
CCCCACCCTGTCCTGCATTTCTGCAAGATCAGGGGCGGCCTGAGGGGGGGTCTAGGGTG
AGGAGATGGGTCCCCTGTACACCAAGGAGGAGTTAGGCAGGTCCCGAGCACTCTTAATT
AAACGACGCCTCGAATGGAACTACTACAACGAATGGTTGCTCTACGTAATGCATTCGCTA
CCTTAGGACCGTTATAGTTAGGCGCGCC

TX-DH114619 (SEQ ID NO:234) includes toxin coding sequence inserted in positions corresponding to D_{H}1-14 to D_{H}6-19:

TACGTATTAATTAAACGACGCCTCGAATGGAACTACTACAACGAATGGTTGCTCTCCCCA
TTGAGGCTGACCTGCCCAGAGAGTCCTGGGCCCACCCCACACACCGGGGCGGAATGTGT
GCAGGCCTCGGTCTCTGTGGGTGTTCCGCTAGCTGGGGCTCACAGTGCTCACCCCACACC
TAAAATGAGCCACAGCCTCCGGAGCCCCCGCAGGAGACCCCGCCCACAAGCCCAGCCCC
CACCCAGGAGGCCCCAGAGCTCAGGGCGCCCCGTCGGATTTTGTACAGCCCCGAGTCAC
TGTGGAGAGATGCTGCAATGGCAGACGCGGCTGCAGCAGATGGTGCCGCGATCATA
GCAGGTGCTGCCACAGTGAGAATAGCTACGTCAAAAACCGTCCAGTGGCCACTGCCGGA
GGCCCCGCCAGAGAGGGCAGCAGCCACTCTGATCCCATGTCCTGCCGGCTCCCATGACCC
CCAGCACGCGGAGCCCCACAGTGTCCCCACTGGATGGGAGGACAAGAGCTGGGGATTCC
GGCGGGTCGGGGCAGGGGCTTGATCGCATCCTTCTGCCGTGGCTCCAGTGCCCCTGGCTG
GAGTTGACCCTTCTGACAAGTGTCCTCAGAGAGACAGGCATCACCGGCGCCTCCCAACAT
CAACCCCAGGCAGCACAGGCACAAACCCCACATCCAGAGCCAACTCCAGGAGCAGAGA
CACCCCAATACCCTGGGGGACCCCGACCCTGATGACTTCCCACTGGAATTCGCCGTAGAG
TCCACCAGGACCAAAGACCCTGCCTCTGCCTCTGTCCCTCACTCAGGACCTGCTGCCGGG
CGAGGCCTTGGGAGCAGACTTGGGCTTAGGGGACACCAGTGTGACCCCGACCTTGACCA
GGACGCAGACCTTTCCTTCCTTTCCTGGGGCAGCACAGACTTTGGGGTCTGGGCCAGGAG
GAACTTCTGGCAGGTCGCCAAGCACAGAGGCCACAGGCTGAGGTGGCCCTGGAAAGACC
TCCAGGAGGTGGCCACTCCCCTTCCTCCCAGCTGGACCCCATGTCCTCCCCAAGATAAGG
GTGCCATCCAAGGCAGGTGCTCCTTGGAGCCCCATTCAGACTCCTCCCTGGACCCCACTG
GGCCTCAGTCCCAGCTCTGGGGATGAAGCCACCACAAGCACACCAGGCAGCCCAGGCCC

AGCCACCCTGCAGTGCCCAAGCACACACTCTGGAGCAGAGCAGGGTGCCTCTGGGAGGG
GCTGAGCTCCCCACCCCACCCCCACCTGCACACCCCACCCACCCCTGCCCAGCGGCTCTG
CAGGAGGGTCAGAGCCCCACATGGGGTATGGACTTAGGGTCTCACTCACGTGGCTCCCA
TCATGAGTGAAGGGGCCTCAAGCCCAGGTTCCCACAGCAGCGCCTGTCGCAAGTGGAGG
CAGAGGCCCGAGGGCCACCCTGACCTGGTCCCTGAGGTTCCTGCAGCCCAGGCTGCCCTG
CTGTCCCTGGGAGGCCTGGGCTCCACCAGACCACAGGTCCAGGGCACCGGGTGCAGGAG
CCACCCACACACAGCTCACAGGAAGAAGATAAGCTCCAGACCCCCAGGGCCAGAACCTG
CCTTCCTGCTACTGCTTCCTGCCCCAGACCTGGGCGCCCTCCCCCGTCCACTTACACACAG
GCCAGGAAGCTGTTCCCACACAGAACAACCCCAAACCAGGACCGCCTGGCACTCAGGTG
GCTGCCATTTCCTTCTCCATTTGCTCCCAGCGCCTCTGTCCTCCCTGGTTCCTCCTTCGGGG
GAACAGCCTGTGCAGCCAGTCCCTGCAGCCCACACCCTGGGGAGACCCAACCCTGCCTG
GGGCCCTTCCAACCCTGCTGCTCTTACTGCCCACCCAGAAAACTCTGGGGTCCTGTCCCT
GCAGTCCCTACCCTGGTCTCCACCCAGACCCCTGTGTATCACTCCAGACACCCCTCCCAG
GCAAACCCTGCACCTGCAGGCCCTGTCCTCTTCTGTCGCTAGAGCCTCAGTTTCTCCCCCC
TGTGCCCACACCCTACCTCCTCCTGCCCACAACTCTAACTCTTCTTCTCCTGGAGCCCCTG
AGCCATGGCATTGACCCTGCCCTCCCACCACCCACAGCCCATGCCCTCACCTTCCTCCTG
GCCACTCCGACCCCGCCCCCTCTCAGGCCAAGCCCTGGTATTTCCAGGACAAAGGCTCAC
CCAAGTCTTTCCCAGGCAGGCCTGGGCTCTTGCCCTCACTTCCCGGTTACACGGGAGCCT
CCTGTGCACAGAAGCAGGGAGCTCAGCCCTTCCACAGGCAGAAGGCACTGAAAGAAATC
GGCCTCCAGCACCTTGACACACGTCCGCCCGTGTCTCTCACTGCCCGCACCTGCAGGGAG
GCTCCGCACTCCCTCTAAAGACAAGGGATCCAGGCAGCAGCATCACGGGAGAATGCAGG
GCTCCCAGACATCCCAGTCCTCTCACAGGCCTCTCCTGGGAAGAGACCTGCAGCCACCAC
CAAACAGCCACAGAGGCTGCTGGATAGTAACTGAGTCAATGACCGACCTGGAGGGCAGG
GGAGCAGTGAGCCGGAGCCCATACCATAGGGACAGAGACCAGCCGCTGACATCCCGAGC
TCCTCAATGGTGGCCCCATAACACACCTAGGAAACATAACACACCCACAGCCCCACCTG
GAACAGGGCAGAGACTGCTGAGCCCCCAGCACCAGCCCCAAGAAACACCAGGCAACAG
TATCAGAGGGGGCTCCCGAGAAAGAGAGGAGGGGAGATCTCCTTCACCATCAAATGCTT
CCCTTGACCAAAAACAGGGTCCACGCAACTCCCCCAGGACAAAGGAGGAGCCCCCTATA
CAGCACTGGGCTCAGAGTCCTCTCTGAGACACCCTGAGTTTCAGACAACAACCCGCTGGA
ATGCACAGTCTCAGCAGGAGAACAGACCAAAGCCAGCAAAAGGGACCTCGGTGACACC
AGTAGGGACAGGAGGATTTTGTGGGGGCTCGTGTCACTGTGAGGATATTGTAGTGGTAG
CAGCAGATGGGGTAGCTGCTACTCCCACAGTGACACAGACCCATTCAAAAACCCCTACT
GCAAACACACCCACTCCTGGGGCTGAGGGGCTGGGGGAGCGTCTGGGAAGTAGGGTCCA
GGGGTGTCTATCAATGTCCAAAATGCACCAGACTCCCCGCCAAACACCACCCCACCAGC
CAGCGAGCAGGGTAAACAGAAAATGAGAGGCTCTGGGAAGCTTGCACAGGCCCCAAGG
AAAGAGCTTTGGCGGGTGTGCAAGAGGGGATGCAGGCAGAGCCTGAGCAGGGCCTTTTG
CTGTTTCTGCTTTCCTGTGCAGAGAGTTCCATAAACTGGTGTTCAAGATCAGTGGCTGGG
AATGAGCCCAGGAGGGCAGTCTGTGGGAAGAGCACAGGGAAGGAGGAGCAGCCGCTAT
CCTACACTGTCATCTTTCAAAAGTTTGCCTTGTGACCACACTATTGCATCATGGGATGCTT
AAGAGCTGATGTAGACACAGCTAAAGAGAGAATCAGTGAGATGAATTTGCAGCATAGAT
CTGAATAAACTCTCCAGAATGTGGAGCAGTACAGAAGCAAACACACAGAAAGTGCCTGA
TGCAAGGACAAAGTTCAGTGGGCACCTTCAGGCATTGCTGCTGGGCACAGACACTCTGA
AAAGCCTTGGCAGGATCTCCCTGCGACAAAGCAGAACCCTCAGGCAATGCCAGCCCCAG
AGCCCTCCCTGAGAGCGTCATGGGGAAAGATGTGCAGAACAGCTGATTATCATAGACTC
AAACTGAGAACAGAGCAAACGTCCATCTGAAGAACAGTCAAATAAGCAATGGTAGGTTC
ATGCAATGCAAACCCAGACAGCCAGGGGACAACAGTAGAGGGCTACAGGCGGCTTTGCG
GTTGAGTTCATGACAATGCTGAGTAATTGGAGTAACAGAGGAAAGCCCAAAAAATACTT
TTAATGTGATTTCTTCTAAATAAAATTTACACCAGGCAAAATGAACTGTCTTCTTAAGGG

ATAAACTTTCCCCTGGAAAAACTACAAGGAAAATTAAGAAAACGATGATCACATAAACA
CAGTTGTGGTTACTTCTACTGGGGAAGGAAGAGGGTATGAGCTGAGACACACAGAGTCG
GCAAGTCTCCAAGCAAGCACAGAACGAATACATTACAGTACCTTGAATACAGCAGTTAA
ACTTCTAAATCGCAAGAACAGGAAAATGCACACAGCTGTGTTTAGAAAATTCTCAGTCC
AGCACTATTCATAATAGCAAAGACATTAACCCAGGTTGGATAAATAAATGATGACACAG
GCAATTGCACAATGATACAGACATACATTTAGTACATGAGACATCGATGATGTATCCCCA
AAGAAATGACTTTAAAGAGAAAAGGCCTGATGTGTGGTGGCACTCACCTCCCTGGGATC
CCCGGACAGGTTGCAGGCACACTGTGTGGCAGGGCAGGCTGGTACATGCTGGCAGCTCC
TGGGGCCTGATGTGGAGCAAGCGCAGGGCTGTATACCCCCAAGGATGGCACAGTCAGTG
AATTCCAGAGAGAAGCAGCTCAGCCACACTGCCCAGGCAGAGCCCGAGAGGGACGCCC
ACGTACAGGGAGGCAGAGCCCAGCTCCTCCACAGCCACCACCACCTGTGCACGGGCCAC
CACCTTGCAGGCACAGAGTGGGTGCTGAGAGGAGGGGCAGGGACACCAGGCAGGGTGA
GCACCCAGAGAAAACTGCAGAAGCCTCACACATCCACCTCAGCCTCCCCTGACCTGGAC
CTCACCTGGTCTGGACCTCACCTGGCCTGGGCCTCACCTGACCTGGACCTCACCTGGCCT
GGGCTTCACCTGACCTGGACCTCACCTGGCCTCCGGCCTCACCTGCACCTGCTCCAGGTC
TTGCTGGAACCTGAGTAGCACTGAGGCTGCAGAAGCTCATCCAGGGTTGGGGAATGACT
CTGGAACTCTCCCACATCTGACCTTTCTGGGTGGAGGCATCTGGTGGCCCTGGGAATATA
AAAAGCCCCAGAATGGTGCCTGCGTGATTTGGGGGCAATTTATGAACCCGAAAGGACAT
GGCCATGGGGTGGGTAGGGACATAGGGACAGATGCCAGCCTGAGGTGGAGCCTCAGGA
CACAGTTGGACGCGGACACTATCCACATAAGCGAGGGACAGACCCGAGTGTTCCTGCAG
TAGACCTGAGAGCGCTGGGCCCACAGCCTCCCCTCGGTGCCCTGCTGCCTCCTCAGGTCA
GCCCTGGACATCCCGGGTTTCCCCAGGCCAGATGGTAGGATTTTGTTGAGGTCTGTGTCA
CTGTGGTATTATGATTACGAGAGAGCTTGCAATGGCAGACGCGGCTGCAGCAGATGGGC
TCGCGATCATAGCAGGTGCTGCTATCGTTATACCCACAGTGTCACACGGTCCATCAAAAA
CCCATGCCACAGCCCTCCCCGCAGGGGACCGCCGCGTGCCATGTTACGATTTTGATCGAG
GACACAGCGCCATGGGTATGGTGGCTACCACAGCAGTGCAGCCCATGACCCAAACACAC
AGGGCAGCAGGCACAATGGACAGGCCTGTGAGTGACCATGCTGGGCTCCAGCCCGCCAG
CCCCGGAGACCATGAAACAGATGGCCAAGGTCACCCCACAGTTCAGCCAGACATGGCTC
CGTGGGGTCTGCATCGCTGCTGCCCTCTAACACCAGCCCAGATGGGGACAAGGCCAACC
CCACATTACCATCTCCTGCTGTCCACCCAGTGGTCCCAGAAGCCCCTCCCTCATGGCTGA
GCCACATGTGTGAACCCTGAGAGCACCCCATGTCAGAGTAGGGGCAGCAGAAGGGCGGG
GCTGGCCCTGTGCACTGTCCCTGCACCCATGGTCCCTCGCCTGCCTGGCCCTGACACCTG
AGCCTCTTCTGAGTCATTTCTAAGATAGAAGACATTCCCGGGGACAGCCGGAGCTGGGC
GTCGCTCATCCCGCCCGGCCGTCCTGAGTCCTGCTTGTTTCCAGACCTCACCAGGGAAGC
CAACAGAGGACTCACCTCACACAGTCAGAGACAAAGAACCTTCCAGAAATCCCTGTCTC
ACTCCCCAGTGGGCACCTTCTTCCAGGACATTCCTCGGTCGCATCACAGCAGGCACCCAC
ATCTGGATCAGGACGGCCCCCAGAACACAAGATGGCCCATGGGGACAGCCCCACAACCC
AGGCCTTCCCAGACCCCTAAAAGGCGTCCCACCCCCTGCACCTGCCCCAGGGCTAAAAAT
CCAGGAGGCTTGACTCCCGCATACCCTCCAGCCAGACATCACCTCAGCCCCCTCCTGGAG
GGGACAGGAGCCCGGGAGGGTGAGTCAGACCCACCTGCCCTCGATGGCAGGCGGGGAA
GATTCAGAAAGGCCTGAGATCCCCAGGACGCAGCACCACTGTCAATGGGGGCCCCAGAC
GCCTGGACCAGGGCCTGCGTGGGAAAGGCCGCTGGGCACACTCAGGGGGATTTTGTGAA
GGCCCCTCCCACTGTGGAGAGGCTTGCTTGTGGCTTCCCTAAGAGCTGCAGCAGGCAAGC
TAAGCCTCACAGATGCTGCCACAGTGATGAAACTAGCATCAAAAACCGGCCGGACACCC
AGGGACCATGCACACTTCTCAGCTTGGAGCTCTCCAGGACCAGAAGAGTCAGGTCTGAG
GGTTTGTAGCCAGACCCTCGGCCTCTAGGGACACCCTGGCCATCACAGCGGATGGGCTG
GTGCCCCACATGCCATCTGCTCCAAACAGGGGCTTCAGAGGGCTCTGAGGTGACTTCACT
CATGACCACAGGTGCCCTGGCCCCTTCCCCGCCAGCTACACCGAACCCTGTCCCAACAGC

TGCCCCAGTTCCAACAGCCAATTCCTGGGGCCCAGAATTGCTGTAGACACCAGCCTCGTT
CCAGCACCTCCTGCCAATTGCCTGGATTCACATCCTGGCTGGAATCAAGAGGGCAGCATC
CGCCAGGCTCCCAACAGGCAGGACTCCCGCACACCCTCCTCTGAGAGGCCGCTGTGTTCC
GCAGGGCCAGGCCCTGGACAGTTCCCCTCACCTGCCACTAGAGAAACACCTGCCATTGTC
GTCCCCACCTGGAAAAGACCACTCGTGGAGCCCCCAGCCCCAGGTACAGCTGTAGAGAG
ACTCCCCGAGGGATCTAAGAAGGAGCCATGCGCAGTTCTGCCGGGACCCTCGGCCAGGC
CGACAGGAGTGGACACTGGAGCTGGGCCCACACTGGGCCACATAGGAGCTCACCAGTGA
GGGCAGGAGAGCACATGCCGGGGAGCACCCAGCCTCCTGCTGACCAGAGGCCCGTCCCA
GAGCCCAGGAGGCTGCAGAGGCCTCTCCAGGGGGACACTGTGCATGTCTGGTCCCTGAG
CAGCCCCCCACGTCCCCAGTCCTGGGGGCCCCTGGCACAGCTGTCTGGACCCTCCCTGTT
CCCTGGGAAGCTCCTCCTGACAGCCCCGCCTCCAGTTCCAGGTGTGGATTTTGTCAGGGG
GTGTCACACTGTGTACTGCCAGAAGTGGATGTGGACTTGCGATAGTGAGAGGAAGAGCT
GTGAGGGTATGGTATGCCGGCTGTGGAGTAAGAAGAAGCTCTGGCACAGTGGTGCTGCC
CATATCAAAAACCAGGCCAAGTAGACAGGCCCCTGCTGTGCAGCCCCAGGCCTCCACTT
CACCTGCTTCTCCTGGGGCTCTCAAGGTCACTGTTGTCTGTACTCTGCCCTCTGTGGGGAG
GGTTCCCTCAGTGGGAGGTCTGTTCTCAACATCCCAGGGCCTCATGTCTGCACGGAAGGC
CAATGGATGGGCAACCTCACATGCCGCGGCTAAGATAGGGTGGGCAGCCTGGCGGGGGA
CAGTACATACTGCTGGGGTGTCTGTCACTGTGCCTAGTGGGGCACTGGCTCCCAAACAAC
GCAGTCCTCGCCAAAATCCCCACAGCCTCCCCTGCTAGGGGCTGGCCTGATCTCCTGCAG
TCCTAGGAGGCTGCTGACCTCCAGAATGTCTCCGTCCCCAGTTCCAGGGCGAGAGCAGAT
CCCAGGCCGGCTGCAGACTGGGAGGCCACCCCCTCCTTCCCAGGGTTCACTGGAGGTGA
CCAAGGTAGGAAATGGCCTTAACACAGGGATGACTGCGCCATCCCCCAACAGAGTCAGC
CCCCTCCTGCTCTGTACCCCGCACCCCCCAGGCCAGTCCACGAAAACCAGGGCCCCACAT
CAGAGTCACTGCCTGGCCCGGCCCTGGGGCGGACCCCTCAGCCCCCACCCTGTCTAGAGG
ACTTGGGGGGACAGGACACAGGCCCTCTCCTTATGGTTCCCCCACCTGCCTCCGGCCGGG
ACCCTTGGGGTGTGGACAGAAAGGACACCTGCCTAATTGGCCCCCAGGAACCCAGAACT
TCTCTCCAGGGACCCCAGCCCGAGCACCCCCTTACCCAGGACCCAGCCCTGCCCCTCCTC
CCCTCTGCTCTCCTCTCATCACCCCATGGGAATCCGGTATCCCCAGGAAGCCATCAGGAA
GGGCTGAAGGAGGAAGCGGGGCCGTGCACCACCGGGCAGGAGGCTCCGTCTTCGTGAAC
CCAGGGAAGTGCCAGCCTCCTAGAGGGTATGGTCCACCCTGCCTGGGGCTCCCACCGTG
GCAGGCTGCGGGGAAGGACCAGGGACGGTGTGGGGGAGGGCTCAGGGCCCTGCGGGTG
CTCCTCCATCTTCGGTGAGCCTCCCCCTTCACCCACCGTCCCGCCCACCTCCTCTCCACCC
TGGCTGCACGTCTTCCACACCATCCTGAGTCCTACCTACACCAGAGCCAGCAAAGCCAGT
GCAGACAAAGGCTGGGGTGCAGGGGGGCTGCCAGGGCAGCTTCGGGGAGGGAAGGATG
GAGGGAGGGGAGGTCAGTGAAGAGGCCCCCTTCCCCTGGGTCCAGGATCCTCCTCTGGG
ACCCCCGGATCCCATCCCCTCCTGGCTCTGGGAGGAGAAGCAGGATGGGAGAATCTGTG
CGGGACCCTCTCACAGTGGAATATCCCCACAGCGGCTCAGGCCAGACCCAAAAGCCCCT
CAGTGAGCCCTCCACTGCAGTCCTGGGCCTGGGTAGCAGCCCCTCCCACAGAGGACAGA
CCCAGCACCCCGAAGAAGTCCTGCCAGGGGGAGCTCAGAGCCATGAAAGAGCAGGATAT
GGGGTCCCCGATACAGGCACAGACCTCAGCTCCATCCAGGCCCACCGGGACCCACCATG
GGAGGAACACCTGTCTCCGGGTTGTGAGGTAGCTGGCCTCTGTCTCGGACCCCACTCCAG
ACACCAGACAGAGGGGCAGGCCCCCCAAAACCAGGGTTGAGGGATGATCCGTCAAGGC
AGACAAGACCAAGGGGCACTGACCCCAGCAAGGGAAGGCTCCCAAACAGACGAGGAGG
ATTTTGTAGCTGTCTGTATCACTGTGTGCAACTGCAGCAGATGGGCTCGCGACCATAGCA
GGTGCTGCCACAGTGACACTCGCCAGGTCAAAAACCCCGTCCCAAGTCAGCGGAAGCAG
AGAGAGCAGGGAGGACACGTTTAGGATCTGAGGCCGCACCTGACACCCAGGGCAGCAG
ACGTCTCCCCTCCAGGGCACCCTCCACCGTCCTGCGTTTCTTCAAGAATAGGGGCGGCCT
GAGGGGGTCCAGGGCCAGGCGATAGGTCCCCTCTACCCCAAGGAGGAGCCAGGCAGGA

CCCGAGCACCGATGCATCTAACGCAGTCATGTAATGCTGGGTGACAGTCAGTTCGCCTAC
GTA

TX-DH120126 (SEQ ID NO:235) includes toxin coding sequence inserted in positions corresponding to D_{H}1-20 to D_{H}1-26:

TACGTAATGCATCTAACGCAGTCATGTAATGCTGGGTGACAGTCAGTTCGCCTCCCCATT
GAGGCTGACCTGCCCAGACGGGCCTGGGCCCACCCCACACACCGGGGCGGAATGTGTGC
AGGCCCCAGTCTCTGTGGGTGTTCCGCTAGCTGGGGCCCCCAGTGCTCACCCCACACCTA
AAGCGAGCCCCAGCCTCCAGAGCCCCCTAAGCATTCCCCGCCCAGCAGCCCAGCCCCTG
CCCCCACCCAGGAGGCCCCAGAGCTCAGGGCGCCTGGTCGGATTTTGTACAGCCCCGAG
TCACTGTGGAGAGAGCTTGCAATGGCAGACGCGGCTGCAGCAGATGGGCTCGCGATCAT
AGCAGGTGCTGCCACAGTGAGAAAAACTGTGTCAAAAACCGACTCCTGGCAGCAGTCGG
AGGCCCCGCCAGAGAGGGGAGCAGCCGGCCTGAACCCATGTCCTGCCGGTTCCCATGAC
CCCCAGCACCCAGAGCCCCACGGTGTCCCCGTTGGATAATGAGGACAAGGGCTGGGGGC
TCCGGTGGTTTGCGGCAGGGACTTGATCACATCCTTCTGCTGTGGCCCCATTGCCTCTGGC
TGGAGTTGACCCTTCTGACAAGTGTCCTCAGAAAGACAGGGATCACCGGCACCTCCCAAT
ATCAACCCCAGGCAGCACAGACACAAACCCCACATCCAGAGCCAACTCCAGGAGCAGAG
ACACCCCAACACTCTGGGGGACCCCAACCGTGATAACTCCCCACTGGAATCCGCCCCAG
AGTCTACCAGGACCAAAGGCCCTGCCCTGTCTCTGTCCCTCACTCAGGGCCTCCTGCAGG
GCGAGCGCTTGGGAGCAGACTCGGTCTTAGGGGACACCACTGTGGGCCCCAACTTTGAT
GAGGCCACTGACCCTTCCTTCCTTTCCTGGGGCAGCACAGACTTTGGGGTCTGGGCAGGG
AAGAACTACTGGCTGGTGGCCAATCACAGAGCCCCCAGGCCGAGGTGGCCCCAAGAAGG
CCCTCAGGAGGTGGCCACTCCACTTCCTCCCAGCTGGACCCCAGGTCCTCCCCAAGATAG
GGGTGCCATCCAAGGCAGGTCCTCCATGGAGCCCCCTTCAGACTCCTCCCGGGACCCCAC
TGGACCTCAGTCCCTGCTCTGGGAATGCAGCCACCACAAGCACACCAGGAAGCCCAGGC
CCAGCCACCCTGCAGTGGGCAAGCCCACACTCTGGAGCAGAGCAGGGTGCGTCTGGGAG
GGGCTAACCTCCCCACCCCCCACCCCCCATCTGCACACAGCCACCTACCACTGCCCAGAC
CCTCTGCAGGAGGGCCAAGCCACCATGGGGTATGGACTTAGGGTCTCACTCACGTGCCTC
CCCTCCTGGGAGAAGGGGCCTCATGCCCAGATCCCTGCAGCACTAGACACAGCTGGAGG
CAGTGGCCCCAGGGCCACCCTGACCTGGCATCTAAGGCTGCTCCAGCCCAGACAGCACT
GCCGTTCCTGGGAAGCCTGGGCTCCACCAGACCACAGGTCCAGGGCACAGCCCACAGGA
GCCACCCACACACAGCTCACAGGAAGAAGATAAGCTCCAGACCCCAGGGCGGGACCTGC
CTTCCTGCCACCACTTACACACAGGCCAGGGAGCTGTTCCCACACAGATCAACCCCAAAC
CGGGACTGCCTGGCACTAGGGTCACTGCCATTTCCCTCTCCATTCCCTCCCAGTGCCTCTG
TGCTCCCTCCTTCTGGGGAACACCCTGTGCAGCCCCTCCCTGCAGCCCACACGCTGGGGA
GACCCCACCCTGCCTCGGGCCTTTTCTACCTGCTGCACTTGCCGCCCACCCAAACAACCC
TGGGTACGTGACCCTGCAGTCCTCACCCTGATCTGCAACCAGACCCCTGTCCCTCCCTCT
AAACACCCCTCCCAGGCCAACTCTGCACCTGCAGGCCCTCCGCTCTTCTGCCACAAGAGC
CTCAGGTTTTCCTACCTGTGCCCACCCCCTAACCCCTCCTGCCCACAACTTGAGTTCTTCC
TCTCCTGGAGCCCTTGAGCCATGGCACTGACCCTACACTCCCACCCACACACTGCCCATG
CCATCACCTTCCTCCTGGACACTCTGACCCCGCTCCCCTCCCTCTCAGACCCGGCCCTGGT
ATTTCCAGGACAAAGGCTCACCCAAGTCTTCCCCATGCAGGCCCTTGCCCTCACTGCCTG
GTTACACGGGAGCCTCCTGTGCGCAGAAGCAGGGAGCTCAGCTCTTCCACAGGCAGAAG
GCACTGAAAGAAATCAGCCTCCAGTGCCTTGACACACGTCCGCCTGTGTCTCTCACTGCC
TGCACCTGCAGGGAGGCTCCGCACTCCCTCTAAAGATGAGGGATCCAGGCAGCAACATC
ACGGGAGAATGCAGGGCTCCCAGACAGCCCAGCCCTCTCGCAGGCCTCTCCTGGGAAGA
GACCTGCAGCCACCACTGAACAGCCACGGAGGTCGCTGGATAGTAACCGAGTCAGTGAC

CGACCTGGAGGGCAGGGGAGCAGTGAACCGGAGCCCATACCATAGGGACAGAGACCAG
CCGCTAACATCCCGAGCCCCTCACTGGCGGCCCCAGAACACCCCGTGGAAAGAGAACAG
ACCCACAGTCCCACCTGGAACAGGGCAGACACTGCTGAGCCCCCAGCACCAGCCCCAAG
AAACACTAGGCAACAGCATCAGAGGGGGCTCCTGAGAAAGAGAGGAGGGGAGGTCTCC
TTCACCATCAAATGCTTCCCTTGACCAAAAACAGGGTCCACGCAACTCCCCCAGGACAAA
GGAGGAGCCCCCTGTACAGCACTGGGCTCAGAGTCCTCTCTGAGACAGGCTCAGTTTCAG
ACAACAACCCGCTGGAATGCACAGTCTCAGCAGGAGAGCCAGGCCAGAGCCAGCAAGA
GGAGACTCGGTGACACCAGTCTCCTGTAGGGACAGGAGGATTTTGTGGGGGTTCGTGTC
ACTGTGAGCATATTGTCGGAGCAGGCAGTGCTATTCCCACAGTGACACAACCCCATTCAA
AAACCCCTACTGCAAACGCACCCACTCCTGGGACTGAGGGGCTGGGGGAGCGTCTGGGA
AGTATGGCCTAGGGGTGTCCATCAATGCCCAAAATGCACCAGACTCTCCCCAAGACATC
ACCCCACCAGCCAGTGAGCAGAGTAAACAGAAAATGAGAAGCAGCTGGGAAGCTTGCA
CAGGCCCCAAGGAAAGAGCTTTGGCAGGTGTGCAAGAGGGGATGTGGGCAGAGCCTCA
GCAGGGCCTTTTGCTGTTTCTGCTTTCCTGTGCAGAGAGTTCCATAAACTGGTATTCAAGA
TCAATGGCTGGGAGTGAGCCCAGGAGGACAGTGTGGGAAGAGCACAGGGAAGGAGGAG
CAGCCGCTATCCTACACTGTCATCTTTTGAAAGTTTGCCCTGTGCCCACAATGCTGCATCA
TGGGATGCTTAACAGCTGATGTAGACACAGCTAAAGAGAGAATCAGTGAAATGGATTTG
CAGCACAGATCTGAATAAATCCTCCAGAATGTGGAGCAGCACAGAAGCAAGCACACAGA
AAGTGCCTGATGCCAAGGCAAAGTTCAGTGGGCACCTTCAGGCATTGCTGCTGGGCACA
GACACTCTGAAAAGCACTGGCAGGAACTGCCTGTGACAAAGCAGAACCCTCAGGCAATG
CCAGCCCTAGAGCCCTTCCTGAGAACCTCATGGGCAAAGATGTGCAGAACAGCTGTTTGT
CATAGCCCCAAACTATGGGGCTGGACAAAGCAAACGTCCATCTGAAGGAGAACAGACAA
ATAAACGATGGCAGGTTCATGAAATGCAAACTAGGACAGCCAGAGGACAACAGTAGAG
AGCTACAGGCGGCTTTGCGGTTGAGTTCATGACAATGCTGAGTAATTGGAGTAACAGAG
GAAAGCCCAAAAAATACTTTTAATGTGATTTCTTCTAAATAAAATTTACACCCGGCAAAA
TGAACTATCTTCTTAAGGGATAAACTTTCCCCTGGAAAAACTATAAGGAAAATCAAGAA
AACGATGATCACATAAACACAGTGGTGGTTACTTCTACTGGGGAAGGAAGAGGGTATGA
GCTGAGACACACAGAGTCGGCAAGTCTCCTAACAAGAACAGAACAAATACATTACAGTA
CCTTGAAAACAGCAGTTAAACTTCTAAATCGCAAGAAGAGGAAAATGCACACACCTGTG
TTTAGAAAATTCTCAGTCCAGCACTGTTCATAATAGCAAAGACATTAACCCAGGTTGGAT
AAATAAGCGATGACACAGGCAATTGCACAATGATACAGACATACATTCAGTATATGAGA
CATCGATGATGTATCCCCAAAGAAATGACTTTAAAGAGAAAAGGCCTGATGTGTGGTGG
CAATCACCTCCCTGGGCATCCCCGGACAGGCTGCAGGCTCACTGTGTGGCAGGGCAGGC
AGGCACCTGCTGGCAGCTCCTGGGGCCTGATGTGGAGCAGGCACAGAGCTGTATATCCC
CAAGGAAGGTACAGTCAGTGCATTCCAGAGAGAAGCAACTCAGCCACACTCCCTGGCCA
GAACCCAAGATGCACACCCATGCACAGGGAGGCAGAGCCCAGCACCTCCGCAGCCACCA
CCACCTGCGCACGGGCCACCACCTTGCAGGCACAGAGTGGGTGCTGAGAGGAGGGGCAG
GGACACCAGGCAGGGTGAGCACCCAGAGAAAACTGCAGAAGCCTCACACATCCCTCACC
TGGCCTGGGCTTCACCTGACCTGGACCTCACCTGGCCTCGGGCCTCACCTGCACCTGCTC
CAGGTCTTGCTGGAGCCTGAGTAGCACTGAGGCTGTAGGGACTCATCCAGGGTTGGGGA
ATGACTCTGCAACTCTCCCACATCTGACCTTTCTGGGTGGAGGCACCTGGTGGCCCAGGG
AATATAAAAAGCCCCAGAATGATGCCTGTGTGATTTGGGGGCAATTTATGAACCCGAAA
GGACATGGCCATGGGGTGGGTAGGGACAGTAGGGACAGATGTCAGCCTGAGGTGAAGC
CTCAGGACACAGGTGGGCATGGACAGTGTCCACCTAAGCGAGGGACAGACCCGAGTGTC
CCTGCAGTAGACCTGAGAGCGCTGGGCCCACAGCCTCCCCTCGGGGCCCTGCTGCCTCCT
CAGGTCAGCCCTGGACATCCCGGGTTTCCCCAGGCCTGGCGGTAGGATTTTGTTGAGGTC
TGTGTCACTGTGGTATTACTATGAGAGGCTTGCTTGTGGCTTCCCTAAGAGCTGCAGCAG
GCAAGCTAAGCCTCACAGATGCTGCTATTACTACCACAGTGTCACAGAGTCCATCAAAA

ACCCATGCCTGGGAGCCTCCCACCACAGCCCTCCCTGCGGGGGACCGCTGCATGCCGTGT
TAGGATTTTGATCGAGGACACGGCGCCATGGGTATGGTGGCTACCACAGCAGTGCAGCC
CATGACCCAAACACACGGGGCAGCAGAAACAATGGACAGGCCCACAAGTGACCATGAT
GGGCTCCAGCCCACCAGCCCCAGAGACCATGAAACAGATGGCCAAGGTCACCCTACAGG
TCATCCAGATCTGGCTCCAAGGGGTCTGCATCGCTGCTGCCCTCCCAACGCCAAACCAGA
TGGAGACAGGGCCGGCCCCATAGCACCATCTGCTGCCGTCCACCCAGCAGTCCCGGAAG
CCCCTCCCTGAACGCTGGGCCACGTGTGTGAACCCTGCGAGCCCCCCATGTCAGAGTAGG
GGCAGCAGGAGGGCGGGGCTGGCCCTGTGCACTGTCACTGCCCCTGTGGTCCCTGGCCTG
CCTGGCCCTGACACCTGAGCCTCTCCTGGGTCATTTCCAAGACATTCCCAGGGACAGCCG
GAGCTGGGAGTCGCTCATCCTGCCTGGCTGTCCTGAGTCCTGCTCATTTCCAGACCTCAC
CAGGGAAGCCAACAGAGGACTCACCTCACACAGTCAGAGACAACGAACCTTCCAGAAAT
CCCTGTTTCTCTCCCCAGTGAGAGAAACCCTCTTCCAGGGTTTCTCTTCTCTCCCACCCTC
TTCCAGGACAGTCCTCAGCAGCATCACAGCGGGAACGCACATCTGGATCAGGACGGCCC
CCAGAACACGCGATGGCCCATGGGGACAGCCCAGCCCTTCCCAGACCCCTAAAAGGTAT
CCCCACCTTGCACCTGCCCCAGGGCTCAAACTCCAGGAGGCCTGACTCCTGCACACCCTC
CTGCCAGATATCACCTCAGCCCCCTCCTGGAGGGGACAGGAGCCCGGGAGGGTGAGTCA
GACCCACCTGCCCTCAATGGCAGGCGGGGAAGATTCAGAAAGGCCTGAGATCCCCAGGA
CGCAGCACCACTGTCAATGGGGGCCCCAGACGCCTGGACCAGGGCCTGTGTGGGAAAGG
CCTCTGGCCACACTCAGGGGGATTTTGTGAAGGGCCCTCCCACTGTGGAGAGGCTTTGCT
GTGGCTTCCCTAAGAGCTGCCGCAGCAGGCAATGCAAGCCTCACAGATGCTGCCACAGT
GATGAAACCAGCATCAAAAACCGACCGGACTCGCAGGGTTTATGCACACTTCTCGGCTC
GGAGCTCTCCAGGAGCACAAGAGCCAGGCCCGAGGGTTTGTGCCCAGACCCTCGGCCTC
TAGGGACACCCGGGCCATCTTAGCCGATGGGCTGATGCCCTGCACACCGTGTGCTGCCAA
ACAGGGGCTTCAGAGGGCTCTGAGGTGACTTCACTCATGACCACAGGTGCCCTGGTCCCT
TCACTGCCAGCTGCACCAGACCCTGTTCCGAGAGATGCCCCAGTTCCAAAAGCCAATTCC
TGGGGCCGGGAATTACTGTAGACACCAGCCTCATTCCAGTACCTCCTGCCAATTGCCTGG
ATTCCCATCCTGGCTGGAATCAAGAGGGCAGCATCCGCCAGGCTCCCAACAGGCAGGAC
TCCCACACACCCTCCTCTGAGAGGCCGCTGTGTTCCGCAGGGCCAGGCCGCAGACAGTTC
CCCTCACCTGCCCATGTAGAAACACCTGCCATTGTCGTCCCCACCTGGCAAAGACCACTT
GTGGAGCCCCCAGCCCCAGGTACAGCTGTAGAGAGAGTCCTCGAGGCCCCTAAGAAGGA
GCCATGCCCAGTTCTGCCGGGACCCTCGGCCAGGCCGACAGGAGTGGACGCTGGAGCTG
GGCCCACACTGGGCCACATAGGAGCTCACCAGTGAGGGCAGGAGAGCACATGCCGGGG
AGCACCCAGCCTCCTGCTGACCAGAGACCCGTCCCAGAGCCCAGGAGGCTGCAGAGGCC
TCTCCAGGGGGACACAGTGCATGTCTGGTCCCTGAGCAGCCCCCAGGCTCTCTAGCACTG
GGGGCCCCTGGCACAGCTGTCTGGACCCTCCCTGTTCCCTGGGAAGCTCCTCCTGACAGC
CCCGCCTCCAGTTCCAGGTGTGGATTTTGTCAGGGGGTGCCACACTGTGTACTGCCAGAA
GTGGATGTGGACTTGCGATAGTGAGAGGAAGTGCTGTGAGGGTATGGTATGCCGGCTGT
GGTGTAAGAAGAAGCTCTGGCACAGTGGTGCCGCCCATATCAAAAACCAGGCCAAGTAG
ACAGACCCCTGCCACGCAGCCCCAGGCCTCCAGCTCACCTGCTTCTCCTGGGGCTCTCAA
GGCTGCTGTCTGCCCTCTGGCCCTCTGTGGGGAGGGTTCCCTCAGTGGGAGGTCTGTGCT
CCAGGGCAGGGATGACTGAGATAGAAATCAAAGGCTGGCAGGGAAAGGCAGCTTCCCG
CCCTGAGAGGTGCAGGCAGCACCACAGAGCCATGGAGTCACAGAGCCACGGAGCCCCCA
GTGTGGGCGTGTGAGGGTGCTGGGCTCCCGGCAGGCCCAGCCCTGATGGGGAAGCCTGC
CCCGTCCCACAGCCCAGGTCCCCAGGGGCAGCAGGCACAGAAGCTGCCAAGCTGTGCTC
TACGATCCTCATCCCTCCAGCAGCATCCACTCCACAGTGGGGAAACTGAGCCTTGGAGAA
CCACCCAGCCCCCTGGAAACAAGGCGGGGAGCCCAGACAGTGGGCCCAGAGCACTGTGT
GTATCCTGGCACTAGGTGCAGGGACCACCCGGAGATCCCCATCACTGAGTGGCCAGCCT
GCAGAAGGACCCAACCCCAACCAGGCCGCTTGATTAAGCTCCATCCCCCTGTCCTGGGA

ACCTCTTCCCAGCGCCACCAACAGCTCGGCTTCCCAGGCCCTCATCCCTCCAAGGAAGGC
CAAAGGCTGGGCCTGCCAGGGGCACAGTACCCTCCCTTGCCCTGGCTAAGACAGGGTGG
GCAGACGGCTGCAGATAGGACATATTGCTGGGGCATCTTGCTCTGTGACTACTGGGTACT
GGCTCTCAACGCAGACCCTACCAAAATCCCCACTGCCTCCCCTGCTAGGGGCTGGCCTGG
TCTCCTCCTGCTGTCCTAGGAGGCTGCTGACCTCCAGGATGGCTTCTGTCCCCAGTTCTAG
GGCCAGAGCAGATCCCAGGCAGGCTGTAGGCTGGGAGGCCACCCCTGTCCTTGCCGAGG
TTCAGTGCAGGCACCCAGGACAGGAAATGGCCTGAACACAGGGATGACTGTGCCATGCC
CTACCTAAGTCCGCCCCTTTCTACTCTGCAACCCCCACTCCCCAGGTCAGCCCATGACGA
CCAACAACCCAACACCAGAGTCACTGCCTGGCCCTGCCCTGGGGAGGACCCCTCAGCCC
CCACCCTGTCTAGAGGAGTTGGGGGGACAGGACACAGGCTCTCTCCTTATGGTTCCCCCA
CCTGGCTCCTGCCGGGACCCTTGGGGTGTGGACAGAAAGGACGCCTGCCTAATTGGCCCC
CAGGAACCCAGAACTTCTCTCCAGGGACCCCAGCCCGAGCACCCCCTTACCCAGGACCC
AGCCCTGCCCCTCCTCCCCTCTGCTCTCCTCTCATCACTCCATGGGAATCCAGAATCCCCA
GGAAGCCATCAGGAAGGGCTGAAGGAGGAAGCGGGGCCGCTGCACCACCGGGCAGGAG
GCTCCGTCTTCGTGAACCCAGGGAAGTGCCAGCCTCCTAGAGGGTATGGTCCACCCTGCC
TGGGGCTCCCACCGTGGCAGGCTGCGGGGAAGGACCAGGGACGGTGTGGGGGAGGGCT
CAGGGCCCTGCAGGTGCTCCATCTTGGATGAGCCCATCCCTCTCACCCACCGACCCGCCC
ACCTCCTCTCCACCCTGGCCACACGTCGTCCACACCATCCTGAGTCCCACCTACACCAGA
GCCAGCAGAGCCAGTGCAGACAGAGGCTGGGGTGCAGGGGGGCCGCCAGGGCAGCTTT
GGGGAGGGAGGAATGGAGGAAGGGGAGGTCAGTGAAGAGGCCCCCCTCCCCTGGGTCT
AGGATCCACCTTTGGGACCCCCGGATCCCATCCCCTCCAGGCTCTGGGAGGAGAAGCAG
GATGGGAGATTCTGTGCAGGACCCTCTCACAGTGGAATACCTCCACAGCGGCTCAGGCC
AGATACAAAAGCCCCTCAGTGAGCCCTCCACTGCAGTGCAGGGCCTGGGGGCAGCCCCT
CCCACAGAGGACAGACCCAGCACCCCGAAGAAGTCCTGCCAGGGGGAGCTCAGAGCCAT
GAAGGAGCAAGATATGGGGACCCCAATACTGGCACAGACCTCAGCTCCATCCAGGCCCA
CCAGGACCCACCATGGGTGGAACACCTGTCTCCGGCCCCTGCTGGCTGTGAGGCAGCTG
GCCTCTGTCTCGGACCCCCATTCCAGACACCAGACAGAGGGACAGGCCCCCCAGAACCA
GTGTTGAGGGACACCCCTGTCCAGGGCAGCCAAGTCCAAGAGGCGCGCTGAGCCCAGCA
AGGGAAGGCCCCCAAACAAACCAGGAGGTTTCTGAAGCTGTCTGTGTCACAGTCTGCTG
CAACTGCAGCAGCAAATGGTGCCGCGACCATAGCAGGTGCTGCCACAATGACACTGGGC
AGGACAGAAACCCCATCCCAAGTCAGCCGAAGGCAGAGAGAGCAGGCAGGACACATTT
AGGATCTGAGGCCACACCTGACACTCAAGCCAACAGATGTCTCCCCTCCAGGGCGCCCT
GCCCTGTTCAGTGTTCCTGAGAAAACAGGGGCAGCCTGAGGGGATCCAGGGCCAGGAGA
TGGGTCCCCTCTACCCCGAGGAGGAGCCAGGCGGGAATCCCAGCCCCCTCCCCATTGAG
GCCATCCTGCCCAGAGGGGCCCGGACCCACCCCACACACCCAGGCAGAATGTGTGCAGG
CCTCAGGCTCTGTGGGTGCCGCTAGCTGGGGCTGCCAGTCCTCACCCCACACCTAAGGTG
AGCCACAGCCGCCAGAGCCTCCACAGGAGACCCCACCCAGCAGCCCAGCCCCTACCCAG
GAGGCCCCAGAGCTCAGGGCGCCTGGGTGGATTTTGTACAGCCCCGAGTCACTGTGGGT
ATAGTGGGGAGAGGCTTTGTGGCTTCCCTAAGAGCTGCAGCAGGCAAAAGCCTCACAGA
TGCTGCAGCTACTACCACAGTGAGAAAAGCTATGTCAAAAACCGTCTCCCGGCCACTGCT
GGAGGCCCAGCCAGAGAAGGGACCAGCCGCCCGAACATACGACCTTCCCAGACCTCATG
ACCCCCAGCACTTGGAGCTCCACAGTGTCCCCATTGGATGGTGAGGATGGGGGCCGGGG
CCATCTGCACCTCCCAACATCACCCCCAGGCAGCACAGGCACAAACCCCAAATCCAGAG
CCGACACCAGGAACACAGACACCCCAATACCCTGGGGGACCCTGGCCCTGGTGACTTCC
CACTGGGATCCACCCCCGTGTCCACCTGGATCAAAGACCCCACCGCTGTCTCTGTCCCTC
ACTCAGGGCCTGCTGAGGGGCGGGTGCTTTGGAGCAGACTCAGGTTTAGGGGCCACCAT
TGTGGGGCCCAACCTCGACCAGGACACAGATTTTTCTTTCCTGCCCTGGGGCAACACAGA
CTTTGGGGTCTGTGCAGGGAGGACCTTCTGGAAAGTCACCAAGCACAGAGCCCTGACTG

AGGTGGTCTCAGGAAGACCCCCAGGAGGGGGCTTGTGCCCCTTCCTCTCATGTGGACCCC
ATGCCCCCCAAGATAGGGGCATCATGCAGGGCAGGTCCTCCATGCAGCCACCACTAGGC
AACTCCCTGGCGCCGGTCCCCACTGCGCCTCCATCCCGGCTCTGGGGATGCAGCCACCAT
GGCCACACCAGGCAGCCCGGGTCCAGCAACCCTGCAGTGCCCAAGCCCTTGGCAGGATT
CCCAGAGGCTGGAGCCCACCCCTCCTCATCCCCCCACACCTGCACACACACACCTACCCC
CTGCCCAGTCCCCCTCCAGGAGGGTTGGAGCCGCCCATAGGGTGGGGGCTCCAGGTCTC
ACTCACTCGCTTCCCTTCCTGGGCAAAGGAGCCTCGTGCCCCGGTCCCCCCTGACGGCGC
TGGGCACAGGTGTGGGTACTGGGCCCCAGGGCTCCTCCAGCCCCAGCTGCCCTGCTCTCC
CTGGGAGGCCTGGGCACCACCAGACCACCAGTCCAGGGCACAGCCCCAGGGAGCCGCCC
ACTGCCAGCTCACAGGAAGAAGATAAGCTTCAGACCCTCAGGGCCGGGAGCTGCCTTCC
TGCCACCCCTTCCTGCCCCAGACCTCCATGCCCTCCCCCAACCACTTACACACAAGCCAG
GGAGCTGTTTCCACACAGTTCAACCCCAAACCAGGACGGCCTGGCACTCGGGTCACTGCC
ATTTCTGTCTGCATTCGCTCCCAGCGCCCCTGTGTTCCCTCCCTCCTCCCTCCTTCCTTTCT
TCCTGCATTGGGTTCATGCCGCAGAGTGCCAGGTGCAGGTCAGCCCTGAGCTTGGGGTCA
CCTCCTCACTGAAGGCAGCCTCAGGGTGCCCAGGGGCAGGCAGGGTGGGGGTGAGGCTT
CCAGCTCCAACCGCTTCGCTACCTTAGGACCGTTATAGTTAGGCGCGCCGTCGACCAATT
CTCATGTTTGACAGCTTATCATCGAATTTCTACGTA

### DNA constructs

Typically, a polynucleotide molecule containing one or more nucleotide coding sequences that each encodes a non-immunoglobulin polypeptide of interest, or portion thereof (e.g., an extracellular portion of an ACKR2 polypeptide or a portion of a toxin peptide) is inserted into a vector, preferably a DNA vector, in order to replicate the polynucleotide molecule in a suitable host cell.

Due to their size, one or more nucleotide coding sequences can be cloned directly from cDNA sources available from commercial suppliers or designed *in silico* based on published sequences available from GenBank. Alternatively, bacterial artificial chromosome (BAC) libraries can provide heterologous nucleotide coding sequences from genes of interest (e.g., a heterologous ACKR2 gene or a toxin encoding sequence). BAC libraries contain an average insert size of 100-150kb and are capable of harboring inserts as large as 300kb (Shizuya, et al., 1992, Proc. Natl. Acad. Sci., USA 89:8794-8797; Swiatek, et al., 1993, Genes and Development 7:2071-2084; Kim, et al., 1996, Genomics 34 213-218; herein incorporated by reference). For example, human and mouse genomic BAC libraries have been constructed and are commercially available (e.g., Invitrogen, Carlsbad Calif.). Genomic BAC libraries can also serve as a source of heterologous coding sequences as well as transcriptional control regions.

Alternatively, heterologous nucleotide coding sequences may be isolated, cloned and/or transferred from yeast artificial chromosomes (YACs). An entire heterologous gene or locus can be cloned and contained within one or a few YACs. If multiple YACs are employed and contain regions of overlapping homology, they can be recombined within yeast host strains to produce a single construct representing the entire locus. YAC arms can be additionally modified with mammalian selection cassettes by retrofitting to assist in introducing the constructs into embryonic stems cells or embryos by methods known in the art and/or described herein.

As described above, exemplary DNA and amino acid sequences for use in constructing an engineered D_{H} region of an immunoglobulin heavy chain locus are provided in Tables 3 and 4, respectively. Other heterologous nucleotide coding sequences can also be found in the GenBank database or other sequence databases known in the art. For example, the mRNA and amino acid sequences of human ACKR2 can be found at GenBank accession numbers NM_001296.4 and NP_001287.2, respectively, and are hereby incorporated by reference. Also, for example, DNA and amino acid sequences of an α-conotoxin can be found at GenBank accession numbers JX177132.1 and AFR68318.1, respectively; of a δ-conotoxin at GenBank accession numbers KR013220.1 and AKD43185.1, respectively; of a κ-conotoxin at GenBank accession numbers DQ311073.1 and ABD33865.1, respectively; of a µ-conotoxin at GenBank accession numbers AY207469.1 and AAO48588.1, respectively; and/or of an ω-conotoxin at GenBank accession numbers M84612.1 and AAA81590.1, respectively; all of which are hereby incorporated by reference. Further, for example, sequences of a toxin from the tarantula *Grammostola Spatulata* can be found at GenBank accession numbers 1TYK_A and 1LUP_A, of SGTX-I can be found at GenBank accession number 1LA4_A, of Huwentoxin-IV (HWTX-IV) can be found at GenBank accession number P83303.2, of Protoxin-I (ProTxI) at GenBank accession number 2M9L_A, of Protoxin-2 (ProTxII) at GenBank accession number P83476.1; all of which are hereby incorporated by reference.

DNA constructs containing one or more nucleotide coding sequences as described herein, in some embodiments, comprise human ACKR2 DNA sequences encoding an extracellular portion of a human ACKR2 polypeptide operably linked to recombination signal sequences (RSSs, i.e., flanked by a 5' RSS and 3' RSS) for recombination with immunoglobulin gene segments (e.g., V_{H} and J_{H}) in a transgenic non-human animal. In some embodiments, DNA constructs containing one or more nucleotide coding sequences as described herein comprise toxin DNA sequences encoding a portion of a µ-conotoxin and/or tarantula toxin peptide operably linked to recombination signal sequences (RSSs, i.e., flanked by a 5' RSS and 3' RSS) for recombination with immunoglobulin gene segments (e.g., V_{H} and J_{H}) in a transgenic non-human animal. Recombination signal sequences may be identical or substantially identical with recombination signal sequences found in nature (e.g., genomic) or may be engineered by the hand of man (e.g., optimized). In some embodiments, RSSs are genomic in origin, and include a sequence or sequences that are found in an immunoglobulin heavy chain locus found in nature (e.g., a human or rodent immunoglobulin heavy chain locus). For example, a DNA construct can include recombination signal sequences located in the 5'-flanking and/or 3'-flanking regions of a nucleotide coding sequence encoding a non-immunoglobulin polypeptide of interest, or portion thereof (e.g., an extracellular portion of heterologous ACKR2 polypeptide or a portion of a toxin peptide), operably linked to the nucleotide coding sequence in a manner capable of recombining the nucleotide coding sequence with a V_{H} and/or J_{H} gene segment. In some embodiments, recombination signal sequences comprise a sequence naturally associated with a traditional D_{H} gene segment (i.e., an RSS found in nature). In some embodiments, recombination signal sequences comprise a sequence that is not naturally associated with a traditional D_{H} gene segment. In some embodiments, recombination signal sequences comprise a sequence that is optimized for recombination with V_{H} and J_{H} gene segments. In some embodiments, recombination signal sequences operably linked to one or more nucleotide coding sequences each encoding a non-immunoglobulin polypeptide of interest, or portion thereof (e.g., an extracellular portion of an ACKR2 polypeptide or a portion of a toxin peptide) provide for recombination at a level similar to, more or less than that level of recombination in the animal from which the sequence is obtained. If additional flanking sequences are useful in optimizing recombination of the one or more nucleotide coding sequences, such sequences can be cloned using existing sequences as probes. Additional sequences necessary for maximizing recombination and/or expression of a heavy chain variable region containing a nucleotide coding sequence of non-immunoglobulin polypeptide (e.g., an ACKR2 or toxin) can be obtained from genomic sequences or other sources depending on the desired outcome.

In various embodiments, one or more nucleotide coding sequences as described herein are each flanked 5' and/or 3' by optimized RSSs. Exemplary optimized RSSs that may be used are provided in Figure 2 and described in Example 1.

In various embodiments, one or more nucleotide coding sequences as described herein are each flanked 5' by an optimized RSS having a sequence at least 50% (e.g., 50%, 55%, 60%, 65%, 70%, 75%, 80%, 85%, 90%, 91%, 92%, 93%, 94%, 95%, 96%, 97%, 98%, 99% or more) identical to a 5' RSS that appears in Figure 2.

In various embodiments, one or more nucleotide coding sequences as described herein are each flanked 5' by an optimized RSS having a sequence that is substantially identical or identical to a 5' RSS that appears in Figure 2.

In various embodiments, one or more nucleotide coding sequences are each flanked 3' by an optimized RSS having a sequence at least 50% (e.g., 50%, 55%, 60%, 65%, 70%, 75%, 80%, 85%, 90%, 91%, 92%, 93%, 94%, 95%, 96%, 97%, 98%, 99% or more) identical to a 3' RSS that appears in Figure 2.

In various embodiments, one or more nucleotide coding sequences as described herein are each flanked 3' by an optimized RSS having a sequence that is substantially identical or identical to a 5'RSS that appears in Figure 2.

In various embodiments, one or more nucleotide coding sequences as described herein are each flanked 5' and 3' by optimized RSSs each 5' and 3' RSS having a sequence at least 50% (e.g., 50%, 55%, 60%, 65%, 70%, 75%, 80%, 85%, 90%, 91%, 92%, 93%, 94%, 95%, 96%, 97%, 98%, 99% or more) identical to 5' and 3' RSSs that appear in Figure 2.

In various embodiments, one or more nucleotide coding sequences as described herein are each flanked 5' and 3' by optimized RSSs each 5' and 3' RSS having a sequence that is substantially identical or identical to 5' and 3' RSSs that appear in Figure 2.

In various embodiments, one or more nucleotide coding sequences as described herein are each flanked by 5' and 3' RSSs that are selected from Figure 2.

DNA constructs can be prepared using methods known in the art. For example, a DNA construct can be prepared as part of a larger plasmid. Such preparation allows the cloning and selection of the correct constructions in an efficient manner as is known in the art. DNA fragments containing one or more nucleotide coding sequences as described herein can be located between convenient restriction sites on the plasmid so that they can be easily isolated from the remaining plasmid sequences for incorporation into the desired animal.

Various methods employed in preparation of plasmids and transformation of host organisms are known in the art. For other suitable expression systems for both prokaryotic and eukaryotic cells, as well as general recombinant procedures, see Molecular Cloning: A Laboratory Manual, 2nd Ed., ed. by Sambrook, J. et al., Cold Spring Harbor Laboratory Press: 1989.

### Production of Non-Human Animals Having An Engineered D_{H} Region

Non-human animals are provided that express antibodies characterized by heavy chain CDR3 diversity to direct binding to particular antigens resulting from integration of one or more nucleotide coding sequences, which one or more nucleotide coding sequences that each encode a non-immunoglobulin polypeptide of interest, or portion thereof (e.g., an extracellular portion of an atypical chemokine receptor such as, e.g., ACKR2, or a portion of a toxin peptide), into an immunoglobulin heavy chain variable region in the genome of the non-human animal. Suitable examples described herein include rodents, in particular, mice.

One or more heterologous nucleotide coding sequences, in some embodiments, comprise genetic material from a heterologous species (e.g., humans, spiders, scorpions, snails, tarantulas, sea anemones, etc.), wherein the heterologous nucleotide coding sequences each encode a non-immunoglobulin polypeptide of interest, or portion thereof (e.g., an extracellular portion of an ACKR polypeptide or a portion of a toxin peptide) that comprises the encoded portion of the genetic material from the heterologous species. In some embodiments, heterologous nucleotide coding sequences described herein comprise nucleotide coding sequences of a heterologous species that encodes a non-immunoglobulin polypeptide of interest, or portion thereof (e.g., an extracellular portion of a heterologous ACKR polypeptide or a portion of a toxin peptide), which portion of a non-immunoglobulin polypeptide of interest appears in an immunoglobulin heavy chain, in particular, a heavy chain CDR3, that is expressed by a B cell of a non-human animal as described herein. Non-human animals, embryos, cells and targeting constructs for making non-human animals, non-human embryos, and cells containing said heterologous nucleotide coding sequences are also provided.

In various embodiments, one or more heterologous nucleotide coding sequences are inserted into a D_{H} region of an immunoglobulin heavy chain variable region within the genome of a non-human animal. In some embodiments, a D_{H} region (or portion thereof) of an immunoglobulin heavy chain variable region is not deleted (i.e., intact). In some embodiments, a D_{H} region (or portion thereof) of an immunoglobulin heavy chain variable region is altered, disrupted, deleted or replaced with one or more heterologous nucleotide coding sequences (e.g., one or more heterologous ACKR2 or one or more heterologous toxin nucleotide coding sequences). In some embodiments, all or substantially all of a D_{H} region is replaced with one or more heterologous nucleotide coding sequences; in some certain embodiments, one or more traditional D_{H} gene segments are not deleted or replaced in a D_{H} region of an immunoglobulin heavy chain variable region. In some embodiments, a D_{H} region as described herein is a synthetic D_{H} region, which synthetic D_{H} region comprises one or more heterologous nucleotide coding sequences as described herein. In some embodiments, a D_{H} region is a human D_{H} region. In some embodiments, a D_{H} region is a murine D_{H} region. In some embodiments, an engineered D_{H} region (or portion thereof) as described herein is inserted into an immunoglobulin heavy chain variable region so that said engineered D_{H} region (or portion thereof) is operably linked with one or more V_{H} gene segments and/or one or more J_{H} gene segments. In some embodiments, one or more heterologous nucleotide coding sequences is inserted into one of the two copies of an immunoglobulin heavy chain variable region, giving rise to a non-human animal that is heterozygous with respect to the one or more heterologous nucleotide coding sequences (i.e., an engineered D_{H} region). In some embodiments, a non-human animal is provided that is homozygous for one or more heterologous nucleotide coding sequences (i.e., an engineered D_{H} region). In some embodiments, a non-human animal is provided that is heterozygous for one or more heterologous nucleotide coding sequences (i.e., an engineered D_{H} region).

In some embodiments, a non-human animal described herein contains a human immunoglobulin heavy chain variable region that includes a D_{H} region that contains one or more heterologous nucleotide coding sequences within its genome (e.g., randomly integrated). Thus, such non-human animals can be described as having a human immunoglobulin heavy chain transgene containing an engineered D_{H} region. An engineered D_{H} region can be detected using a variety of methods including, for example, PCR, Western blot, Southern blot, restriction fragment length polymorphism (RFLP), or a gain or loss of allele assay. In some embodiments, a non-human animal described herein is heterozygous with respect to an engineered D_{H} region as described herein. In some embodiments, a non-human animal described herein is homozygous with respect to an engineered D_{H} region as described herein. In some embodiments, a non-human animal described herein is hemizygous with respect to an engineered D_{H} region as described herein. In some embodiments, a non-human animal described herein contains one or more copies of an engineered D_{H} region as described herein.

In some embodiments, one or more heterologous ACKR nucleotide coding sequences disclosed herein are heterologous ACKR2 nucleotide coding sequences. In some embodiments, one or more heterologous ACKR2 nucleotide coding sequences are human.

In some embodiments, one or more heterologous toxin nucleotide coding sequences described herein are heterologous µ-conotoxin nucleotide coding sequences, heterologous tarantula toxin nucleotide coding sequences and/or combinations thereof.

In various embodiments, one or more heterologous nucleotide coding sequences described herein includes one or more nucleotide coding sequences that each have a sequence at least 50% (e.g., 50%, 55%, 60%, 65%, 70%, 75%, 80%, 85%, 90%, 91%, 92%, 93%, 94%, 95%, 96%, 97%, 98%, 99% or more) identical to one or more nucleotide coding sequences that appear in Table 3 or Table 4.

In various embodiments, one or more heterologous nucleotide coding sequences described herein includes one or more nucleotide coding sequences that each have a sequence that is substantially identical or identical to one or more nucleotide coding sequences that appear in Table 3 or Table 4.

In various embodiments, one or more heterologous nucleotide coding sequences described herein are selected from Table 3 and/or Table 4.

In various embodiments, an engineered D_{H} region described herein comprises one or more heterologous nucleotide coding sequences that each have a sequence that is at least 50% (e.g., 50%, 55%, 60%, 65%, 70%, 75%, 80%, 85%, 90%, 91%, 92%, 93%, 94%, 95%, 96%, 97%, 98%, 99% or more) identical to one or more nucleotide coding sequences that appear in Table 3 or Table 4.

In various embodiments, an engineered D_{H} region described herein comprises one or more heterologous nucleotide coding sequences that each have a sequence that is substantially identical or identical to one or more nucleotide coding sequences that appear in Table 3 or Table 4.

In various embodiments, an engineered D_{H} region described herein comprises 5, 10, 15, 20 or 25 heterologous ACKR2 nucleotide coding sequences that each have a sequence that is identical to 5, 10, 15, 20 or 25 ACKR2 nucleotide coding sequences that appear in Table 3.

In various embodiments, an engineered D_{H} region described herein comprises 5, 10, 15, 20, 25 or 26 heterologous toxin nucleotide coding sequences that each have a sequence that is identical to 5, 10, 15, 20, 25 or 26 toxin nucleotide coding sequences that appear in Table 4.

In various embodiments, an engineered D_{H} region described herein comprises one or more heterologous nucleotide coding sequences that are each flanked by a 5' recombination signal sequence (5' RSS) and a 3' recombination signal sequence (3' RSS), which 5' RSS and 3' RSS each have a sequence that is at least 50% (e.g., 50%, 55%, 60%, 65%, 70%, 75%, 80%, 85%, 90%, 91%, 92%, 93%, 94%, 95%, 96%, 97%, 98%, 99% or more) identical to a 5' RSS and a 3' RSS that appear in Figure 2.

In various embodiments, an engineered D_{H} region described herein comprises one or more heterologous nucleotide coding sequences that are each flanked by a 5' recombination signal sequence (5' RSS) and a 3' recombination signal sequence (3' RSS), which 5' RSS and 3' RSS each have a sequence that is substantially identical or identical to a 5' RSS and a 3' RSS that appear in Figure 2.

In various embodiments, an engineered D_{H} region described herein comprises one or more heterologous nucleotide coding sequences that are each flanked by a 5' recombination signal sequence (5' RSS) and a 3' recombination signal sequence (3' RSS), which 5' RSS and 3' RSS are selected from Figure 2.

In various embodiments, an engineered D_{H} region described herein comprises
(a) SEQ ID NO:39 flanked 5' by SEQ ID NO:59 and flanked 3' by SEQ ID NO:60;
(b) SEQ ID NO:7 flanked 5' by SEQ ID NO:69 and flanked 3' by SEQ ID NO:70;
(c) SEQ ID NO:1 flanked 5' by SEQ ID NO:77 and flanked 3' by SEQ ID NO:78;
(d) SEQ ID NO:33 flanked 5' by SEQ ID NO:87 and flanked 3' by SEQ ID NO:88;
(e) SEQ ID NO:3 flanked 5' by SEQ ID NO:95 and flanked 3' by SEQ ID NO:96; and
(f) SEQ ID NO:5 flanked 5' by SEQ ID NO:103 and flanked 3' by SEQ ID NO:104.

In various embodiments, an engineered D_{H} region described herein comprises
(a) SEQ ID NO:41 flanked 5' by SEQ ID NO:61 and flanked 3' by SEQ ID NO:62;
(b) SEQ ID NO:15 flanked 5' by SEQ ID NO:71 and flanked 3' by SEQ ID NO:72;
(c) SEQ ID NO:37 flanked 5' by SEQ ID NO:79 and flanked 3' by SEQ ID NO:80;
(d) SEQ ID NO:13 flanked 5' by SEQ ID NO:81 and flanked 3' by SEQ ID NO:82;
(e) SEQ ID NO:29 flanked 5' by SEQ ID NO:89 and flanked 3' by SEQ ID NO:90;
(f) SEQ ID NO:11 flanked 5' by SEQ ID NO:97 and flanked 3' by SEQ ID NO:98; and
(g) SEQ ID NO:9 flanked 5' by SEQ ID NO:105 and flanked 3' by SEQ ID NO:106;

In various embodiments, an engineered D_{H} region described herein comprises
(a) SEQ ID NO:43 flanked 5' by SEQ ID NO:63 and flanked 3' by SEQ ID NO:64;
(b) SEQ ID NO:23 flanked 5' by SEQ ID NO:73 and flanked 3' by SEQ ID NO:74;
(c) SEQ ID NO:17 flanked 5' by SEQ ID NO:83 and flanked 3' by SEQ ID NO:84;
(d) SEQ ID NO:35 flanked 5' by SEQ ID NO:91 and flanked 3' by SEQ ID NO:92;
(e) SEQ ID NO:19 flanked 5' by SEQ ID NO:99 and flanked 3' by SEQ ID NO:100; and
(f) SEQ ID NO:21 flanked 5' by SEQ ID NO:107 and flanked 3' by SEQ ID NO:108.

In various embodiments, an engineered D_{H} region described herein comprises
(a) SEQ ID NO:45 flanked 5' by SEQ ID NO:65 and flanked 3' by SEQ ID NO:66;
(b) SEQ ID NO:31 flanked 5' by SEQ ID NO:75 and flanked 3' by SEQ ID NO:76;
(c) SEQ ID NO:25 flanked 5' by SEQ ID NO:85 and flanked 3' by SEQ ID NO:86;
(d) SEQ ID NO:49 flanked 5' by SEQ ID NO:93 and flanked 3' by SEQ ID NO:94;
(e) SEQ ID NO:27 flanked 5' by SEQ ID NO:101 and flanked 3' by SEQ ID NO:102; and
(f) SEQ ID NO:47 flanked 5' by SEQ ID NO:67 and flanked 3' by SEQ ID NO:68.

In various embodiments, an engineered D_{H} region described herein comprises one or more DNA fragments that each have a sequence that is at least 50% (e.g., 50%, 55%, 60%, 65%, 70%, 75%, 80%, 85%, 90%, 91%, 92%, 93%, 94%, 95%, 96%, 97%, 98%, 99% or more) identical to a DNA fragment selected from the group consisting of SEQ ID NO:131, SEQ ID NO:132, SEQ ID NO:133, SEQ ID NO:134, and combinations thereof.

In various embodiments, an engineered D_{H} region described herein comprises one or more DNA fragments that each have a sequence that is substantially identical or identical to a DNA fragment selected from the group consisting of SEQ ID NO:131, SEQ ID NO:132, SEQ ID NO:133, SEQ ID NO:134, and combinations thereof.

In various embodiments, an engineered D_{H} region described herein comprises any one of SEQ ID NO:131, SEQ ID NO:132, SEQ ID NO:133 and SEQ ID NO:134.

In various embodiments, an engineered D_{H} region described herein comprises SEQ ID NO:131, SEQ ID NO:132, SEQ ID NO:133 and SEQ ID NO:134. In some certain embodiments, an engineered D_{H} region described herein comprises, from 5' to 3', SEQ ID NO:131, SEQ ID NO:132, SEQ ID NO:133 and SEQ ID NO:134.

In various embodiments, an engineered D_{H} region described herein comprises
(a) SEQ ID NO:180 flanked 5' by SEQ ID NO:59 and flanked 3' by SEQ ID NO:60;
(b) SEQ ID NO:182 flanked 5' by SEQ ID NO:69 and flanked 3' by SEQ ID NO:70;
(c) SEQ ID NO:184 flanked 5' by SEQ ID NO:77 and flanked 3' by SEQ ID NO:78;
(d) SEQ ID NO:186 flanked 5' by SEQ ID NO:87 and flanked 3' by SEQ ID NO:88;
(e) SEQ ID NO:188 flanked 5' by SEQ ID NO:95 and flanked 3' by SEQ ID NO:96; and
(f) SEQ ID NO:190 flanked 5' by SEQ ID NO:103 and flanked 3' by SEQ ID NO: 104.

In various embodiments, an engineered D_{H} region described herein comprises
(a) SEQ ID NO:192 flanked 5' by SEQ ID NO:61 and flanked 3' by SEQ ID NO:62;
(b) SEQ ID NO:194 flanked 5' by SEQ ID NO:71 and flanked 3' by SEQ ID NO:72;
(c) SEQ ID NO:196 flanked 5' by SEQ ID NO:79 and flanked 3' by SEQ ID NO:80;
(d) SEQ ID NO:198 flanked 5' by SEQ ID NO:81 and flanked 3' by SEQ ID NO:82;
(e) SEQ ID NO:200 flanked 5' by SEQ ID NO:89 and flanked 3' by SEQ ID NO:90;
(f) SEQ ID NO:202 flanked 5' by SEQ ID NO:97 and flanked 3' by SEQ ID NO:98; and
(g) SEQ ID NO:204 flanked 5' by SEQ ID NO:105 and flanked 3' by SEQ ID NO: 106;

In various embodiments, an engineered D_{H} region described herein comprises
(a) SEQ ID NO:206 flanked 5' by SEQ ID NO:63 and flanked 3' by SEQ ID NO:64;
(b) SEQ ID NO:208 flanked 5' by SEQ ID NO:73 and flanked 3' by SEQ ID NO:74;
(c) SEQ ID NO:210 flanked 5' by SEQ ID NO:83 and flanked 3' by SEQ ID NO:84;
(d) SEQ ID NO:212 flanked 5' by SEQ ID NO:91 and flanked 3' by SEQ ID NO:92;
(e) SEQ ID NO:214 flanked 5' by SEQ ID NO:99 and flanked 3' by SEQ ID NO: 100; and
(f) SEQ ID NO:216 flanked 5' by SEQ ID NO:107 and flanked 3' by SEQ ID NO: 108.

In various embodiments, an engineered D_{H} region described herein comprises
(a) SEQ ID NO:218 flanked 5' by SEQ ID NO:65 and flanked 3' by SEQ ID NO:66;
(b) SEQ ID NO:220 flanked 5' by SEQ ID NO:75 and flanked 3' by SEQ ID NO:76;
(c) SEQ ID NO:222 flanked 5' by SEQ ID NO:85 and flanked 3' by SEQ ID NO:86;
(d) SEQ ID NO:224 flanked 5' by SEQ ID NO:93 and flanked 3' by SEQ ID NO:94;
(e) SEQ ID NO:226 flanked 5' by SEQ ID NO:101 and flanked 3' by SEQ ID NO: 102;
(f) SEQ ID NO:228 flanked 5' by SEQ ID NO:109 and flanked 3' by SEQ ID NO: 110; and
(g) SEQ ID NO:230 flanked 5' by SEQ ID NO:67 and flanked 3' by SEQ ID NO:68.

In various embodiments, an engineered D_{H} region described herein comprises one or more DNA fragments that each have a sequence that is at least 50% (e.g., 50%, 55%, 60%, 65%, 70%, 75%, 80%, 85%, 90%, 91%, 92%, 93%, 94%, 95%, 96%, 97%, 98%, 99% or more) identical to a DNA fragment selected from the group consisting of SEQ ID NO:232, SEQ ID NO:233, SEQ ID NO:234, SEQ ID NO:235, and combinations thereof.

In various embodiments, an engineered D_{H} region described herein comprises one or more DNA fragments that each have a sequence that is substantially identical or identical to a DNA fragment selected from the group consisting of SEQ ID NO:232, SEQ ID NO:233, SEQ ID NO:234, SEQ ID NO:235, and combinations thereof.

In various embodiments, an engineered D_{H} region described herein comprises any one of SEQ ID NO:232, SEQ ID NO:233, SEQ ID NO:234 and SEQ ID NO:235.

In various embodiments, an engineered D_{H} region described herein comprises SEQ ID NO:232, SEQ ID NO:233, SEQ ID NO:234 and SEQ ID NO:235. In some certain embodiments, an engineered D_{H} region described herein comprises, from 5' to 3', SEQ ID NO:232, SEQ ID NO:233, SEQ ID NO:234 and SEQ ID NO:235.

In various embodiments, an antibody produced by a non-human animal described herein comprises a heavy chain variable region that includes a CDR3 having an amino acid sequence that is or comprises an amino acid sequence that is at least 50% (e.g., 50%, 55%, 60%, 65%, 70%, 75%, 80%, 85%, 90%, 91%, 92%, 93%, 94%, 95%, 96%, 97%, 98%, 99% or more) identical to an amino acid sequence that appears in Table 3 or Table 4.

In various embodiments, an antibody produced by a non-human animal described herein comprises a heavy chain variable region that includes a CDR3 having an amino acid sequence that is or comprises an amino acid sequence that is substantially identical or identical to an amino acid sequence that appears in Table 3 or Table 4.

In various embodiments, an antibody produced by a non-human animal described herein comprises a heavy chain variable region that includes a CDR3 having an amino acid sequence that is a variant (i.e., somatically mutated variant thereof) of an amino acid sequence that is or comprises an amino acid sequence that appears in Table 3 or Table 4.

Compositions and methods for making non-human animals whose genome comprises an immunoglobulin heavy chain variable region that includes an engineered D_{H} region, wherein the engineered D_{H} region includes one or more nucleotide coding sequences that each encode a portion of a polypeptide of interest, including nucleotide coding sequences of specific polymorphic forms of a polypeptide of interest, allelic variants of a polypeptide of interest (e.g., single amino acid differences) or alternatively spliced isoforms of a polypeptide of interest, are provided, including compositions and methods for making non-human animals that express antibodies comprising a heavy chain variable region that includes a CDR3 having an amino acid sequence encoded by such nucleotide coding sequences from an immunoglobulin heavy chain locus that contains human V_{H} and J_{H} gene segments operably linked to one or more non-human heavy chain constant region genes. In some embodiments, compositions and methods for making non-human animals that express such antibodies under the control of an endogenous enhancer(s) and/or an endogenous regulatory sequence(s) are also provided. In some embodiments, compositions and methods for making non-human animals that express such antibodies under the control of a heterologous enhancer(s) and/or a heterologous regulatory sequence(s) are also provided. Methods include inserting one or more nucleotide coding sequences that each encode a portion of a polypeptide of interest, or one or more DNA fragments that each contain a plurality of nucleotide coding sequences that each encode a portion of a polypeptide of interest, in the genome of a non-human animal so that an antibody is expressed which includes said portion of a polypeptide of interest.

In some embodiments, methods include inserting about 10,050bp of DNA that includes six nucleotide coding sequences corresponding to extracellular portions of a heterologous ACKR2 polypeptide, about 9,768bp of DNA that includes seven nucleotide coding sequences corresponding to extracellular portions of a heterologous ACKR2 polypeptide, about 9,788bp of DNA that includes six nucleotide coding sequences corresponding to extracellular portions of a heterologous ACKR2 polypeptide, and/or about 11,906bp of DNA that includes six nucleotide coding sequences corresponding to extracellular portions of a heterologous ACKR2 polypeptide. Together, such DNA includes 25 coding sequences corresponding to extracellular portions of a heterologous ACKR2 polypeptide. In some embodiments, methods include inserting DNA that further comprises recombination signal sequences that flank each of the nucleotide coding sequences corresponding to extracellular portions of a heterologous ACKR2 polypeptide. Genetic material that includes the nucleotide coding sequences corresponding to extracellular portions of a heterologous ACKR2 polypeptide and flanking recombination signal sequences described above may be inserted into the genome of a non-human animal, thereby creating a non-human animal having an engineered D_{H} region that contains nucleotide coding sequences corresponding to extracellular portions of a heterologous ACKR2 polypeptide and the necessary recombination signal sequences to allow for recombination with adjacent V_{H} and J_{H} gene segments.

In some embodiments, methods include inserting about 9,812bp of DNA that includes six nucleotide coding sequences corresponding to portions of one or more toxins (e.g., a µ-conotoxin and/or a tarantula toxin), about 9,512bp of DNA that includes seven nucleotide coding sequences corresponding to portions of one or more toxins (e.g., a µ-conotoxin and/or a tarantula toxin), about 9,691bp of DNA that includes six nucleotide coding sequences corresponding to portions of one or more toxins (e.g., a µ-conotoxin and/or a tarantula toxin), and/or about 11,896bp of DNA that includes seven nucleotide coding sequences corresponding to portions of one or more toxins (e.g., a µ-conotoxin and/or a tarantula toxin). Together, such DNA includes 26 coding sequences corresponding to portions of one or more toxin peptide(s). In some embodiments, methods include inserting DNA that further comprises recombination signal sequences that flank each of the nucleotide coding sequences corresponding to portions of one or more toxin peptide(s). Genetic material that includes the nucleotide coding sequences corresponding to portions of one or more toxin peptide(s) and flanking recombination signal sequences described above may be inserted into the genome of a non-human animal, thereby creating a non-human animal having an engineered D_{H} region that contains nucleotide coding sequences corresponding to portions one or more toxin peptide(s) and the necessary recombination signal sequences to allow for recombination with adjacent V_{H} and J_{H} gene segments.

Where appropriate, nucleotide coding sequences corresponding to portions of a heterologous polypeptide of interest may be modified to include codons that are optimized for expression in the non-human animal (e.g., see U.S. Patent Nos. 5,670,356 and 5,874,304). Codon optimized sequences are synthetic sequences, and preferably encode the identical polypeptide (or a biologically active fragment of a full-length polypeptide which has substantially the same activity as the full-length polypeptide) encoded by the non-codon optimized parent polynucleotide. In some embodiments, nucleotide coding sequences corresponding to portions of a heterologous polypeptide of interest may include an altered sequence to optimize codon usage for a particular cell type (e.g., a rodent cell). For example, the codons of the nucleotide coding sequences corresponding to portions of a heterologous polypeptide of interest to be inserted into the genome of a non-human animal (e.g., a rodent) may be optimized for expression in a cell of the non-human animal. Such a sequence may be described as a codon-optimized sequence.

Insertion of nucleotide coding sequences corresponding to portions of a heterologous polypeptide of interest into a D_{H} region so that said nucleotide coding sequences are operably linked to V_{H} and J_{H} gene segments (e.g., a plurality of V_{H} and J_{H} gene segments) employs a relatively minimal modification of the genome and results in expression of antibodies comprising heavy chains characterized by CDR3s having amino acid sequences corresponding to portions of a heterologous polypeptide of interest in the non-human animal.

Methods for generating transgenic non-human animals, including knockouts and knock-ins, are well known in the art (see, e.g., Gene Targeting: A Practical Approach, Joyner, ed., Oxford University Press, Inc. (2000)). For example, generation of transgenic rodents may optionally involve disruption of the genetic loci of one or more endogenous rodent genes (or gene segments) and introduction of one or more heterologous genes (or gene segments or nucleotide coding sequences) into the rodent genome, in some embodiments, at the same location as an endogenous rodent gene (or gene segments). In some embodiments, nucleotide coding sequences corresponding to portions of a heterologous polypeptide of interest are introduced into a D_{H} region of a randomly inserted immunoglobulin heavy chain locus in the genome of a rodent. In some embodiments, nucleotide coding sequences corresponding to portions of a heterologous polypeptide of interest are introduced into a D_{H} region of an endogenous immunoglobulin heavy chain locus in the genome of a rodent; in some certain embodiments, an endogenous immunoglobulin heavy chain locus is altered, modified, or engineered to contain human gene segments (e.g., V and/or J) operably linked to one or more constant region genes (e.g., human or murine).

A schematic illustration (not to scale) of an exemplary strategy for construction of a targeting vector for integration into rodent embryonic stem (ES) cells to create a rodent whose genome comprises an immunoglobulin heavy chain variable region that includes an engineered diversity cluster (i.e., D_{H} region), which diversity cluster includes one or more nucleotide coding sequences that each encode a portion of a heterologous ACKR2 polypeptide (e.g., an extracellular portion of a D6 chemokine decoy receptor) is provided in Figures 3A and 3B, or that each encode a portion of a toxin peptide (e.g., a portion of a µ-conotoxin and/or a tarantula toxin peptide) is provided in Figures 7A and 7B. As illustrated, DNA fragments each containing various numbers of nucleotide coding sequences that each encode an extracellular portion of a heterologous ACKR2 polypeptide (Figures 3A-3B) or that each encode a portion of a toxin peptide (Figures 7A-7B) are assembled together using isothermic assembly along with a selection cassette (e.g., neomycin) flanked by site-specific recombination recognition sites (e.g., *lox*P). An alternative strategy that employs sequential ligation of DNA fragments is illustrated in Figures 4A and 4B (for ACKR2 nucleotide coding sequences) and 8A and 8B (for toxin nucleotide coding sequences), respectively. The DNA fragments include recombination signal sequences that flank (5' and 3') each of the nucleotide coding sequences to allow for recombination with V_{H} and J_{H} gene segments once integrated into an immunoglobulin heavy chain locus. The recombination signal sequences may be obtained from genomic sources or, alternatively, they may be optimized as described herein to provide for efficient recombination of the nucleotide coding sequences with operably linked V_{H} and J_{H} gene segments. The nucleotide coding sequences themselves may be optimized such that somatic hypermutation during affinity maturation of antibody sequences occurs at, near or higher than the level of somatic hypermutation observed in wild-type non-human animals which comprise an immunoglobulin heavy chain locus that includes traditional D_{H} gene segments or a non-human animal that is transgenic for a human immunoglobulin heavy (and/or light chain) locus that includes traditional human D_{H} gene segments. Once assembled, the assembled DNA fragments (i.e., engineered D_{H} region) are ligated into a BAC vector that contains genomic heavy chain variable region DNA (e.g., human) to create a targeting vector for integration into an immunoglobulin heavy chain locus. The ligation is performed such that the engineered D_{H} region is flanked 5' by V_{H} genomic DNA and 3' by J_{H} genomic DNA and non-human (e.g., rodent) genomic heavy chain constant region DNA (e.g., intronic enhancer and a IgM constant region gene). The final targeting vector for incorporation into the genome of a non-human cell (e.g., a rodent embryonic stem cell) contains V_{H} genomic DNA (e.g., containing one or more V_{H} gene segments), an engineered D_{H} region, 3' J_{H} genomic DNA, and non-human (e.g., rodent) genomic heavy chain constant region DNA, all of which are operably linked to allow for recombination between V_{H} gene segments, a nucleotide coding sequence within the engineered D_{H} region and a J_{H} gene segment once integrated into the genome of a non-human animal.

The targeting vector is introduced into rodent (e.g., mouse) embryonic stem cells so that the sequence contained in the targeting vector (i.e., an engineered D_{H} region) results in the capacity of a non-human cell or non-human animal (e.g., a mouse) that expresses antibodies that contain CDR3s having amino acids encoded by the inserted nucleotide coding sequences.

As described herein, a transgenic rodent is generated where an engineered D_{H} region has been introduced into an endogenous immunoglobulin heavy chain locus of the rodent genome (e.g., an endogenous immunoglobulin heavy chain locus engineered to contain human variable region gene segments). Immunoglobulins are expressed on murine cells, which immunoglobulins have CDR3s containing amino acids encoded by the nucleotide coding sequences from the engineered D_{H} region. The immunoglobulins further comprise murine constant regions and, therefore, provide for the necessary effector functions for various immune cells in the rodent's immune system. When an endogenous immunoglobulin heavy chain locus of the rodent genome is not targeted by the targeting vector, the engineered D_{H} region is preferably inserted into an immunoglobulin heavy chain locus at a location other than that of the endogenous murine immunoglobulin heavy chain locus (e.g., randomly inserted). In such cases, the endogenous murine immunoglobulin heavy chain locus may be deleted or otherwise rendered non-functional (e.g., by targeted deletion, insertion, inversion, etc.) so that antibodies produced by the non-human animal utilize gene segments and sequences from the immunoglobulin heavy chain locus containing the inserted engineered D_{H} region.

In some embodiments, the genome of a non-human animal described herein further comprises one or more human immunoglobulin heavy and/or light chain genes (see, e.g., U.S. Patent No. 8,502,018; U.S. Patent No. 8,642,835; U.S. Patent No. 8,697,940; U.S. Patent No: 8,791,323; and U.S. Patent Application Publication No. 2013/0096287 A1; herein incorporated by reference). Alternatively, an engineered D_{H} region can be introduced into an embryonic stem cell of a different modified strain such as, e.g., a VELOCIMMUNE^{®} strain (see, e.g., U.S. Patent No. 8,502,018 or U.S. Patent No. 8,642,835; herein incorporated by reference). In some embodiments, an engineered D_{H} region can be introduced into an embryonic stem cell of a modified strain as described in U.S. Patent Nos. 8,697,940 and 8,642,835; herein incorporated by reference.

In some embodiments, the genome of a non-human animal described herein further comprises (e.g., via cross-breeding or multiple targeting strategies) one or more human immunoglobulin light chain genes or loci (e.g., transgenic, endogenous, etc.) as described in U.S. Patent Application Publication Nos. 2011-0195454 A1, 2012-0021409 A1, 2012-0192300 A1, 2013-0045492 A1, 2013-0185821 A1, 2013-0198880 A1, 2013-0302836 A1, 2015-0059009 A1; International Patent Application Publication Nos. WO 2011/097603, WO 2012/148873, WO 2013/134263, WO 2013/184761, WO 2014/160179, WO 2014/160202; all of which are hereby incorporated by reference.

The genetic engineering of a single rearranged light chain, e.g., a light chain comprising a rearranged light chain variable region has been described. For example, generation of a universal light chain mouse (ULC) comprising a single rearranged variable gene sequence V_{L}:J_{L} and generation of antigen-specific antibodies in those mice is described in, e.g., U.S. Patent Application Nos. 13/022,759, 13/093,156, 13/412,936, 13/488,628, 13/798,310, and 13/948,818 (Publication Nos. 2011/0195454, 2012/0021409, 2012/0192300, 2013/0045492, US20130185821, and US20130302836 respectively), each of which is incorporated herein by reference in its entirety. The engineered common light chain mouse described in U.S. Application Publication Nos. 2011/0195454, 2012/0021409, 2012/0192300 and 2013/0045492 comprised nucleic acid sequence encoding a limited repertoire of light chain options, e.g., common or universal light chain "ULC" that comprised no more than two VL gene segments or a single rearranged human immunoglobulin light chain variable region sequence. To achieve such limited repertoire, a mouse was engineered to render nonfunctional or substantially nonfunctional its ability to make, or rearrange, a native mouse light chain variable domain. In one aspect, this was achieved, e.g., by deleting the mouse's light chain variable region gene segments. As previously described, the endogenous mouse locus can then be modified by exogenous suitable light chain variable region gene segments of choice, preferably human light chain variable region gene segments, operably linked to the endogenous mouse light chain constant domain, in a manner such that the exogenous variable region gene segments can combine with the endogenous mouse light chain constant region gene and form a rearranged reverse chimeric light chain gene (human variable, mouse constant). In various embodiments, the light chain variable region is capable of being somatically mutated. In various embodiments, to maximize ability of the light chain variable region to acquire somatic mutations, the appropriate enhancer(s) is retained in the mouse. In one aspect, in modifying a mouse κ light chain locus to replace endogenous mouse κ light chain gene segments with human κ light chain gene segments, the mouse κ intronic enhancer and mouse κ 3' enhancer are functionally maintained, or undisrupted.

Thus, provided was a genetically engineered mouse that expresses a limited repertoire of reverse chimeric (human variable, mouse constant) light chains associated with a diversity of reverse chimeric (human variable, mouse constant) heavy chains comprising amino acids encoded by a genetically engineered D_{H} region (or portion thereof) as described herein . In various embodiments, the endogenous mouse κ light chain gene segments are deleted and replaced with a single (or two) rearranged human light chain region, operably linked to the endogenous mouse C κ gene. In embodiments for maximizing somatic hypermutation of the rearranged human light chain region, the mouse κ intronic enhancer and the mouse κ 3' enhancer are maintained. In various embodiments, the mouse also comprises a nonfunctional κ light chain locus, or a deletion thereof or a deletion that renders the locus unable to make a κ light chain.

Thus, in one embodiment, provided herein is a non-human animal (e.g., a rodent, e.g., a mouse or a rat) that comprises in its genome, e.g., in its germline, a limited repertoire of preferably human light chain variable regions, or a single rearranged human light chain variable region, from a limited repertoire of preferably human light chain variable gene segments, wherein the non-human animal also comprises in its genome, e.g., in its germline, an immunoglobulin heavy chain variable region that includes an engineered D_{H} region, wherein the engineered D_{H} region includes one or more nucleotide sequences that each encode a non-immunoglobulin polypeptide of interest, or portion thereof.

Genetically engineered animals are provided that express a limited repertoire of human light chain variable domains, or a single human light chain variable domain, from a limited repertoire of human light chain variable region gene sequences. In one embodiment, the single rearranged V/J human light chain sequence is selected from Vκ1-39Jκ and Vκ3-20Jκ, e.g., Vκ1-39Jκ5 and Vκ3-20Jκ1. In some embodiments, a non-human animal as disclosed herein comprises a modified light chain locus comprising a replacement all endogenous functional V_{L} and all endogenous functional J_{L} gene segments with the single rearranged V/J light chain sequence, wherein the single rearranged V/J light chain sequence is operably linked to an endogenous light chain constant region gene. In some embodiments, the modified light chain locus is in the germline genome of the non-human animal. In one embodiment, the non-human animal comprises in its germline genome a single rearranged light chain variable gene sequence operably linked to a light chain constant region gene sequence, wherein the single rearranged light chain variable region gene sequence comprises human germline V_{L} and human germline J_{L} gene segments, e.g., human germline Vκ1-39 and human germline Jκ5 or human germline Vκ3-20 and Jκ1. In some embodiments, a non-human animal as disclosed herein comprises a B cell, e.g., a B cell that has not undergone class switching, comprising in its genome a single rearranged V/J light chain sequence operably linked to an endogenous light chain constant region gene, wherein the single rearranged V/J light chain does not comprise somatic mutations compared to a single rearranged V/J light chain sequence operably linked to an endogenous light chain constant region gene found in the germline genome of the non-human animal. In other embodiments, a non-human animal as disclosed herein comprises a B cell, e.g., a B cell that has undergone class switching, comprising in its genome a single rearranged V/J light chain sequence operably linked to an endogenous light chain constant region gene, wherein the single rearranged V/J light chain comprises somatic mutations compared to a single rearranged V/J light chain sequence operably linked to an endogenous light chain constant region gene found in the germline genome of the non-human animal.

Accordingly, a genetically modified non-human animal is provided, along with methods and compositions for making the animal, wherein the genetic modification comprises an engineered D_{H} region and a single rearranged light chain locus, and wherein the animal further expresses a genetically engineered single rearranged light chain, e.g., an engineered common light chain (ULC), which may associate with a heavy chain containing amino acids encoded by the engineered D_{H} region in tissues or cells of the non-human animal.

A transgenic founder non-human animal can be identified based upon the presence of an engineered D_{H} region in its genome and/or expression of antibodies containing amino acids encoded by the nucleotide coding sequences in tissues or cells of the non-human animal. A transgenic founder non-human animal can then be used to breed additional non-human animals carrying the engineered D_{H} region thereby creating a series of non-human animals each carrying one or more copies of an engineered D_{H} region. Moreover, transgenic non-human animals carrying an engineered D_{H} region can further be bred to other transgenic non-human animals carrying other transgenes (e.g., human immunoglobulin genes) as desired.

Transgenic non-human animals may also be produced to contain selected systems that allow for regulated or directed expression of the transgene. Exemplary systems include the Cre/loxP recombinase system of bacteriophage P1 (see, e.g., Lakso, M. et al., 1992, Proc. Natl. Acad. Sci. USA 89:6232-6236) and the FLP/Frt recombinase system of S. cerevisiae (O'Gorman, S. et al, 1991, Science 251:1351-1355). Such animals can be provided through the construction of *"double"* transgenic animals, e.g., by mating two transgenic animals, one containing a transgene comprising a selected modification (e.g., an engineered D_{H} region) and the other containing a transgene encoding a recombinase (e.g., a Cre recombinase).

The non-human animals described herein may be prepared as described above, or using methods known in the art, to comprise additional human or humanized genes, oftentimes depending on the intended use of the non-human animal. Genetic material of such additional human or humanized genes may be introduced through the further alteration of the genome of cells (e.g., embryonic stem cells) having the genetic modifications as described above or through breeding techniques known in the art with other genetically modified strains as desired. In some embodiments, non-human animals described herein are prepared to further comprise transgenic human immunoglobulin heavy and light chain genes (see e.g., Murphy, A.J. et al., (2014) Proc. Natl. Acad. Sci. U.S.A. 111(14):5153-5158; U.S. Patent No. 8,502,018; U.S. Patent No. 8,642,835; U.S. Patent No. 8,697,940; U.S. Patent No: 8,791,323; and U.S. Patent Application Publication No. 2013/0096287 A1; herein incorporated by reference). In some embodiments, non-human animals described herein are prepared to further comprise one or more human immunoglobulin light chain genes or loci (e.g., transgenic, endogenous, etc.) as described in U.S. Patent Application Publication Nos. 2011-0195454 A1, 2012-0021409 A1, 2012-0192300 A1, 2013-0045492 A1, 2013-0185821 A1, 2013-0198880 A1, 2013-0302836 A1, 2015-0059009 A1; International Patent Application Publication Nos. WO 2011/097603, WO 2012/148873, WO 2013/134263, WO 2013/184761, WO 2014/160179, WO 2014/160202; all of which are hereby incorporated by reference.

In some embodiments, non-human animals described herein may be prepared by introducing a targeting vector, as described herein, into a cell from a modified strain. To give but one example, a targeting vector, as described above, may be introduced into a VELOCIMMUNE^{®} mouse. VELOCIMMUNE^{®} mice express antibodies that have fully human variable regions and mouse constant regions. In some embodiments, non-human animals described herein are prepared to further comprise human immunoglobulin genes (variable and/or constant region genes). In some embodiments, non-human animals described herein comprise an engineered D_{H} region, as described herein, and genetic material from a heterologous species (e.g., humans), wherein the genetic material encodes, in whole or in part, one or more human heavy and/or light chain variable regions.

For example, as described herein, non-human animals comprising an engineered D_{H} region may further comprise (e.g., via cross-breeding or multiple gene targeting strategies) one or more modifications as described in Murphy, A.J. et al., (2014) Proc. Natl. Acad. Sci. U.S.A. 111(14):5153-5158; U.S. Patent No. 8,502,018; U.S. Patent No. 8,642,835; U.S. Patent No. 8,697,940; U.S. Patent No: 8,791,323; U.S. Patent Application Publication Nos. 2011-0195454 A1, 2012-0021409 A1, 2012-0192300 A1, 2013-0045492 A1, 2013-0096287 A1, 2013-0185821 A1, 2013-0198880 A1, 2013-0302836 A1, 2015-0059009 A1; International Patent Application Publication Nos. WO 2011/097603, WO 2012/148873, WO 2013/134263, WO 2013/184761, WO 2014/160179 or WO 2014/160202; all of these applications are incorporated herein by reference in their entirety. In some embodiments, a rodent comprising an engineered D_{H} region as described herein is crossed to a rodent comprising a humanized immunoglobulin heavy and/or light chain variable region locus (see, e.g., U.S. Patent No. 8,502,018 or U.S. Patent No. 8,642,835; incorporated herein by reference). In some embodiments, a rodent comprising an engineered D_{H} region as described herein is crossed to a rodent comprising a humanized immunoglobulin light chain genes or loci as described in U.S. Patent Application Publication Nos. 2011-0195454 A1, 2012-0021409 A1, 2012-0192300 A1, 2013-0045492 A1, 2013-0185821 A1, 2013-0198880 A1, 2013-0302836 A1, 2015-0059009 A1; International Patent Application Publication Nos. WO 2011/097603, WO 2012/148873, WO 2013/134263, WO 2013/184761, WO 2014/160179, WO 2014/160202; all of which are hereby incorporated by reference.

Although embodiments employing an engineered D_{H} region in a mouse (i.e., a mouse with an engineered D_{H} region operably linked with human V_{H} and J_{H} gene segments, all of which are operably linked with one or more murine heavy chain constant region genes) are extensively discussed herein, other non-human animals that comprise an engineered D_{H} region are also provided. In some embodiments, such non-human animals comprise an engineered D_{H} region operably linked to endogenous V_{H} and J_{H} gene segments. In some embodiments, such non-human animals comprise an engineered D_{H} region operably linked to humanized V_{H} and J_{H} gene segments. Such non-human animals include any of those which can be genetically modified to express antibodies having CDR3s that include amino acids encoded by nucleotide coding sequences corresponding to a portion of a polypeptide of interest as disclosed herein, including, e.g., mammals, e.g., mouse, rat, rabbit, pig, bovine (e.g., cow, bull, buffalo), deer, sheep, goat, chicken, cat, dog, ferret, primate (e.g., marmoset, rhesus monkey), etc. For example, for those non-human animals for which suitable genetically modifiable ES cells are not readily available, other methods are employed to make a non-human animal comprising the genetic modification. Such methods include, e.g., modifying a non-ES cell genome (e.g., a fibroblast or an induced pluripotent cell) and employing somatic cell nuclear transfer (SCNT) to transfer the genetically modified genome to a suitable cell, e.g., an enucleated oocyte, and gestating the modified cell (e.g., the modified oocyte) in a non-human animal under suitable conditions to form an embryo.

Methods for modifying a non-human animal genome (e.g., a pig, cow, rodent, chicken, etc.) include, e.g., employing a zinc finger nuclease (ZFN), a transcription activator-like effector nuclease (TALEN) or a Cas protein (i.e., a CRISPR/Cas system) to modify a genome to include an engineered D_{H} region as described herein. Guidance for methods for modifying the germline genome of a non-human animal can be found in, e.g., U.S. Patent Application Publication Nos. 2015-0376628 A1, US 2016-0145646 A1 and US 2016-0177339 A1; incorporated herein by reference.

In some embodiments, a non-human animal described herein is a mammal. In some embodiments, a non-human animal described herein is a small mammal, e.g., of the superfamily Dipodoidea or Muroidea. In some embodiments, a genetically modified animal described herein is a rodent. In some embodiments, a rodent described herein is selected from a mouse, a rat, and a hamster. In some embodiments, a rodent described herein is selected from the superfamily Muroidea. In some embodiments, a genetically modified animal described herein is from a family selected from Calomyscidae (e.g., mouse-like hamsters), Cricetidae (e.g., hamster, New World rats and mice, voles), Muridae (true mice and rats, gerbils, spiny mice, crested rats), Nesomyidae (climbing mice, rock mice, with-tailed rats, Malagasy rats and mice), Platacanthomyidae (e.g., spiny dormice), and Spalacidae (e.g., mole rates, bamboo rats, and zokors). In some certain embodiments, a genetically modified rodent described herein is selected from a true mouse or rat (family Muridae), a gerbil, a spiny mouse, and a crested rat. In some certain embodiments, a genetically modified mouse described herein is from a member of the family Muridae. In some embodiment, a non-human animal described herein is a rodent. In some certain embodiments, a rodent described herein is selected from a mouse and a rat. In some embodiments, a non-human animal described herein is a mouse.

In some embodiments, a non-human animal described herein is a rodent that is a mouse of a C57BL strain selected from C57BL/A, C57BL/An, C57BL/GrFa, C57BL/KaLwN, C57BL/6, C57BL/6J, C57BL/6ByJ, C57BL/6NJ, C57BL/10, C57BL/10ScSn, C57BL/10Cr, and C57BL/O1a. In some certain embodiments, a mouse described herein is a 129 strain selected from the group consisting of a strain that is 129P1, 129P2, 129P3, 129X1, 129S1 (e.g., 129S1/SV, 129S1/SvIm), 129S2, 129S4, 129S5, 129S9/SvEvH, 129/SvJae, 129S6 (129/SvEvTac), 129S7, 129S8, 129T1, 129T2 (see, e.g., Festing et al., 1999, Mammalian Genome 10:836; Auerbach, W. et al., 2000, Biotechniques 29(5):1024-1028, 1030, 1032). In some certain embodiments, a genetically modified mouse described herein is a mix of an aforementioned 129 strain and an aforementioned C57BL/6 strain. In some certain embodiments, a mouse described herein is a mix of aforementioned 129 strains, or a mix of aforementioned BL/6 strains. In some certain embodiments, a 129 strain of the mix as described herein is a 129S6 (129/SvEvTac) strain. In some embodiments, a mouse described herein is a BALB strain, e.g., BALB/c strain. In some embodiments, a mouse described herein is a mix of a BALB strain and another aforementioned strain.

In some embodiments, a non-human animal described herein is a rat. In some certain embodiments, a rat described herein is selected from a Wistar rat, an LEA strain, a Sprague Dawley strain, a Fischer strain, F344, F6, and Dark Agouti. In some certain embodiments, a rat strain as described herein is a mix of two or more strains selected from the group consisting of Wistar, LEA, Sprague Dawley, Fischer, F344, F6, and Dark Agouti.

### Methods Employing Non-human Animals Having An Engineered Diversity Cluster

Several *in vitro* and *in vivo* technologies have been developed for the production of antibody-based therapeutics. In particular, *in vivo* technologies have featured the production of transgenic animals (i.e., rodents) containing human immunoglobulin genes either randomly incorporated into the genome of the animal (e.g., see U.S. Patent No. 5,569,825) or precisely placed at an endogenous immunoglobulin locus in operable linkage with endogenous immunoglobulin constant regions of the animal (e.g., see U.S. Patent Nos. 8,502,018; 8,642,835; 8,697,940; and 8,791,323). Both approaches have been productive in producing promising antibody therapeutic candidates for use in humans. Further, both approaches have the advantage over *in vitro* approaches in that antibody candidates are chosen from antibody repertoires generated *in vivo,* which includes selection for affinity and specificity for antigen within the internal milieu of the host's immune system. In this way, antibodies bind to naturally presented antigen (within relevant biological epitopes and surfaces) rather than artificial environments or *in silico* predictions that can accompany *in vitro* technologies. Despite the robust antibody repertoires produced from *in vivo* technologies, antibodies to complex (e.g., membrane-spanning polypeptides) or cytoplasmic antigens remains difficult. Further, generating antibodies to polypeptides that share a high degree of sequence identity between species (e.g., human and mouse) remains a challenge due to immune tolerance.

Thus, described herein is, among other things, the recognition that the construction of an *in vivo* system characterized by the production of antibodies having added diversity in CDRs, in particular, CDR3s, generated from non-traditional sequences (i.e., not traditional D_{H} gene segments or D_{H} gene segments that appear in nature) can be made using one or more nucleotide coding sequences (i.e., synthetic sequences) that each encode a portion of a polypeptide of interest. Such added diversity can direct binding to particular antigens (e.g., membrane-spanning polypeptides). For example, as described herein, antibodies that block one or more inflammatory cytokines (e.g., β-chemokines) can be generated by non-human animals engineered to contain an immunoglobulin heavy chain locus that contains an engineered D_{H} region, which engineered D_{H} region includes one or more nucleotide coding sequences that each encode an extracellular portion of a cytokine receptor (e.g., a β-chemokine receptor). Also described herein, antibodies that block or inhibition the activity and/or function of an ion channel (e.g., a Na_{V} channel) can be generated by non-human animals engineered to contain an immunoglobulin heavy chain locus that contains an engineered D_{H} region, which engineered D_{H} region includes one or more nucleotide coding sequences that each encode a portion of a toxin (e.g., a µ-conotoxin and/or a tarantula toxin). Upon recombination of a V_{H} gene segment, a nucleotide coding sequence, and a J_{H} gene segment, a heavy chain variable coding sequence is formed that contains a CDR3 region having a sequence encoded, in part, by the nucleotide coding sequence and thereby directs binding to a particular antigen associated with the nucleotide coding sequence.

Non-human animals described herein may be employed for making a human antibody, which human antibody comprises variable domains derived from one or more variable region nucleic acid sequences encoded by genetic material of a cell of a non-human animal as described herein. For example, a non-human animal described herein is immunized with a β-chemokine (or Na_{V} channel polypeptide, in whole or in part) under conditions and for a time sufficient that the non-human animal develops an immune response to said β-chemokine (or Na_{V} channel polypeptide, in whole or in part). Antibodies are isolated from the non-human animal (or one or more cells, for example, one or more B cells) and characterized using various assays measuring, for example, affinity, specificity, epitope mapping, ability for blocking ligand-receptor interaction, inhibition receptor activation, etc. In various embodiments, antibodies produced by non-human animals described herein comprise one or more human variable domains that are derived from one or more human variable region nucleotide sequences isolated from the non-human animal. In some embodiments, anti-drug antibodies (e.g., anti-idiotype antibody) may be raised in non-human animals described herein.

Non-human animals described herein provide an improved *in vivo* system and source of biological materials (e.g., cells) for producing human antibodies that are useful for a variety of assays. In various embodiments, non-human animals described herein are used to develop therapeutics that target one or more cytokines and/or modulate cytokine activity and/or modulate cytokine interactions with other binding partners (e.g., a cytokine receptor). In various embodiments, non-human animals described herein are used to develop therapeutics that target one or more ion channels and/or modulate ion channel activity and/or modulate ion channel interactions with other binding partners. In various embodiments, non-human animals described herein are used to identify, screen and/or develop candidate therapeutics (e.g., antibodies, siRNA, etc.) that bind one or more human cytokine polypeptides or ion channels (e.g., a potassium channel, calcium channel or sodium channel). In various embodiments, non-human animals described herein are used to screen and develop candidate therapeutics (e.g., antibodies, siRNA, etc.) that block activity of one or more human β-chemokine polypeptides or that block the activity of one or more human voltage-gated sodium (Na_{V}) channels. In various embodiments, non-human animals described herein are used to determine the binding profile of antagonists and/or agonists of one or more human β-chemokine polypeptides or of one or more human Na_{V} channels. In some embodiments, non-human animals described herein are used to determine the epitope or epitopes of one or more candidate therapeutic antibodies that bind one or more human β-chemokine polypeptides or that bind one or more human Na_{V} channels.

In various embodiments, non-human animals described herein are used to determine the pharmacokinetic profiles of anti-β-chemokine or anti-Na_{V} antibodies. In various embodiments, one or more non-human animals described herein and one or more control or reference non-human animals are each exposed to one or more candidate therapeutic anti-β-chemokine or anti-Na_{V} antibodies at various doses (e.g., 0.1 mg/kg, 0.2 mg/kg, 0.3 mg/kg, 0.4 mg/kg, 0.5 mg/kg, 1 mg/kg, 2 mg/kg, 3 mg/kg, 4 mg/kg, 5 mg/mg, 7.5 mg/kg, 10 mg/kg, 15 mg/kg, 20 mg/kg, 25 mg/kg, 30 mg/kg, 40 mg/kg, or 50 mg/kg or more). Candidate therapeutic antibodies may be dosed via any desired route of administration including parenteral and non-parenteral routes of administration. Parenteral routes include, e.g., intravenous, intraarterial, intraportal, intramuscular, subcutaneous, intraperitoneal, intraspinal, intrathecal, intracerebroventricular, intracranial, intrapleural or other routes of injection. Non-parenteral routes include, e.g., oral, nasal, transdermal, pulmonary, rectal, buccal, vaginal, ocular. Administration may also be by continuous infusion, local administration, sustained release from implants (gels, membranes or the like), and/or intravenous injection. Blood is isolated from non-human animals (humanized and control) at various time points (e.g., 0 hr, 6 hr, 1 day, 2 days, 3 days, 4 days, 5 days, 6 days, 7 days, 8 days, 9 days, 10 days, 11 days, or up to 30 or more days). Various assays may be performed to determine the pharmacokinetic profiles of administered candidate therapeutic antibodies using samples obtained from non-human animals as described herein including, but not limited to, total IgG, anti-therapeutic antibody response, agglutination, etc.

In various embodiments, non-human animals described herein are used to measure the therapeutic effect of blocking or modulating β-chemokine activity (or β-chemokine signaling, or β-chemokine mediated interactions) and the effect on gene expression as a result of cellular changes or the β-chemokine receptor density of cells of non-human animals as described herein. In various embodiments, a non-human animal described herein or cells isolated therefrom are exposed to a candidate therapeutic that binds a human β-chemokine polypeptide (or a portion of a human β-chemokine polypeptide) and, after a subsequent period of time, analyzed for effects on β-chemokine-dependent processes (or interactions), for example, ligand-receptor interactions or β-chemokine signaling.

In various embodiments, non-human animals described herein are used to measure the therapeutic effect of blocking or modulating Na_{V} channel activity (or Na_{V} channel signaling, or Na_{V}-mediated interactions, or Na_{V} channel action potentials) and the effect on gene expression as a result of cellular changes or the Na_{V} channel density of cells of non-human animals as described herein. In various embodiments, a non-human animal described herein or cells isolated therefrom are exposed to a candidate therapeutic that binds a human Na_{V} channel (or a portion of a human Na_{V} channel) and, after a subsequent period of time, analyzed for effects on Na_{V} channel-dependent processes (or interactions), for example, ligand-receptor interactions or Na_{V} channel action potentials.

In various embodiments, non-human animals described herein may be employed for making a human antibody that acts on a voltage-gated sodium channel (e.g., Na_{V}1.7). Such a human antibody may be tested and/or developed in a non-human animal having a modification different than described herein such as, for example, a modification as described in U.S. Patent Nos. 8,871,996 and 8,486,647, herein incorporated by reference.

Non-human animals described herein express antibodies that bind one or more β-chemokines (or Na_{V} channels), thus cells, cell lines, and cell cultures can be generated to serve as a source of anti-β-chemokine (or anti-Na_{V}) antibodies for use in binding and functional assays, e.g., to assay for binding or function of a human β-chemokine (or Na_{V} channel) antagonist or agonist, particularly where the antagonist or agonist is specific for a human β-chemokine sequence (or human Na_{V} channel sequence) or epitope or, alternatively, specific for a human β-chemokine sequence (or human Na_{V} channel sequence) or epitope that functions in ligand-receptor interaction (binding). In various embodiments, β-chemokine epitopes (or Na_{V} channel epitopes) bound by candidate therapeutic antibodies or siRNAs can be determined using cells isolated from non-human animals described herein.

Cells from non-human animals described herein can be isolated and used on an ad hoc basis, or can be maintained in culture for many generations. In various embodiments, cells from a non-human animal described herein are immortalized (e.g., via use of a virus) and maintained in culture indefinitely (e.g., in serial cultures).

Non-human animals described herein provide an *in vivo* system for the generation of variants of an antibody that binds one or more β-chemokine polypeptides (or Na_{V} channels). Such variants include antibodies having a desired functionality, specificity, low cross-reactivity to a common epitope shared by two or more β-chemokine polypeptides (or Na_{V} channels). In some embodiments, non-human animals described herein are employed to generate panels of antibodies to generate a series of variant antibodies that are screened for a desired or improved functionality.

Non-human animals described herein provide an *in vivo* system for generating anti-β-chemokine (or anti-Na_{V}) antibody libraries. Such libraries provide a source for heavy and light chain variable region sequences that may be grafted onto different Fc regions based on a desired effector function and/or used as a source for affinity maturation of the variable region sequence using techniques known in the art (e.g., site-directed mutagenesis, error-prone PCR, etc.).

Non-human animals described herein provide an *in vivo* system for the analysis and testing of a drug or vaccine. In various embodiments, a candidate drug or vaccine may be delivered to one or more non-human animals described herein, followed by monitoring of the non-human animals to determine one or more of the immune response to the drug or vaccine, the safety profile of the drug or vaccine, or the effect on a disease or condition and/or one or more symptoms of a disease or condition. Exemplary methods used to determine the safety profile include measurements of toxicity, optimal dose concentration, antibody (i.e., anti-drug) response, efficacy of the drug or vaccine, and possible risk factors. Such drugs or vaccines may be improved and/or developed in such non-human animals.

Vaccine efficacy may be determined in a number of ways. Briefly, non-human animals described herein are vaccinated using methods known in the art and then challenged with a vaccine or a vaccine is administered to already-infected non-human animals. The response of a non-human animal(s) to a vaccine may be measured by monitoring of, and/or performing one or more assays on, the non-human animal(s) (or cells isolated therefrom) to determine the efficacy of the vaccine. The response of a non-human animal(s) to the vaccine is then compared with control animals, using one or more measures known in the art and/or described herein.

Vaccine efficacy may further be determined by viral neutralization assays. Briefly, non-human animals as described herein are immunized and serum is collected on various days post-immunization. Serial dilutions of serum are pre-incubated with a virus during which time antibodies in the serum that are specific for the virus will bind to it. The virus/serum mixture is then added to permissive cells to determine infectivity by a plaque assay or microneutralization assay. If antibodies in the serum neutralize the virus, there are fewer plaques or lower relative luciferase units compared to a control group.

Non-human animals described herein provide an improved *in vivo* system for development and characterization of antibody-based therapeutics for use in cancer and/or inflammatory diseases. Inflammation has long been associated with cancer (reviewed in, e.g., Grivennikov, S.I. et al., 2010, Cell 140:883-99; Rakoff-Nahoum, S., 2006, Yale J. Biol. Med. 79:123-30). Indeed, a developing tumor environment is characterized, in part, by infiltration of various inflammatory mediators. Also, persistent inflammation can lead to a higher probability of developing cancer. Thus, in some embodiments, a non-human animal described herein provides for an *in vivo* system for the development and/or identification of anti-cancer and/or anti-inflammatory therapeutics. In various embodiments, non-human animals described herein or control non-human animals (e.g., having a genetic modification different than described herein or no genetic modification, i.e., wild-type) may be implanted with a tumor (or tumor cells), followed by administration of one or more candidate therapeutics. In some embodiments, candidate therapeutics may include a multi-specific antibody (e.g., a bi-specific antibody) or an antibody cocktail. In some embodiments, candidate therapeutics include combination therapy such as, for example, administration of two or more mono-specific antibodies dosed sequentially or simultaneously. The tumor may be allowed sufficient time to be established in one or more locations within the non-human animal prior to administration of one or more candidate therapeutics. Tumor cell proliferation, growth, survival, etc. may be measured both before and after administration with the candidate therapeutic(s). Cytotoxicity of candidate therapeutics may also be measured in the non-human animal as desired.

Non-human animals described herein provide an improved *in vivo* system for development and characterization of antibody-based therapeutics for use in the treatment of pain (e.g., neuropathic) or for use as an analgesic. In some embodiments, a non-human animal described herein provides for an *in vivo* system for the development and/or identification of anti-pain therapeutics. In various embodiments, non-human animals described herein or control non-human animals (e.g., having a genetic modification different than described herein [such as, for example, a modification as described in U.S. Patent Nos. 8,871,996 and 8,486,647; herein incorporated by reference], or no genetic modification, i.e., wild-type) may be subjected to a pain stimulus (e.g., incisional, chemically induced, etc.; see, e.g., Recognition and Alleviation of Pain in Laboratory Animals. Washington D.C.: National Academies Press, 2009. Print), followed by administration of one or more candidate therapeutics. In some embodiments, candidate therapeutics may include a multi-specific antibody (e.g., a bi-specific antibody) or an antibody cocktail. In some embodiments, candidate therapeutics include combination therapy such as, for example, administration of two or more mono-specific antibodies dosed sequentially or simultaneously. A sufficient period of time may be allowed to prior to administration of one or more candidate therapeutics. Various measurements and/or tests commonly associated with assessment of pain management and/or pain therapeutic efficacy may be recorded both before and after administration with the candidate therapeutic(s).

### Kits

Also described herein is a pack or kit comprising one or more containers filled with at least one non-human animal, non-human cell, DNA fragment, and/or targeting vector as described herein. Kits may be used in any applicable method (e.g., a research method). Optionally associated with such container(s) can be a notice in the form prescribed by a governmental agency regulating the manufacture, use or sale of pharmaceuticals or biological products, which notice reflects (a) approval by the agency of manufacture, use or sale for human administration, (b) directions for use, or both, or a contract that governs the transfer of materials and/or biological products (e.g., a non-human animal or non-human cell as described herein) between two or more entities.

Other features of the described embodiments will become apparent in the course of the following descriptions of exemplary embodiments, which are given for illustration and are not intended to be limiting thereof.

### EXAMPLES

The following examples are provided so as to describe to those of ordinary skill in the art how to make and use methods and compositions disclosed herein and are not intended to limit the scope of what the inventors regard as their invention. Unless indicated otherwise, temperature is indicated in Celsius, and pressure is at or near atmospheric.

### Example 1. Construction of an engineered diversity cluster that includes nucleotide coding sequences of a D6 chemokine decoy receptor within an immunogloblin heavy chain variable region

This example illustrates exemplary methods of constructing a targeting vector for insertion into the genome of a non-human animal such as a rodent (e.g., a mouse). In particular, the methods described in this example demonstrate the production of a targeting vector for insertion into the genome of rodent (e.g., a mouse) embryonic stem (ES) cells to produce a rodent whose genome comprises an immunoglobulin heavy chain variable region that includes a engineered heavy chain diversity (D_{H}) region, which engineered D_{H} region includes one or more nucleotide sequences that each encode a a non-immunoglobulin polypeptide or portion thereof. In this example, coding sequences of the extracellular domain of an atypical chemokine receptor (ACKR), for example, D6 chemokine decoy receptor, were employed in the construction of a targeting vector for integration into an immunoglobulin heavy chain variable region. As described below, the coding sequences were placed in operable linkage with heavy chain variable (V_{H}) and heavy chain joining (J_{H}) segments in the place of traditional D_{H} gene segments so that, upon VDJ recombination, antibodies having V_{H} CDR3s generated from the coding sequences are expressed.

Targeting vectors containing coding sequences from extracellular domains of D6 chemokine decoy receptor for insertion into an immunoglobulin heavy chain variable region were created using VELOCIGENE^{®} technology (see, e.g., U.S. Patent No. 6,586,251 and Valenzuela et al., 2003, Nature Biotech. 21(6):652-659; herein incorporated by reference) and molecular biology techniques known in the art. The methods described in this example can be employed to utilize any set of coding sequences derived from any desired polypeptide, or combination of coding sequences (or coding sequence fragments) as desired. An alternative and non-limiting exemplary strategy for constructing a targeting vector using coding sequences from extracellular domains of D6 chemokine decoy receptor is set forth in Figures 3 and 4.

Briefly, a series of four DNA fragments (D₆-DH1116, D₆-DH17613, D₆-DH114619, and D₆DH120126) each containing several D6 coding sequences were made by *de novo* DNA synthesis (Table 5; Blue Heron Biotech, Bothell, WA). Various restriction enzyme sites and/or overlap regions (∼40 bp, which included multiple restriction enzyme sites) were included at the ends of each DNA fragment to allow for subsequent cloning in tandem (see below). A single human D_{H} segment (i.e., D_{H}6-25) remained intact in a D_{H} region engineered to contain nucleotide coding sequences corresponding to portions of an extracellular domain of a D6 decoy chemokine receptor. Immunoglobulin gene segments (e.g., D_{H} segments) can, in some embodiments, be associated with suboptimal or defective recombination signal sequences (e.g., a heptamer and/or nonamer sequence) such that usage of such D_{H} segments is substaintially less than usage of D_{H} segments associated with recombination signal sequences characterized as wild-type, normal and/or not defective. Thus, such D_{H} segments (e.g. D_{H}6-25, RSS sequences shown in Fig 2) may be left intact or deleted when engineering a D_{H} region to contain nucleotide coding sequences that each encode a non-immunoglobulin polypeptide of interest, or portion thereof (e.g., a D6 chemokine decoy receptor).

Immunoglobulins participate in a cellular mechanism, termed somatic hypermutation, which produces affinity-matured antibody variants characterized by high affinity to their target. Although somatic hypermutation largely occurs within the CDRs of antibody variable regions, mutations are preferentially targeted to certain sequence motifs that are referred to as hot spots, e.g., RGYW activation-induced cytidine deaminase (AID) hotspots (see, e.g., Li, Z. et al., 2004, Genes Dev. 18:1-11; Teng, G. and F.N. Papavasiliou, 2007, Annu. Rev. Genet. 41:107-20; hereby incorporated by reference). The nucleic acid sequence of each D6 coding sequence naturally contained such hot spot sequences, however, artificial hot spots were introduced into selected D6 coding sequences to optimize the potential for somatic hypermutation during clonal selection of B cell receptors. Artificial hot spots for SHM were introduced into D6 coding sequences *in silico* prior to *de novo* synthesis. Figure 1 set forths exemplary analysis of natural and artificial hotspots employed in D6 coding sequences described herein.

V(D)J recombination is guided by flanking DNA sequences, termed recombination signal sequences (RSSs), that ensure DNA rearrangements at precise locations relative to V, D and J coding sequences. Each RSS consists of a conserved block of seven nucleotides (heptamer) that is contiguous with the coding sequence followed by a nonconserved spacer (either 12 or 23bp) and a second conserved block of nine nucleotides (nonamer). Each of the D6 coding sequences were designed with optimized RSSs to allow for better recombination frequency and equal usage. Briefly, all heptamers and nonamers in D_{H} RSS sequences were replaced with consensus sequences (Figure 2). RSS sequences of non-functional *"ORF only"* D_{H} segments were repaired to allow usage with D6 coding sequences. As described herein, *"ORF only"* are D_{H} segments that include a coding sequence that can be translated in at least one open reading frame, but have at least one non-functional RSS sequence (not recognized by *RAG* recombinase). The inventors recognized that optimizing heptamers and nonamers did not guarantee that all D6 coding sequences would be used at equal frequencies due, at least in part, to unpredictable affects from spacers, coding end sequences, flanking intergenic sequences, etc. As described above for somatic hypermutation hot spots, optimized RSSs were designed *in silico* prior to *de novo* synthesis of D6 DNA fragments.

The D6 DNA fragments were prepared for assembly together in tandem employing the restriction enzyme sites and overlap regions designed into each fragment. The D6-DH1166 fragment and a plasmid carrying a neomycin cassette were prepared for ligation by restriction digest using AgeI and EcoRI (Figure 3A, top). The AgeI/EcoRI digested plasmid and D6-DH1166 fragment were ligated together using a DNA ligase according to manufacturer's specifications. The ligation product and the remaining D6 DNA fragments were digested with SnaBI. A BAC vector (pBACE3.6) was separately digested with NotI and AscI to accept the D6 DNA fragments (Figure 3A, middle). The D6-DH1166/neomycin cassette ligation product and remaining D6 DNA fragments were assembled using a one-step isothermal assembly as previously described (Figure 3A, bottom; Barnes, W.M., 1994, Proc. Natl. Acad. Sci. 91(6):2216-20; Gibson, D.G. et al., 2009, Nat. Methods 6(5):343-5; Gibson, D.G. et al., 2010, Nat. Methods 7(11):901-3). The assembled D6 DNA fragment (Figure 3B, top) was then digested with PI-SceI and I-CeuI and ligated together with a BAC clone containing 5' and 3' homology arms containing ∼50 kb of human variable region genomic DNA and a human J_{H} cluster, respectively, via compatible ends (Figure 3B, middle). The final targeting vector contained, from 5' to 3', a ∼50 kb 5' homology arm containing human variable region DNA, a neomycin cassette, a ∼41 kb DNA fragment containing 25 extracellular coding sequences of a D6 chemokine decoy receptor, a human J_{H} cluster, a mouse heavy chain intronic enhancer (Ei), a mouse IgM constant region gene and a lox site (Figure 3B, bottom).

Alternatively, the D6 DNA fragments described above may be assembled by using molecular techniques that differ from those described above, yet known in the art. For example, because the D6 DNA fragments are arranged in tandem, various different designs (e.g., order of coding sequences) can be achieved through the use of different restriction sites/overlap regions designed into the ends of the fragments as desired. An exemplary alternative method of assembly of the D6 DNA fragments described above by sequential ligation is set forth in Figures 4A and 4B.

A D6-D_{H} targeting vector described above was introduced into mouse embryonic stem (ES) cells for producing modified ES cells containing an immunoglobulin heavy chain locus containing the engineered diversity cluster, e.g., engineered D_{H} region.

**TABLE 5**

| DNA fragment (size) | D6 coding sequence name (SEQ ID NO) | D_{H} position |
|---|---|---|
| D6-DH1166 (∼10,050 bp) SEQ ID NO:131 | D6 Nterm-C (39) | D_{H}1-1 |
| | D6 EC3 (7) | D_{H}2-2 |
| | D6 Nterm (1) | D_{H}3-3 |
| | D6 Nterm+EC3 (33) | D_{H}4-4 |
| | D6 EC1 (3) | D_{H}5-5 |
| | D6 EC2 (5) | D_{H}6-6 |
| D6-DH17613 (∼9,768 bp) SEQ ID NO:132 | D6 EC1-N (41) | D_{H}1-7 |
| | D6 EC3-S (15) | D_{H}2-8 |
| | D6 Nterm-N (37) | D_{H}3-9 |
| | D6 EC2-S (13) | D_{H}3-10 |
| | D6 EC1-EC2-S (29) | D_{H}4-11 |
| | D6 EC1-S (11) | D_{H}5-12 |
| | D6 Nterm-S (9) | D_{H}6-13 |
| D6-DH114619 (∼9,788 bp) SEQ ID NO:133 | D6 EC2-N (43) | D_{H}1-14 |
| | D6 EC2-EC1 (23) | D_{H}2-15 |
| | D6 Nterm-EC3 (17) | D_{H}3-16 |
| | D6 EC1+EC2 (35) | D_{H}4-17 |
| | D6 EC3-Nterm (19) | D_{H}5-18 |
| | D6 EC1-EC2 (21) | D_{H}6-19 |
| | D6 EC2-C (45) | D_{H}1-20 |
| D6-DH120126 (∼11,906 bp) SEQ ID NO:134 | D6 EC2-EC1-S (31) | D_{H}2-21 |
| | D6 Nterm-EC3-S (25) | D_{H}3-22 |
| | D6 EC3-C (49) | D_{H}4-23 |
| | D6 EC3-Nterm-S (27) | D_{H}5-24 |
| | D6 EC3-N (47) | D_{H}1-26 |

### Example 2. Generation of rodents having an engineered diversity cluster that includes nucleotide coding sequences of a D6 chemokine decoy receptor within an immunoglobulin heavy chain variable region

This example demonstrates the production of non-human animals (e.g., rodents) whose genome comprises an immunoglobulin heavy chain variable region that includes an engineered heavy chain diversity (D_{H}) region, wherein the engineered D_{H} region includes one or more nucleotide sequences that each encode a portion of a non-immunoglobulin polypeptide, in particular, an extracellular portion of an atypical chemokine receptor (ACKR) such as, for example, D6 chemokine decoy receptor.

Correct assembly of the D6-D_{H} targeting vector described in Example 1 and targeted insertion of the D6 DNA fragments into the diversity cluster of BAC DNA was confirmed by sequencing and polymerase chain reaction throughout the construction of the targeting vector using primers set forth in Table 6. Targeted BAC DNA, confirmed by polymerase chain reaction, was then introduced into F1 hybrid (129S6SvEvTac/C57BL6NTac) mouse embryonic stem (ES) cells via electroporation followed by culturing in selection medium. The ES cells used for electroporation had a genome that included human V_{H} and J_{H} gene segments operably linked with rodent immunoglobulin heavy chain constant regions (e.g., IgM), lacked all or part of a human D_{H} region (i.e., deleted), and contained an inserted sequence encoding one or more murine Adam6 genes (see, e.g., U.S. Patent Nos. 8,642,835 and 8,697,940). Drug-resistant colonies were picked 10 days after electroporation and screened by TAQMAN^{™} and karyotyping for correct targeting as previously described (Valenzuela et al., *supra*; Frendewey, D. et al., 2010, Methods Enzymol. 476:295-307) using primer/probe sets that detected proper integration of the D6 coding sequences into the diversity cluster of an immunoglobulin variable region (Table 7 [F: forward primer, P: probe, R: reverse primer] and Figure 5).

The VELOCIMOUSE^{®} method (DeChiara, T.M. et al., 2010, Methods Enzymol. 476:285-294; Dechiara, T.M., 2009, Methods Mol. Biol. 530:311-324; Poueymirou et al., 2007, Nat. Biotechnol. 25:91-99) was used, in which targeted ES cells were injected into uncompacted 8-cell stage Swiss Webster embryos, to produce healthy fully ES cell-derived F0 generation mice heterozygous for the engineered D_{H} region and that express antibodies containing heavy chain variable regions that include CDR3 regions generated from recombination of D6 coding sequences. F0 generation heterozygous male were crossed with C57B16/NTac females to generate F1 heterozygotes that were intercrossed to produce F2 generation homozygotes and wild-type mice for phenotypic analyses.

The drug selection cassette may optionally be removed by the subsequent addition of a recombinase (e.g., by Cre treatment) or by breeding to a Cre deleter mouse strain (see, e.g., International Patent Application Publication No. WO 2009/114400) in order to remove any loxed selected cassette introduced by the targeting construct that is not removed, e.g., at the ES cell stage or in the embryo. Optionally, the selection cassette is retained in the mice.

Taken together, this example illustrates the generation of a rodent (e.g., a mouse) whose genome comprises an immunoglobulin heavy chain variable region that includes an engineered heavy chain diversity (D_{H}) region, which engineered D_{H} region is characterized by the inclusion of one or more nucleotide sequences that each encode an extracellular portion of an atypical chemokine receptor (ACKR) such as, for example, D6 chemokine decoy receptor. The strategy described herein for inserting D6 coding sequences into the place of traditional D_{H} segments enables the construction of a rodent that expresses a plurality of antibodies, each of which comprises a heavy chain CDR3 generated from recombination of a D6 coding sequence. Leveraged with the presence of human V_{H} and J_{H} segments, rodents described herein provide an *in vivo* system for the production of human antibody-based therapeutics that characterized by diversity that binds one or more CCL and, in some embodiments, provides antibody-based anti-inflammatory drugs for human use.

**TABLE 6**

| Primer Name | Sequence (5'-3') | Size (bp) |
|---|---|---|
| *DH1166*/*neomycin cassette ligation* | | |
| M13 reverse | CACAGGAAACAGCTATGACC (SEQ ID NO:113) | 412 |
| 3' ub pro- 200 | CCAGTGCCCTAGAGTCACCCA (SEQ ID NO:114) | |
| 5' neo detect | CTCCCACTCATGATCTATAGA (SEQ ID NO:115) | 409 |
| 3' up detect hD_{H}1-1-hD_{H}6-6 | CTGGGGCTCGCTTTAGTTG (SEQ ID NO:116) | |

| *pBACe3.6* + *DNA fragment isothermal assembly* | | |
|---|---|---|
| 5' up detect SacB | TGATAGCCGTTGTATTCAGC (SEQ ID NO:117) | 693 |
| 3' ub pro-200 | CCAGTGCCCTAGAGTCACCCA (SEQ ID NO:118) | |
| 5' down detect hD_{H}1-20- hD_{H}1- 26 | GCCATTTCTGTCTGCATTCG (SEQ ID NO:119) | 444 |
| 5' cm down detect | GGTTCATCATGCCGTTTGTGA (SEQ ID NO:120) | |

| *Primers for sequencing DH120126 DNA fragment* | | |
|---|---|---|
| HD-1 seq For | TGGGCTCGTAGTTTGACGTG (SEQ ID NO:121) | - |
| HD-1 seq Rev | TTACCCACACTTCACGCACG (SEQ ID NO:122) | - |
| HD-2 seq For | TTAAACGACGCCTCGAATG (SEQ ID NO:123) | - |
| HD-2 seq Rev | GCAACCATTCGTTGTAGTAG (SEQ ID NO:124) | - |
| HD-3 seq For | CTAACGCAGTCATGTAATGC (SEQ ID NO:125) | - |
| HD-3 seq Rev | GACTGTCACCCAGCATTAC (SEQ ID NO:126) | - |

| *Targeting vector post PI-SceI*/*I-CeuI ligation* | | |
|---|---|---|
| hIgHD up detect | CGTCGCCTCTACGGGAAATC (SEQ ID NO:127) | 695 |
| 3' ub pro-200 | CCAGTGCCCTAGAGTCACCCA (SEQ ID NO:128) | |
| 5' down detect hD_{H}1-20- hD_{H}1-26 | GCCATTTCTGTCTGCATTCG (SEQ ID NO:129) | 553 |
| hIgHD down detect | AAACACCACGTAGGATTTACGC (SEQ ID NO:130) | |

**TABLE 7**

| Primer/Probe | | Sequence (5'-3') |
|---|---|---|
| Hyg | F | TGCGGCCGATCTTAGCC (SEQ ID NO:135) |
| | P | ACGAGCGGGTTCGGCCCATTC (SEQ ID NO:136) |
| | R | TTGACCGATTCCTTGCGG (SEQ ID NO:137) |
| hIgH DH-1 | F | CGGGTCACTGCCATTTCTG (SEQ ID NO:138) |
| | P | TCTGCATTCGCTCCCAGCGC (SEQ ID NO:139) |
| | R | TCTGCGGCATGAACCCAAT (SEQ ID NO:140) |
| hIgH DH-4 | F | TGGCCAGAACTGACCCTAC (SEQ ID NO:141) |
| | P | ACCGACAAGAGTCCCTCAGG (SEQ ID NO:142) |
| | R | GGAGTCGGCTCTGGATGTG (SEQ ID NO:143) |
| HD jxn-1 | F | GGAGCCAGGCAGGACACA (SEQ ID NO:144) |
| | P | TGGGCTCGTAGTTTGACGT (SEQ ID NO:145) |
| | R | GGGACTTTCTTACCCACACTTCA (SEQ ID NO:146) |
| HDjxn-2 | F | GGTCCCGAGCACTCTTAATTAAAC (SEQ ID NO:147) |
| | P | CCTCGAATGGAACTAC (SEQ ID NO:148) |
| | R | GGGAGAGCAACCATTCGTTGT (SEQ ID NO:149) |
| HDjxn-3 | F | CCGAGCACCGATGCATCTA (SEQ ID NO:150) |
| | P | CGCAGTCATGTAATGC (SEQ ID NO:151) |
| | R | GGGAGGCGAACTGACTGTCA (SEQ ID NO:152) |
| Neo | F | GGTGGAGAGGCTATTCGGC (SEQ ID NO:153) |
| | P | TGGGCACAACAGACAATCGGCTG (SEQ ID NO:154) |
| | R | GAACACGGCGGCATCAG (SEQ ID NO:155) |
| hIgH1 | F | CAGTCCCGTTGATCCAGCC (SEQ ID NO:156) |
| | P | CCCATCAGGGATTTTGTATCTCTGTGGACG (SEQ ID NO:157) |
| | R | GGATATGCAGCACTGTGCCAC (SEQ ID NO:158) |
| hIgH9 | F | TCCTCCAACGACAGGTCCC (SEQ ID NO:159) |
| | P | TCCCTGGAACTCTGCCCCGACACA (SEQ ID NO:160) |
| | R | GATGAACTGACGGGCACAGG (SEQ ID NO:161) |
| hIgH31 | F | ATCACACTCATCCCATCCCC (SEQ ID NO:162) |
| | P | CCCTTCCCTAAGTACCACAGAGTGGGCTC (SEQ ID NO:163) |
| | R | CACAGGGAAGCAGGAACTGC (SEQ ID NO:164) |
| mIgHp2 | F | GCCATGCAAGGCCAAGC (SEQ ID NO:165) |
| | P | CCAGGAAAATGCTGCCAGAGCCTG (SEQ ID NO:166) |
| | R | AGTTCTTGAGCCTTAGGGTGCTAG (SEQ ID NO:167) |
| mIgHA8 | F | CCCCACAGCAAATCACAACC (SEQ ID NO:168) |
| | P | ATGCAGTTGTCACCCTTGAGGCCATTC (SEQ ID NO:169) |
| | R | TGTTTCCCAGGCGTCACTG (SEQ ID NO:170) |
| mIgHA1 | F | CTCAGTGATTCTGGCCCTGC (SEQ ID NO:171) |
| | P | TGCTCCACAGCTACAAACCCCTTCCTATAATG (SEQ ID NO:172) |
| | R | GGATGATGGCTCAGCACAGAG (SEQ ID NO:173) |
| mIgHA7 | F | TGGTCACCTCCAGGAGCCTC (SEQ ID NO:174) |
| | P | AGTCTCTGCTTCCCCCTTGTGGCTATGAGC (SEQ ID NO:175) |
| | R | GCTGCAGGGTGTATCAGGTGC (SEQ ID NO:176) |
| D_{H}6-25 | F | GTGTCACAGTCGGGCATA (SEQ ID NO:177) |
| | P | CCACGGCTACCACAATGACACTGG (SEQ ID NO:178) |
| | R | CCTTCGGCTGACTTGGGATG (SEQ ID NO:179) |

### Example 3. Construction of an engineered diversity cluster that includes nucleotide coding sequences of one or more toxin peptides within an immunogloblin heavy chain variable region

This example illustrates exemplary methods of constructing a targeting vector for insertion into the genome of a non-human animal such as a rodent (e.g., a mouse). In particular, the methods described in this example demonstrate the production of a targeting vector for insertion into the genome of rodent (e.g., a mouse) embryonic stem (ES) cells to produce a rodent whose genome comprises an immunoglobulin heavy chain variable region that includes a engineered heavy chain diversity (D_{H}) region, which engineered D_{H} region includes one or more nucleotide sequences that each encode a portion of a non-immunoglobulin polypeptide. In this example, coding sequences of two toxins (e.g., a conotoxin such as, for example, µ-conotoxin, and a tarantula toxin such as, for example, ProTxII) were employed in the construction of a targeting vector for integration into an immunoglobulin heavy chain variable region. As described below, the coding sequences were placed in operable linkage with heavy chain variable (V_{H}) and heavy chain joining (J_{H}) segments in the place of traditional D_{H} gene segments so that, upon VDJ recombination, antibodies having CDR3s generated from the coding sequences are expressed.

Targeting vectors containing coding sequences from µ-conotoxin and a tarantula toxin (e.g., ProTxII) for insertion into an immunoglobulin heavy chain variable region were created as described above in Example 1. The methods described herein can be employed to utilize any set of coding sequences derived from any desired conotoxin (e.g., α-conotoxin, δ-conotoxin, κ-conotoxin, µ-conotoxin and/or ω-conotoxin), tarantula toxin (e.g., ProTxI, ProTxII, Huwentoxin-IV [HWTX-IV], etc.) or combination of coding sequences (or coding sequence fragments) as desired. An exemplary strategy for constructing a targeting vector using coding sequences from µ-conotoxin and a tarantula toxin is set forth in Figures 7A and 7B.

Briefly, a series of four DNA fragments, each containing several µ-conotoxin (µCTX) and tarantula toxin (ProTxII) coding sequences were made by *de novo* DNA synthesis (Table 8; Blue Heron Biotech, Bothell, WA). As described in Example 1, various restriction enzyme sites and/or overlap regions were included at the ends of each DNA fragment to allow for subsequent cloning in tandem. A total of 26 toxin coding sequences were employed in constructing an engineered D_{H} region, which included replacement of entire D_{H} coding sequences and insertion into D_{H} coding sequences (i.e., only partially replace a D_{H}). Insertions of toxin coding sequences were made at D_{H} positions 1-26, 2-2, 2-8, 2-15, 2-21, 3-3, 3-9, 3-10, 3-16, and 3-22 with the corresponding toxin coding sequence, while replacement of D_{H} sequence with toxin coding sequences were made at D_{H} positions 1-1, 1-7, 1-14, 1-20, 4-4, 4-11, 4-17, 4-23, 5-5, 5-12, 5-18, 5-24, 6-6, 6-13, 6-19 and 6-25 (see Table 8). In the case of insertions, toxin sequences were inserted in open reading frame two (ORF2) and flanked by D_{H} consensus sequences (e.g., see Figure 2). Retaining native flanking D_{H} sequences provides natural flexible linker sequence and/or use of the disulfide bond in D_{H}2 segments to cyclize the encoded toxin peptide. In contrast to the engineered D_{H} region constructed using D6 coding sequences described in Example 1, D_{H}6-25 was replaced with full length µCTX KIIIA (µCPTX-KIIIA fl; SEQ ID NO:228, see Table 8) in constructing a D_{H} region that included toxin coding sequences.

As described in Example 1, the nucleic acid sequence of each toxin coding sequence naturally contained such hot spot sequences, however, artificial hot spots were introduced into selected toxin coding sequences *in silico* prior to *de novo* synthesis to optimize the potential for somatic hypermutation during clonal selection of B cell receptors (see Figures 6A-6L). Further, each of the toxin coding sequences was designed with optimized RSSs *in silico* prior to *de novo* synthesis to allow for better recombination frequency and equal usage (Figure 2). Assembly of the toxin DNA fragments was prepared as described in Example 1 (see Figures 7A and 7B).

Alternatively, the toxin DNA fragments described above may be assembled by using molecular techniques that differ from those described above, yet known in the art. For example, because the toxin DNA fragments are arranged in tandem, various different designs (e.g., order of coding sequences) can be achieved through the use of different restriction sites/overlap regions designed into the ends of the fragments as desired. An exemplary alternative method of assembly of the toxin DNA fragments described above by sequential ligation is set forth in Figures 8A and 8B.

The TX-D_{H} targeting vector described above was introduced into mouse embryonic stem (ES) cells for producing modified ES cells containing an immunoglobulin heavy chain locus containing the engineered diversity cluster.

**TABLE 8**

| DNA fragment (size) | Toxin coding sequence name (SEQ ID NO) | D_{H} position |
|---|---|---|
| TX-DH1166 (∼9,812 bp) SEQ ID NO:232 | µCTX-SmIIIA C1SC4S | D_{H}1-1 |
| | (180) | D_{H}2-2 |
| | µCTX-CSSRWC (182) | D_{H}3-3 |
| | µOPTX-KIIIA mini (184) | D_{H}4-4 |
| | µCTX-PIIIA C1SC4S (186) | D_{H}5-5 |
| | ProTxII C1SC4S (188) | D_{H}6-6 |
| | µCTX-KIIIA C1SC4S (190) | |
| TX-DH17613 (∼9,512 bp) SEQ ID NO:233 | µCTX-SmIIIA mini (192) | D_{H}1-7 |
| | µCTX-RSRQ insertion (194) | D_{H}2-8 |
| | µCTX-KIIIA midi (196) | D_{H}3-9 |
| | µCTX-SmIIIA mini (198) | D_{H}3-10 |
| | µCTX-PIIIA mini (200) | D_{H}4-11 |
| | ProTxII C2SC5S (202) | D_{H}5-12 |
| | µCTX-KIIIA mini (204) | D_{H}6-13 |
| TX-DH114619 (∼9,691 bp) SEQ ID NO:234 | µCTX-SmIIIA fl (206) | D_{H}1-14 |
| | µCTX-SSRW insertion (208) | D_{H}2-15 |
| | µCTX-SmIIIA midi (210) | D_{H}3-16 |
| | µCTX-PIIIA midi (212) | D_{H}4-17 |
| | ProTxII C3SC6S (214) | D_{H}5-18 |
| | µCTX-KIIIA midi (216) | D_{H}6-19 |
| TX-DH120126 (∼11,896 bp) SEQ ID NO:235 | µCTX-SmIIIA midi (218) | D_{H}1-20 |
| | µCTX-CRSRQC (220) | D_{H}2-21 |
| | µCTX-PIIIA midi (222) | D_{H}3-22 |
| | µCTX-PIIIA fl (224) | D_{H}4-23 |
| | ProTxII fl (226) | D_{H}5-24 |
| | µCTX-KIIIA fl (228) | D_{H}6-25 |
| | µCTX-PIIIA mini (230) | D_{H}1-26 |

### Example 4. Generation of rodents having an engineered diversity cluster that includes nucleotide coding sequences of one or more toxin peptides within an immunoglobulin heavy chain variable region

This example demonstrates the production of non-human animals (e.g., rodents) whose genome comprises an immunoglobulin heavy chain variable region that includes an engineered heavy chain diversity (D_{H}) region, wherein the engineered D_{H} region includes one or more nucleotide sequences that each encode a portion of a non-immunoglobulin polypeptide, in particular, a toxin such as, for example, a conotoxin (e.g., µ-conotoxin), tarantula toxin (e.g., ProTxII) or combinations thereof.

Correct assembly of the TX-D_{H} targeting vector described in Example 3 and targeted insertion of the toxin DNA fragments into the diversity cluster of BAC DNA was confirmed by sequencing and polymerase chain reaction throughout the construction of the targeting vector using primers set forth in Table 6. Targeted BAC DNA, confirmed by polymerase chain reaction, was then introduced into F1 hybrid (129S6SvEvTac/C57BL6NTac) mouse embryonic stem (ES) cells via electroporation followed by culturing in selection medium. The ES cells used for electroporation had a genome that included human V_{H} and J_{H} gene segments operably linked with rodent immunoglobulin heavy chain constant regions (e.g., IgM), lacked a D_{H} region (i.e., deleted), and contained an inserted sequence encoding one or more murine Adam6 genes (see, e.g., U.S. Patent Nos. 8,642,835 and 8,697,940). Drug-resistant colonies were picked 10 days after electroporation and screened by TAQMAN^{™} and karyotyping for correct targeting as previously described (Valenzuela et al., *supra*; Frendewey, D. et al., 2010, Methods Enzymol. 476:295-307) using primer/probe sets that detected proper integration of the toxin coding sequences into the diversity cluster of an immunoglobulin variable region (Table 7 and Figure 5).

The VELOCIMOUSE^{®} method (DeChiara, T.M. et al., 2010, Methods Enzymol. 476:285-294; Dechiara, T.M., 2009, Methods Mol. Biol. 530:311-324; Poueymirou et al., 2007, Nat. Biotechnol. 25:91-99) was used, in which targeted ES cells were injected into uncompacted 8-cell stage Swiss Webster embryos, to produce healthy fully ES cell-derived F0 generation mice heterozygous for the engineered D_{H} region and that express antibodies containing heavy chain variable regions that include CDR3 regions generated from recombination of toxin coding sequences. F0 generation heterozygous male were crossed with C57B16/NTac females to generate F1 heterozygotes that were intercrossed to produce F2 generation homozygotes and wild-type mice for phenotypic analyses.

The drug selection cassette may optionally be removed by the subsequent addition of a recombinase (e.g., by Cre treatment) or by breeding to a Cre deleter mouse strain (see, e.g., International Patent Application Publication No. WO 2009/114400) in order to remove any loxed selected cassette introduced by the targeting construct that is not removed, e.g., at the ES cell stage or in the embryo. Optionally, the selection cassette is retained in the mice.

Taken together, this example illustrates the generation of a rodent (e.g., a mouse) whose genome comprises an immunoglobulin heavy chain variable region that includes an engineered heavy chain diversity (D_{H}) region, which engineered D_{H} region is characterized by the inclusion of one or more nucleotide sequences that each encode a portion of a toxin peptide such as, for example, µ-conotoxin, ProTxII or combinations thereof. The strategy described herein for inserting toxin coding sequences into the place of traditional D_{H} segments enables the construction of a rodent that expresses antibodies configured such that they contain heavy chain CDR3s that are generated from recombination of the toxin coding sequences. Leveraged with the presence of human V_{H} and J_{H} segments, rodents described herein provide an *in vivo* system for the production of human antibody-based therapeutics that characterized by diversity that directs binding to ion channels (e.g., voltage-gated sodium channels such as, Na_{V}1.7) and, in some embodiments, provides antibody-based anti-pain drugs for human use.

### Example 5. Phenotypic assessment of rodents having an engineered diversity cluster

This example demonstrates the characterization of various immune cell populations in rodents (e.g., mice) whose genome comprises an immunoglobulin heavy chain variable region that includes an engineered heavy chain diversity (D_{H}) region, wherein the engineered D_{H} region includes one or more nucleotide sequences that each encode a portion of a non-immunoglobulin polypeptide. In this example, rodents homozygous for an engineered D_{H} region that includes toxin coding sequences (e.g., µ-conotoxins and tarantula toxin) as described in Example 3 were analyzed by flow cytometry for identification of immune cells in harvested spleen and bone marrow using several fluorescently-labeled antibodies. This example also demonstrates the identification of immune cells in harvested spleen and bone marrow of mice heterozygous for an engineered D_{H} region that includes coding sequences of the extracellular portion of an ACKR2 (also known as D6 chemokine decoy receptor) as described in Example 1. As described below, mice containing an engineered D_{H} region as described herein demonstrate similar levels of B cells in the splenic and bone marrow compartments as compared to control engineered mice. Importantly, this Example also demonstrates that mice containing an engineered D_{H} region as described herein demonstrate a B cell development and maturation free of any defects or deficiencies in the B-cell immune response, and produce antibodies containing CDR3 regions generated from rearrangement of the inserted coding sequences and adjacent immunoglobulin gene segments (i.e., human V_{H} and J_{H}).

Briefly, spleens and femurs were harvested from VELOCIMMUNE^{®} mice (n=3, 26% C57BL/6 22% 129S6/SvEvTac 50% Balb/cAnNTac; see, e.g., U.S. Patent Nos. 8,502,018 and 8,642,835) and 6579ho/1293ho mice ("TX-D_{H} ho", n=3; 38% C57BL/6NTac 36% 129S6/SvEvTac 25% Balb/cAnNTac; mice homozygous for an immunoglobulin heavy chain locus containing a plurality of human V_{H}, engineered D_{H} segments including toxin coding sequences in the place of traditional D_{H} segments, and J_{H} segments operably linked to a rodent immunoglobulin heavy chain constant region including rodent heavy chain enhancers and regulatory regions, and containing an inserted nucleotide sequence encoding one or more murine Adam6 genes [e.g., U.S. Patent Nos. 8,642,835 and 8,697,940]; and a homozygous immunoglobulin κ light chain locus containing human Vκ and Jκ gene segments operably linked to a rodent Cκ region gene including rodent κ light chain enhancers). Bone marrow was collected from femurs by centrifugation. Red blood cells from spleen and bone marrow preparations were lysed with ACK lysis buffer (Gibco) followed by washing with 1xPBS with 2% FBS. Isolated cells (1×10⁶) were incubated with selected antibody cocktails for 30 min at +4°C: Stain 1: rat anti-mouse CD43-FITC (Biolegend 121206, clone 1B11), rat anti-mouse c-kit-PE (Biolegend 105808, clone 2B8), rat anti-mouse IgM-PeCy (eBiosciences 25-5790-82, clone II/41), rat anti-mouse IgD-PerCP-Cy5.5 (Biolegend 405710, clone 11-26c.2a), rat anti-mouse CD3-PB (Biolegend 100214, clone 17-A2), rat anti-mouse B220-APC (eBiosciences 17-0452-82, clone RA3-6B2), and rat anti-mouse CD19-APC-H7 (BD 560143 clone 1D3). Stain 2: rat anti-mouse kappa-FITC (BD 550003, clone 187.1), rat anti-mouse lambda-PE (Biolegend 407308, clone RML-42), rat anti-mouse IgM-PeCy (eBiosciences 25-5790-82, clone II/41), rat anti-mouse IgD-PerCP-Cy5.5 (Biolegend 405710, clone 11-26c.2a), rat anti-mouse CD3-PB (Biolegend 100214, clone 17-A2), rat anti-mouse B220-APC (eBiosciences 17-0452-82, clone RA3-6B2), and rat anti-mouse CD19-APC-H7 (BD 560143 clone 1D3). Stain 3: rat anti-mouse CD23-FITC (Biolegend 101606 clone B3B4), rat anti-mouse CD93 (Biolegend 136504, clone AA4.1), rat anti-mouse IgM-PeCy (eBiosciences 25-5790-82, clone II/41), rat anti-mouse IgD-PerCP-Cy5.5 (Biolegend 405710, clone 11-26c.2a), rat anti-mouse CD19-V450 (BD 560375, clone 1D3), rat anti-mouse CD21/35-APC (BD 558658, clone 7G6), and rat anti-mouse B220-APC-eF1780 (eBioscences 47-0452-82, clone RA3-6B2). Following staining, cells were washed and fixed in 2% formaldehyde. Data acquisition was performed on a BD LSRFORTESSA^{™} flow cytometer and analyzed with FLOWJO^{™} software. Representative results are set forth in Figures 9A-9D and Figures 10A-10D.

In a similar experiment, spleens and femurs were harvested from VELOCIMMUNE^{®} mice (n=3, 26% C57BL/6 23% 129SvEvTac 51% Balb/cAnNTac; see, e.g., U.S. Patent Nos. 8,502,018 and 8,642,835) and 6590hetmice ("D6-D_{H} het", n=3; 75% C57BL/6 25% 129SvEvTac; mice heterozygous for an immunoglobulin heavy chain locus containing a plurality of human V_{H}, engineered D_{H} segments including D6 chemokine decoy receptor coding sequences in the place of traditional D_{H} segments, and J_{H} segments operably linked to a rodent immunoglobulin heavy chain constant region including rodent heavy chain enhancers and regulatory regions, and containing an inserted nucleotide sequence encoding one or more murine Adam6 genes [e.g., U.S. Patent Nos. 8,642,835 and 8,697,940]; and analyzed by flow cytometry (as described above). Representative results are set forth in Figures 11A-11D and Figures 12A-12D.

In another similar experiment, spleens and femurs were harvested from VELOCIMMUNE^{®} mice (n=3, 38% C57BL/6 36% 129SvEvTac 25% Balb/cAnNTac; see, e.g., U.S. Patent Nos. 8,502,018 and 8,642,835) and 6590ho/1293ho mice ("D6-D_{H} ho", n=3; 38% C57BL/6 36% 129SvEvTac 25% Balb/cAnNTac) mice homozygous for an immunoglobulin heavy chain locus containing a plurality of human V_{H}, engineered D_{H} segments including D6 chemokine decoy receptor coding sequences in the place of one or more traditional D_{H} segments, and J_{H} segments operably linked to a rodent immunoglobulin heavy chain constant region including rodent heavy chain enhancers and regulatory regions, and containing an inserted nucleotide sequence encoding one or more murine Adam6 genes [e.g., U.S. Patent Nos. 8,642,835 and 8,697,940]; and homozygous for an immunoglobulin κ light chain locus containing human Vκ and Jκ gene segments operably linked to a rodent Cκ region gene including rodent κ light chain enhancers) and analyzed by flow cytometry (as described above). Representative results are set forth in Figures 13A-13D and Figures 14A-14D.

The results showed that TX-D_{H} ho mice demonstrated similar percentages of B cells in the splenic and bone marrow compartments as compared to control engineered mice. For some TX-D_{H} ho mice, a higher percentage of Igλ positive cells was observed (e.g., see Figures 9C and 10D). Overall, B cell maturation in the spleen demonstrated a normal progression in TX-D_{H} ho mice when compared to control engineered mice.

D6-D_{H} het mice also demonstrated similar B cell percentages in both the splenic and bone marrow compartments (Figures 11A-12D). D6-D_{H} het mice also appeared to have similar light chain ratios as compared to control mice (Figures 11C and 12D). D6-D_{H} het also demonstrated an overall normal development and maturation of B cells.

### Example 6. Human gene usage and rearrangement of coding sequences in engineered D_{H} regions

This Example demonstrates human V(D)J gene segment usage in rodents containing an engineered D_{H} region as described herein using Next Generation Sequencing (NGS)-antibody repertoire analysis. In particular, RT-PCR sequencing was conducted on RNA isolated from sorted spleen and unsorted bone marrow of mice homozygous for an engineered D_{H} region containing toxin coding sequences and mice heterozygous for an engineered D_{H} region containing ACKR2 coding sequences. As described below, all engineered D_{H} segments were observed in analyzed sequences from mice harboring an engineered D_{H} region containing toxin coding sequences in the place of traditional D_{H} segments. Also described below, a majority (i.e., 16 of 25) of engineered D_{H} segments were observed in analyzed sequences from mice harboring an engineered D_{H} region containing coding sequences of an extracellular portion of an ACKR2 (D6 chemokine decoy receptor).

Briefly, spleen and bone marrow was harvested from 6579ho/1293ho mice ("TX-D_{H} ho", n=3; *supra*) and B cells were positively enriched from total splenocytes by magnetic cell sorting using mouse anti-CD19 magnetic beads and MACS^{®} columns (Miltenyi Biotech). Total RNA was isolated from enriched B cells in sorted splenocytes and unsorted total bone marrow using an RNeasy Plus RNA isolation kit (Qiagen) according to manufacturer's specifications. Immunoglobulin heavy chain (µ chain, IgM) cDNA was then synthesized from total RNA using SuperScript III Reverse Transcriptase (Thermo Fisher Scientific) and a mouse IgM constant region primer (Table 9, IgM RT) according to manufacturer's specifications. IgM cDNA was amplified using a multiplex PCR strategy employing a mixture of V_{H} family-specific primers (Table 9, VH multi-1, 2, 3, 4, 5 & 6) designed to anneal to V_{H} leader sequences, and a primer (Table 9, IgM PCR1) designed to anneal to IgM constant region. PCR products ranging from ∼450-800bp were isolated using Pippin Prep (SAGE Science) and then a second PCR using PCR2 primers (Table 9, PCR2-F & PCR2-R, "XXXXXX" is a 6nt index to distinguish sequencing libraries and to allow sequencing multiple libraries at the same time) were performed to attach sequencing adaptors and indexes. PCR products ranging from ∼490bp-800bp were isolated, purified, and quantified by qPCR using a KAPA Library Quantification Kit (KAPA Biosystems) before loading onto a MiSeq sequencer (Illumina) for sequencing using MiSeq Reagent Kits v3 (2x300 cycles). Table 9 sets forth the sequences of selected primers used for repertoire library construction.

For bioinformatic analysis, Raw Illumina sequences were de-muliplexed and filtered based on quality, length and match to corresponding constant region gene primer. Overlapping paired-end reads were merged and analyzed using custom in-house pipeline. The pipeline used local installation of IgBLAST (NCBI, v2.2.25+) to align rearranged light chain sequences to human germline V_{H} and J_{H} gene segment database, and denoted productive and non-productive joins along with the presence of stop codons. CDR3 sequences and expected non-template nucleotides were extracted using boundaries as defined in International Immunogenetics Information System (IMGT). Representative results are set forth in Table 10 (total matches to toxin coding sequences among sequence reads using each V_{H} family-specific primer mixture) and Figures 15-17.

The results demonstrated that the majority of sequences analyzed contained toxin sequences within the CDR3 region of V_{H} region sequences. In particular, about 87% of sequences corresponding to productive rearrangements in mice harboring an engineered D_{H} region containing multiple toxin coding sequences in the place of traditional D_{H} segments contained toxin amino acid sequences within CDR3 regions. In addition, all 26 toxin coding sequences positioned within the place of traditional D_{H} segments in these mice were detected at various frequencies. The most highly utilized toxin coding sequences observed were the short µCTX four (4)-amino acid loop SmIIIa (D_{H} position D_{H}2-15), the µCTX-SmIIIA midi insert (D_{H} position D_{H}3-16) and the µCTX-PIIIA midi (D_{H} position D_{H}3-22).

In a similar experiment, human V_{H}, engineered D_{H} and human J_{H} segment usage was analyzed in 6590het mice ("D6-D_{H} het", n=3; *supra*) using Next Generation Sequencing (NGS)-antibody repertoire analysis as described above. RT-PCR sequencing was conducted on RNA isolated from sorted spleen and unsorted bone marrow using a multiplex PCR strategy as described above. Representative results are set forth in Table 11 (number of in-frame amino acid (AA) sequences among nucleotide (NT) sequence reads in RNA amplified from bone marrow and spleen), Table 12 (percent of selected D6 coding sequences in amplified RNA from spleen and bone marrow [combined all V_{H}-families, not reflective of quantitative V_{H} usage]) and Figures 18-20.

The results demonstrated that the majority of D6 coding sequences used in RNA amplified from bone marrow and spleen were about 30 to 40 nucleotides in length. In particular, the longest (e.g., Nterm+EC3 (216 nt)) and shortest (e.g., EC1-N (9 nt)) D6 coding sequences were not initially observed in the sequences analyzed from D6-D_{H} het mice. The inventors observed certain limitations when assessing the usage of D6 coding sequences from the engineered D_{H} region. For example, the inventors note that IgM sequence containing full-length (or longer than full-length) longer D6 coding sequences (e.g., D6 Nterm+EC3, D_{H} position D_{H}4-4; SEQ ID NO:33) are likely excluded from sequences due to the gel size selection step (see above), and shorter D6 coding sequences (e.g., D6 EC1, D_{H} position D_{H}5-5; SEQ ID NO:3) are likely excluded due the trimming selection step. For shorter D6 coding sequences, however, manual inspection of sequences indicated that these D6 coding sequences were, in fact, present among the sequences obtained from amplified RNA. Further, the inventors observed incorrect alignment of sequences when comparing to databases due to similarity between selected J_{H} segments (e.g., J_{H}1) and D6 coding sequences. Such sequence similarity resulted in the identification of shorter D_{H} sequences and the possibility of ambiguous annotations by certain databases. As a result, several sequences required individual analysis using alignments conducted by hand. Of all the sequences analyzed from D6-D_{H} het mice, about 50-60% of amino acid sequences were in-frame and utilized a D6 coding sequence within CDR3 regions of amplified heavy chains. Further, 16 of the 25 D6 engineered D_{H} segments were detected in amplified sequences from RNA (Table 12), while D6 Nterm (D_{H}3-3), D6 Nterm+EC3 (D_{H}4-4), D6 EC1 (D_{H}5-5), D6 EC1-N (D_{H}1-7), D6 EC1-S (D_{H}5-12), D6 Nterm-S (D_{H}6-13), D6 Nterm-EC3 (D_{H}3-16), D6 EC1+EC2 (D_{H}4-17) and D6 EC3-C (D_{H}4-23) were not detected in analyzed sequences. As described above, usage of some of these D6 coding sequences, in particular, the longer D6 coding sequences, could not be confirmed in the analyzed set of sequences. Overall, no major differences in usage of D6 coding sequences between bone marrow and spleen derived samples was observed. Also, there appeared no negative selection bias against rearrangement of D6 coding sequences among the analyzed sequences.

Taken together, this example demonstrates that mice harboring engineered immunoglobulin heavy chain loci characterized by the presence of an engineered D_{H} region that contains coding sequences of a non-immunoglobulin polypeptide (e.g., a D6 chemokine decoy receptor or one or more toxins) in the place of traditional D_{H} segments are capable of rearranging the engineered coding sequences with adjacent human V_{H} and J_{H} segments to form functional heavy chains. Further, provided engineered mice demonstrate a robust antibody repertoire utilizing multiple coding sequences engineered into a synthetic D_{H} region in the context of several human V_{H} and J_{H} segment families.

**TABLE 9**

| Primer Name | 5' to 3' Sequence (SEQ ID NO:) |
|---|---|
| IgM RT | TCTTATCAGACAGGGGGCTCTC (SEQ ID NO:236) |
| VH multi-1 | GTGACTGGAGTTCAGACGTGTGCTCTTCCGATCT TCACCATGGACTGSACCTGGA (SEQ ID NO:237) |
| VH multi-2 | GTGACTGGAGTTCAGACGTGTGCTCTTCCGATCT CCATGGACACACTTTGYTCCAC (SEQ ID NO:238) |
| VH multi-3 | GTGACTGGAGTTCAGACGTGTGCTCTTCCGATCT TCACCATGGAGTTTGGGCTGAGC (SEQ ID NO:239) |
| VH multi-4 | GTGACTGGAGTTCAGACGTGTGCTCTTCCGATCT AGAACATGAAACAYCTGTGGTTCTT (SEQ ID NO:240) |
| VH multi-5 | GTGACTGGAGTTCAGACGTGTGCTCTTCCGATCT ATGGGGTCAACCGCCATCCT (SEQ ID NO:241) |
| VH multi-6 | GTGACTGGAGTTCAGACGTGTGCTCTTCCGATCT ACAATGTCTGTCTCCTTCCTCAT (SEQ ID NO:242) |
| IgM PCR1 | ACACTCTTTCCCTACACGACGCTCTTCCGATCT GGGAAGACATTTGGGAAGGAC (SEQ ID NO:243) |
| PCR2-F | CAAGCAGAAGACGGCATACGAGATXXXXXX GTGACTGGAGTTCAGACGTGTGCTCTTCCGATCT (SEQ ID NO:244) |
| PCR2-R | AATGATACGGCGACCACCGAGATCTACACXXXXXX ACACTCTTTCCCTACACGACGCTCTTCCGATCT (SEQ ID NO:245) |

**TABLE 10**

| V_{H} family | Total sequence reads | Total matches to TX sequences (percent of total) |
|---|---|---|
| V_{H}1 | 35,784 | 34,175 (95.5%) |
| V_{H}2 | 12,651 | 12,149 (96.0%) |
| V_{H}3 | 497,715 | 463,839 (93.2%) |
| V_{H}4 | 19,576 | 16,715 (85.4%) |
| V_{H}5 | 29,102 | 27,201 (93.5%) |
| V_{H}6 | 3,405 | 2,743 (80.6%) |
| Tx: toxin | | |

**TABLE 11**

| | Bone marrow | | | Spleen | | |
|---|---|---|---|---|---|---|
| | M1 | M2 | M3 | M1 | M2 | M3 |
| NT | 26,031 | 23,298 | 20,949 | 17,623 | 14,687 | 19,834 |
| AA (in frame) | 13,741 | 12,655 | 7,433 | 11,112 | 7,780 | 9,057 |
| % AA (in frame) | 52.8 | 54.3 | 35.5 | 63.1 | 53.0 | 45.7 |

**TABLE 12**

| D6-D_{H} (position) | Length (NT) | Bone marrow | | | Spleen | | |
|---|---|---|---|---|---|---|---|
| | | M1 | M2 | M3 | M1 | M2 | M3 |
| D6 Nterm-C (D_{H}1-1) | 42 | 30.19 | 34.29 | 13.50 | 19.46 | 20.19 | 14.53 |
| D6 EC3 (D_{H}2-2) | 66 | 3.42 | 2.70 | 1.37 | 1.56 | 2.85 | 1.41 |
| D6 EC2 (D_{H}6-6) | 102 | 0.00 | 0.00 | 0.00 | 0.00 | 0.01 | 0.00 |
| D6 EC3-S (D_{H}2-8) | 66 | 6.33 | 3.67 | 1.58 | 4.72 | 3.80 | 3.13 |
| D6 Nterm-N (D_{H}3-9) | 105 | 0.14 | 0.03 | 0.05 | 0.02 | 0.08 | 0.05 |
| D6 EC2-S (D_{H}3-10) | 102 | 0.00 | 0.00 | 0.00 | 0.00 | 0.01 | 0.00 |
| D6 EC1-EC2-S (D_{H}4-11) | 78 | 0.04 | 0.02 | 0.00 | 0.02 | 0.00 | 0.00 |
| D6 EC2-N (D_{H}1-14) | 33 | 11.23 | 9.65 | 5.48 | 9.83 | 15.71 | 11.68 |
| D6 EC2-EC1 (D_{H}2-15) | 39 | 1.02 | 1.25 | 0.19 | 1.82 | 1.05 | 0.82 |
| D6 EC3-Nterm (D_{H}5-18) | 81 | 0.03 | 0.01 | 0.00 | 0.00 | 0.00 | 0.00 |
| D6 EC1-EC2 (D_{H}6-19) | 78 | 1.89 | 0.82 | 0.57 | 0.70 | 1.21 | 0.73 |
| D6 EC2-C (D_{H}1-20) | 66 | 4.87 | 3.46 | 2.35 | 2.41 | 3.61 | 2.67 |
| D6 EC2-EC1-S (D_{H}2-21) | 39 | 2.63 | 2.44 | 2.20 | 7.29 | 3.41 | 2.64 |
| D6 Nterm-EC3-S (D_{H}3-22) | 135 | 0.02 | 0.00 | 0.00 | 0.00 | 0.02 | 0.00 |
| D6 EC3-Nterm-S (D_{H}5-24) | 81 | 0.02 | 0.01 | 0.00 | 0.01 | 0.03 | 0.00 |
| D6 EC3-N (D_{H}1-26) | 36 | 38.17 | 41.67 | 72.71 | 52.14 | 48.03 | 62.32 |

### Example 7. Production of antibodies in rodents containing engineered D_{H} regions

This example demonstrates production of antibodies in a rodent whose genome comprises an immunoglobulin heavy chain variable region that includes an engineered D_{H} region as described herein. The methods described in this example, and/or immunization methods well known in the art, can be used to immunize rodents containing an engineered D_{H} region as described herein with polypeptides or fragments thereof (e.g., peptides derived from a desired epitope), or combination of polypeptides or fragments thereof, as desired.

Briefly, cohorts of mice that include an engineered D_{H} region as described herein are challenged with an antigen of interest using immunization methods known in the art. The antibody immune response is monitored by an ELISA immunoassay (i.e., serum titer).

Generation of a common light chain mouse (also referred to as universal light chain or ULC mice) comprising a single rearranged variable gene sequence V:J (e.g., Vκ1-39Jκ5 or Vκ3-20Jκ1 common light chain mouse) and generation of antigen-specific antibodies in those mice is described in, e.g., U.S. Patent Application Nos. 13/022,759, 13/093,156, 13/412,936, 13/488,628, 13/798,310, and 13/948,818 (Publication Nos. 2011/0195454, 2012/0021409, 2012/0192300, 2013/0045492, US20130185821, and US20130302836 respectively), each of which is incorporated herein by reference in its entirety. Specifically, mice that express the genetically engineered Vκ1-39Jκ5 kappa light chain (1633 HO or 1634 HO) or the genetically engineered Vκ3-20Jκ1 kappa light chain (1635 HO or 1636 HO) in their germline were made.

VELOCIMMUNE^{®} mice containing a single rearranged human germline light chain region (ULC Vκ1-39Jκ5; 1633 or 1634) or (ULC Vκ3-20Jκ1; 1635 or 1636) are bred to mice carrying a modified IgG constant region. Specifically, such ULC mice were bred to mice having a genome comprising a homozygous immunoglobulin heavy chain locus containing a plurality of human V_{H}, engineered D_{H} segments including toxin coding sequences in the place of traditional D_{H} segments, and J_{H} segments operably linked to a mouse immunoglobulin heavy chain constant region including mouse heavy chain enhancers and regulatory regions, and containing an inserted nucleotide sequence encoding one or more murine Adam6 genes [e.g., U.S. Patent Nos. 8,642,835 and 8,697,940], to obtain progeny mice heterozygous or homozygous for the heavy chain locus comprising toxin coding sequence and heterozygous or homozygous for the universal light chain. As shown in **Figure 21****,** mice homozygous for an engineered D_{H} region containing toxin coding sequences and homozygous for a universal light chain (6579HO/1634HO) had an antibody response after immunization with a soluble protein comparable to a control animal (1460/1634).

### Example 8. Isolation of cells expressing and/or nucleic acids encoding antibodies produced in rodents containing engineered D_{H} regions

When a desired immune response is achieved, splenocytes (and/or other lymphatic tissue) are harvested and fused with mouse myeloma cells to preserve their viability and form immortal hybridoma cell lines. The hybridoma cell lines are screened (e.g., by an ELISA assay) and selected to identify hybridoma cell lines that produce antigen-specific antibodies. Hybridomas may be further characterized for relative binding affinity and isotype as desired. Using this technique several antigen-specific chimeric antibodies (i.e., antibodies possessing human variable domains and rodent constant domains) are obtained.

DNA encoding the variable regions of heavy chain and light chains may be isolated and linked to desirable isotypes (constant regions) of the heavy chain and light chain for the preparation of fully-human antibodies. Such an antibody protein may be produced in a cell, such as a CHO cell. Fully human antibodies are then characterized for relative binding affinity and/or neutralizing activity of the antigen of interest.

DNA encoding the antigen-specific chimeric antibodies or the variable domains of light and heavy chains may be isolated directly from antigen-specific lymphocytes. Initially, high affinity chimeric antibodies are isolated having a human variable region and a rodent constant region and are characterized and selected for desirable characteristics, including affinity, selectivity, epitope, etc. Rodent constant regions are replaced with a desired human constant region to generate fully-human antibodies. While the constant region selected may vary according to specific use, high affinity antigen-binding and target specificity characteristics reside in the variable region. Antigen-specific antibodies are also isolated directly from antigen-positive B cells (from immunized mice) without fusion to myeloma cells, as described in, e.g., U.S. Patent No. 7,582,298, specifically incorporated herein by reference in its entirety. Using this method, several fully human antigen-specific antibodies (i.e., antibodies possessing human variable domains and human constant domains) are made.

### EQUIVALENTS

Having thus described several aspects of at least one embodiment of this invention, it is to be appreciated by those skilled in the art that various alterations, modifications, and improvements will readily occur to those skilled in the art. Such alterations, modifications, and improvements are intended to be part of this disclosure, and are intended to be within the spirit and scope of the invention. Accordingly, the foregoing description and drawing are by way of example only and the invention is described in detail by the claims that follow.

Use of ordinal terms such as "first," "second," "third," etc., in the claims to modify a claim element does not by itself connote any priority, precedence, or order of one claim element over another or the temporal order in which acts of a method are performed, but are used merely as labels to distinguish one claim element having a certain name from another element having a same name (but for use of the ordinal term) to distinguish the claim elements.

The articles "a" and "an" in the specification and in the claims, unless clearly indicated to the contrary, should be understood to include the plural referents. Claims or descriptions that include "or" between one or more members of a group are considered satisfied if one, more than one, or all of the group members are present in, employed in, or otherwise relevant to a given product or process unless indicated to the contrary or otherwise evident from the context. The invention includes embodiments in which exactly one member of the group is present in, employed in, or otherwise relevant to a given product or process. The invention also includes embodiments in which more than one, or the entire group members are present in, employed in, or otherwise relevant to a given product or process. Furthermore, it is to be understood that the invention encompasses all variations, combinations, and permutations in which one or more limitations, elements, clauses, descriptive terms, etc., from one or more of the listed claims is introduced into another claim dependent on the same base claim (or, as relevant, any other claim) unless otherwise indicated or unless it would be evident to one of ordinary skill in the art that a contradiction or inconsistency would arise. Where elements are presented as lists, (e.g., in Markush group or similar format) it is to be understood that each subgroup of the elements is also disclosed, and any element(s) can be removed from the group. It should be understood that, in general, where the invention, or aspects of the invention, is/are referred to as comprising particular elements, features, etc., certain embodiments of the invention or aspects of the invention consist, or consist essentially of, such elements, features, etc. For purposes of simplicity those embodiments have not in every case been specifically set forth in so many words herein. It should also be understood that any embodiment or aspect of the invention can be explicitly excluded from the claims, regardless of whether the specific exclusion is recited in the specification.

Those skilled in the art will appreciate typical standards of deviation or error attributable to values obtained in assays or other processes described herein.

The publications, websites and other reference materials referenced herein to describe the background of the invention and to provide additional detail regarding its practice are hereby incorporated by reference.

### Aspects of the invention

1. A rodent whose genome comprises an immunoglobulin heavy chain variable region that includes an engineered D_{H} region, wherein the engineered D_{H} region includes one or more nucleotide sequences that each encode a non-immunoglobulin polypeptide of interest, or portion thereof.
2. The rodent of aspect 1, wherein the immunoglobulin heavy chain variable region is a human immunoglobulin heavy chain variable region.
3. The rodent of aspect 1 or 2, wherein the immunoglobulin heavy chain variable region is operably linked to an immunoglobulin heavy chain constant region.
4. The rodent of aspect 3, wherein the immunoglobulin heavy chain constant region is an endogenous immunoglobulin heavy chain constant region.
5. The rodent of any one of the preceding aspects, wherein the non-immunoglobulin polypeptide of interest is a chemokine receptor.
6. The rodent of aspect 5, wherein the chemokine receptor is an atypical chemokine receptor (ACKR).
7. The rodent of aspect 6, wherein the ACKR is D6 chemokine decoy receptor.
8. The rodent of aspect 7, wherein the engineered D_{H} region includes 5, 10, 15, 20, 25 or more nucleotide sequences that each encode an extracellular portion of a D6 chemokine decoy receptor.
9. The rodent of aspect 8, wherein the engineered D_{H} region includes 25 nucleotide sequences that each encode an extracellular portion of a D6 chemokine decoy receptor.
10. The rodent of aspect 8 or 9, wherein the extracellular portion of a D6 chemokine decoy receptor is selected from the group consisting of an N-terminal region, an extracellular loop, and combinations thereof.
11. The rodent of aspect 9, wherein the 25 nucleotide sequences each encode a sequence that is at least 95% identical to a sequence that appears in Table 3.
12. The rodent of aspect 9, wherein the 25 nucleotide sequences each encode a sequence that is substantially identical to a sequence that appears in Table 3.
13. The rodent of aspect 9, wherein the 25 nucleotide sequences each encode a sequence that is identical to a sequence that appears in Table 3.
14. The rodent of any one of the preceding aspects, wherein the one or more nucleotide sequences comprise one or more nucleotide substitutions that increase somatic hypermutation of the one or more nucleotide sequences.
15. The rodent of any one aspects 1-4, wherein the non-immunoglobulin polypeptide of interest is a conotoxin or a tarantula toxin.
16. The rodent of aspect 15, wherein the conotoxin is selected from the group consisting of α-conotoxin, δ-conotoxin, κ-conotoxin, µ-conotoxin, ω-conotoxin and combinations thereof.
17. The rodent of aspect 16, wherein the conotoxin is µ-conotoxin.
18. The rodent of aspect 17, wherein the engineered D_{H} region includes 5, 10, 15, 20, 25 or more nucleotide sequences that each encode a portion of µ-conotoxin, a tarantula toxin, or combinations thereof.
19. The rodent of aspect 18, wherein the engineered D_{H} region includes 26 nucleotide sequences that each encode a portion of a µ-conotoxin and/or a tarantula toxin.
20. The rodent of aspect 19, wherein the 26 nucleotide sequences each encode a sequence that is at least 95% identical to a sequence that appears in Table 4.
21. The rodent of aspect 19, wherein the 26 nucleotide sequences each encode a sequence that is substantially identical to a sequence that appears in Table 4.
22. The rodent of aspect 19, wherein the 26 nucleotide sequences each encode a sequence that is identical to a sequence that appears in Table 4.
23. The rodent of any one of aspects 15-22, wherein the one or more nucleotide sequences comprise one or more nucleotide substitutions that increase somatic hypermutation of the one or more nucleotide sequences.
24. The rodent of any one of the preceding aspects, wherein the engineered D_{H} region further includes a first and a second recombination signal sequence flanking each of the one or more nucleotide sequences.
25. The rodent of aspect 24, wherein the first recombination signal sequence comprises a sequence that is at least 95% identical to a first recombination signal sequence that appears in Figure 2.
26. The rodent of aspect 24, wherein the first recombination signal sequence comprises a sequence that is substantially identical to a first recombination signal sequence that appears in Figure 2.
27. The rodent of aspect 24, wherein the first recombination signal sequence comprises a sequence that is identical to a first recombination signal sequence that appears in Figure 2.
28. The rodent of aspect 24, wherein the second recombination signal sequence comprises a sequence that is at least 95% identical to a second recombination signal sequence that appears in Figure 2.
29. The rodent of aspect 24, wherein the second recombination signal sequence comprises a sequence that is substantially identical to a second recombination signal sequence that appears in Figure 2.
30. The rodent of aspect 24, wherein the second recombination signal sequence comprises a sequence that is identical to a second recombination signal sequence that appears in Figure 2.
31. The rodent of aspect 24, wherein the first and second recombination signal sequences are selected from Figure 2.
32. The rodent of any one of the preceding aspects, wherein the genome of the rodent lacks one or more wild-type endogenous D_{H} gene segments.
33. The rodent of any one of the preceding aspects, wherein the genome of the rodent lacks one or more wild-type endogenous recombination signal sequences.
34. The rodent of any one of aspects 1-33, wherein the rodent is a rat or a mouse.
35. An isolated rodent cell or tissue whose genome comprises an immunoglobulin heavy chain variable region that includes an engineered D_{H} region, wherein the engineered D_{H} region includes one or more nucleotide sequences that each encode a non-immunoglobulin polypeptide of interest, or portion thereof.
36. An immortalized cell made from the isolated rodent cell of aspect 35.
37. A rodent embryonic stem cell whose genome comprises an immunoglobulin heavy chain variable region that includes an engineered D_{H} region, wherein the engineered D_{H} region includes one or more nucleotide sequences that each encode a non-immunoglobulin polypeptide of interest, or portion thereof.
38. A rodent embryo generated from the rodent embryonic stem cell of aspect 37.
39. A method of making a rodent whose genome contains an immunoglobulin heavy chain variable region that includes an engineered D_{H} region, the method comprising
   (a) inserting a DNA fragment into a rodent embryonic stem cell, said DNA fragment comprising one or more nucleotide sequences that each encode a non-immunoglobulin polypeptide of interest, or portion thereof;
   (b) obtaining the rodent embryonic stem cell generated in (a); and
   (c) creating a rodent using the embryonic stem cell of (b).
40. The method of aspect 39, wherein the non-immunoglobulin polypeptide of interest is a chemokine receptor.
41. The method of aspect 40, wherein the chemokine receptor is an atypical chemokine receptor (ACKR).
42. The method of aspect 41, wherein the ACKR is D6 chemokine decoy receptor.
43. The method of aspect 42, wherein the DNA fragment includes 5, 10, 15, 20, 25 or more nucleotide sequences that each encode an extracellular portion of a D6 chemokine decoy receptor.
44. The method of aspect 43, wherein the DNA fragment includes 25 nucleotide sequences that each encode an extracellular portion of a D6 chemokine decoy receptor.
45. The method of aspect 43 or 44, wherein the extracellular portion of a D6 chemokine decoy receptor is selected from the group consisting of an N-terminal region, an extracellular loop, and combinations thereof.
46. The method of aspect 39, wherein the non-immunoglobulin polypeptide of interest is a conotoxin or a tarantula toxin.
47. The method of aspect 46, wherein the conotoxin is selected from the group consisting of α-conotoxin, δ-conotoxin, κ-conotoxin, µ-conotoxin, ω-conotoxin and combinations thereof.
48. The method of aspect 47, wherein the conotoxin is µ-conotoxin.
49. The method of aspect 48, wherein the DNA fragment includes 5, 10, 15, 20, 25 or more nucleotide sequences that each encode a portion of µ-conotoxin, a tarantula toxin, or combinations thereof.
50. The method of aspect 49, wherein the DNA fragment includes 26 nucleotide sequences that each encode a portion of µ-conotoxin and/or a tarantula toxin.
51. The method of aspect 44 or 50, wherein the DNA fragment further comprises first and second recombination signal sequences flanking each of the 25 or 26 nucleotide sequences.
52. A method of making a rodent whose genome contains an immunoglobulin heavy chain variable region that includes an engineered D_{H} region, the method comprising
   modifying the genome of a rodent so that it comprises an immunoglobulin heavy chain variable region that includes an engineered D_{H} region, which engineered D_{H} region comprises one or more nucleotide sequences that each encode a non-immunoglobulin polypeptide of interest, or portion thereof, thereby making said rodent.
53. The method of aspect 52, wherein the non-immunoglobulin polypeptide of interest is a chemokine receptor.
54. The method of aspect 53, wherein the chemokine receptor is an atypical chemokine receptor (ACKR).
55. The method of aspect 54, wherein the ACKR is D6 chemokine decoy receptor.
56. The method of aspect 55, wherein the genome of the rodent is modified to include 5, 10, 15, 20, 25 or more nucleotide sequences that each encode an extracellular portion of a D6 chemokine decoy receptor.
57. The method of aspect 56, wherein the genome of the rodent is modified to include 25 nucleotide sequences that each encode an extracellular portion of a D6 chemokine decoy receptor.
58. The method of aspect 56 or 57, wherein the extracellular portion of a D6 chemokine decoy receptor is selected from the group consisting of an N-terminal region, an extracellular loop, and combinations thereof.
59. The method of aspect 52, wherein the non-immunoglobulin polypeptide of interest is a conotoxin or a tarantula toxin.
60. The method of aspect 59, wherein the conotoxin is selected from the group consisting of α-conotoxin, δ-conotoxin, κ-conotoxin, µ-conotoxin, ω-conotoxin and combinations thereof.
61. The method of aspect 60, wherein the conotoxin is µ-conotoxin.
62. The method of aspect 61, wherein the genome of the rodent is modified to include 5, 10, 15, 20, 25 or more nucleotide sequences that each encode a portion of µ-conotoxin, a tarantula toxin, or combinations thereof.
63. The method of aspect 62, wherein the genome of the rodent is modified to include 26 nucleotide sequences that each encode a portion of µ-conotoxin and/or a tarantula toxin.
64. The method of aspect 57 or 63, wherein the genome of the rodent is modified to further include first and second recombination signal sequences flanking each of the 25 or 26 nucleotide sequences.
65. The method of any one of aspects 39-64, wherein the immunoglobulin heavy chain variable region is a human immunoglobulin heavy chain variable region.
66. The method of any one of aspects 39-64, wherein the immunoglobulin heavy chain variable region is operably linked to an immunoglobulin heavy chain constant region.
67. The method of aspect 66, wherein the immunoglobulin heavy chain constant region is an endogenous immunoglobulin heavy chain constant region.
68. A method of producing an antibody in a rodent, the method comprising the steps of
   (a) immunizing a rodent with an antigen, which rodent has a genome comprising an immunoglobulin heavy chain variable region that includes an engineered D_{H} region, wherein the engineered D_{H} region includes one or more nucleotide sequences that each encode a non-immunoglobulin polypeptide of interest, or portion thereof;
   (b) maintaining the rodent under conditions sufficient that the rodent produces an immune response to the antigen; and
   (c) recovering an antibody from the rodent, or a rodent cell, that binds the antigen.
69. The method of aspect 68, wherein the rodent cell is a B cell.
70. The method of aspect 68, wherein the rodent cell is a hybridoma.
71. The method of any one of aspects 68-70, wherein the non-immunoglobulin polypeptide of interest is a chemokine receptor.
72. The method of aspect 71, wherein the chemokine receptor is an atypical chemokine receptor (ACKR).
73. The method of aspect 72, wherein the ACKR is D6 chemokine decoy receptor.
74. The method of aspect 73, wherein the engineered D_{H} region includes 5, 10, 15, 20, 25 or more nucleotide sequences that each encode an extracellular portion of a D6 chemokine decoy receptor.
75. The method of aspect 74, wherein the engineered D_{H} region includes 25 nucleotide sequences that each encode an extracellular portion of a D6 chemokine decoy receptor.
76. The method of aspect 74 or 75, wherein the extracellular portion of a D6 chemokine decoy receptor is selected from the group consisting of an N-terminal region, an extracellular loop, and combinations thereof.
77. The method of any one of aspects 68-70, wherein the non-immunoglobulin polypeptide of interest is a conotoxin or a tarantula toxin.
78. The method of aspect 77, wherein the conotoxin is selected from the group consisting of α-conotoxin, δ-conotoxin, κ-conotoxin, µ-conotoxin, ω-conotoxin and combinations thereof.
79. The method of aspect 78, wherein the conotoxin is µ-conotoxin.
80. The method of aspect 79, wherein the engineered D_{H} region includes 5, 10, 15, 20, 25 or more nucleotide sequences that each encode a portion of µ-conotoxin, a tarantula toxin, or combinations thereof.
81. The method of aspect 80, wherein the engineered D_{H} region includes 26 nucleotide sequences that each encode a portion of µ-conotoxin and/or a tarantula toxin.
82. The method of aspect 75 or 81, wherein the engineered D_{H} region further comprises first and second recombination signal sequences flanking each of the 25 or 26 nucleotide sequences.
83. The method of any one of aspects 68-82, wherein the immunoglobulin heavy chain variable region is a human immunoglobulin heavy chain variable region.
84. The method of any one of aspects 68-83, wherein the immunoglobulin heavy chain variable region is operably linked to an immunoglobulin heavy chain constant region.
85. The method of aspect 84, wherein the immunoglobulin heavy chain constant region is an endogenous immunoglobulin heavy chain constant region.
86. The method of any one of aspects 39-85, wherein the rodent is a rat or a mouse.
87. A rodent whose genome comprises
   a human immunoglobulin heavy chain variable region that comprises one or more human V_{H} gene segments, an engineered D_{H} region, and one or more human J_{H} gene segments, which engineered D_{H} region includes
      (i) one or more nucleotide sequences that each encode an extracellular portion of an atypical chemokine receptor (ACKR); and
      (ii) first and second recombination signal sequences flanking each of the one or more nucleotide sequences of (i);
   wherein the human immunoglobulin heavy chain variable region is operably linked to one or more endogenous immunoglobulin constant region genes so that the rodent is characterized in that when it is immunized with an antigen, it generates antibodies comprising human heavy chain variable domains encoded by the one or more human V_{H} gene segments, engineered D_{H} region, and one or more human J_{H} gene segments operably linked to rodent heavy chain constant domains encoded by the one or more endogenous immunoglobulin constant region genes, and wherein the antibodies show specific binding to the antigen.
88. The rodent of aspect 87, wherein the ACKR is D6 chemokine decoy receptor.
89. The rodent of aspect 88, wherein the engineered D_{H} region includes 25 nucleotide sequences that each encode an extracellular portion of a D6 chemokine decoy receptor.
90. The rodent of aspect 89, wherein the extracellular portion of a D6 chemokine decoy receptor is selected from the group consisting of an N-terminal region, an extracellular loop, and combinations thereof.
91. A rodent whose genome comprises
   a human immunoglobulin heavy chain variable region that comprises one or more human V_{H} gene segments, an engineered D_{H} region, and one or more human J_{H} gene segments, which engineered D_{H} region includes
      (i) one or more nucleotide sequences that each encode a portion of a toxin; and
      (ii) first and second recombination signal sequences flanking each of the one or more nucleotide sequences of (i);
   wherein the human immunoglobulin heavy chain variable region is operably linked to one or more endogenous immunoglobulin constant region genes so that the rodent is characterized in that when it is immunized with an antigen, it generates antibodies comprising human heavy chain variable domains encoded by the one or more human V_{H} gene segments, an engineered D_{H} region, and one or more human J_{H} gene segments operably linked to rodent heavy chain constant domains encoded by the one or more endogenous immunoglobulin constant region genes, and wherein the antibodies show specific binding to the antigen.
92. The rodent of aspect 91, wherein the one or more nucleotide sequences each encode a portion of a µ-conotoxin, a tarantula toxin, or combinations thereof.
93. The rodent of aspect 92, wherein the engineered D_{H} region includes 26 nucleotide sequences that each encode a portion of µ-conotoxin and/or a tarantula toxin.
94. The rodent of aspect 87 or 91, wherein the one or more nucleotide sequences comprise one or more nucleotide substitutions that increase somatic hypermutation of the one or more nucleotide sequences.
95. The rodent of any one of aspect 87-94, further comprising an insertion of one or more human V_{L} gene segments and one or more human J_{L} gene segments into an endogenous light chain locus.
96. The rodent of aspect 95, wherein the human V_{L} and J_{L} segments are Vκ and Jκ gene segments and are inserted into an endogenous κ light chain locus.
97. The rodent of aspect 96, wherein the human Vκ and Jκ gene segments are operably linked to a rodent Cκ gene.
98. The rodent of aspect 95, wherein the human V_{L} and J_{L} segments are Vλ and Jλ gene segments and are inserted into an endogenous λ light chain locus.
99. The rodent of aspect 98, wherein the human Vλ and Jλ gene segments are operably linked to a rodent Cλ gene.
100. The rodent of any one of aspects 87-99, wherein the rodent is a rat or a mouse.

## Claims

1. A targeting vector, for integration into a rodent endogenous immunoglobulin heavy chain locus, comprising an unrearranged human immunoglobulin variable region that comprises at least one human V_{H} gene segment and at least one human J_{H} gene segment flanking an engineered D_{H} region that comprises one or more nucleotide sequences that each
(a) encode a non-immunoglobulin polypeptide of interest, or portion thereof, and
(b) is flanked by a first recombination signal and second recombination signal sequence
wherein the immunoglobulin V_{H} gene segment, the one or more nucleotide sequences, and the immunoglobulin J_{H} gene segment recombine in a B cell to form a rearranged heavy chain variable region V_{H}/engineered D_{H}/J_{H} sequence.

2. The targeting vector of claim 1, for integration of the one or more nucleotide sequences into a DH region of the endogenous immunoglobulin heavy chain locus.

3. The targeting vector of claim 1, for replacement of all or substantially all of a DH region of the endogenous immunoglobulin heavy chain locus with the one or more nucleotide sequences.

4. The targeting vector of any one of claims 1-3, wherein the one or more nucleotide sequences comprises a sequence that increases somatic hypermutation of the one or more nucleotide sequences.

5. The targeting vector of any one of the preceding claims, wherein
(a) the first recombination signal sequence comprises a sequence that is at least 95% identical to a recombination signal sequence set forth as any one of SEQ ID NOs: 51-112,
(b) the first recombination signal sequence comprises a sequence that is 95% to 99% identical to a recombination signal sequence set forth as any one of SEQ ID NOs: 51-112,
(c) the first recombination signal sequence comprises a sequence that is identical to a recombination signal sequence set forth as any one of SEQ ID NOs:51-112,
(d) the second recombination signal sequence comprises a sequence that is at least 95% identical to a recombination signal sequence set forth as any one of SEQ ID NOs: 51-112,
(e) the second recombination signal sequence comprises a sequence that is 95% to 99% identical to a recombination signal sequence set forth as any one of SEQ ID NOs: 51-112,
(f) the second recombination signal sequence comprises a sequence that is identical to a recombination signal sequence set forth as any one of SEQ ID NOs:51-112, and/or
(g) the first recombination signal and second recombination signal sequences are selected from a sequence set forth as any one of SEQ ID NOs:51-112.

6. The targeting vector of any one of the preceding claims, wherein the engineered D_{H} region further comprises one or more wild-type human D_{H} gene segments.

7. The targeting vector of any one of the preceding claims, wherein the unrearranged human variable region comprises a plurality of human V_{H} gene segments and/or a plurality of human J_{H} gene segments.

8. An isolated rodent cell whose genome comprises, at an endogenous heavy chain locus, the targeting vector of any one of the preceding claims, optionally wherein the rodent cell is a rat cell or a mouse cell.

9. The isolated rodent cell of claim 8, wherein the cell is a germ cell or an embryonic stem cell.

10. The isolated rodent cell of claim 9, wherein the cell is an embryonic stem cell.

11. A rodent generated from the rodent cell of claim 9 or 10.

12. The rodent of claim 11, wherein the rodent is a rat or mouse.

13. A method of making a rodent whose genome contains an immunoglobulin heavy chain variable region that includes an engineered D_{H} region, the method comprising:
(a) introducing a targeting vector according to any one of claims 1-7 into a rodent embryonic stem cell; and
(b) making a rodent using the rodent embryonic stem cell.

14. The method of claim 13, wherein the rodent is a rat or mouse.

15. A method of providing a nucleotide sequence comprising a rearranged heavy chain variable region V_{H}/engineered D_{H}/J_{H} sequence, comprising isolating said nucleotide sequence from a rodent according to claim 11 or 12.
